# EUROPEAN PATENT SPECIFICATION

(11) **EP 1 689 858 B1**
(45) Date of publication and mention of the grant of the patent: **15.05.2013**
(21) Application number: 04810564.7
(22) Date of filing: 08.11.2004
(51) Int. Cl.: C07K 14/08

(54) **METHODS OF CHARACTERIZING INFECTIOUS BURSAL DISEASE VIRUS**
VERFAHREN ZUR CHARAKTERISIERUNG DES IBD (INFECTIOUS BURSAL DISEASE)-VIRUS
PROCEDES DE CARACTERISATION DU VIRUS DE LA BURSITE INFECTIEUSE

(30) Priority: 13.11.2003 US 519571 P
(43) Date of publication of application: 16.08.2006
(73) Proprietor: UNIVERSITY OF GEORGIA RESEARCH FOUNDATION, INC., Athens, Georgia 30602 (US)
(72) Inventor: BROWN, Thomas, Paul, North Andover, MA 01845 (US)
(74) Representative: Harding, Charles Thomas
(86) International application number: PCT/US2004/037255
(87) International publication number: WO 2005/049794

(56) References cited:
- WO-A-00/12677
- WO-A-03/074552
- US-A- 6 114 112
- MUNDT E: "Tissue culture infectivity of different strains of infectious bursal disease virus is determined by distinct amino acids in VP2" JOURNAL OF GENERAL VIROLOGY, SOCIETY FOR GENERAL MICROBIOLOGY, SPENCERS WOOD, GB, vol. 80, 1999, pages 2067-2076, XP002169605 ISSN: 0022-1317
- BAYLISS C D ET AL: "A COMPARISON OF THE SEQUENCES OF SEGMENT A OF FOUR INFECTIOUS BURSAL DISEASE VIRUS STRAINS AND IDENTIFICATION OF A VARIABLE REGION IN VP2" JOURNAL OF GENERAL VIROLOGY, READING, BERKS, GB, vol. 71, no. 6, 1990, pages 1303-1312, XP009007233 ISSN: 0022-1317
- VAKHARIA V.N. ET AL: 'Molecular basis of antigenic variation in infectious bursal disease virus' VIRUS RESEARCH vol. 31, 1994, pages 265 - 273, XP002092967

## Description

### FIELD OF THE INVENTION

The present description relates to the characterization of infectious bursal disease virus ("IBDV") for use in vaccine identification and production. An IBDV cDNA, e.g., from a tissue sample of an avian suspected of being infected with IBDV is generated and sequenced, the sequenced IBDV is aligned with other IBDV sequences, and the relatedness of the aligned IBDV sequences is determined. The methods allow rapid selection of a specific vaccine strain with an IBDV sequence most related to the IBDV in the sample that gives the greatest protection against that strain of virus without virus isolation, cross neutralization studies, or importation of live virus. Alternatively, if the IBDV sequence from the sample does not closely match a known IBDV sequence, the description provides for the identification of a novel strain of IBDV.

Novel IBDV strains identified by the present description are useful for the preparation of immunogenic compositions and vaccines against diseases caused by the viruses. Such novel IBDV strains can also be used to provide attenuated, inactivated and sub-unit immunogenic compositions and vaccines.

### BACKGROUND OF THE INVENTION

Infectious Bursal disease (IBD), also called Gumboro disease, is an acute, highly-contagious viral infection in chickens that has lymphoid tissue as its primary target, with a selective tropism for cells of the bursa of Fabricius. The morbidity rate in susceptible flocks is high, with rapid weight loss and moderate mortality rates. Chicks that recover from the disease may have immune deficiencies because of the destruction of the bursa of Fabricius, which is an essential component of the chicken immune system. IBDV causes severe immunosuppression in chickens younger than 3 weeks of age and induces bursal lesions in chicks up to 3 months old.

For many years, the disease could be prevented by inducing high levels of antibodies in breeder flocks, by the application of an inactivated vaccine to chickens that had been primed with attenuated live IBDV vaccine. This has kept economic losses caused by IBD to a minimum. Maternal antibodies in chickens derived from vaccinated breeders prevent early infection with IBDV and diminish problems associated with immunosuppression. In addition, attenuated live vaccines have also been used successfully in commercial chicken flocks after maternal antibodies had declined.

Recently, very virulent strains of IBDV have caused outbreaks of disease with high mortality in Europe. The current vaccination programs failed to protect chicks sufficiently. Vaccination failures were mainly due to the inability of live vaccines to infect the birds before challenge with virulent field virus.

Therefore, a constant need exists to improve existing vaccines and to develop new types of vaccines. For the development of live vaccines, IBD viruses in attenuated form are required. Conventionally, this can be achieved by serial passaging of IBDV field isolates on an appropriate substrate. For the development of inactivated IBDV vaccines, an appropriate substrate is necessary for the generation of high amounts of IBDV antigen mass resulting from the propagation of IBD viruses on the substrate.

It is known that field IBDVs can readily be propagated in vivo in the bursa of infected birds or in embryonated eggs. However, although, the successful adaptation and propagation of some IBDV strains to in vitro cell culture of chicken embryo origin has been reported, it is generally acknowledged that most IBDV strains isolated from infected bursa in the field, in particular the so-called virulent- or very virulent IBDV strains cannot be adapted to cells of chicken embryo origin, such as chicken embryo fibroblasts (CEF) or cells from other organs such as the kidney and liver (see, e.g., Brown et al., J Gen Virol 1994 Mar;75 ( Pt 3):675-80; and Van Loon, et al., Proceedings of the International symposium on infectious bursal disease and chicken infectious anaemia, Rauischholzhausen, Germany, 179-187, 1994).

The drawbacks of the in vivo culture substrates are obvious. Such culture methods are animal unfriendly, need a lot of animals, are time consuming and cannot be carried out under standardised and stringent conditions. In addition, the limited number of IBDV strains which are not refractory to adaptation to in vitro cell culture substrates suffer from the disadvantage that, as a result of the serial passaging process leading to the adaptation of the IBDV strains, random mutations can be introduced in the genome of the virus in an uncontrolled manner. Such mutations may influence properties of the virus other than that associated with the adaptation of the virus to the cell culture, e.g., properties related to the immunogenicity of the virus. Such additional, random mutations are not desired. The adaptation of the IBDVs by passaging of the virus in vitro in CEF cell cultures has been associated with attenuation of the virulence as demonstrated by a reduction of the virus' ability to induce lesions in the bursa of the infected bird.

There exists a need for a more efficient method for the rapid identification of an appropriate vaccine for an avian infected with IBDV as well as the identification of novel strains of IBDV, especially very virulent strains of IBDV.

Citation or identification of any document in this application is not an admission that such document is available as prior art to the present invention.

### SUMMARY OF THE INVENTION

In one aspect, the present invention provides an isolated IBDV polypeptide sequence that consists of the amino acid residues of SEQ ID NO: 2, SEQ ID NO: 4 or SEQ ID NO: 6.

In another aspect, the present invention provides an isolated IBDV polynucleotide sequence that consists of the sequence of SEQ ID NO: 1, SEQ ID NO: 3 or SEQ ID NO: 5, or an antisense strand that is fully complementary thereto.

In a further aspect, the present invention provides an expression vector comprising a polynucleotide sequence according to the present invention.

The present invention provides in another aspect a preparation comprising one or more expression vectors according to the present invention.

In a further aspect, the present invention provides the use of an isolated IBDV polypeptide sequence according to the invention, or an isolated IBDV polynucleotide sequence according to the invention, or an expression vector according to the invention for the preparation of an immunogenic composition.

The present invention provides, in a further aspect, an isolated IBDV polypeptide sequence according to the invention, or an isolated IBDV polynucleotide sequence according to the invention, or an expression vector according to the invention, or a preparation according to the invention, for use in inducing an immunological response to IBDV in an avian.

In another aspect, the present invention provides the use of an isolated IBDV polypeptide sequence according to the invention, or an isolated IBDV polynucleotide sequence according to the invention, or an expression vector according to the invention, or a preparation according to the invention, for the preparation of a medicament for inducing an immunological response to IBDV in an avian.

The present description is based, in part, on a method to rapidly characterize IBDV from a tissue or cell sample suspected of being infected with IBDV without virus isolation, cross neutralization studies, or importation of samples containing live virus from foreign countries.

The description provides for a method of characterizing a strain of IBDV comprising: generating and sequencing an IBDV cDNA from a sample suspected of having a strain of IBDV, aligning the sequenced IBDV with one or more IBDV sequences, and comparing relatedness of aligned IBDV sequences, thereby characterizing a strain of IBDV.

The sample is any sample suspected of having a strain of IBDV. In an advantageous embodiment, the sample is a tissue sample, advantageously a paraffin-embedded tissue sample. The sample can also be a cell suspected of being infected with IBDV. In an advantageous embodiment, IBDV cDNAs are generated by extracting RNA from the paraffin-embedded tissue sample and RT-PCR amplification of the IBDV cDNA with IBDV-specific primers. Advantageously, the IBDV-specific primers amplify a hypervariable portion of IBDV, such as VP1, VP2, VP3, VP4 or VP5.

In another embodiment, the sequences are compared with a dendritogram. In one advantageous embodiment, the IBDV sequences are nucleic acid sequences. In another advantageous embodiment, an amino acid sequence is deduced from the IBDV cDNA and the one or more IBDV sequences are amino acid sequences.

The description also provides for the identification of a novel strain of IBDV wherein the IBDV sequence does not align to any of the one or more IBDV sequences with close homology. The method comprises (a) generating an IBDV cDNA from the strain of IBDV, (b) aligning the IBDV cDNA with IBDV sequences, (c) comparing relatedness of aligned IBDV sequences, and (d) identifying a novel strain of IBDV if the IBDV cDNA is less than 95%, advantageously less than about 98% to about 99.9%, more advantageously less than about 99.6% or about 99.8%, homologous to any one of the known IBDV sequences. It is advantageous that the novel strain of IBDV has less than 50%, less than 60%, less than 70%, less than 75%, less than 80%, less than 85%, less than 90%, less than 93%, less than 95%, less than 97%, less than 98%, less than 98.1%, less than 98.2%, less than 98.3%, less than 98.4%, less than 98.5%, less than 98.6%, less than 98.7%, less than 98.8%, less than 98.9%, less than 99%, less than 99.1%, less than 99.2%, less than 99.3%, less than 99.4%, less than 99.5%, less than 99.6%, less than 99.7%, less than 99.8%, less than 99.9%, most advantageously less than about 99.6% or 99.8%, homology or identity with any known IBDV sequence. The present description further provides isolating the novel strain of IBDV.

The description encompasses new IBDV strains identified by the methods described herein. Advantageously, the new IBDV strains are identified by VGIS (Viral Genomic Identification System). The description provides for IBDV strains, nucleic acids, polypeptides, as well as analogues and fragments thereof, for new sequences identified using VGIS: Sequence No. 1631, a new vvIBDV-like strain; Sequence No. 087, a new IBDV Variant strain and Sequence No. 077, a new previously unidentified IBDV strain. The description provides for isolated IBDV strains, isolated polypeptides (e.g., SEQ ID NO: 2) and isolated IBDV polynucleotides (e.g., SEQ ID NO: 1), or antisense strands fully complementary thereto, of Sequence No. 1631. The description provides for isolated IBDV strains, isolated polypeptides (e.g., SEQ ID NO: 4) and isolated IBDV polynucleotides (e.g., SEQ ID NO: 3), or antisense strands fully complementary thereto, of Sequence No. 087. The description provides for isolated IBDV strains, isolated polypeptides (e.g., SEQ ID NO: 6) and isolated IBDV polynucleotides (e.g., SEQ ID NO: 5), or antisense strands fully complementary thereto, of Sequence No. 077. The polynucleotides can be DNA or RNA molecules.

The present description also provides for selecting a vaccine to protect an avian against the strain of IBDV, wherein the vaccine has an IBDV sequence most closely matched to the IBDV cDNA. The method comprises (a) generating an IBDV cDNA from the strain of IBDV, (b) aligning the IBDV cDNA with IBDV sequences, (c) comparing relatedness of aligned IBDV sequences, and (d) identifying a vaccine for a strain of IBDV if the IBDV cDNA is at least 95% homologous, advantageously about 98% to about 99.9% homologous, to any known IBDV sequences that correspond to a known IBDV virus. It is advantageous that the IBDV strain will have at least 50%, at least 60%, at least 70%, at least 75%, at least 80%, at least 85%, at least 90%, at least 93%, at least 95%, at least 97%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% homology or identity with any known IBDV sequence in order for the known IBDV vaccine corresponding to the known IBDV sequence to be effective. It is advantageous that the IBDV strain will have at least 98%, at least 98.7%, at least 99.3%, at least 99.6%, at least 99.8%, most advantageously at least about 99.3% or 99.6%, homology or identity with any known IBDV sequence in order for the known IBDV vaccine to be effective.

The present description also relates to a computer-assisted method for characterizing a strain of IBDV by using a computer system, e.g., a programmed computer comprising a processor, a data storage system, an input device, and an output device, the steps of: (a) inputting into the programmed computer through the input device data comprising sequences of IBDV generated from a sample suspected of having a strain of IBDV, thereby generating a data set; (b) comparing, using the processor, the data set to a computer database of IBDV sequences stored in the computer data storage system; (c) selecting from the database, using computer methods, IBDV sequences stored in the computer data storage system having a portion that is about 95%, advantageously about 98% to about 99.9%, more advantageously about 99.3% or about 99.6%, homologous to the data set; (d) and outputting to the output device the selected IBDV sequences having a portion that is at least about 98% to about 99.9%, more advantageously about 99.3% or about 99.6%, homologous to the data set, or optionally outputting to the output device indicating the absence of IBDV sequences having a portion that is at least about 98% to about 99.9%, more advantageously about 99.3% or about 99.6%, homologous to the data set if no IBDV sequences have a portion that is at least about 98% to about 99.8%, more advantageously about 99.3% or about 99.6%, homologous to the data set, thereby characterizing a strain of IBDV.

In one embodiment, the sample is a paraffin-embedded tissue sample. In an advantageous embodiment, the IBDV sequences correspond to one or more hypervariable portions of IBDV, such as VP1, VP2, VP3, VP4 or VP5. The IBDV sequences in the storage system are nucleic acid sequences or amino acid sequences. In another embodiment, a data set of amino acid sequences is deduced if the input sequences are nucleotide sequences.

The present description also provides for a method of transmitting data comprising transmission of information from such methods herein discussed or steps thereof, e.g., via telecommunication, telephone, video conference, mass communication, e.g., presentation such as a computer presentation (e.g. POWERPOINT), internet, email, documentary communication such as a computer program (e. g. WORD) document and the like.

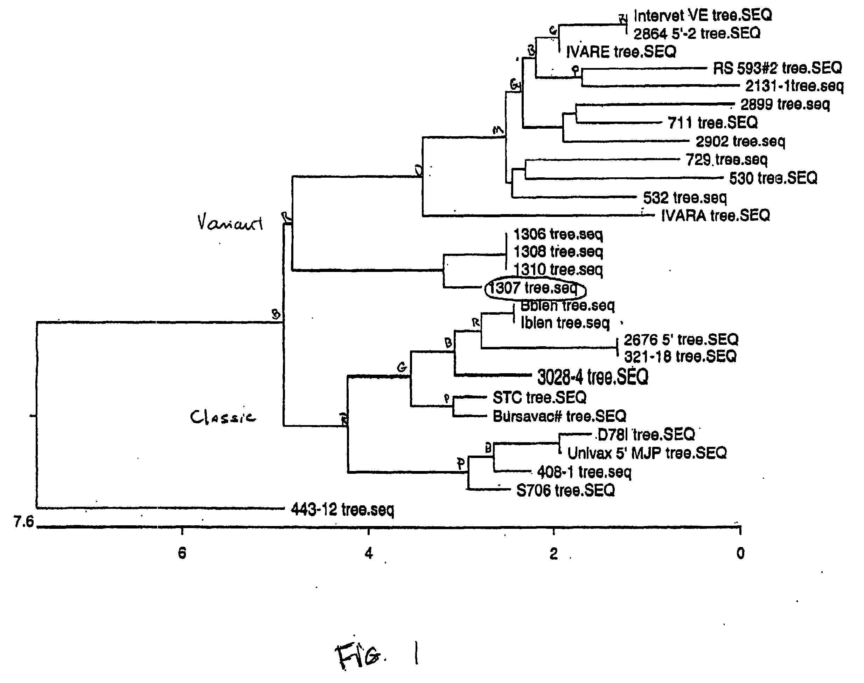

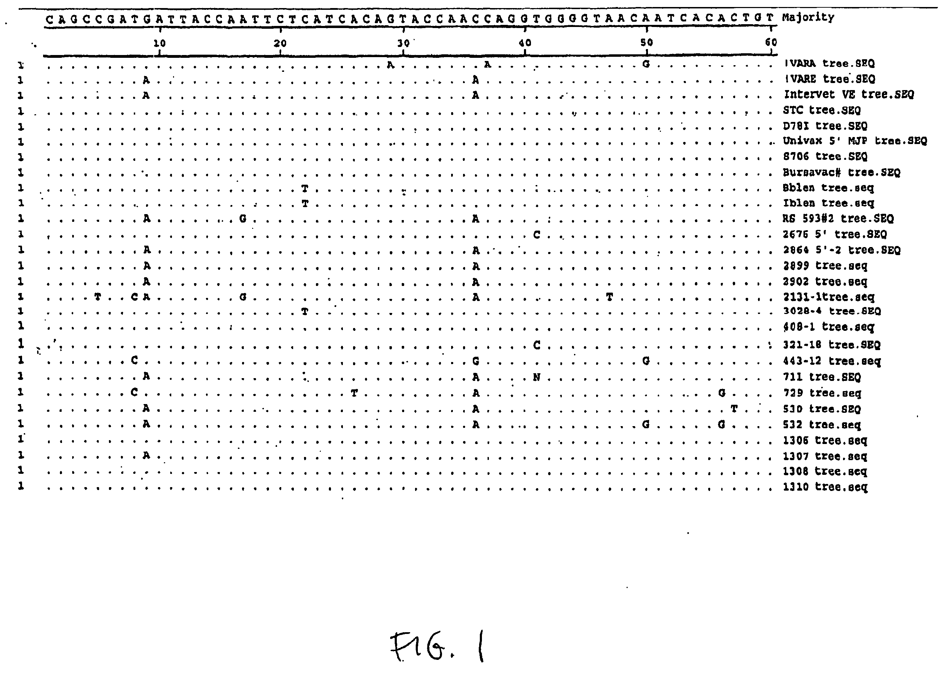

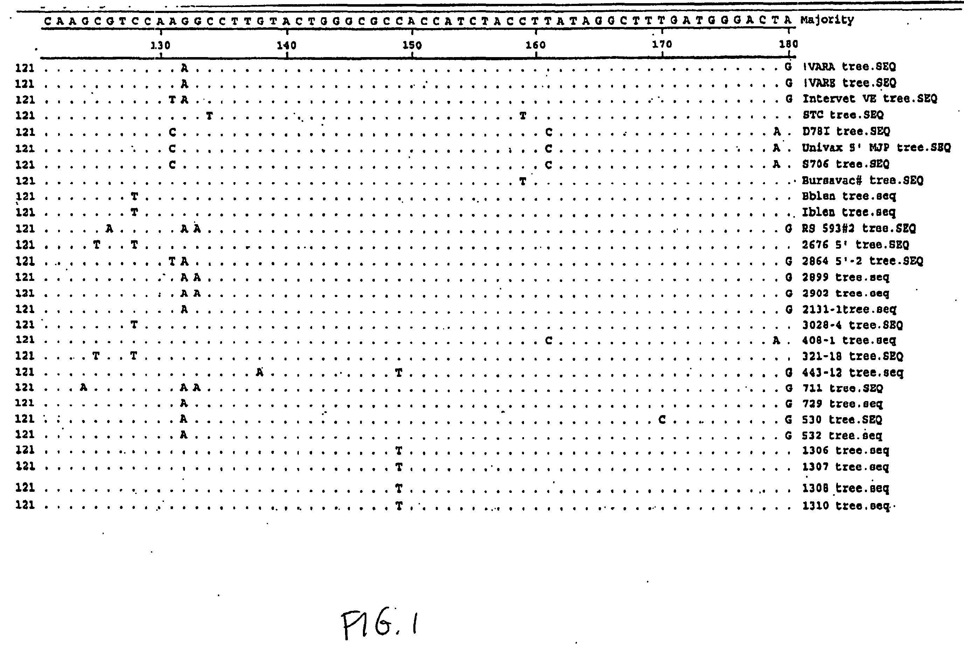

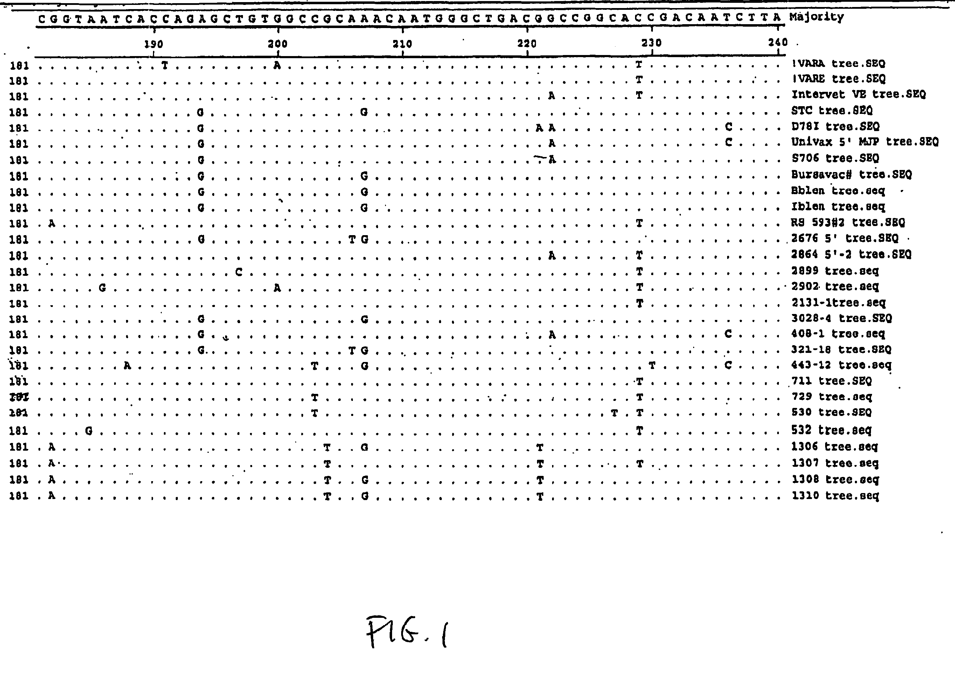

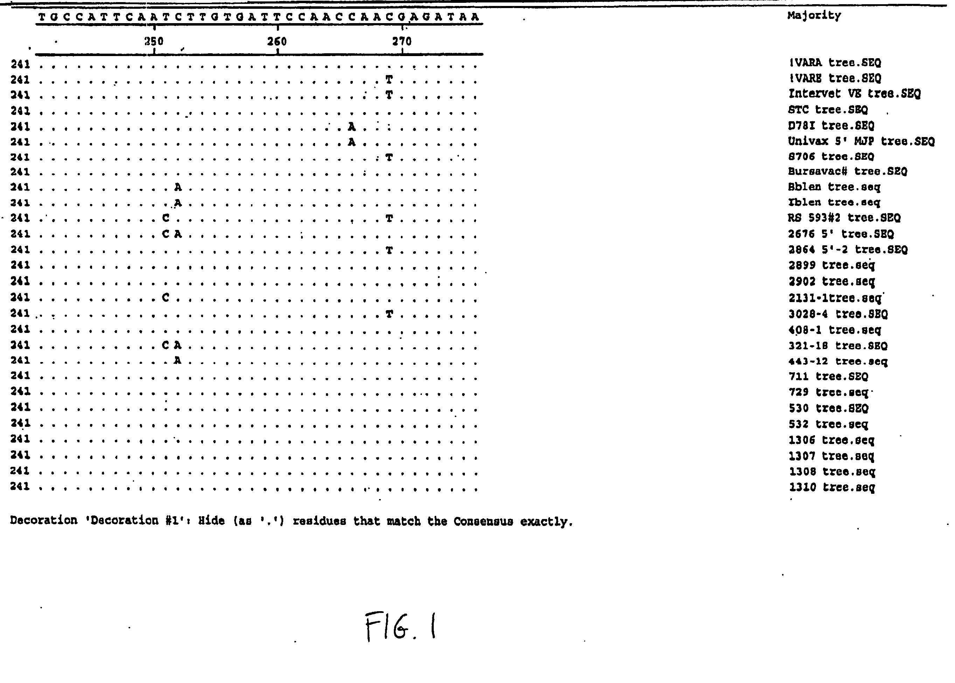

The description relates to a computer system and a computer readable media for characterizing a strain of IBDV, the system containing either: IBDV nucleotide sequences according to Table 2 and/or FIG. 1 or IBDV amino acid sequences of Table 3 or IBDV amino acid sequences derived from the nucleotide sequences according to Table 2 and/or FIG. 1. A computer readable media containing either: IBDV nucleotide sequences according to Table 2 and/or FIG. 1 or IBDV amino acid sequences of Table 3 or IBDV amino acid sequences derived from the nucleotide sequences according to Table 2 and/or FIG. 1. The description also relates to a method of doing business comprising providing to a user the computer system described herein or the media described herein or either: IBDV nucleotide sequences according to Table 2 and/or FIG. 1 or IBDV amino acid sequences of Table 3 or IBDV amino acid sequences derived from the nucleotide sequences according to Table 2 and/or FIG. 1.

The description also provides for the use of new IBDV strains identified by the methods of the invention as vaccines. The description also relates to methods for obtaining an epitope, antigen, or immunogen of a novel strain of IBDV comprising isolating the epitope, antigen, or immunogen from a novel strain of IBDV identified by the methods of the invention. The epitope, antigen, or immunogen can be an expression product of a nucleic acid molecule that is heterologous to the virus.

The description provides for methods and compositions for eliciting an immune response and/or inducing an immunological or protective response comprising administering the novel IBDV or an epitope, antigen, or immunogen thereof in an effective amount to elicit the immune response to an animal, advantageously an avian. The description also relates to the administration of an adjuvant and or a cytokine, including a cytokine is expressed by the virus. The IBDV can be inactivated or attenuated.

The present description relates to characterizing IBDVs that infect avians. The avian can be a chicken, duck, goose, pheasant, quail or turkey. In other embodiments, the description also relates to characterizing birnaviruses in aquatic animals such as, but not limited to, fish and shellfish.

It is noted that in this disclosure and particularly in the claims and/or paragraphs, terms such as "comprises", "comprised", "comprising" and the like can have the meaning attributed to it in U.S. Patent law; e.g., they can mean "includes", "included", "including", and the like; and that terms such as "consisting essentially of" and "consists essentially of" have the meaning ascribed to them in U.S. Patent law, e.g., they allow for elements not explicitly recited, but exclude elements that are found in the prior art or that affect a basic or novel characteristic of the invention.

These and other embodiments are disclosed or are obvious from and encompassed by, the following Detailed Description.

### BRIEF DESCRIPTION OF THE DRAWINGS

The following detailed description, given by way of example, but not intended to limit the invention solely to the specific embodiments described, may best be understood in conjunction with the accompanying drawings, in which:
FIG. 1 shows a phylogenetic tree of nucleic acid sequences aligned using Clustal method with Weighted residue weight table. The majority sequence (SEQ ID NO: 32) is indicated on top of the alignment.
FIG. 2A shows a histological section (formalin-fixed) of normal bursa.
FIG. 2B shows a histological section (formalin-fixed) of acute bursal necrosis.
FIG. 3 shows an agarose gel of RT-PCR results showing an amplified segment shared by IBDVs. Lane 1 is the size ladder, lane 2 is the water negative control, and lanes 3 to 5 are individual samples that were formalin fixed, paraffin embedded, sections taken from three blocks, RNA extracted, RT-PCR completed as per protocol, and products run on gel. The band is at expected size.
FIG. 4A shows sequence variations in an IBDV VP2 amplicon of nucleic acid sequences.
FIG. 4B shows sequence variations in IBDV VP2 deduced amino acid sequences.

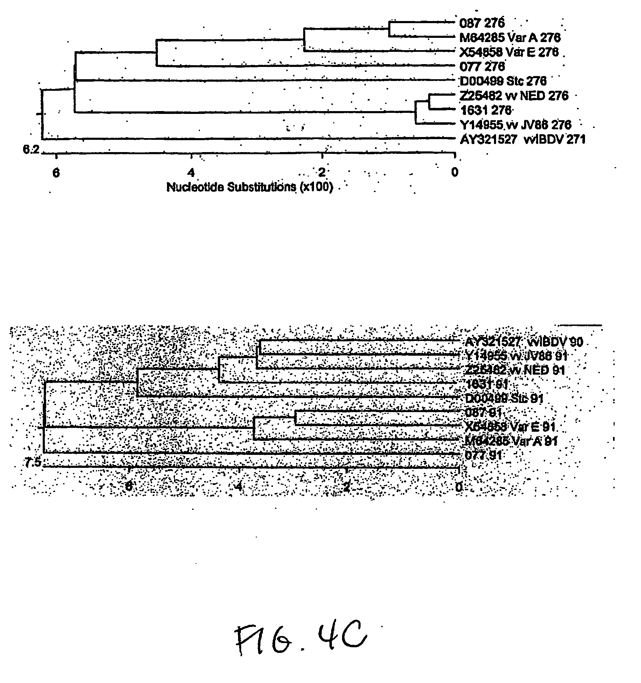

FIG. 4C shows sequence variations of new sequences identified using new VGIS (Viral Genomic Identification System). Nucleotide sequences 1631 276 is a new vvIBDV-like strain, 087 276 is a new IBDV Variant strain, and 077 276 is a new previously unidentified IBDV strain. Amino acid sequences 1631 91 is a new vvIBDV-like strain, 087 91 is a new IBDV Variant strain, and 077 91 is a new previously unidentified IBDV strain. Genebank posted sequences for comparison: #AY321527 is a vvIBDV sequence, #Y14955 is another vvIBDV sequence, #Z25482 is a third vvIBDV sequence, #D00499 is an IBDV USDA Standard Challenge Strain (STC), #X54858 is an IBDV Variant E strain, #M64285 is an IBDV Variant A strain.
FIG. 5 shows a flowchart illustrating the general overview of input, an intermediate step, and output.

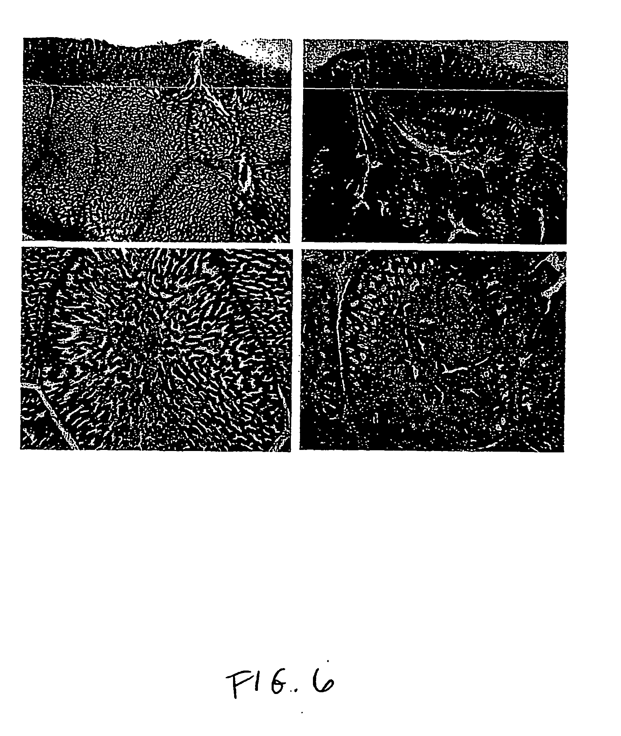

FIG. 6 shows photomicrographs of proventriculi from a normal broiler chicken (A and C), and from a broiler chicken with naturally occurring proventriculitis (B and D). H&E, 25X and 40X.

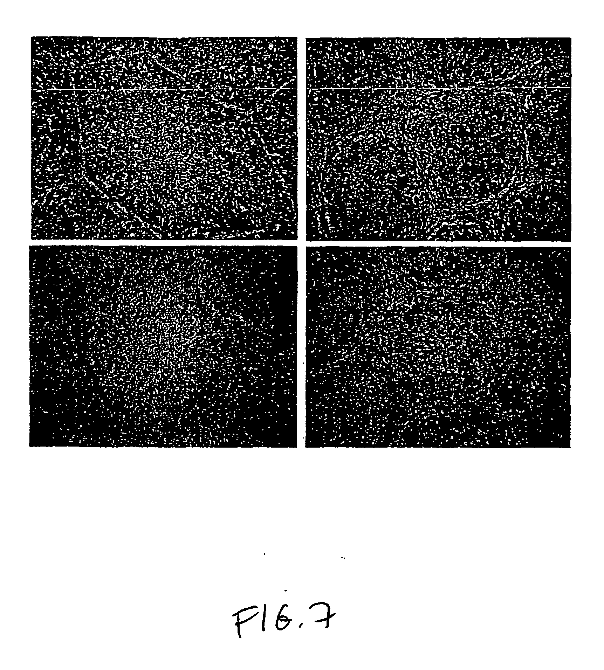

FIG. 7. shows photomicrographs of bursas from broiler chickens, control and challenged with IBDV (STC strain). (A) IBDV antigen staining by IHC, negative control. (B) IBDV antigen staining by IHC, challenged. (C) Apoptosis staining by TUNEL method, negative control. (D) Apoptosis staining by TUNEL method, challenged. 100X.
FIG. 8 shows (A) Proventriculitis in a commercial chicken inoculated with an infectious proventricular homogenate at day of age (14 dpi). (B) Comparison between the proventricular wall of a normal chicken (upper section), and the proventricular wall of a chicken with proventriculitis (lower section) where the wall is thickened, with a white lobular pattern.
FIG. 9 shows (A and B) Proventriculi of a normal chicken (upper and on left) and a chicken with proventriculitis (lower and on right). The proventriculus is enlarged and the gastric isthmus distended in proventriculitis.
FIG. 10 shows photomicrographs of proventriculi from a normal chicken (A) and from chicken with proventriculitis (B, C, and D). Degeneration and necrosis of glandular epithelium with coalescing of glands and lymphocytic infiltration in mucosa and glands (B). Dilation of glandular sinus with separation of epithelial cells from basement membrane (C). Lymphocytic infiltration in the glandular interstitium with ductal epithelial hyperplasia (D). H&E ,10 and 25X.
FIG. 11 shows photomicrographs of proventriculi from a normal chicken (A) and from chicken with proventriculitis (B, C, and D). Nuclei of affected glandular epithelial cells are enlarged and pale with marginated chromatin (B). Columnar ductal epithelium replacing secretory glandular epithelium (C). Hypertrophy and hyperplasia of ductal epithelium (D). H&E, 40X.
FIG. 12 shows photomicrographs of proventriculi from a normal chicken (A) and from chicken with proventriculitis (B, C, and D) after immunofluorescent staining using as primary antibody convalescent sera from inoculated chickens. 25, 40X.
FIG. 13 shows photographs of proventriculi from broiler chickens (14 days of age): inoculated with saline (A, and C), or with infectious proventricular homogenate (B, and D). Increase in size of the proventriculus and gastric isthmus and a white lobular pattern in a thickened mucosa can be observed in chickens with induced proventriculitis.
FIG. 14 shows photomicrographs of proventriculi: A, normal proventriculus of chickens inoculated with saline (negative control) (7 dpi). B, proventriculitis in chickens inoculated with positive proventricular homogenate (+PV) (7 dpi) with necrosis of the glandular epithelium, coalescing of glands, and diffuse lymphocytic infiltration in glands and mucosa. C, proventriculitis in chickens inoculated with +PV (14 dpi), with ductal epithelium replacing glandular epithelium. D, proventriculus in SPF broilers inoculated with +PV (21 dpi), with small germinal centers. HE, 10X.
FIG. 15 shows photomicrographs of proventriculi from broiler chickens inoculated with positive proventricular homogenate (+PV) (14 dpi). A and B, treated with CP and +PV, with metaplastic replacement of proventricular glandular epithelium by ductal epithelium with minimal necrosis. C and D, treated with CS and +PV, with acute necrosis of the epithelium with coalescing glands and variable germinal center formation. HE, 10, and 25X.

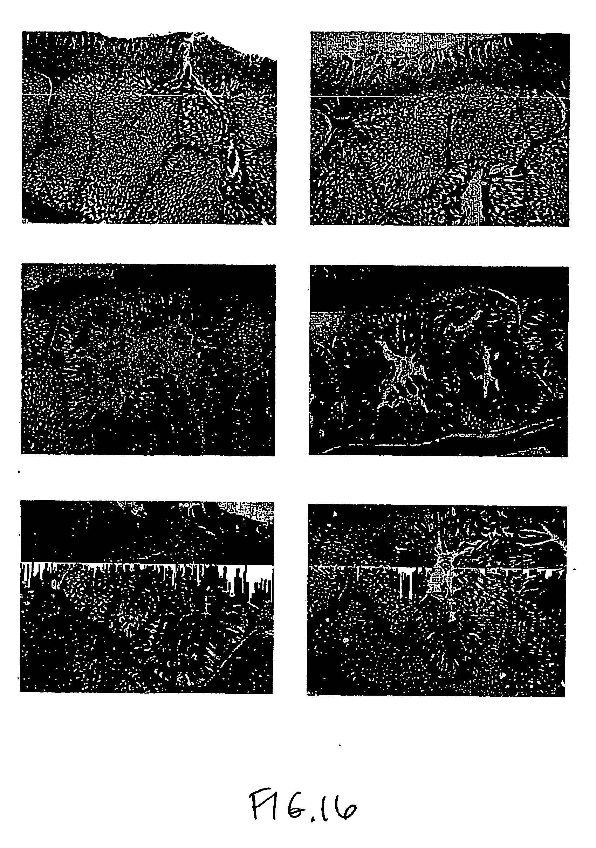

FIG. 16 shows photomicrographs of proventriculi: A. From chicken inoculated with -PV at 7 dpi. Lymphocytic infiltration in the lamina propria of the mucosa and surrounding the orifice of the secretory duct. B. From chicken inoculated with -PV at 21 dpi. Small lymphocyte aggregations are present in the proventricular gland. C. From chicken inoculated with positive +PV at 7 dpi. Severe necrosis of the glandular epithelium, dilation of sinus with desquamated epithelium and lymphocytic infiltration of the proventricular gland. D. and E. From chickens inoculated with +PV at 14 dpi. Diffuse lymphocytic infiltration in the proventricular gland and the lamina propria of the mucosa. Tubular epithelium replacing glandular epithelium F. From chicken inoculated with +PV at 21 dpi. Lymphocyte aggregations present in the proventricular glands. HE, 25X.

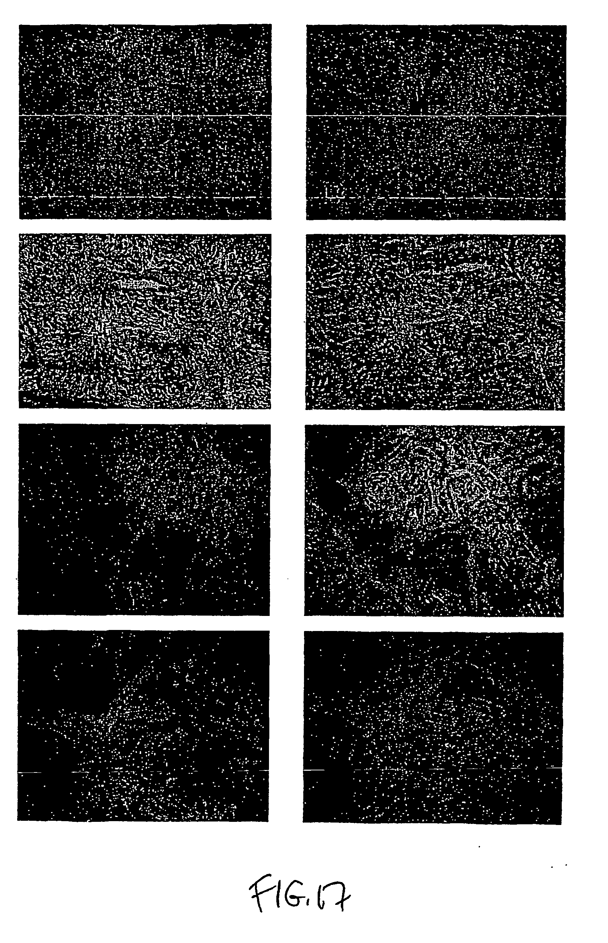

FIG. 17 shows immunohistochemistry (IHC) staining of proventricular lymphocytes: A. From chicken inoculated with -PV, B cell staining in the lamina propria of the mucosa, 7dpi. B. From chicken inoculated with -PV, CD3+ T cell staining in the lamina propria of the mucosa, interstitium between proventricular glands, and deep in the glands. 7dpi. C. From +PV-inoculated chicken, B cell staining in the proventricular gland, 7 dpi. D. From +PV-inoculated chicken, CD3+ T cell staining in the proventricular gland, 7 dpi. E. and G. From +PV-inoculated chicken, B cell staining of lymphocyte aggregations in the gland and mucosa, 14 dpi. F. and H. From +PV-inoculated chicken, CD3+ T cell staining in the glands and mucosa, 14 dpi. 25 and 50X.

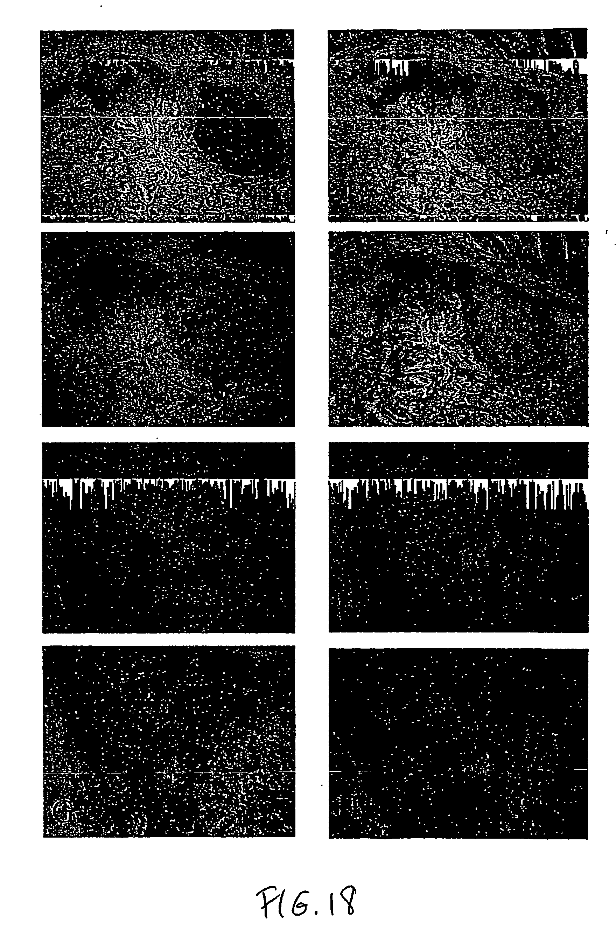

FIG. 18 shows immunohistochemistry (IHC) staining of proventricular lymphocytes from +PV-inoculated chicken at 14 dpi. A. and E. B cell staining. B. and F. CD3+T cell staining. C. and G. CD4+ T cell staining. D. and H. CD8+ T cell staining. 50 and 100X.
FIG. 19 shows immunohistochemistry (IHC) staining of proventricular lymphocyte aggregations from +PV-inoculated chicken at 14 dpi. A. B cell stain. B. CD3+ T cell stain. 10X.

### DETAILED DESCRIPTION

The present description is based, in part, on a method to rapidly characterize IBDV from a tissue or cell sample suspected of being infected with IBDV without virus isolation, cross neutralization studies, or importation of samples containing live virus from foreign countries. The present description relates to characterizing IBDVs that infect avians. The avian can be a chicken, duck, goose, pheasant, quail or turkey. In other embodiments, the description also relates to characterizing birnaviruses in aquatic animals such as, but not limited to, fish and shellfish.

Although the advantageous embodiment of the present description is the characterization of IBDV, the methods described herein can be applied to other viruses, specifically avian viruses. An advantageous alternate avian virus to which methods of the present description can be applied is reovirus. Other avian viruses to which the present description can be applied include, but are not limited to, arbovirus, astrovirus, avian adenovirus, avian circovirus, avian encephalomyelitis virus, avian infectious laryngeotracheitis virus, avian influenza virus, avian leukosis virus, avian polyomavirus, avipox virus, birnavirus, canarypox virus, chicken anemia virus, coranovirus, duck enteritis virus, duck hepatitis virus, enterovirus, falcon herpesvirus, flavovirus, fowlpox virus, herpes virus of turkeys, infectious bronchitis virus, infectious bursal disease virus (IBDV), Newcastle disease virus, oncornavirus, orthomyxovirus, Pacheco's disease, paramyxovirus group 2-9 viruses (PMV 2-9), parvovirus, pigeon herpesvirus, pigeon pox virus, pneumovirus, psittacine herpesvirus (Pachecco's herpesvirus), quail pox virus, reovirus, rotavirus, rous sarcoma virus, swine influenza virus, turkey herpesvirus, turkey rhinotracheitis virus, vaccinia virus, and West Nile virus (WNV).

The description provides for a method of characterizing a strain of IBDV comprising: generating and sequencing an IBDV cDNA from a sample suspected of having, i.e., infected with, a strain of IBDV, aligning the sequenced IBDV with one or more IBDV sequences, and comparing relatedness of aligned IBDV sequences, thereby characterizing a strain of IBDV.

In one embodiment, the sample is a paraffin-embedded tissue sample. In an advantageous embodiment, IBDV cDNAs are generated by extracting RNA from the paraffin-embedded tissue sample and reverse transcriptase-polymerase chain reaction (RT-PCR) amplification of the IBDV cDNA with IBDV-specific primers. Methods of extracting RNA and RT-PCR amplification of a cDNA from a paraffin-embedded tissue sample are well known in the art (see, e.g., Brown et al., Vet Pathol. 2003;40(5):613, and U.S. Patent Nos. 6,248,535; 6,428,963 and 6,610,488).

Advantageously, the IBDV-specific primers amplify a hypervariable portion of IBDV, such as VP1, VP2, VP3, VP4 or VP5. In an embodiment wherein VP2 is amplified, advantageous primer pairs for amplification are B5 5': GGTATGTGAGGCTTGGTGAC (SEQ ID NO: 7) and B5 3': TTATCTCGTTGGTTGGAATC (SEQ ID NO: 8), or alternatively, B4 5': TCTTGGGTATGTGAGGCTTG (SEQ ID NO: 9) and B4 3': GGATGTGATTGGCTGGGTTA (SEQ ID NO: 10).

In one advantageous embodiment, the IBDV sequences are nucleic acid sequences. In another advantageous embodiment, an amino acid sequence is deduced from the IBDV cDNA and the one or more IBDV sequences is an amino acid sequence. Methods for determining sequences of nucleic acids and amino acids are well known to one of skill in the art.

Advantageously, the nucleic acid sequencing is by automated methods (reviewed by Meldrum, Genome Res. 2000 Sep;10(9):1288-303). Methods for sequencing nucleic acids include, but are not limited to, automated fluorescent DNA sequencing (see, e.g., Watts & MacBeath, Methods Mol Biol. 2001;167:153-70 and MacBeath et al., Methods Mol BioL 2001;167:119-52), capillary electrophoresis (see, e.g., Bosserhoff et al., Comb Chem High Throughput Screen. 2000 Dec;3(6):455-66), DNA sequencing chips (see, e.g., Jain, Pharmacogenomics. 2000 Aug;1(3):289-307), mass spectrometry (see, e.g., Yates, Trends Genet. 2000 Jan;16(1):5-8), pyrosequencing (see, e.g., Ronaghi, Genome Res. 2001 Jan;11(1):3-11), and ultrathin-layer gel electrophoresis (see, e.g., Guttman & Ronai, Electrophoresis. 2000 Dec;21(18):3952-64). The sequencing can also be done by any commercial company. Examples of such companies include, but are not limited to, the University of Georgia Molecular Genetics Instrumentation Facility (Athens, Georgia) or SeqWright DNA Technologies Services (Houston, Texas).

Advantageously, the amino acid sequencing is by automated methods. Methods for sequencing amino acids include, but are not limited to, alkylated-thiohydantoin method (see, e.g., Dupont et al., EXS. 2000;88:119-31), chemical protein sequencing (see, e.g., Stolowitz, Curr Opin Biotechnol. 1993 Feb;4(1):9-13), Edman degradation (see, e.g., Prabhakaran et al., J Pept Res. 2000 Jul;56(1):12-23), and mass spectrometry (see, e.g., McDonald et al., Dis Markers. 2002;18(2):99-105). Alternatively, amino acid sequences can be deduced from nucleic acid sequences. Such methods are well known in the art, e.g., EditSeq from DNASTAR, Inc.

The invention provides for the comparison of IBDV sequences. Advantageously, the sequenced IBDV is compared to a library of known IBDV sequences. Such known IBDV sequences include, but are not limited to, the sequences of Figure 1 and the sequenced referenced by the accession numbers in Table 2 (see Example 4, infra).

Alternatively, IBDV can be isolated from an avian infected with the virus (see, e.g., Zorman-Rojs et al., Avian Dis 2003 Jan-Mar;47(1):186-92, Phong et al., Avian Dis 2003 Jan-Mar;47(1):154-62, and Banda et al., Avian Dis 2003 Jan-Mar;47(1):87-95), or alternatively, IBDV can be purchased from a commercial source (see, e.g., Jackwood & Sommer, Virology 2002 Dec 5;304(1):105-13), or recombinant forms (wildtype and mutant) of IBDV can be utilized (see, e.g., U.S. Patent No. 6,492,148 and Martinez-Torrecuadrada et al., Vaccine 2003 May 16;21(17-18):1952-1960). The sequences of these IBDVs can be determined by methods well known in the art, if not readily available. The term IBDV also encompasses all strains of IBDV, such as, but not limited to Bursal Disease Vaccine, Lukert strain, live virus, which is obtained from either Vineland Laboratories (Vineland, N.J.) or Salsbury Laboratories (Charles City, Iowa), the Bursal Disease Virulent Challenge Virus, which is obtained from the United States Department of Agriculture in Ames, Iowa (original isolate from S. A. Edgar), and Infectious Bursal Disease Virus strain VR2161, disclosed in U.S. Patent No. 4,824,668.

In an advantageous embodiment, the sequences are compared with a dendritogram (see, e.g., Figure 1 and Example 3). In an advantageous embodiment, the program MegAlign is used to align sequences and make a tree: MegAlign (DNASTAR, Inc.) and EditSeq is used to convert the nucleic acid sequence to amino acids (DNASTAR, Inc.). Several publications describe the use of RT-PCR and cDNA sequencing to generate dendograms for IBDV virulent, very virulent and vaccinal strains in order to characterize new strains (see, e.g., Sun et al. J. Vet. Med. B Infect. Dis. Vet. Public Health, 2003, 50, 148-154; Parede et al. Avian Pathol. 2003, 32, 511-518; Bais et al Acta Virol. 2003, 47, 73-77 and Phong et al Avian Dis. 2003, 47, 154-162).

The description encompasses new IBDV strains identified by the methods described herein. Advantageously, the new IBDV strains are identified by VGIS (Viral Genomic Identification System). The description provides for IBDV strains, nucleic acids, polypeptides, as well as analogues and fragments thereof, for new sequences identified using VGIS: Sequence No. 1631, a new vvIBDV-like strain; Sequence No. 087, a new IBDV Variant strain and Sequence No. 077, a new previously unidentified IBDV strain.

The description encompasses new IBDV strains identified by the methods described herein. Advantageously, the new IBDV strains are identified by VGIS (Viral Genomic Identification System). The description provides for IBDV strains, nucleic acids, polypeptides, as well as analogues and fragments thereof, for the new IBDV strains identified using VGIS: The description provides for isolated IBDV strains, isolated polypeptides and isolated IBDV polynucleotides, or antisense strands fully complementary thereto, of Sequence No. 1631, Sequence No. 087 and Sequence No. 077. The polynucleotides can be DNA or RNA molecules.

Advantageously, the description provides for an isolated IBDV strain, an isolated polypeptide (SEQ ID NO: 2) and an isolated IBDV polynucleotide, or antisense strands fully complementary thereto, of Sequence No. 1631 (SEQ ID NO: 1). In another advantageous embodiment, the description provides for an isolated IBDV strain, an isolated polypeptide (SEQ ID NO: 4) and an isolated IBDV polynucleotide, or antisense strands fully complementary thereto, of Sequence No. 087 (SEQ ID NO: 3). In yet another advantageous embodiment, the description provides for an isolated IBDV strain, an isolated polypeptide (SEQ ID NO: 6) and an isolated IBDV polynucleotide, or antisense strands fully complementary thereto, Sequence No. 077 (SEQ ID NO: 5).

For the purposes of the present description, sequence identity or homology is determined by comparing the sequences when aligned so as to maximize overlap and identity while minimizing sequence gaps. In particular, sequence identity may be determined using any of a number of mathematical algorithms. A nonlimiting example of a mathematical algorithm used for comparison of two sequences is the algorithm of Karlin & Altschul, Proc. Natl. Acad. Sci. USA 1990;87: 2264-2268, modified as in Karlin & Altschul, Proc. Natl. Acad. Sci. USA 1993;90: 5873-5877.

Another example of a mathematical algorithm used for comparison of sequences is the algorithm of Myers & Miller, CABIOS 1988;4: 11-17. Such an algorithm is incorporated into the ALIGN program (version 2.0) which is part of the GCG sequence alignment software package. When utilizing the ALIGN program for comparing amino acid sequences, a PAM120 weight residue table, a gap length penalty of 12, and a gap penalty of 4 can be used. Yet another useful algorithm for identifying regions of local sequence similarity and alignment is the FASTA algorithm as described in Pearson & Lipman, Proc. Natl. Acad. Sci. USA 1988;85: 2444-2448.

Advantageous for use according to the present description is the WU-BLAST (Washington University BLAST) version 2.0 software. WU-BLAST version 2.0 executable programs for several UNIX platforms can be downloaded from ftp ://blast. wustl. edu/blast/executables. This program is based on WU-BLAST version 1.4, which in turn is based on the public domain NCBI-BLAST version 1.4 (Altschul & Gish, 1996, Local alignment statistics, Doolittle ed., Methods in Enzymology 266: 460-480; Altschul et al., Journal of Molecular Biology 1990;215: 403-410; Gish & States, 1993;Nature Genetics 3: 266-272; Karlin & Altschul, 1993;Proc. Natl. Acad. Sci. USA 90: 5873-5877).

In general, comparison of amino acid sequences is accomplished by aligning an amino acid sequence of a polypeptide of a known structure with the amino acid sequence of a the polypeptide of unknown structure. Amino acids in the sequences are then compared and groups of amino acids that are homologous are grouped together. This method detects conserved regions of the polypeptides and accounts for amino acid insertions and deletions. Homology between amino acid sequences can be determined by using commercially available algorithms (see also the description of homology above). In addition to those otherwise mentioned herein, mention is made too of the programs BLAST, gapped BLAST, BLASTN, BLASTP, and PSI-BLAST, provided by the National Center for Biotechnology Information. These programs are widely used in the art for this purpose and can align homologous regions of two amino acid sequences.

In all search programs in the suite the gapped alignment routines are integral to the database search itself. Gapping can be turned off if desired. The default penalty (Q) for a gap of length one is Q=9 for proteins and BLASTP, and Q=10 for BLASTN, but may be changed to any integer. The default per-residue penalty for extending a gap (R) is R=2 for proteins and BLASTP, and R=10 for BLASTN, but may be changed to any integer. Any combination of values for Q and R can be used in order to align sequences so as to maximize overlap and identity while minimizing sequence gaps. The default amino acid comparison matrix is BLOSUM62, but other amino acid comparison matrices such as PAM can be utilized.

Alternatively or additionally, the term "homology " or "identity", for instance, with respect to a nucleotide or amino acid sequence, can indicate a quantitative measure of homology between two sequences. The percent sequence homology can be calculated as (N*_{ref} -* N*_{dif}*)*100/N*_{ref},* wherein N*_{dif}* is the total number of non-identical residues in the two sequences when aligned and wherein N*_{ref}* is the number of residues in one of the sequences. Hence, the DNA sequence AGTCAGTC will have a sequence identity of 75% with the sequence AATCAATC (N*_{ref}* = 8; N*_{dif}=*2).

Alternatively or additionally, "homology" or "identity" with respect to sequences can refer to the number of positions with identical nucleotides or amino acids divided by the number of nucleotides or amino acids in the shorter of the two sequences wherein alignment of the two sequences can be determined in accordance with the Wilbur and Lipman algorithm (Wilbur & Lipman, Proc Natl Acad Sci USA 1983;80:726 ), for instance, using a window size of 20 nucleotides, a word length of 4 nucleotides, and a gap penalty of 4, and computer-assisted analysis and interpretation of the sequence data including alignment can be conveniently performed using commercially available programs (e.g., Intelligenetics ^{™} Suite, Intelligenetics Inc. CA). When RNA sequences are said to be similar, or have a degree of sequence identity or homology with DNA sequences, thymidine (T) in the DNA sequence is considered equal to uracil (U) in the RNA sequence. Thus, RNA sequences are within the scope of the invention and can be derived from DNA sequences, by thymidine (T) in the DNA sequence being considered equal to uracil (U) in RNA sequences.

And, without undue experimentation, the skilled artisan can consult with many other ' programs or references for determining percent homology.

In a less advantageous embodiment, the IBDV sequences are compared by melting point curve analysis (see, e.g., U.S. Patent No. 6,495,326 ). Instead of comparing the IBDV sequences, the melting temperature of the nucleic acid sequence is determined and the patterns from the melting curve analysis are compared. Briefly, the PCR products are melted, e.g., from about 55 C to about 95 C in about 10 minutes and the shape of the melting curve is a function of GC content, length and sequence. The use of RT-PCR and probes with different melting temperatures (Tₘ) to characterize IBDV strains has been described (see, e.g., Jackwood et al., Avian Dis. 2003, 47, 738-744).

The description also provides for the identification of a novel strain of IBDV wherein the IBDV. sequence does not align to any of the one or more IBDV. sequences with close homology. The method comprises (a) generating an IBDV cDNA from the strain of IBDV, (b) aligning the IBDV cDNA with IBDV sequences, (c) comparing relatedness of aligned IBDV sequences, and (d) identifying a novel strain of IBDV if the IBDV cDNA is less than 95%, advantageously less than about 98% to about 99.9%, more advantageously less than about 99.6% or about 99.8%, homologous to any one of the known IBDV sequences. It is advantageous that the novel strain of IBDV has less than 50%, less than 60%, less than 70%, less than 75%, less than 80%, less than 85%, less than 90%, less than 93%, less than 95%, less than 97%, less than 98%, less than 98.1%, less than 98.2%, less than 98.3%, less than 98.4%, less than 98.5%, less than 98.6%, less than 98.7%, less than 98.8%, less than 98.9%, less than 99%, less than 99.1%, less than 99.2%, less than 99.3%, less than 99.4%, less than 99.5%, less than 99.6%, less than 99.7%, less than 99.8%, less than 99.9%, most advantageously less than about 99.6% or 99.8%, homology or identity with any known IBDV sequence.

The present description further provides isolating the novel strain of IBDV. Methods for isolating novel nucleic acids, such as viruses, are well known to one of skill in the art (see, e.g., protocols in Ausubel et al., Current Protocols in Molecular Biology, 1991, John Wiley and Sons, New York; Sambrooket al., Molecular Cloning: A laboratory manual, 1989, Cold Spring Harbor Laboratory, Cold Spring Harbor, New York). In an advantageous embodiment, the IBDV cDNA generated from the sample suspected of having IBDV is used as a probe to screen cDNA or genomic libraries specific to the sample (e.g., of a similar cell or tissue type) to isolate a full length clone corresponding to the novel strain of IBDV.

The present description also provides for selecting a vaccine to protect an avian against the strain of IBDV, wherein the vaccine has an IBDV sequence most closely matched to the IBDV cDNA. The method comprises (a) generating an IBDV cDNA from the strain of IBDV, (b) aligning the IBDV cDNA with IBDV sequences, (c) comparing relatedness of aligned IBDV sequences, and (d) identifying a vaccine for a strain of IBDV if the IBDV cDNA is at least 95% homologous, advantageously about 98% to about 99.9% homologous, to any known IBDV sequences that correspond to a known IBDV virus. It is advantageous that the IBDV strain will have at least 50%, at least 60%, at least 70%, at least 75%, at least 80%, at least 85%, at least 90%, at least 93%, at least 95%, at least 97%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% homology or identity with any known IBDV sequence in order for the known IBDV vaccine corresponding to the known IBDV sequence to be effective. It is advantageous that the IBDV strain will have at least 98%, at least 98.7%, at least 99.3%, at least 99.6%, at least 99.8%, most advantageously at least 99.3% or 99.6%, homology or identity with any known IBDV sequence in order for the known IBDV vaccine to be effective.

The known IBDV vaccine can be selected from any available IBDV vaccine. The IBDV strain will have at least 50%, at least 60%, at least 70%, at least 75%, at least 80%, at least 85%, at least 90%, at least 93%, at least 95%, at least 97%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% homology or identity with any known IBDV sequence correlating with the known IBDV vaccine to be effective. Advantageously, the IBDV vaccine correlates with the IBDV sequence, wherein the sequence of the IBDV strain will have at least 98%, at least 98.7%, at least 99.3%, at least 99.6%, at least 99.8%, most advantageously at least 99.3% or 99.6%, homology or identity with any known IBDV sequence correlating with the known IBDV vaccine to be effective.

Alternatively, or in addition to the homology analysis described above, an IBDV vaccine is selected, in part, by identifying critical amino acid residues that are involved in viral functions (e.g., virulence) that are identical in the known IBDV sequence corresponding to the vaccine that are also present in the IBDV strain of interest. Such critical amino acid residues include, but are not limited to, 222 (Ala) (see, e.g., Cao et al., Avian Dis. 1998 Apr-Jun;42(2):340-51, Hoque et al., J Biochem Mol Biol Biophys. 2002 Apr;6(2):93-9, Kwon et al., Avian Dis. 2000 Jul-Sep;44(3):691-6 and Parede et al., Avian Pathol. 2003 Oct;32(5):511-8), 242 (Ile) (see, e.g., Rudd et al., Arch Virol. 2002 Jul;17(7):1303-22), 249 (Lys) (see, e.g., Cao et al., Avian Dis. 1998 Apr-Jun;42(2):340-51), 253 (Gln) (see, e.g., Brandt et al., J Virol. 2001 Dec;75(24):11974-82), 254 (Gly) (see, e.g., Cao et al., Avian Dis. 1998 Apr-Jun;42(2):340-51 and Hoque et al., J Biochem Mol Biol Biophys. 2002 Apr;6(2):93-9), 256 (Ile) (see, e.g., Cao et al., Avian Dis. 1998 Apr-Jun;42(2):340-51, Hoque et al., J Biochem Mol Biol Biophys. 2002 Apr;6(2):93-9, Kwon et al., Avian Dis. 2000 Jul-Sep;44(3):691-6, Parede et al., Avian Pathol. 2003 Oct;32(5):511-8 and Rudd et al., Arch Virol. 2002 Jul;147(7):1303-22), 270 (Ala) (see, e.g., Hoque et al., J Biochem Mol Biol Biophys. 2002 Apr;6(2):93-9), 279 (Asp) (see, e.g., Brandt et al., J Virol. 2001 Dec;75(24):11974-82, Cao et al., Avian Dis. 1998 Apr-Jun;42(2):34.0-51, Lim et al., J Virol. 1999 Apr;73(4):2854-62 and Yamaguchi et al., Virology. 1996 Sep 1;223(1):219-23), 284 (Ala) (see, e.g., Brandt et al., J Virol. 2001 Dec;75(24):11974-82, Cao et al., Avian Dis. 1998 Apr-Jun;42(2):340-51, Lim et al., J Virol. 1999 Apr;73(4):2854-62 and Yamaguchi et al., Virology. 1996 Sep 1;223(1):219-23), 294 (Ile) (see, e.g., Cao et al., Avian Dis. 1998 Apr-Jun;42(2):340-51, Hoque et al., J Biochem Mol Biol Biophys. 2002 Apr;6(2):93-9, Kwon et al., Avian Dis. 2000 Jul-Sep;44(3):691-6, Parede et al., Avian Pathol. 2003 Oct;32(5):511-8 and Rudd et al., Arch Virol. 2002 Jul;147(7):1303-22) and 299 (Ser) (see, e.g., Hoque et al., J Biochem Mol Biol Biophys. 2002 Apr;6(2):93-9 and Kwon et al., Avian Dis. 2000 Jul-Sep;44(3):691-6). Other critical residues include the nucleotides and amino acids described *infra* in Example 3. It is understood that the corresponding nucleic acid codon (except for the degenerate third nucleotide) and location in the nucleotide sequence can be determined by one of ordinary skill in the art.

It is understood by one of skill in the art that the sequence identity or homology is not limited and includes regions of the IBDV sequence that are compared. In an advantageous embodiment, the IBDV sequences correspond to one or more hypervariable portions of IBDV, such as VP1, VP2, VP3, VP4 or VP5. It is understood by one of skill in the art that if the IBDV sequence corresponds to VP2, then the known IBDV sequence also corresponds to VP2.

Advantageously, the IBDV vaccine is manufactured by Merial, including but not limited to Bur-Cell series, Bursa Blen™ M, IBD Blen™, S-706 or SVS-510. In another advantageous embodiment, the IBDV vaccine correlates with any of the IBDV nucleotide sequences according to Table 2 and/or FIG. 1. In another advantageous embodiment, the IBDV vaccine is an avian polynucleotide vaccine formula (GenBank Accession Nos. BD009825, BD009826, BD009827, BD009829, BD009830, BD009832 and BD009833), broad-spectrum infectious bursal disease virus vaccine (GenBank Accession Nos. BD144646 and BD144647), infectious bursa disease virus partial VP2 gene, genomic RNA, isolate Ventri (GenBank Accession No. AJ586960), infectious bursa disease virus partial VP2 gene, genomic RNA, isolate BURSINE Plus (GenBank Accession No. AJ586961), infectious bursa disease virus partial VP2 gene, genomic RNA, isolate MB (GenBank Accession No. AJ586962), infectious bursa disease virus partial VP2 gene, genomic RNA, isolate D78 (GenBank Accession No. AJ586963), infectious bursa disease virus partial VP2 gene, genomic RNA, isolate NVRI-VOM (GenBank Accession No. AJ586964), infectious bursa disease virus partial VP2 gene, genomic RNA, isolate IBA (GenBank Accession No. AJ586965), infectious bursa disease virus partial VP2 gene, genomic RNA, isolate Nobilis Gumboro 228E (GenBank Accession No. AJ586966), infectious bursa disease virus partial VP2 gene, genomic RNA, isolate Bursaplex (GenBank Accession No. AJ586967), or infectious bursa disease virus partial VP2 gene, genomic RNA, isolate V877 (GenBank Accession No. AJ586968).

The present description further provides a computer-assisted method for characterizing a strain of IBDV by using a computer system, e.g., a programmed computer comprising a processor, a data storage system, an input device, and an output device, the steps of: (a) inputting into the programmed computer through the input device data comprising sequences of IBDV generated from a sample suspected of having, i.e., infected with, a strain of IBDV, thereby generating a data set; (b) comparing, using the processor, the data set to a computer database of IBDV sequences stored in the computer data storage system; (c) selecting from the database, using computer methods, IBDV sequences stored in the computer data storage system having a portion that is about 95%, advantageously about 98% to about 99.8%, most advantageously about 99.3% to about 99.6%, homologous to the data set; (d) and outputting to the output device the selected IBDV sequences having a portion that is at least about 95%, advantageously about 98% to about 99.8%, most advantageously about 99.3% to about 99.6%, homologous to the data set, or optionally outputting to the output device indicating the absence of IBDV sequences having a portion that is at least about 95%, advantageously about 98% to about 99.8%, most advantageously about 99.3% to about 99.6%, homologous to the data set if no IBDV sequences have a portion that is at least about 95%, advantageously about 98% to about 99.8%, most advantageously about 99.3% to about 99.6%, homologous to the data set, thereby characterizing a strain of IBDV.

The systems, such as computer systems, are intended to characterize a strain of IBDV from a sample suspected of having, i.e., infected with, a strain of IBDV. The system can contain known IBDV sequences which include, but are not limited to, the sequences of Figure 1 and the sequenced referenced by the accession numbers in Table 2 (see Example 4, infra), as well as the amino acid sequences deduced from the nucleotide sequences and the nucleotide sequences amplified by the primers referenced by the accession numbers and the amino acid sequences deduced therefrom. The description also involves computer readable media with IBDV sequences that include, but are not limited to, the sequences of Figure 1 and the sequenced referenced by the accession numbers in Table 2 (see Example 4, infra), as well as the amino acid sequences deduced from the nucleotide sequences and the nucleotide sequences amplified by the primers referenced by the accession numbers and the amino acid sequences deduced therefrom.

In one embodiment, the sample is a paraffin-embedded tissue sample. In an advantageous embodiment, IBDV cDNAs are generated by extracting RNA from the paraffin-embedded tissue sample and RT-PCR amplification of the IBDV cDNA with IBDV-specific primers. Methods of extracting RNA and RT-PCR amplification of a cDNA from a paraffin-embedded tissue sample are well known in the art (see, e.g., Brown et al., Vet Pathol. 2003;40(5):613, and U.S. Patent Nos. 6,248,535; 6,428,963 and 6,610,488)).

In an advantageous embodiment, the IBDV sequences correspond to one or more hypervariable portions of IBDV, such as VP1, VP2, VP3, VP4 or VP5. The IBDV sequences in the storage system are nucleic acid sequences or amino acid sequences. The IBDV sequences in the storage system include, but are not limited to; the sequences of Figure 1 and the sequenced referenced by the accession numbers in Table 2 (see Example 4, infra), as well as the amino acid sequences deduced from the nucleotide sequences and the nucleotide sequences amplified by the primers referenced by the accession numbers and the amino acid sequences deduced therefrom. In another embodiment, a data set of amino acid sequences is deduced if the input sequences are nucleotide sequences, e.g., by the EditSeq program from DNASTAR, Inc.

The description also provides for the identification of a novel strain of IBDV wherein the IBDV sequence does not align to any of the one or more IBDV sequences with close homology. The method comprises identifying a novel strain of IBDV if no IBDV sequences have a portion that is at least about 95%, advantageously about 98% to about 99.8%, most advantageously about 99.3% to about 99.6%, homologous to the data set. It is advantageous that the novel strain of IBDV has has less than 50%, less than 60%, less than 70%, less than 75%, less than 80%, less than 85%, less than 90%, less than 93%, less than 95%, less than 97%, less than 98%, less than 98.1%, less than 98.2%, less than 98.3%, less than 98.4%, less than 98.5%, less than 98.6%, less than 98.7%, less than 98.8%, less than 98.9%, less than 99%, less than 99.1%, less than 99.2%, less than 99.3%, less than 99.4%, less than 99.5%, less than 99.6%, less than 99.7%, less than 99.8%, less than 99.8%, most advantageously less than about 99.6% or 99.8%, homology or identity with any known IBDV sequence. Alignment programs, such as but not limited to, ALIGN, FASTA, MegAlign, NCBI-BLAST (e.g., BLAST, gapped BLAST, BLASTN, BLASTP, and PSI-BLAST), and WU-BLAST (Washington University BLAST), can be used for characterization of the IBDV sequence in comparison to the known IBDV sequences in the database. Such alignment programs can be used as algorithms for calculating homology or identity between the IBDV sequence to be characterized and the known IBDV sequences in the database. One of skill in the art could adapt these algorithms into computer programs with routine experimentations for purposes of this description. The present description further provides isolating the novel strain of IBDV.

The present description also provides for selecting a vaccine to protect an avian against the strain of IBDV, wherein the vaccine has an IBDV sequence most closely matched to the IBDV cDNA. The method comprises identifying IBDV strains with one or more IBDV sequences having a portion that is at least about 95%, advantageously about 98% to about 99.8%, most advantageously about 99.3% to about 99.6%, homologous to the data set It is advantageous that the IBDV strain will be have at least 50%, at least 60%, at least 70%, at least 75%, at least 80%, at least 85%, at least 90%, at least 93%, at least 95%, at least 97%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, at least 99.9% homology or identity with any known IBDV sequence in order for the known IBDV vaccine to be effective. It is advantageous that the IBDV strain will have at least 98%, at least 98.7%, at least 99.3%, at least 99.6%, at least 99.8%, most advantageously at least 99.3% or 99.6%, homology or identity with any known IBDV sequence in order for the known IBDV vaccine to be effective. Alignment programs, such as but not limited to, ALIGN, FASTA, MegAlign, NCBI-BLAST (e.g., BLAST, gapped BLAST, BLASTN, BLASTP, and PSI-BLAST), and WU-BLAST (Washington University BLAST), can be used for characterization of the IBDV sequence in comparison to the known IBDV sequences in the database. Such alignment programs can be used as algorithms for calculating homology or identity between the IBDV sequence to be characterized and the known IBDV sequences in the database. One of skill in the art could adapt these algorithms into computer programs with routine experimentations for purposes of this description.

The present description also provides for a method of transmitting data comprising transmission of information from such methods herein discussed or steps thereof, e.g., via telecommunication, telephone, video conference, mass communication, e.g., presentation such as a computer presentation (e.g. POWERPOINT), internet, email, documentary communication such as a computer program (e.g. WORD) document and the like.

The description relates to a computer system and a computer readable media for characterizing a strain of IBDV, the system containing either: IBDV nucleotide sequences according to Table 2 and/or FIG. 1 or IBDV amino acid sequences of Table 3 or IBDV amino acid sequences derived from the nucleotide sequences according to Table 2 and/or FIG. 1. A computer readable media containing either: IBDV nucleotide sequences according to Table 2 and/or FIG. 1 or IBDV amino acid sequences of Table 3 or IBDV amino acid sequences derived from the nucleotide sequences according to Table 2 and/or FIG. 1. The description also relates to a method of doing business comprising providing to a user the computer system described herein or the media described herein or either: IBDV nucleotide sequences according to Table 2 and/or FIG. 1 or IBDV amino acid sequences of Table 3 or IBDV amino acid sequences derived from the nucleotide sequences according to Table 2 and/or FIG. 1.

"Computer readable media" refers to any media which can be read and accessed directly by a computer, and includes, but is not limited to: magnetic storage media such as floppy discs, hard storage medium and magnetic tape; optical storage media such as optical discs or CD-ROM; electrical storage media such as RAM and ROM; and hybrids of these categories, such as magnetic/optical media. By providing such computer readable media, the IBDV sequence data can be routinely accessed to characterize an IBDV sequence isolated from a sample suspected of having, i.e., being infected with, IBDV. Alignment programs, such as but not limited to, ALIGN, FASTA, MegAlign, NCBI-BLAST (e.g., BLAST, gapped BLAST, BLASTN, BLASTP, and PSI-BLAST), and WU-BLAST (Washington University BLAST), can be used for characterization of the IBDV sequence in comparison to the known IBDV sequences in the database. Such alignment programs can be used as algorithms for calculating homology or identity between the IBDV sequence to be characterized and the known IBDV sequences in the database. One of skill in the art could adapt these algorithms into computer programs with routine experimentations for purposes of this description.

The description further comprehends methods of doing business by providing access to such computer readable media and/or computer systems and/or sequence data to users; e.g., the media and/or sequence data can be accessible to a user, for instance on a subscription basis, via the Internet or a global communication/computer network; or, the computer system can be available to a user, on a subscription basis.

A "computer system" refers to the hardware means, software means and data storage means used to analyze the IBDV sequence of the present description. The minimum hardware means of computer-based systems of the invention may comprise a central processing unit (CPU), input means, output means, and data storage means. Desirably, a monitor is provided to visualize structure data. The data storage means may be RAM or other means for accessing computer readable media of the invention. Examples of such systems are microcomputer workstations available from Silicon Graphics Incorporated and Sun Microsystems running Unix based, Linux, Windows NT or IBM OS/2 operating systems.

Accordingly, the description further comprehends methods of transmitting information obtained in any method or step thereof described herein or any information described herein, e.g., via telecommunications, telephone, mass communications, mass media, presentations, internet, email, etc.

The apparatus and method for storing and/or retrieving a target data sequence in response to an input data sequence described in U.S. Patent No. 6,643,653. is advantageous for the present description. The apparatus of U.S. Patent No. 6,643,653 requires a relatively small amount of storage space and thus provides a high speed of operation (e.g., retrieval of the target sequence).

Applying the method and apparatus of U.S. Patent No. 6,643,653 to the present description, key data sequence (i.e., the IBDV sequence to be characterized) is received, which may be a nucleotide sequence, an amino acid sequence, or melting curve data, and an equivalent, translated, normalized or other related target data sequence is retrieved if it exists. A target data sequence (e.g., a specific IBDV vaccine) may be identified and retrieved for any number of different given data sequences, assuming that there is minimum homology between the IBDV sequences.

In an embodiment of the description, the data structure used to store and retrieve target sequences may be considered a virtual tree. Illustratively, the virtual tree starts at a root, the size of which (e.g., number of cells) may be equivalent to the possible values of the first datum, item or other unit of the given data sequence (e.g., nucleotide or amino acid sequence). The virtual tree also includes virtual blocks of variable sizes (i.e., comprising a variable number of nodes), and leaves that are also of variable sizes and which contain target data sequences of variable lengths. The virtual tree is traversed for a given or key data sequence by first locating a root cell that corresponds to the first unit within the key sequence. That cell will identify (e.g., by memory address or offset) the virtual block that contains a node corresponding to the next unit. That node will also store a memory offset or pointer to the next virtual block having a node corresponding to the next item, and so on. The node corresponding to the final item of the key sequence identifies the leaf node that contains the target data sequence.

The description also provides for the use of new IBDV strains identified by the methods of the description as vaccines. It is advantageous for the new IBDV strain to be cloned in an expression vector and expressed in a cell. Alternatively, the new IBDV can be isolated and cultured in a cell culture system.

The description provides for isolated IBDV strains, isolated polypeptides and isolated IBDV. polynucleotides, or antisense strands fully complementary thereto of, Sequence No. 1631, Sequence No. 087 and Sequence No. 077. The polynucleotides of Sequence No. 1631, Sequence No. 087 and Sequence No. 077 (SEQ ID NOS: 1, 3 and 5) can be be cloned in an expression vector and expressed in a cell. Alternatively, the IBDV strains of Sequence No. 1631, Sequence No. 087 and Sequence No. 077 can be isolated and cultured in a cell culture system.

Elements for the expression of the novel IBDV are advantageously present in an inventive vector. In minimum manner, this comprises, consists essentially of, or consists of an initiation codon (ATG), a stop codon and a promoter, and optionally also a polyadenylation sequence for certain vectors such as plasmid and certain viral vectors, e.g., viral vectors other than poxviruses. When the polynucleotide encodes a polyprotein fragment, e.g. VP2, VP3 or VP4 advantageously, in the vector, an ATG is placed at 5' of the reading frame and a stop codon is placed at 3'. Other elements for controlling expression may be present, such as enhancer sequences, stabilizing sequences and signal sequences permitting the secretion of the protein.

Methods for making and/or administering a vector or recombinants or plasmid for expression of gene products of genes of the description either *in vivo* or *in vitro* can be any desired method, e.g., a method which is by or analogous to the methods disclosed in, or disclosed in documents cited in: U.S. Patent Nos. 4,603,112; 4,769,330; 4,394,448; 4,722,848; 4,745,051; 4,769,331; 4,945,050; 5,494,807; 5,514,375; 5,744,140; 5,744,141; 5,756,103; 5,762,938; 5,766,599; 5,990,091; 5,174,993; 5,505,941; 5,338,683; 5,494,807; 5,591,639; 5,589,466; 5,677,178; 5,591,439; 5,552,143; 5,580,859; 6,130,066; 6,004,777; 6,130,066; 6,497,883; 6,464,984; 6,451,770; 6,391,314; 6,387,376; 6,376,473; 6,368,603; 6,348,196; 6,306,400; 6,228,846; 6,221,362; 6,217,883; 6,207,166; 6,207,165; 6,159,477; 6,153,199; 6,090,393; 6,074,649; 6,045,803; 6,033,670; 6,485,729; 6,103,526; 6,224,882; 6,312,682; 6,348,450 and 6; 312,683; WO 90/01543; WO91/11525; WO 94/16716; WO 96/39491; WO 98/33510; EP 265785; EP 0 370 573; Andreansky et al., Proc. Natl. Acad. Sci. USA 1996;93:11313-11318; Ballay et al., EMBO J. 1993;4:3861-65; Felgner et al., J. Biol. Chem. 1994;269:2550-2561; Frolov et al., Proc. Natl. Acad. Sci. USA 1996;93:11371-11377; Graham, Tibtech 1990;8:85-87; Grunhaus et al., Sem. Virol. 1992;3:237-52; Ju et al., Diabetologia 1998;41:736-739; Kitson et al., J. Virol. 1991;65:3068-3075; McClements et al., Proc. Natl. Acad. Sci. USA 1996;93:11414-11420; Moss, Proc. Natl. Acad. Sci. USA 1996;93:11341-11348; Paoletti, Proc. Natl. Acad. Sci. USA 1996;93:11349-11353; Pennock et al., Mol. Cell. Biol. 1984;4:399-406; Richardson (Ed), Methods in Molecular Biology 1995;39, "Baculovirus Expression Protocols," Humana Press Inc.; Smith et al. (1983) Mol. Cell. Biol. 1983;3:2156-2165; Robertson et al., Proc. Natl. Acad. Sci. USA 1996;93:11334-11340; Robinson et al., Sem. Immunol. 1997;9:271; and Roizman, Proc. Natl. Acad. Sci. USA 1996;93:11307-11312. Thus, the vector in the description can be any suitable recombinant virus or virus vector, such as a poxvirus (e.g., vaccinia virus, avipox virus, canarypox virus, fowlpox virus, raccoonpox virus, swinepox virus, etc.), adenovirus (e.g., canine adenovirus), herpesvirus, baculovirus, retrovirus, etc.; or the vector can be a plasmid. The herein cited documents, in addition to providing examples of vectors useful in the practice of the description, can also provide sources for non-IBDV proteins or epitopes thereof, e.g., non-IBDV immunogens or epitopes thereof, cytokines, etc. to be expressed by vector or vectors in, or included in, multivalent or cocktail immunogenic compositions or vaccines of the description.

The present description also relates to preparations comprising vectors, such as expression vectors, e.g., vaccines or immunogenic compositions. The preparations can comprise, consist essentially of, or consist of one or more vectors, e.g., expression vectors, such as *in vivo* expression vectors, comprising, consisting essentially or consisting of (and advantageously expressing) one or more of the IBDV polynucleotides and, advantageously, the vector contains and expresses a polynucleotide that includes, consists essentially of, or consists of a coding region encoding IBDV, in a pharmaceutically or veterinarily acceptable carrier, excipient or vehicle. Thus, according to an embodiment of the description, the other vector or vectors in the preparation comprises, consists essentially of or consists of a polynucleotide that encodes, and under appropriate circumstances the vector expresses one or more other proteins of IBDV or an epitope thereof.

According to another embodiment, the vector or vectors in the preparation comprise, or consist essentially of, or consist of polynucleotide(s) encoding one or more proteins or epitope(s) thereof of IBDV, e.g., of one or more IBDV strains or isolates; and, advantageously, in a suitable host cell or under appropriate conditions, the vector or vectors have express of the polynucleotide(s). The description preparation advantageously comprises, consists essentially of, or consists of, at least two vectors comprising, consisting essentially of, or consisting of, and advantageously also expressing, preferably *in vivo* under appropriate conditions or suitable conditions or in a suitable host cell, polynucleotides from different IBDV strains or isolates encoding the same proteins and/or for different proteins, but preferably for the same proteins. As to preparations containing one or more vectors containing, consisting essentially of or consisting of polynucleotides encoding, and preferably expressing, advantageously *in vivo*, IBDV, or an epitope thereof, it is preferred that the expression products be from two, three or more different IBDV strains or isolates, advantageously strains. The description is also directed at mixtures of vectors that contain, consist essentially of, or consist of coding for, and express, IBDV of different strains.

According to yet another embodiment and as will be shown in greater detail hereinafter, the other vector or vectors in the preparation comprise and express one or more cytokines and/or one or more immunogens of one or more other pathogenic agents. Sources for cytokines, immunogens for other pathogenic agents or epitope(s) thereof, and nucleic acid molecules encoding the same, may be found in herein cited documents, as well as in, WO02096349, WO0208162, WO0020025, WO00152888, WO0145735, WO00127097, WO0116330, WO0077210, WO0077188, W00077043, WO9842743, WO9833928, WO9749826, WO9749825, U.S. Patents Nos. 6,387,376, 6,306,400, 6,159,477, 6,156,567, 6,153,199, 6,090,393, 6,074,649, 6,033,670.

According to an embodiment of the description, the vectors, e.g., *in vivo* expression vectors, are viral vectors. Viral vectors, e.g., viral expression vectors are advantageously: poxviruses, e.g. vaccinia virus or an attenuated vaccinia virus, (for instance, MVA, a modified Ankara strain obtained after more than 570 passages of the Ankara vaccine strain on chicken embryo fibroblasts; see Stickl & Hochstein-Mintzel, Munch. Med. Wschr., 1971, 113, 1149-1153; Sutter et al., Proc. Natl. Acad. Sci. U.S.A., 1992, 89,10847-10851; available as ATCC VR-1508; or NYVAC, *see* U.S. Patent No. 5,494,807, for instance, Examples 1 to 6 and *et seq* of U.S. Patent No. 5,494,807 which discuss the construction of NYVAC, as well as variations of NYVAC with additional ORFs deleted from the Copenhagen strain vaccinia virus genome, as well as the insertion of heterologous coding nucleic acid molecules into sites of this recombinant, and also, the use of matched promoters; see also WO96140241), avipox virus or an attenuated avipox virus (e.g., canarypox, fowlpox, dovepox, pigeonpox, quailpox, ALVAC or TROVAC; see, e.g., U.S. Patent No. 5,505,941, 5,494,807), swinepox, raccoonpox, camelpox, or myxomatosis virus; adenoviruses, such as avian, canine, porcine, bovine, human adenoviruses; or herpes viruses, such as canine herpes virus (CHV), Marek's disease virus (MDV serotypes 1 and 2), turkey herpes virus (HVT or MDV serotype 3), or duck herpes virus. When a herpes virus is used, the vector HVT is preferred for the vaccination of the avian species and the vector EHV for the vaccination of horses.

According to another embodiment of the description, the poxvirus vector, e.g., expression vector, is a canarypox virus or a fowlpox virus vector, advantageously an attenuated canarypox virus or fowlpox virus. In this regard, is made to the canarypox available from the ATCC under access number VR-111. Attenuated canarypox viruses are described in U.S. Patent No. 5,756,103 (ALVAC) and WO01/05934. Numerous fowlpox virus vaccination strains are also available, e.g. the DEFROSEC CT strain marketed by MERIAL and the NOBILIS VARIOLE vaccine marketed by Intervet; and, reference is also made to U.S. Patent No. 5,766,599 which pertains to the atenuated fowlpox strain TROVAC.

For information on poxviruses and how to generate recombinants thereof and how to administer recombinants thereof, the skilled artisan can refer documents cited herein and to WO90/12882, e.g., as to vaccinia virus mention is made of U.S. Patents Nos. 4,769,330, 4,722,848, 4,603,112, 5,110,587, 5,494,807, and 5,762,938 *inter alia*; as to fowlpox, mention is made of U.S. Patents Nos. 5,174,993, 5,505,941 and US-5,766,599 *inter alia*; as to canarypox mentionis made of U.S. Patent No. 5,756,103 *inter alia*; as to swinepox mention is made of U.S. Patent No. 5,382,425 *inter alia*; and, as to raccoonpox, mention is made of WO00/03030 *inter alia*.

When the expression vector is a vaccinia virus, insertion site or sites for the polynucleotide or polynucleotides to be expressed are advantageously at the thymidine kinase (TK) gene or insertion site, the hemagglutinin (HA) gene or insertion site, the region encoding the inclusion body of the A type (ATI); see also documents cited herein, especially those pertaining to vaccinia virus. In the case of canarypox, advantageously the insertion site or sites are ORF(s) C3, C5 and/or C6; see also documents cited herein, especially those pertaining to canarypox virus. In the case of fowlpox, advantageously the insertion site or sites are ORFs F7 and/or F8; see also documents cited herein, especially those pertaining to fowlpox virus. The insertion site or sites for MVA virus area advantageously as in various publications, including Carroll M. W. et al., Vaccine, 1997, 15 (4), 387-394; Stittelaar K. J. et al., J. Virol., 2000, 74 (9), 4236-4243; Sutter G. et al., 1994, Vaccine, 12 (11), 1032-1040; and, in this regard it is also noted that the complete MVA genome is described in Antoine G., Virology, 1998, 244, 365-396, which enables the skilled artisan to use other insertion sites or other promoters.

Preferably, when the expression vector is a poxvirus, the polynucleotide to be expressed is inserted under the control of a specific poxvirus promoter, e.g., the vaccinia promoter 7.5 kDa (Cochran et al., J. Virology, 1985, 54, 30-35), the vaccinia promoter I3L (Riviere et al., J. Virology, 1992,66,3424-3434), the vaccinia promoter HA (Shida, Virology, 1986, 150, 451-457), the cowpox promoter ATI (Funahashi et al., J. Gen. Virol., 1988, 69, 35-47), the vaccinia promoter H6 (Taylor J. et al., Vaccine, 1988, 6, 504-508; Guo P. et al. J. Virol., 1989, 63, 4189-4198; Perkus M. et al., J. Virol., 1989, 63, 3829-3836), *inter alia*.

Preferably, for the vaccination of mammals the expression vector is a canarypox or a fowlpox. In this way, there can be expression of the heterologous proteins with limited or no productive replication. Preferably, for the vaccination of avians, the expression vector is a canarypox or a fowlpox.

When the expression vector is a herpes virus of turkeys or HVT, advantageous insertion site or sites are located in the BamHI I fragment or in the BamHI M fragment of HVT. The HVT BamHI I restriction fragment comprises several open reading frames (ORFs) and three intergene regions and comprises several preferred insertion zones, such as the three intergene regions 1, 2 and 3, which are preferred regions, and ORF UL55 (see, e.g., FR-A-2 728 795, U.S. Patent No. 5,980,906). The HVT BamHI M restriction fragment comprises ORF UL43, which is also a preferred insertion site (see, e.g., FR-A-2 728 794, U.S. Patent No. 5,733,554).

Preferably, when the expression vector is a herpes virus, the polynucleotide to be expressed is inserted under the control of a eukaryotic promoter, such as a strong eukaryote promoter, preferably a CMV-IE (murine or human) promoter; that is, in embodiments herein, the polynucleotide to be expressed is operably linked to a promoter, and in herpes virus embodiments, advantageously the polynucleotide to be expressed is operably linked to a strong eukatyotic promoter such as a mCMV-IE or hCMV-IE promoter.

In an advantageous embodiment, the Semliki Forest virus (SFV) expression system is used for the expression of IBDV, particularly as a basis for avian vaccine development (see, e.g., Phenix et al., Vaccine. 2001 Apr 30;19(23-24):3116-23).

According to another embodiment of the invention, the expression vectors are expression vectors used for the *in vitro* expression of proteins in an appropriate cell system. The expressed proteins can be harvested in or from the culture supernatant after, or not after secretion (if there is no secretion a cell lysis typically occurs or is performed), optionally concentrated by concentration methods such as ultrafiltration and/or purified by purification means, such as affinity, ion exchange or gel filtration-type chromatography methods.

The present description includes infection of an appropriate host cell with IBDV and provides methods of culturing IBDV in the host cell. The host cell is contacted with the virus under conditions which result in viral infection of the host cell e.g., according to a specific multiplicity of infection (MOI) specific to IBDV and the cell type. It is well known to one of skill in the art that the MOI can according to the particular virus and host cell and that routine experimentation is necessary at times to determine the optimal MOI for a particular virus, and in some instances, the particular cell to be infected. The infected cells are then incubated for a period of time sufficient to allow for viral replication. In one embodiment, the virus is harvested from the culture of infected cells. In another embodiment, the culture of infected cells is frozen, e.g., at -70° C. In yet another embodiment, the method further involves the measurement of viral multiplication, e.g., by measuring the cytopathic effect (CPE) on cells or by comparing the virus titer at varying timepoints during inoculation.

Host cells that can be used in the present description include, but are not limited to, 293-EBNA cells, avian stem cells, BGM-70 cells, chicken B-lymphocyte cell line (RP9), chicken embryo bursal cells, chicken embryo fibroblasts (CEF), chicken kidney embryo cells, chicken macrophage [MQ-NCSU] cells, cotton rat lung cells, HRT-18 cell line, HuTu 80 cells, LSCC-RP12 B-lymphoblastoid cells, LSCC-RP9 B-lymphoblastoid cells, MOP-8 cells, PANC-1 cells, quail [QT35] cells and Vero cells. It is understood to one of skill in the art that conditions for infecting a host cell varies according to the particular virus and that routine experimentation is necessary at times to determine the optimal conditions for culturing IBDV depending on the host cell.

The term of "immunogenic composition" covers herein any composition able, once it has been administered to an animal, e.g., avian, to elicit an immune response against the virus or antigen or immunogen or epitope. The term of "vaccine" covers herein any composition able, once it has been administered to the animal, e.g., avian, to induce a protective immune response against the virus, or to efficaciously protect the animal against said virus.

In an advantageous embodiment, the compositions or vaccines of the present description encompass the novel IBDV strains described herein, i.e., Sequence No. 1631, Sequence No. 087 and Sequence No. 077.

Immunogenic compositions or vaccines according to the description can include the virus culture or preparation or antigen or immunogen or epitope of the virus, and at least one immunogen, antigen or epitope of another pathogen or another pathogen (e.g., inactivated or attenuated pathogen). Such an immunogen, antigen or epitope may e.g. be of bacterial, or parasitic or viral origin or an inactivated or attenuated form of the pathogen. The description also comprehends kits to prepare these combination compositions, as well as methods for making these combination compositions and the use of the components of these combination compositions to prepare the combination compositions. Accordingly, the description involves a kit for preparing the combination immunogenic or vaccine compositions of the description; for instance, such a kit that comprises (a) an organism, pathogen or virus or antigen or epitope thereof (advantageously a virus as mentioned herein) and (b) an organism, pathogen or virus or immunogen, antigen or epitope thereof (advantageously a virus or immunogen, antigen or epitope thereof, but other pathogens as herein mentioned are also contemplated) that is different than (a), in separate containers, optionally in the same package, and optionally with instructions for admixture and/or administration.

Immunogenic compositions and/or vaccines according to the description can include IBDV culture or preparation (e.g., inactivated or attenuated IBDV, or an immunogen or antigen or epitope thereof), and at least one immunogen, antigen or epitope of another avian pathogen (including without limitation the pathogen in inactivated or attenuated form). For avian multivalent immunogenic compositions and multivalent vaccines, the additional avian pathogen(s), as to which additional avian antigen(s) or immunogen(s) or epitope(s) thereof are included in and/or expressed by the multivalent immunogenic compositions and multivalent vaccines, are viruses, diseases, or pathogens of the Marek's disease virus (MDV) (e.g., serotypes 1 and 2, advantageously 1), Newcastle disease virus (NDV), paramyxoviruses other than Newcastle disease (PMV2 to PMV7), infectious bronchitis virus (IBV), infectious anaemia virus or chicken anemia virus (CAV), infectious laryngotracheitis virus (ILTV), encephalomyelitis virus or avian encephalomyelitis virus (AEV or avian leukosis virus ALV), virus of hemorragic enteritis of turkeys (HEV), pneumovirosis virus (TRTV), fowl plague virus (avian influenza), chicken hydropericarditis virus, avian reoviruses, coccidiosis, egg drop syndrome (EDS76), fowl pox, inclusion body hepatitis (adenovirus), lymphoproliferative disease of turkeys, reticuloendotheliosis in chickens, reticuloendotheliosis in turkeys, rotavirus enteritis, and turkey rhinotracheitis, *Escherichia coli, Mycoplasma gallinarum*, *Mycoplasma gallisepticum*, *Haemophilus avium*, *Pasteurella gallinarum*, *Pasteurella multocida gallicida*, and mixtures thereof. Advantageously, for MDV the immunogen is advantageously gB and/or gD, e.g., gB and gD, for NDV the immunogen is advantageously HN and/or F, e.g., HN and F; for IBDV the immunogen advantageously isVP2; for IBV the immunogen is advantageously S (more advantageously S1) and/or M and/or N, e.g., S (or S1) and M and/or N; for CAV the immunogen is advantageously VP1 and/or VP2; for ILTV the immunogen is advantageously gB and/or gD; for AEV the immunogen advantageously is env and/or gag/pro, e.g., env and gag/pro or gag/pro; for HEV the immunogen is advantageously the 100K protein and/or hexon; for TRTV the immunogen is advantageously F and/or G, and for fowl plague the immunogen is advantageously HA and/or N and/or NP, e.g., HA and N and/or NP. Thus, the description also involves methods for making these compositions, as well as kits therefor.

An immunogenic composition or vaccine according to the description that also comprises such an additional immunogenic component (additional immunogen, antigen or epitope) has the advantage that it induces an immune response or protection against several infections or maladies or causative agents thereof at the same time. This additional immunogenic component can be an attenuated or inactivated micro-organism, a recombinant construct or sub-units (e.g. proteins, glycoproteins, polypeptides, or epitopes). Epitope determination procedures, such as, generating overlapping peptide libraries (Hemmer et al., Immunology Today, 1998, 19 (4), 163-168), Pepscan (Geysen H. M. et al., Proc. Nat. Acad. Sci. USA, 1984, 81 (13), 3998-4002; Geysen H. M. et al., Proc. Nat. Acad. Sci. USA, 1985, 82 (1), 178-182; Van der Zee R. et al., Eur. J. Immunol., 1989, 19 (1), 43-47; Geysen H. M., Southeast Asian J. Trop. Med. Public Health, 1990, 21 (4), 523-533; Multipin Peptide Synthesis Kits de Chiron) and algorithms (De Groot A. et al., Nature Biotechnology, 1999, 17, 533-561), can be used in the practice of the description, to determine epitopes of immunogens, antigens, polypeptides, glycoproteins and the like, without undue experimentation. From that information, one can construct nucleic acid molecules encoding such an epitope, and from that knowledge and knowledge in the art, one can construct vectors or constructs, e.g., recombinant viruses or vectors or plasmids that express immunogens, epitopes or antigens; all without undue experimentation.

The pharmaceutically or veterinarily acceptable carriers or vehicles or excipients are well known to the one skilled in the art. For example, a pharmaceutically or veterinarily acceptable carrier or vehicle or excipient can be a 0.9% NaCl (e.g., saline) solution or a phosphate buffer. The pharmaceutically or veterinarily acceptable carrier or vehicle or excipients may be any compound or combination of compounds facilitating the administration of the vector (or protein expressed from an inventive vector *in vitro*); advantageously, the carrier, vehicle or excipient may facilitate transfection and/or improve preservation of the vector (or protein). Doses and dose volumes are herein discussed in the general description of immunization and vaccination methods, and can also be determined by the skilled artisan from this disclosure read in conjunction with the knowledge in the art, without any undue experimentation.

The immunogenic compositions and vaccines according to the description preferably comprise or consist essentially of one or more adjuvants. Advantageously, the adjuvant used for inactivated IBDV is water-in-oil emulsion, based on paraffin oil (see, e.g., Vaccine Design The Subunit and Adjuvant Approach Edited by Powel and Newman Plenum Press NY 1995 page 219, Woodard Bacterial vaccines Edited by Riss 1990 pages 281-306; and Brugh et al Am. J. Vet. Res. 1983, 44, 72-75).

Other suitable adjuvants for use in the practice of the present description are (1) polymers of acrylic or methacrylic acid, maleic anhydride and alkenyl derivative polymers, (2) immunostimulating sequences (ISS), such as oligodeoxyribonucleotide sequences having one ore more non-methylated CpG units (Klinman D. M. et al., Proc. Natl. Acad. Sci., USA, 1996, 93, 2879-2883; WO98/16247), (3) an oil in water emulsion, such as the SPT emulsion described on p 147 of "Vaccine Design, The Subunit and Adjuvant Approach" published by M. Powell, M. Newman, Plenum Press 1995, and the emulsion MF59 described on p 183 of the same work, (4) cation lipids containing a quaternary ammonium salt, (5) cytokines, (6) aluminum hydroxide or aluminum phosphate or (7) other adjuvants discussed in any document cited, or (8) any combinations or mixtures thereof.

The oil in water emulsion (3), which is especially appropriate for viral vectors, can be based on: light liquid paraffin oil (European pharmacopoeia type), isoprenoid oil such as squalane, squalene, oil resulting from the oligomerization of alkenes, e.g. isobutene or decene, esters of acids or alcohols having a straight-chain alkyl group, such as vegetable oils, ethyl oleate, propylene glycol, di(caprylate/caprate), glycerol tri(caprylate/caprate) and propylene glycol dioleate, or esters of branched, fatty alcohols or acids, especially isostearic acid esters. The oil is used in combination with emulsifiers to form an emulsion. The emulsifiers may be nonionic surfactants, such as: esters of on the one hand sorbitan, mannide (e.g. anhydromannitol oleate), glycerol, polyglycerol or propylene glycol and on the other hand oleic, isostearic, ricinoleic or hydroxystearic acids, said esters being optionally ethoxylated, or polyoxypropylene-polyoxyethylene copolymer blocks, such as Pluronic, e.g., L121.

Among the type (1) adjuvant polymers, preference is given to polymers of crosslinked acrylic or methacrylic acid, especially crosslinked by polyalkenyl ethers of sugars or polyalcohols. These compounds are known under the name carbomer (Pharmeuropa, vol. 8, no. 2, June 1996). One skilled in the art can also refer to U.S. Patent No. 2,909,462, which provides such acrylic polymers crosslinked by a polyhydroxyl compound having at least three hydroxyl groups, preferably no more than eight such groups, the hydrogen atoms of at least three hydroxyl groups being replaced by unsaturated, aliphatic radicals having at least two carbon atoms. The preferred radicals are those containing 2 to 4 carbon atoms, e.g. vinyls, allyls and other ethylenically unsaturated groups. The unsaturated radicals can also contain other substituents, such as methyl. Products sold under the name Carbopol (BF Goodrich, Ohio, USA) are especially suitable. They are crosslinked by allyl saccharose or by allyl pentaerythritol. Among them, reference is made to Carbopol 974P, 934P and 971P.

As to the maleic anhydride-alkenyl derivative copolymers, preference is given to EMA (Monsanto), which are straight-chain or crosslinked ethylene-maleic anhydride copolymers and they are, for example, crosslinked by divinyl ether. Reference is also made to J. Fields et al., Nature 186: 778-780, June 4, 1960.

With regard to structure, the acrylic or methacrylic acid polymers and EMA are preferably formed by basic units having the following formula: 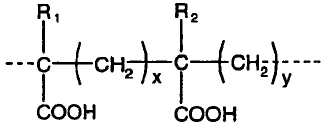 in which:
- R₁ and R₂, which can be the same or different, represent H or CH₃
- x = 0 or 1, preferably x = 1
- y = 1 or 2, with x + y = 2.

For EMA, x = 0 and y = 2 and for carbomers x = y = 1.

These polymers are soluble in water or physiological salt solution (20 g/l NaCl) and the pH can be adjusted to 7.3 to 7.4, e.g., by soda (NaOH), to provide the adjuvant solution in which the expression vector(s) can be incorporated. The polymer concentration in the final vaccine composition can range between 0.01 and 1.5% w/v, advantageously 0.05 to 1% w/v and preferably 0.1 to 0.4% w/v.

The cationic lipids (4) containing a quaternary ammonium salt which are advantageously but not exclusively suitable for plasmids, are preferably those having the following formula: 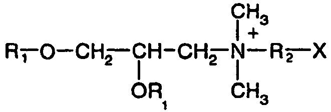 in which R₁ is a saturated or unsaturated straight-chain aliphatic radical having 12 to 18 carbon atoms, R₂ is another aliphatic radical containing 2 or 3 carbon atoms and X is an amine or hydroxyl group.

Among these cationic lipids, preference is given to DMRIE (N-(2-hydroxyethyl)-N,N-dimethyl-2,3-bis(tetradecyloxy)-1-propane ammonium; WO96/34109), preferably associated with a neutral lipid, preferably DOPE (dioleoyl-phosphatidyl-ethanol amine; Behr J. P., 1994, Bioconjugate Chemistry, 5, 382-389), to form DMRIE-DOPE.

Preferably, the plasmid mixture with the adjuvant is formed extemporaneously and preferably contemporaneously with administration of the preparation or shortly before administration of the preparation; for instance, shortly before or prior to administration, the plasmid-adjuvant mixture is formed, advantageously so as to give enough time prior to administration for the mixture to form a complex, e.g. between about 10 and about 60 minutes prior to administration, such as approximately 30 minutes prior to administration.

When DOPE is present, the DMRIE:DOPE molar ratio is preferably about 95: about 5 to about 5:about 95, more preferably about 1: about 1, e.g., 1:1.

The DMRIE or DMRIE-DOPE adjuvant:plasmid weight ratio can be between about 50: about 1 and about 1: about 10, such as about 10: about 1 and about 1:about 5, and preferably about 1: about 1 and about 1: about 2, e.g., 1:1 and 1:2.

The cytokine or cytokines (5) can be in protein form in the immunogenic or vaccine composition, or can be co-expressed in the host with the immunogen or immunogens or epitope(s) thereof. Preference is given to the co-expression of the cytokine or cytokines, either by the same vector as that expressing the immunogen or immunogens or epitope(s) thereof, or by a separate vector therefor.

The description comprehends preparing such combination compositions; for instance by admixing the active components, advantageously together and with an adjuvant, carrier, cytokine, and/or diluent

Cytokines that may be used in the present description include, but are not limited to, granulocyte colony stimulating factor (G-CSF), granulocyte/macrophage colony stimulating factor (GM-CSF), interferon α (IFN α), interferon β (IFN β), interferon γ, (IFN γ), interleukin-1α (IL-1 α), interleukin-1 β (IL-1 β), interleukin-2 (IL-2), interleukin-3 (IL-3), interleukin-4 (IL-4), interleukin-5 (IL-5), interleukin-6 (IL-6), interleukin-7 (IL-7), interleukin-8 (IL-8), interleukin-9 (IL-9), interleukin-10 (IL-10), interleukin-11 (IL-11), interleukin-12 (IL-12), tumor necrosis factor α (TNF α), tumor necrosis factor β (TNF β), and transforming growth factor β (TGF β). It is understood that cytokines can be co-administered and/or sequentially administered with the immunogenic or vaccine composition of the present description. Thus, for instance, a virus propagated in the instant description can contain an exogenous nucleic acid molecule and express in vivo a suitable cytokine, e.g., a cytokine matched to this host to be vaccinated or in which an immunological response is to be elicited (for instance, an avian cytokine for preparations to be administered to birds).

The description provides for methods and compositions for eliciting an immune response to a virus in an animal. A host cell is contacted with a virus under conditions which result in viral infection of the host cell. The culture of infected cells is incubated for a sufficient period of time sufficient to allow for viral replication. The virus is optionally harvested from the culture of infected cells. In one embodiment, the virus is attenuated. In another embodiment, the virus is inactivated. Either the infected cell, harvested virus, or an immunogen, antigen, or epitope thereof, i.e., an immunogenic or vaccine composition, is administered to the animal in an effective amount to elicit an immune response to the virus sufficient to provide an immunological or protective response.

Another aspect of the present description is a method of immunization or a method of vaccination using the immunogenic compositions or the vaccine compositions according to the description, respectively.

The method includes at least one administration to an animal of an efficient amount of the immunogenic composition or vaccine according to the description. The animal may be male, female, pregnant female and newborn. This administration may be notably done by intramuscular (IM), intradermal (ID) or subcutaneous (SC) injection or via intranasal or oral administration. The immunogenic composition or the vaccine according to the description can be administered by a syringe or a needleless apparatus (like for example Pigjet or Biojector (Bioject, Oregon, USA)).

An inactivated vaccine may be prepared as well from the harvested culture fluid. Inactivation may be achieved by treating the viruses by any of the methods commonly employed to make inactivated vaccines. These methods include but are not limited to formaldehyde treatment, betapropriolactone treatment, ethylene-imine treatment, treatment with a plurality of organic solvents, treatment with a plurality of detergents, treatment with gamma radiation or X-rays, or treatment with ultraviolet light. The methods recited herein serve as art-known examples for inactivating virus. Inactivated virus vaccines are usually administered mixed with an adjuvant such as aluminum hydroxide, and an emulsifier such as oil, or a detergent. The inactivated vaccine can be administered to the animal by any of a plurality of methods which include but are not limited to inoculation intramuscularly or subcutaneously, spraying, ocularly, nasally, orally, or in ovo.

For attenuated compositions the doses of the virus or organism or pathogen produced on the new cell culture may be between about 10³ and about 10⁷ CCID₅₀ (median Cell Culture Infectious Doses), advantageously between about 10⁴ and about 10⁶ CCID₅₀ and more advantageously about 10⁵ CCID₅₀. The volumes are from 0.2 to 2.0 ml, advantageously about 2.0 ml. One or more administrations can be done; e.g. with two injections at 2-4 weeks interval, and advantageously with a boost about 3 weeks after the first injection.

With inactivated compositions of the virus or organism or pathogen produced on the new cell culture, the animal may be administered approximately 10⁴-10⁹ equivalent CCID₅₀ (titer before inactivation), advantageously approximately 10⁵-10⁸ equivalent CCID₅₀ in a single dosage unit. The volume of one single dosage unit can be between 0.2 ml and 5.0 ml and advantageously between 0.5 ml and 2.0 ml and more advantageously about 2.0 ml. One or more administrations can be done; e.g. with two injections at 2-4 weeks interval, and advantageously with a boost about 3 weeks after the first injection.

With sub-unit compositions, e.g., from the virus or pathogen or organism produced on the new cell culture, the animal may be administered approximately 5 µg to 500 µg, advantageously 20 µg to 50 µg. The volumes are from 0.2 to 2.0 ml, advantageously about 2.0 ml. One or more administrations can be done; e.g. with two injections 2-4 weeks apart, and advantageously with a boost about 3 weeks after the first injection.

The compositions according to the description may also be administered to other mammals, e.g. mice or laboratory animal, for instance to generate polyclonal antibodies, or to prepare hybridomas for monoclonal antibodies.

The present description provides for the immunization of animals, advantageously avians. Methods for administering IBDV vaccines are described in U.S. Patent Nos. 5,595,912; 5,614,409; 5,632,989; 5,849,575; 6,054,126; 6,451,321 and 6,528,063. A method for administration of the immunogenic or vaccine composition to an avian is described in U.S. Patent No. 6,506,385. Exemplary means of administration are oral administration (e.g., in the feed or drinking water), intramuscular injection, subcutaneous injection, intravenous injection, intra-abdominal injection, eye drop, or nasal spray. Birds may also be administered vaccines in a spray cabinet, i.e., a cabinet in which the birds are placed and exposed to a vapor containing vaccine, or by course spray. When administering the description vaccines to birds post-hatch, administration by subcutaneous injection or spray cabinet is advantageous. Birds may also be administered the vaccine in ovo, as described in U.S. Pat. No. 4,458,630. In ovo administration of vaccine is most advantageous. As a practical matter, it may be desirable to administer compositions including two or more vaccines to the animal at the same time.

The in ovo administration of vaccine, as described hereinabove, involves the administration of the vaccine to the avian embryo while contained in the egg. The vaccine may be administered to any suitable compartment of the egg (e.g., allantois, yolk sac, amnion, air cell, or into the avian embryo itself), as would be apparent to one skilled in the art. Advantageously, the vaccine is administered to the amnion. Eggs administered the vaccines of the present description are fertile eggs which are advantageously in the last half, more advantageously the last quarter, of incubation. Chicken eggs are treated on about the twelfth to twentieth day of incubation, more advantageously the fifteenth to nineteenth day of incubation, and are most advantageously treated on about the eighteenth day of incubation (the eighteenth day of embryonic development). Turkey eggs are advantageously treated on about the fourteenth to twenty-sixth day of incubation, more advantageously on about the twenty-first to twenty-seventh day of incubation, most advantageously on about the twenty-fifth day of incubation. Those skilled in the art will appreciate that the present invention can be carried out at any predetermined time in ovo, as long as the embryo is able to mount an immune response to the virus vaccine.

Eggs may be administered the vaccines by any means which transports the compound through the shell. The advantageous method of administration is, however, by injection. The substance may be placed within an extraembryonic compartment of the egg (e.g., yolk sac, amnion, allantois, air cell) or within the embryo itself. The site of injection is advantageously within the region defined by the amnion, including the amniotic fluid and the embryo itself. By the beginning of the fourth quarter of incubation, the amnion is sufficiently enlarged that penetration thereof is assured nearly all of the time when the injection is made from the center of the large end of the egg along the longitudinal axis.

The mechanism of egg injection is not critical, but it is advantageous that the method not unduly damage the tissues and organs of the embryo or the extraembryonic membranes surrounding it so that the treatment will not decrease hatch rate. A hypodermic syringe fitted with a needle of about 18 to 22 gauge is suitable for the purpose. To inject into the air cell, the needle need only be inserted into the egg by about two millimeters. A one-inch needle, when fully inserted from the center of the large end of the egg, will penetrate the shell, the outer and inner shell membranes enclosing the air cell, and the amnion. Depending on the precise stage of development and position of the embryo, a needle of this length will terminate either in the fluid above the chick or in the chick itself. A pilot hole may be punched or drilled through the shell prior to insertion of the needle to prevent damaging or dulling of the needle. If desired, the egg can be sealed with a substantially bacteria-impermeable sealing material such as wax or the like to prevent subsequent entry of undesirable bacteria.

It is envisioned that a high-speed automated egg injection system for avian embryos will be particularly suitable for practicing the present invention. Numerous such devices are available, exemplary being those disclosed in U.S. Patent Nos. 4,040,388; 4,469,047; 4,593,646; 4,681,063; and 4,903,635. All such devices, as adapted for practicing the present invention, comprise an injector containing the vaccine described herein, with the injector positioned to inject an egg carried by the apparatus with the vaccine. Other features of the apparatus are discussed above. In addition, if desired, a sealing apparatus operatively associated with the injection apparatus may be provided for sealing the hole in the egg after injection thereof.

The invention will now be further described by way of the following non-limiting examples.

### EXAMPLES

### Example 1: Primers For Amplifying VP2 regions of IBDV

B5 5': GGTATGTGAGGCTTGGTGAC (SEQ ID NO: 7)
B5 3': TTATCTCGTTGGTTGGAATC (SEQ ID NO: 8)
B4 5': TCTTGGGTATGTGAGGCTTG (SEQ ID NO: 9)
B4 3': GGATGTGATTGGCTGGGTTA (SEQ ID NO: 10)

FIG. 1 shows a phylogenetic tree of nucleic acid sequences aligned using Clustal method with Weighted residue weight table. Sequences were determined by either the University of Georgia Molecular Genetics Instrumentation Facility (Athens, Georgia) or SeqWright DNA Technologies Services (Houston, Texas). The MegAlign program (DNASTAR, Inc., 1228 S. Park St., Madison, WI 53715) was used to align and make tree and SeqEdit (DNASTAR, Inc., 1228 S. Park St., Madison, WI 53715) was the program used to convert the sequences to amino acids. It is apparent to one of skill in the art that the IBDV sequences of FIG. 1 encompass the majority sequence as well as the tree sequences with the nucleotide substitutions indicated therein.

### Example 2: Pathology of IBDV

Tissue collection-Sample Selection. Sick or acute birds were selected for specific etiologies. Routine healthy birds were selected for routine monitoring. Broilers were 14, 21, 28 or 35 days of age. Pullets are 21, 28, 35, 48 or 60 days of age. Sentinel birds were collected 3-5 days after placing. For mycotoxin documentation, birds were placed on suspected feeds and killed sequentially 2-3 days after first exposure.

The samples collected were immune organs, such as bursa, thymus, spleen and marrow. For sample preservation, the thickness was limited to 0.5 cm. The bone was split to expose the marrow. The sample was fixed in 10% neutral buffered formalin for 24 hours. After 24 hours, the sample was stored in H₂O, PBS and alcohol.

Sample transportation. The samples were stable at room temperature after fixation. The samples were protected from freezing and not packaged with frozen serum or tissues. The shipping weight was reduced by pouring off the formalin, which reduced the spillage of formalin in the mail. The sample was kept moist in an airtight containiner.

Sample preparation. Tissues were handled as follows after receipt. First, the tissues were processed for routine histopathology through alcohols, clearings and paraffin. The tissues were sectioned at 5 microns for hematoxylin and eosin (HE) staining. Routine processing was usually completed in 24 hours. Nucleic acid analysis was investigated using routine sections for nucleic acid extraction for pathogen identification.

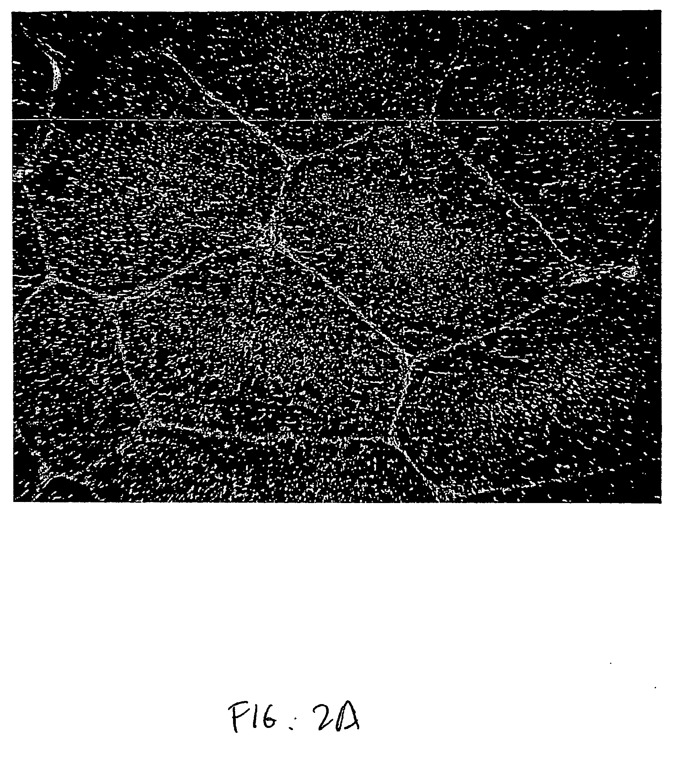

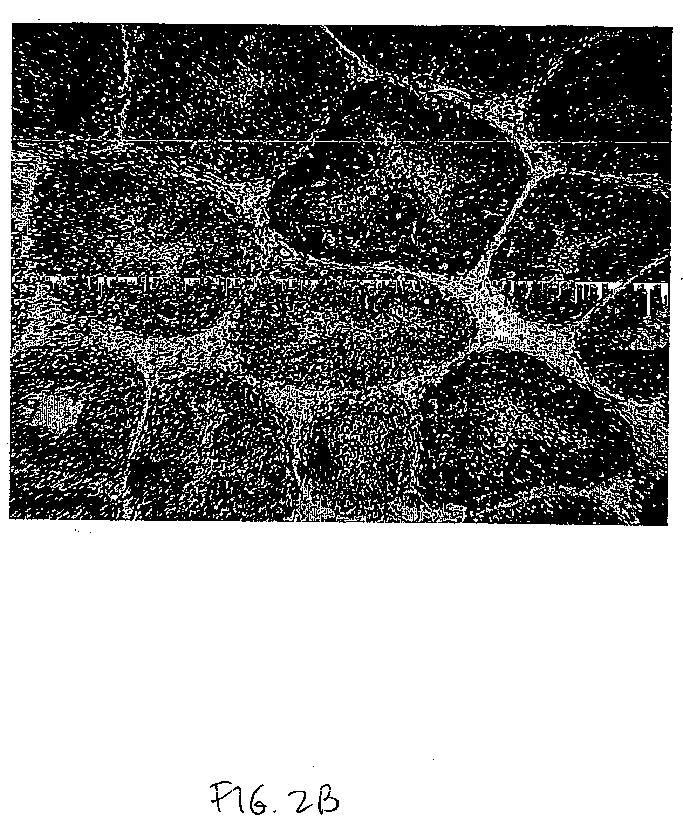

Infectious bursal disease (IBD). The first effect (1-3 days post infection) was lymphocyte lysis in bursa of Fabricus (BF) with no significant lesions in other organs. The second effect (3-5 days post infection) was continued lymphoid depletion in variant strains and acute fibrinoid necrosis in classical strains. The third effect (5-8 days post infection) was diffuse lymphoid depletion which affects all follicles uniformly. The fourth effect (10 days post infection) was the regeneration of some follicles, which if not present by 10 days will usually not occur. Immune system suppression was transient if regeneration occurs and permanent if no regeneration occurs. A histological section (formalin-fixed) of normal bursa is presented in FIG. 2A and a section of acute bursal necrosis is presented in FIG. 2B.

IBD interpretation. Acute damage to the bursa showed that protection is inadequate. Chronic atrophy means that there was impaired B cell development and release. Variation suggests maternal immunity was suspect. Vaccine strains were capable of producing severe lesions and immune suppression. The production of lesions meant either the vaccine strain or a field isolate penetrated the existed antibody and damaged the immune system.

### Example 3: Characterization of IBDV by RT-PCR

Introduction. IBDVs belong to the Birnaviridae family and the Virnavirus genus. IBDVs are icosahedral, nonenveloped, and have no surface projections. The virion is 60 nm in diameter with a 45 nm in transmission electron microscopy (TEM). IBDV is an RNA, double stranded bisegmented virus. The major external capsid protein is VP2, which is glycosylated and contains major neutralizing epitopes.

Birnaviral infections include infectious pancreatic necrosis virus which infects fish, skin tumor virus which infects eel, gill lamellar pillar cell necrosis virus which infects eel, marine birnavirus which infections oysters and fish, and IBDV in which serotype 1 infects chickens and serotype 2 infects turkeys.

IBDV is typed by antigenic subtypes, pathotypes and molecular groups by restriction enzyme fragment length polymorphism (RFLP). Antigenic subtypes include Serotype 1 (including Classic and Variants A and E) and Serotype 2. Pathotypes are apathogenic, mild, intermediate, intermediate plus, classical, variant and very virulent. Molecular groups by RFLP have been classified in six groups and were identified by extracting RNA from either a fresh sample or a sample stored in phenol/chloroform, RT-PCR of the RNA generating a cDNA, enzyme restriction of the cDNA, gel electrophoresis of the restricted cDNA, comparing RFLP profiles, and diagnosis of the molecular group.

Issues impacting current RT-PCR molecular typing of IBDVs. The current method of typing IBDVs into molecular groups by RFLP does not allow correlation of lesions with viral identity in the same sample. The correlation requires dual sampling by histopath and RT-PCR. A second problem is that samples are difficult to ship internationally. A third problem is that retrospective examination is difficult for epidemiological analysis. A fourth problem is that RT-PCR samples are not stable over time and require specialized reagents not commonly available.

The current molecular grouping system is designed for categorization only. The molecular group identity cannot be used for prospective design of vaccination strategies. The molecular group identity also does not allow identification of newly emergent agents without additional use of classical isolation techniques.

Early IBDV detection in paraffin-embedded tissues. RT-PCR of IBDV in routine tissues in routine paraffin-embedded blocks would allow (1) correlation of damage with virus, (2) stable blocks for transport, (3) killed agents, therefore fewer import restrictions, (4) retrospective viral analysis and (5) formalin-inactivated RNases. An early studied trial to detect IBDV in formalin-fixed, paraffin-embedded tissues was carried out by histopathology, immunohistochemistry (IHC) and RT-PCR.

Field cases of IBDV included bursas and proventriculi that were collected in 10% buffered formalin, fixed for 24 hours and processed routinely into paraffin blocks for histopathology. Histopathology, HIC and RT-PCR were performed on all field cases.

For experimental IBDV exposure, antibody free SPF broilers (n=32) were hatched and raised in isolators to 28 days of age and exposed orally to IBDV strains (10³ tissue culture infective dose₅₀ (TCID₅₀)). IBDV strains included USDA standard challenge strain of IBDV (STC), Lukert, Bursine 2, D78, Variant E, Variant A and an IBDV strain patented by Intervet (GLS) from proventriculitis. The animals were necropsied at 4 or 6 days post-exposure. Bursa, proventriculus and thymus were collected, fixed (10% neutral buffered formalin (NBF) for 24 hours) and processed for histopathology.

Histopathology for IBDV Lesions. Tissue samples bursa, thymus and proventriculus were examined and lesions were scored as 1=Normal, 2=Mild, 3=Moderate and 4=Severe.

Immunohistochemistry for IBDV. IHC was run in an automated immunostainer. The primary antibody was a mouse antibody reactive to all IBDVs. The secondary antibody was an antimouse antibody conjugated with nonbiotin peroxidase (Dako, Envision). The staining of IBDV antigen in tissue sections was scored as 1=None, 2=Mild, 3=Moderate and 4=Diffuse.

RT-PCR for IBDV. RNA was isolated from paraffin-embedded tissues as follows. Tissue (30 microns) was cut from blocks. Deparaffinization was with HemoDe and 100% ethanol. Digestion was with 10% Proteinase K for 1 hour at 50 C. RNA was extracted with Trizol® (Gibco BRL).

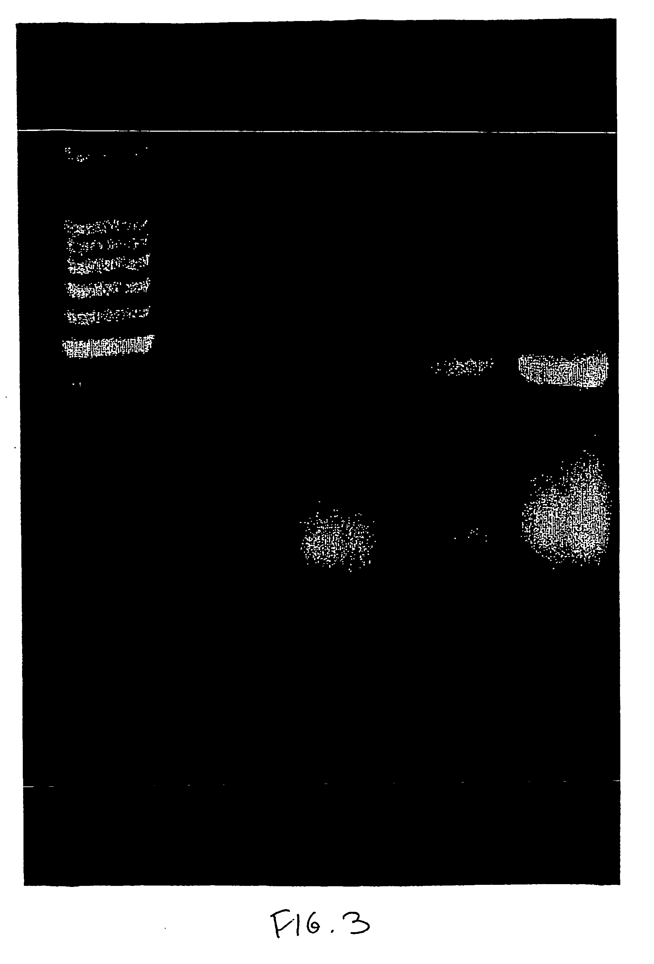

RNA extraction. Formalin-fixed tissue samples preserved in paraffin were deparaffinized with HemoDe and digested with Proteinase K. RNA was extracted using Triazol® (Gibco BRL). The sample RNA was diluted in 90% DMSO. The RNA was denatured for 5 minutes at 95 C and put on ice before RT-PCR. FIG. 3 shows an agarose gel of RT-PCR results showing an amplified segment shared by IBDVs.

All methods used successfully detected IBDV in formalin fixed paraffin-embedded tissues where expected. For bursal sections, histopathology demonstrated acute/subacute necrosis, immunohistochemistry revealed staining for IBDV antigen present in all sections, and RT-PCR was IBDV positive for all samples. For thymus sections, histopathology demonstrated no lesions, immunohistochemistry revealed some IBDV staining, and RT-PCR was IBDV positive for most samples. For proventricular sections, histopathology demonstrated no or low lesion scores, immunohistochemistry revealed no IBDV staining, and RT-PCR had a weak IBDV positive in some samples.

Can real time RT-PCR and RFLP be adapted for IBDV? RNA is extracted from a paraffin-embedded tissue sample, subjected to real time RT-PCR and enzyme restriction, followed by melting curve analysis, a comparison of patterns, and diagnosis of molecular groups.

Real-time RT-PCR. Real-time RT-PCR is ultra-rapid cycling with cycle-by-cycle monitoring. A DNA binding dye was used in the PCR mix. In this instance, SYBR Green dye was used for monitoring PCR. SYBR Green I fluoresces when bound to dsDNA.

Melting curve analysis. PCR products were differentiated by analysis of melting curves whose shape is a function of GC content, length and sequence.

RT-PCR. SYBR Green I and LightCycler Instrument (Roche Diagnostics) were used for RT-PCR. The conditions were 10 minutes of RT followed by 45 PCR cycles in 20 minutes. Primers that resulted in the amplification of a 400 bp fragment in the VP2 region of IBDV were used.

Real time analysis with RT-PCR. Using RNA extracts from paraffin blocks, RNA was denatured at 95 C for 5 minutes and RT-PCR was done using LightCycler-RNA Amplification SYBR Green I Kit and LightCycler (Roche). Primers were designed to amplify a 400 bp segment shared by all IBDVs. This segment has a slight variation by strain. Amplification and detection of specific products was based on fluorescence of DNA binding dye SYBR Green (Roche). The products were melted at 55 C to 95 C in 10 minutes. Strains were identified by DNA melting curve analysis.

RFLP of RT-PCR products. Restriction enzymes StyI, SacI and NarI were used. In separate capilolaries, 1 µl of PCR product was cut with each enzyme in a 10 µl reaction for one hour at 37 C. 2 µl of SYBR Green dye was added to each tube. A melting curve analysis was done on the restricted products and melting peaks were compared.

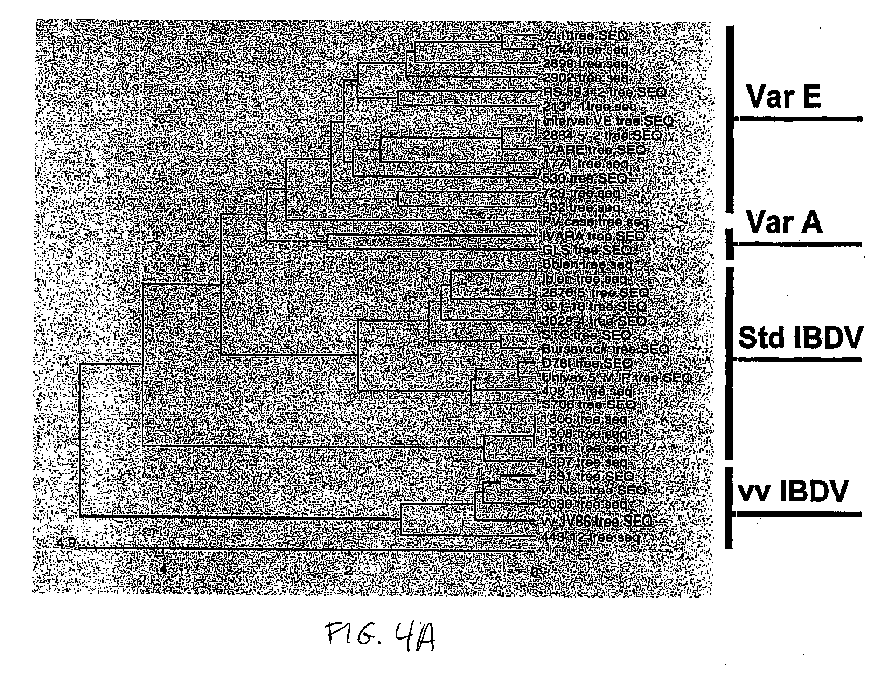

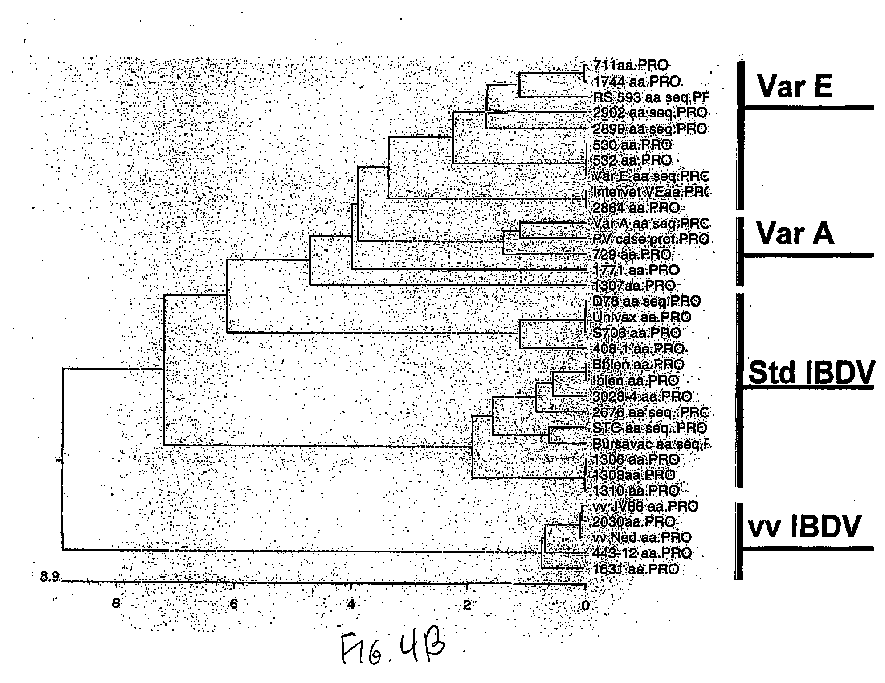

Can real time RT-PCR products be sequenced to develop an IBDV alignment library? Molecular grouping by RFLP has little correlation with antigenic serotypes. Sequence comparisons may yield more relevant information. A logical place to start is sequence for hypervariable VP2. FIG. 4A shows sequence variations in an IBDV VP2 amplicon of nucleic acid sequences. FIG 4B shows sequence variations in IBDV VP2 deduced amino acid sequences.

Nucleotides 36, 68, 74, 96, 98, 107, 138, 146, 149, 218, 230, 236, 252 and 268 were cited as "unique" to very virulent IBDVS (vvIBDV). Amino acids 12, 32, 46, 84 and 89 were cited as "unique' to vvIBDVs. A comparison of two IBDVs 2030 (vvIBDV) versus 1307 (standard) is shown in Table 1.

**Table 1: Comparison of IBDV 2030 (vvIBDV) versus 1307 (standard)**

| Nucleotides: | 36 | 68 | 74 | 96 | 98 | 107 | 138 | 146 | 149 | 218 | 230 | 236 | 252 | 268 |
|---|---|---|---|---|---|---|---|---|---|---|---|---|---|---|
| #2030 | + | + | + | + | + | + | + | + | + | + | + | + | + | + |
| #1307 | - | - | + | - | + | - | - | - | - | - | - | - | + | - |

| Amino acids: | 12 | 32 | 46 | 84 | 89 | | | | | | | | | |
|---|---|---|---|---|---|---|---|---|---|---|---|---|---|---|
| #2030 | + | + | + | + | + | | | | | | | | | |
| #1307 | - | - | - | - | + | | | | | | | | | |

Conclusion on identification of Very Virulent IBDVs (vvIBDV). At the present time, one nucleotide or amino acid marker cannot be depended on to differentiate vvIBDVs from other strains. Proteomic identification of the pathogenic mechanisms for the increased virulence of vvIBDV will allow determination of the requirements for this increased virulence.

In understanding IBDV, a sequence library may be used for purposes other than vaccine matching. Specifically, isolates may vary in their abilities to induce apoptotic and necrotic cell death.

IBDV induced apoptosis. In addition to causing necrosis in the lymphoid cells of the bursa, IBDV induces apoptosis (see, e.g., Vasconcelos & Lam, J Gen Virol. 1994 Jul;75 (Pt 7):1803-6, Tham & Moon, Avian Dis. 1996 Jan-Mar;40(1):109-13, Fernandez-Arias et al., J Virol. 1997 Oct;71(10):8014-8, Ojeda et al., Avian Dis. 1997 Apr-Jun;41(2):312-6 and Tanimura & Sharma, J Comp Pathol. 1998 Jan;118(1):15-27).

Apoptosis in infected cells may contribute to the pathogenesis of IBDV (see, e.g., Jungmann et al., J Gen Virol. 2001 May;82(Pt 5):1107-15 and Ojeda et al., Avian Dis. 1997 Apr-Jun;41(2):312-6). The induction of apoptosis has been reported in IBDV-infected chicken peripheral blood lymphocytes (see, e.g., Vasconcelos & Lam, J Gen Virol. 1994 Jul;75 (Pt 7):1803-6) and in the thymus (see, e.g., Inoue et al., Avian Dis. 1994 Oct-Dec;38(4):839-46 and Tanimura & Sharma, J Comp Pathol. 1998 Jan;118(1):15-27).

IBDV-induced apoptosis occurs in the proventriculus of IBDV challenged SPF leghorn chickens. IBDV induced apoptosis was studied using a modified terminal deoxynucleotidyl transferase-mediated dUTP nick end labeling (TUNEL) method on sections of bursa, thymus and proventriculus of IBDV infected birds.

Materials and Methods. Formalin-fixed paraffin-embedded tissue samples from birds from the IBDV studies described above were used for the terminal deoxynucleotidyl transferase-mediated dUTP nick end labeling (TUNEL) assay. For the detection of apoptotic cells, the *in situ* cell death detection kit (DeadEnd Colorimetric TUNEL System, Promega Corp., Madison WI) was used according to the manufacturer's instructions.

Results. Bursas from birds challenged with IBDV had intense apoptosis staining in both follicular cortex and medulla. Apoptosis present in follicles that were IHC antigen positive. Distribution within positive follicles of apoptosis and IHC was the same within a strain.

Apoptosis was observed in some samples with proventriculitis, mostly in infiltrating lymphocytes. The results suggested IBDV induced systemic lymphocyte apoptotic lysis in addition to that in primary lymphoid organs.

Are B cells the sole target cells for all isolates of IBDV or is there T cell lysis and diversity? IBDV-induced lymphoid infiltrates and target cell necrosis *in situ* by immunohistochemistry was characterized. Monoclonals with IHC for T cells were CD3, CD4 and CD8 and for B lymphocytes, HIS-C1. Multiplex labeling of T lymphocytes using IHC for IBDV, apoptotic cell lysis, and confirmation of strain identity using real-time PCR will answer the question.

Conclusions. Melting curve analysis is a simple way of differentiating strains into molecular groups and eliminates the need to run gels. Sequence data can be obtained quickly from the real time RT-PCR products without further clean-up or gel purification. VP2 hypervariable region sequence data generated using these techniques is a manageable data set that will allow timely and targeted vaccine applications and identification of the sequence of emergent variants for future biologics use.

To date, 61 cases of IBD have been assayed, 34 cases with a positive RT-PCR, 33 cases have been sequenced, and 27 had a negative RT-PCR. To date, 66 cases of REO have been assayed, 20 with a positive RT-PCR, 20 cases have been sequenced, and 46 had a negative RT-PCR.

### Example 4: Novel IBDV Strains Identified Using Viral Genomic Identification System

New Sequences identified using new VGIS (Viral Genomic Identification System). 1631 is a new vvIBDV-like strain, 087 is a new IBDV Variant strain, and 077 is a new previously unidentified IBDV strain.

FIG. 4C shows sequence variations of new sequences identified using new VGIS (Viral Genomic Identification System). Nucleotide sequences 1631 276 is a new vvIBDV-like strain, 087 276 is a new IBDV Variant strain, and 077 276 is a new previously unidentified IBDV strain. Amino acid sequences 1631 91 is a new vvIBDV-like strain, 087 91 is a new IBDV Variant strain, and 077 91 is a new previously unidentified IBDV strain. Genebank posted sequences for comparison: #AY321527 is a vvIBDV sequence, #Y14955 is another vvIBDV sequence, #Z25482 is a third vvIBDV sequence, #D00499 is an IBDV USDA Standard Challenge Strain (STC), #X54858 is an IBDV Variant E strain, #M64285 is an IBDV Variant A strain.
Sequence Number 1631 - New vvIBDV-like strain nucleic acid sequence:
Sequence Number 1631 - New vvIBDV-like strain translated amino acid sequence:
Sequence Number 087 - New IBDV Variant strain nucleic acid sequence:
Sequence Number 087 - New IBDV Variant strain translated amino acid sequence:
Sequence Number 077 - New Previously Unidentified strain nucleic acid sequence:
Sequence Number 077 - New Previously Unidentified strain translated amino acid sequence: Genebank posted sequences for comparison: #AY321527 is a vvIBDV sequence, #Y14955 is another vvIBDV sequence, #Z25482 is a third vvIBDV sequence, #D00499 is an IBDV USDA Standard Challenge Strain (STC), #X54858 is an IBDV Variant E strain, #M64285 is an IBDV Variant A strain.
AY321527 vvIBDV strain of IBDV 1-271 Classical. Gene Bank Accession Number: AY321527, DEFINTION: Infectious bursal disease virus isolate VV/2003 VP2 gene, partial cds., REFERENCE: bases 1 to 271.
AY321527 vvIBDV strain of IBDV 1-271 Classical nucleic acid sequence:
AY321527 vvIBDV strain of IBDV 1-271 Classical Translated amino acid sequence:
Y14955 vvJV86 276 83-309 Classic. Gene Bank Accession Number: Y14955, DEFINITION: Infectious bursal disease virus partial VP2 gene for structural protein VP2, genomic RNA, strain 94432, REFERENCE: bases 83 to 309.
Y14955 vvJV86 276 83-309 Classic nucleic acid sequence:
Y14955 vvJV86 276 83-309 Classic translated amino acid sequence:
Z25482 vv NED 276 59-335 Classic. Gene Bank Accession Number: Z25482, DEFINITION: Infectious bursal disease virus VP2 protein, partial CDS, REFERENCE: bases 59 to 335.
Z25482 vv NED 276 59-335 Classic nucleic acid sequence:
Z25482 vv NED 276 59-335 Classic translated amino acid sequence:
D00499 STC strain of IBDV 725-1001 Classic. Gene Bank Accession Number: D00499, DEFINITION: Infectious bursal disease virus genomic RNA, segment A containing large ORF and small ORF, complete cds., REFERENCE:Bases 725 to 1001.
D00499 STC strain of IBDV 725-1001 Classic nucleic acid sequence:
D00499 STC strain of IBDV 725-1001 Classic translated amino acid sequence:
X54858 Variant E strain of IBDV 680-956 Variant. Gene Bank Accession Number: X54858. DEFINITION: Avian infectious bursal disease virus RNA for VP2 and (partial)VP4 proteins. REFERENCE: bases 680 to 956.
X54858 Variant E strain of IBDV 680-956 Variant nucleic acid sequence:
M64285 Variant A strain of IBDV 688-964 Variant. Gene Bank Accession Number M64285, DEFINITION: Infectious bursal disease virus polyprotein (encoding VP2 and VP4) mRNA, 5'end, REFERENCE: Bases 688 to 964.
M64285 Variant A strain of IBDV 688-964 Variant nucleic acid sequence:

### Example 5: Flowchart

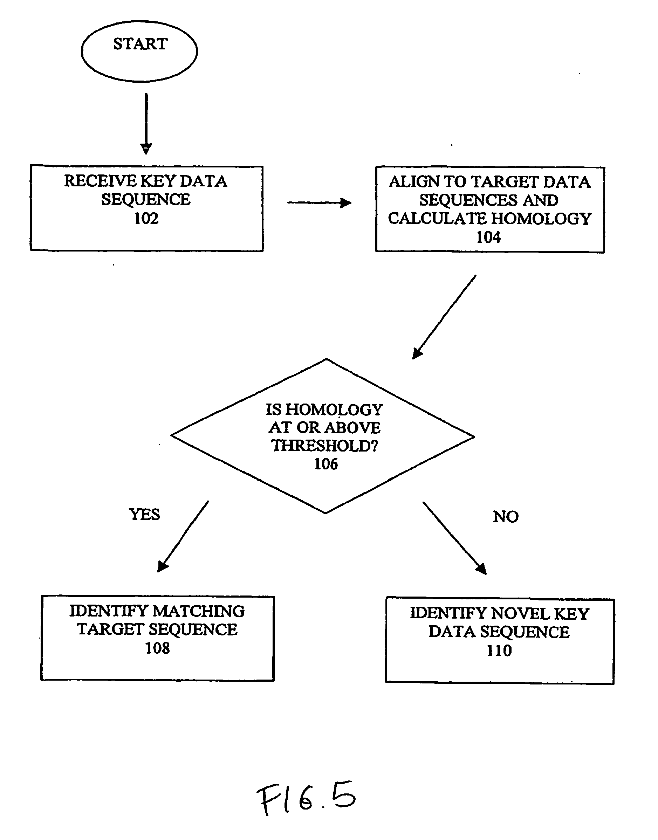

FIG. 5 shows a flowchart illustrating the general overview of input, an intermediate, step, and output. FIG. 5 demonstrates one method of retrieving a target data sequence in response to a key data sequence or the identification of a new sequence in the absence of a match. This embodiment of the description may be configured to handle data sequences of one type (e.g., nucleotide sequences) or multiple types (e.g., nucleic acids and/or amino acids), and the key and target data sequences may be of any lengths.

In state 102, a key data sequence is received. The key data may be in any suitable notation (e.g., nucleotide or amino acid) or may be converted to a suitable notation (e.g., an amino acid can be converted to a nucleotide triplet wherein the nucleotide at the third position is unspecified or a nucleotide sequence converted into an amino acid sequence).

In state 104 the key data sequence is aligned to a database of target data sequences, e.g., tree data comprising a plurality of nucleotide or amino acid sequences. A percentage homology or identity is calculated with alignment programs, such as but not limited to, ALIGN, FASTA, MegAlign, NCBI-BLAST (e.g., BLAST, gapped BLAST, BLASTN, BLASTP, and PSI-BLAST), and WU-BLAST (Washington University BLAST) for the key data sequence with each putative target data sequence.

In state 106 the percent homology or identity is subject to a threshold determination. If the percent homology or identity is greater than about 95%, advantageously about 98% to about 99.8%, most advantageously about 99.3% to about 99.6%, then a suitable match is identified in state 108. Otherwise, if the percent homology or identity is less than the threshold, then a new sequence is identified in state 110.

### Example 6: IBDV sequences

| **Accession No.** | **Description** |
|---|---|
| A12620 | IBDV RNA segment |
| A28793 | p501 DNA fragment from patent WO9015140 |
| A28794 | p502 DNA fragment from patent WO9015140 |
| A28795 | p601 DNA fragment from patent WO9015140 |
| A28796 | p611 DNA fragment from patent WO9015140 |
| A28797 | primer DNA N527 from patent WO9015140 |
| A28798 | primer DNA N528 from patent WO9015140 |
| A28799 | primer DNA N531 from patent WO9015140 |
| A28800 | primer DNA N526 from patent WO9015140 |
| A28801 | primer DNA N533 from patent WO9015140 |
| A28803 | pIP41 (002-73 IBVD) DNA from patent WO9015140 |
| A28805 | pIPZ01 (E IBVD) DNA from patent WO9015140 |
| A33255 | IBDV Edgar strain segment A |
| A33259 | Synthetic IBDV Edgar strain segment A primer 0 |
| A33260 | Synthetic IBDV Edgar strain segment A primer 1 |
| A33261 | Synthetic IBDV Edgar strain segment A primer 1b |
| A33262 | Synthetic IBV Edgar strain segment A primer 2 |
| A33263 | Synthetic IBDV Edgar strain segment A primer 3 |
| A33264 | Synthetic IBDV Edgar strain segment A primer 4 |
| A33265 | Synthetic IBDV Edgar strain segment A primer 5 |
| A33266 | Synthetic IB DV Edgar strain segment A primer 6 |
| A38328 | Sequence 1 from Patent WO9410321 |
| AB024076 | Infectious bursal disease virus mRNA for VP2-4-3 polyprotein, partial cds |
| AF006694 | Infectious bursal disease virus segment A structural protein (VP2) mRNA, partial cds |
| AF006695 | Infectious bursal disease virus segment A structural protein (VP2) mRNA, partial cds |
| AF006696 | Infectious bursal disease virus segment A structural protein (VP2) mRNA, partial cds |
| AF006697 | Infectious bursal disease virus segment A structural protein (VP2) mRNA, partial cds |
| AF06698 | Infectious bursal disease virus segment A structural protein (VP2) mRNA, partial cds |
| AF06699 | Infectious bursal disease virus segment A structural protein (VP2) mRNA, partial cds |
| AF006700 | Infectious bursal disease virus segment A structural protein (VP2) mRNA, partial cds |
| AF006701 | Infectious bursal disease virus segment A structural protein (VP2) mRNA, partial cds |
| AF051837 | Infectious bursal disease virus strain GZ29112 structural polyprotein VP2, VP4, VP3 gene, complete cds |
| AF051838 | Infectious bursal disease virus strain HK46 structural polyprotein VP2, VP4, VP3 gene, partial cds |
| AF051839 | Infectious bursal disease virus strain HKL6 structural polyprotein VP2 precusor gene, partial cds |
| AF069577 | Infectious bursal disease virus strain V877 polyprotein VP2 hypervariable region mRNA, partial cds |
| AF069578 | Infectious bursal disease virus strain V877/K polyprotein VP2 hypervariable region mRNA, partial cds |
| AF069579 | Infectious bursal disease virus strain GT 101 polyprotein VP2 hypervariable region mRNA, partial cds |
| AF076223 | Infectious bursal disease virus isolate C4-2 VP2 protein gene, partial cds |
| AF076224 | Infectious bursal disease virus isolate AH-2 VP2 protein gene, partial cds |
| AF076225 | Infectious bursal disease virus isolate B2/28 VP2 protein gene, partial cds |
| AF076226 | Infectious bursal disease virus isolate HD96 VP2 protein gene, partial cds |
| AF076227 | Infectious bursal disease virus isolate JS-18 VP2 protein gene, partial cds |
| AF076228 | Infectious bursal disease virus isolate D11-2 VP2 protein gene, partial cds |
| AF076229 | Infectious bursal disease virus isolate HN3 VP2 protein gene, partial cds |
| AF076230 | Infectious bursal disease virus isolate TS VP2 protein gene, partial cds |
| AF076231 | Infectious bursal disease virus isolate BJ-1 VP2 protein gene, partial cds |
| AF076232 | Infectious bursal disease virus isolate Ark VP2 protein gene, partial cds |
| AF076233 | Infectious bursal disease virus isolate Ga VP2 protein gene, partial cds |
| AF076234 | Infectious bursal disease virus isolate Miss VP2 protein gene, partial cds |
| AF076235 | Infectious bursal disease virus isolate BV3 VP2 protein gene, partial cds |
| AF076236 | Infectious bursal disease virus isolate Univax VP2 protein gene, partial cds |
| AF083092 | Infectious bursal disease virus segment B strain Winterfield-2512 VP1 gene, complete cds |
| AF083093 | Infectious bursal disease virus segment B strain IL3 VP1 gene, complete cds |
| AF083094 | Infectious bursal disease virus segment B strain IL4 VP1 gene, complete cds |
| AF091097 | Infectious bursal disease virus isolate 3212 viral protein 2 (VP2) mRNA, partial cds |
| AF091098 | Infectious bursal disease virus isolate Miss viral protein 2 (VP2) mRNA, partial cds |
| AF0791099 | Infectious bursal disease virus isolate U28 viral protein 2 (VP2) mRNA, partial cds |
| AF092171 | Infectious bursal disease virus VP5 and polyprotein genes, complete cds |
| AF092943 | Infectious bursal disease virus VP5 (VP5) and structural polyprotein (VP2-3) genes, complete cds |
| AF092944 | Infectious bursal disease virus RNA-dependent RNA polymerase (VP1) gene, complete cds |
| AF109154 | Infectious bursal disease virus structural polyprotein gene, complete cds |
| AF121256 | Infectious bursal disease virus segment A major capsid protein VP2 (VP2) gene, partial cds |
| AF133904 | Infectious bursal disease virus strain variant E segment A polyprotein VP0 (vp0) and vp5 genes, complete cds |
| AF133905 | Infectious bursal disease virus strain variant E segment B double-stranded RNA-dependent RNA polymerase VP1 (vp1) gene, complete cds |
| AF140705 | Infectious bursal disease virus segment A polyprotein gene, partial cds |
| AF148073 | Infectious bursal disease virus isolate 002/73 segment A VP2 protein mRNA, partial cd |
| AF148074 | Infectious bursal disease virus isolate V877/K segment A VP2 protein mRNA, partial cds |
| AF148075 | Infectious bursal disease virus isolate Bursavac live segment A VP2 protein mRNA, partial cds |
| AF148076 | Infectious bursal disease virus isolate 01/94 segment A VP2 protein mRNA, partial cds |
| AF148077 | Infectious bursal disease virus isolate 02/95 segment A VP2 protein mRNA, partial cds |
| AF148078 | Infectious bursal disease virus isolate 03/95 segment A VP2 protein mRNA, partial cds |
| AF148079 | Infectious bursal disease virus isolate 04/95 segment A VP2 protein mRNA, partial cds |
| AF148080 | Infectious bursal disease virus isolate 06/95 VP2 protein gene, partial cds |
| AF148081 | Infectious bursal disease virus isolate 08/95 VP2 protein gene, partial cds |
| AF155123 | Infectious bursal disease virus XJ-9 RNA A polyprotein gene, partial cds |
| AF159207 | Infectious bursal disease virus N4 segment A VP2 gene, partial cds |
| AF159208 | Infectious bursal disease virus N6 segment A VP2 gene, partial cds |
| AF159209 | Infectious bursal disease virus N7 segment A VP2 gene, partial cds |
| AF159210 | Infectious bursal disease virus N8 segment A VP2 gene, partial cds |
| AF159211 | Infectious bursal disease virus N9 segment A VP2 gene, partial cds |
| AF159212 | Infectious bursal disease virus N10 segment A VP2 gene, partial cds |
| AF159213 | Infectious bursal disease virus N11 segment A VP2 gene, partial cds |
| AF159214 | Infectious bursal disease virus N13 segment A VP2 gene, partial cds |
| AF159215 | Infectious bursal disease virus N14 segment A VP2 gene, partial cds |
| AF159216 | Infectious bursal disease virus K357/88 segment A VP2 gene, partial cds |
| AF159217 | Infectious bursal disease virus K280/89 segment A VP2 gene, partial cds |
| AF159218 | Infectious bursal disease virus K406/89 segment A VP2 gene, partial cds |
| AF159219 | Infectious bursal disease virus Cu-1 wt segment A VP2 gene, partial cds |
| AF165149 | Infectious bursal disease virus segment A strain K310 VP5 and polyprotein genes, complete cds |
| AF165150 | Infectious bursal disease virus segment A strain KK1 VP5 and polyprotein genes, complete cds |
| AF165151 | Infectious bursal disease virus segment A strain KSH VP5 and polyprotein genes, complete cds |
| AF194428 | Infectious bursal disease virus isolate CEF94 VP5 and polyprotein mRNAs, complete cds |
| AF194429 | Infectious bursal disease virus isolate CEF94 VP1 mRNA, complete cds |
| AF203880 | Infectious bursal disease virus strain Ts segment B VP1 gene, complete cds |
| AF240686 | Infectious bursal disease virus segment A viral protein 5 and polyprotein mRNA, complete cds |
| AF240687 | Infectious bursal disease virus segment B viral protein 1 mRNA, complete cds |
| AF247006 | Infectious bursal disease virus segment A VP5 and polyprotein genes, complete cds |
| AF248612 | Infectious bursal disease virus /UPM94/273 segment A VP2 protein (VP2) gene, partial cds |
| AF260317 | Infectious bursal disease virus BLRI94/B551 segment A polyprotein gene, partial cds |
| AF262030 | Infectious bursal disease virus UPM92-04 segment A VP2 protein (VP2) gene, partial sequence |
| AF279287 | Infectious bursal disease virus strain V97/TW segment A polyprotein VP2 mRNA, partial cds |
| AF279288 | Infectious bursal disease virus strain 2512 segment A polyprotein VP2 mRNA, partial cds |
| AF279691 | Infectious bursal disease virus isolate Ca586-BR segment A VP2 protein gene, partial cds |
| AF281220 | Infectious bursal disease virus isolate 1174 VP2 protein gene, partial cds |
| AF281221 | Infectious bursal disease virus isolate 1568 VP2 protein gene, partial cds |
| AF281222 | Infectious bursal disease virus isolate 1610 VP2 protein gene, partial cds |
| AF281223 | Infectious bursal disease virus isolate 43 VP2 protein gene, partial cds |
| AF281224 | Infectious bursal disease virus isolate 404 VP2 protein gene, partial cds |
| AF281225 | Infectious bursal disease virus isolate 405 VP2 protein gene, partial cds |
| AF281226 | Infectious bursal disease virus isolate E3 VP2 protein gene, partial cds |
| AF281227 | Infectious bursal disease virus isolate E6 VP2 protein gene, partial cds |
| AF281228 | Infectious bursal disease virus isolate GER VP2 protein gene, partial cds |
| AF281229 | Infectious bursal disease virus isolate Int20 VP2 protein gene, partial cds |
| AF281230 | Infectious bursal disease virus isolate RS593 VP2 protein gene, partial cds |
| AF281231 | Infectious bursal disease virus isolate BursinePlus viral protein 2 variable region (VP2) gene, partial cds |
| AF281232 | Infectious bursal disease virus isolate Bursine2 viral protein 2 variable region (VP2) gene, partial cds |
| AF281233 | Infectious bursal disease virus isolate F3 viral protein 2 variable region (VP2) gene, partial cds |
| AF281234 | Infectious bursal disease virus isolate H2 viral protein 2 variable region (VP2) gene, partial cds |
| AF281235 | Infectious bursal disease virus isolate V1 viral protein 2 variable region (VP2) gene, partial cds |
| AF281236 | Infectious bursal disease virus isolate R1 viral protein 2 variable region (VP2) gene, partial cds |
| AF281237 | Infectious bursal disease virus isolate S 1 viral protein 2 variable region (VP2) gene, partial cds |
| AF281238 | Infectious bursal disease virus isolate T1 viral protein 2 variable region (VP2) gene, partial cds |
| AF281239 | Infectious bursal disease virus isolate U1 viral protein 2 variable region (VP2) gene, partial cds |
| AF281240 | Infectious bursal disease virus isolate Q2 viral protein 2 variable region (VP2) gene, partial cds |
| AF281311 | Infectious bursal disease virus isolate NZ2103/97 viral protein 2 gene, partial cds |
| AF281312 | Infectious bursal disease virus isolate NZ1105/98 viral protein 2 gene, partial cds |
| AF281651 | Infectious bursal disease virus segment A polyprotein gene, partial cds |
| AF293774 | Infectious bursal disease virus segment A isolate G81 VP2 (VP2) mRNA, partial cds |
| AF293775 | Infectious bursal disease virus segment A isolate G07 VP2 (VP2) mRNA, partial cds |
| AF293776 | Infectious bursal disease virus segment A isolate G11 VP2 (VP2) mRNA, partial cds |
| AF293777 | Infectious bursal disease virus segment A isolate G16 VP2 (VP2) mRNA, partial cds |
| AF293778 | Infectious bursal disease virus segment A isolate G2369 VP2 (VP2) mRNA, partial cds |
| AF293779 | Infectious bursal disease virus segment A isolate G48 VP2 (VP2) mRNA, partial cds |
| AF293780 | Infectious bursal disease virus segment A isolate G52 VP2 (VP2) mRNA, partial cds |
| AF293781 | Infectious bursal disease virus segment A isolate G67 VP2 (VP2) mRNA, partial cds |
| AF293782 | Infectious bursal disease virus segment A isolate G68 VP2 (VP2) mRNA, partial cds |
| AF293783 | Infectious bursal disease virus segment A isolate G71 VP2 (VP2) mRNA, partial cds |
| AF293784 | Infectious bursal disease virus segment A isolate G72 VP2 (VP2) mRNA, partial cds |
| AF293785 | Infectious bursal disease virus segment A isolate G75 VP2 (VP2) mRNA, partial cds |
| AF293786 | Infectious bursal disease virus segment A isolate G79 VP2 (VP2) mRNA, partial cds |
| AF293787 | Infectious bursal disease virus segment A isolate G02 VP2 (VP2) mRNA, partial cds |
| AF293788 | Infectious bursal disease virus segment A isolate Variant E VP2 (VP2) mRNA, partial cds |
| AF293789 | Infectious bursal disease virus segment A isolate Edgar VP2 (VP2) mRNA, partial cds |
| AF293790 | Infectious bursal disease virus segment A isolate Lukert VP2 (VP2) mRNA, partial cds |
| AF293791 | Infectious bursal disease virus segment A isolate Mississippi VP2 (VP2) mRNA, partial cds |
| AF293792 | Infectious bursal disease virus segment A isolate Variant A VP2 (VP2) mRNA, partial cds |
| AF293793 | Infectious bursal disease virus segment A isolate aphis VP2 (VP2) mRNA, partial cds |
| AF293794 | Infectious bursal disease virus segment A isolate AvimmuneF VP2 (VP2) mRNA, partial cds |
| AF293795 | Infectious bursal disease virus segment A isolate Bursine II VP2 (VP2) mRNA, partial cds |
| AF293796 | Infectious bursal disease virus segment A isolate Gumboral CT VP2 (VP2) mRNA, partial cds |
| AF293797 | Infectious bursal disease virus segment A isolate Gumboro Nobilis VP2 (VP2) mRNA, partial cds |
| AF293798 | Infectious bursal disease virus segment A isolate Gumborvet VP2 (VP2) mRNA, partial cds |
| AF293799 | Infectious bursal disease virus segment A isolate Gumbovax VP2 (VP2) mRNA, partial cds |
| AF293800 | Infectious bursal disease virus segment A isolate Matternalin VP2 (VP2) mRNA, partial cds |
| AF293801 | Infectious bursal disease virus segment A isolate Ultravac VP2 (VP2) mRNA, partial cds |
| AF293802 | Infectious bursal disease virus segment A isolate Des603-BR VP2 (VP2) mRNA, partial cds |
| AF293803 | Infectious bursal disease virus segment A isolate MC597-BR VP2 (VP2) mRNA, partial cds |
| AF293804 | Infectious bursal disease virus segment A isolate MC599-BR VP2 (VP2) mRNA, partial cds |
| AF303219 | Infectious bursal disease virus strain T1/TW segment A VP2 protein gene, partial cds |
| AF303895 | Infectious bursal disease virus isolate 1174 VP2 protein gene, partial cds |
| AF303896 | Infectious bursal disease virus isolate V1 VP2 protein gene, partial cds |
| AF304025 | Infectious bursal disease virus isolate 92-12-12 viral protein 2 (VP2) gene, partial cds |
| AF304026 | Infectious bursal disease virus isolate 94-3-6 viral protein 2 (VP2) gene, partial cds |
| AF304027 | Infectious bursal disease virus isolate 94-3-10 viral protein 2 (VP2) gene, partial cds |
| AF304028 | Infectious bursal disease virus isolate MB viral protein 2 (VP2) gene, partial cds |
| AF305736 | Infectious bursal disease virus isolate L1 VP2 protein gene, partial cds |
| AF305737 | Infectious bursal disease virus isolate L1 VP2 protein gene, partial cds |
| AF305738 | Infectious bursal disease virus isolate 586 VP2 protein gene, partial cds |
| AF305739 | Infectious bursal disease virus isolate 586 VP2 protein gene, partial cds |
| AF305740 | Infectious bursal disease virus isolate U2 VP2 protein gene, partial cds |
| AF305741 | Infectious bursal disease virus isolate U2 VP2 protein gene, partial cds |
| AF305742 | Infectious bursal disease virus isolate Q2 VP2 protein gene, partial cds |
| AF305743 | Infectious bursal disease virus isolate Q2 VP2 protein gene, partial cds |
| AF312371 | Infectious bursal disease virus VP2 protein (VP2) gene, partial cds |
| AF312793 | Infectious bursal disease virus segment A polyprotein mRNA, partial cds |
| AF321054 | Infectious bursal disease virus strain HZ2 segment A VP5 and polyprotein mRNAs, complete cds |
| AF321055 | Infectious bursal disease virus strain JD1 segment A VP5 and polyprotein mRNAs, complete cds |
| AF321056 | Infectious bursal disease virus strain ZJ2000 segment A VP5 and polyprotein mRNAs, complete cds |
| AF322444 | Infectious bursal disease virus segment A VP5 protein and polyprotein genes, complete cds |
| AF322445 | Infectious bursal disease virus segment B VP1 protein gene, complete cds |
| AF362747 | Infectious bursal disease virus Cu-1 wt polyprotein mRNA, complete cds |
| AF362748 | Infectious bursal disease virus Cu-1wt RNA-dependent RNA-polymerase mRNA, complete cds |
| AF362770 | Infectious bursal disease virus strain BD 3/99 segment B RNA-dependent RNA polymerase VP1 gene, complete cds |
| AF362771 | Infectious bursal disease virus strain Cu-1 M segment A structural polyprotein gene, partial cds |
| AF362772 | Infectious bursal disease virus strain Cu-1 M segment B RNA-dependent RNA polymerase VP1 gene, complete cds |
| AF362773 | Infectious bursal disease virus strain 23/82 segment A structural polyprotein gene, complete cds |
| AF362774 | Infectious bursal disease virus strain 23/82 segment B RNA-dependent RNA polymerase VP1 gene, complete cds |
| AF362775 | Infectious bursal disease virus strain Cu-1 segment B RNA-dependent RNA polymerase VP1 gene, complete cds |
| AF362776 | Infectious bursal disease virus strain BD 3/99 segment A structural polyprotein gene, complete cds |
| AF381000 | Infectious bursal disease virus isolate 01/96 VP2 protein gene, partial cds |
| AF381001 | Infectious bursal disease virus isolate A-1 VP2 protein gene, partial cds |
| AF381002 | Infectious bursal disease virus isolate M-1 VP2 protein gene, partial cds |
| AF381003 | Infectious bursal disease virus isolate R-1 VP2 protein mRNA, partial cds |
| AF381004 | Infectious bursal disease virus isolate SS-1 VP2 protein gene, partial cds |
| AF381005 | Infectious bursal disease virus isolate N1/99 VP2 protein gene, partial cds |
| AF381006 | Infectious bursal disease virus isolate K-2 VP2 protein gene, partial cds |
| AF381007 | Infectious bursal disease virus isolate T-4 VP2 protein mRNA, partial cds |
| AF381008 | Infectious bursal disease virus isolate Y5-3 VP2 protein gene, partial cds |
| AF381009 | Infectious bursal disease virus isolate H-1 VP2 protein mRNA, partial cds |
| AF381010 | Infectious bursal disease virus isolate C-1 VP2 protein gene, partial cds |
| AF381011 | Infectious bursal disease virus isolate 05-5 VP2 protein gene, partial cds |
| AF413069 | Infectious bursal disease virus strain BJ836 VP2 protein mRNA, partial cds |
| AF413070 | Infectious bursal disease virus strain BX viral protein 2 mRNA, partial cds |
| AF413071 | Infectious bursal disease virus strain LM viral protein 2 mRNA, partial cds |
| AF413072 | Infectious bursal disease virus strain HD98 viral protein 2 mRNA, partial cds |
| AF413073 | Infectious bursal disease virus strain HB97 viral protein 2 mRNA, partial cds |
| AF413074 | Infectious bursal disease virus strain FJ viral protein 2 mRNA, partial cds |
| AF413075 | Infectious bursal disease virus strain SC viral protein 2 mRNA, partial cds |
| AF413076 | Infectious bursal disease virus strain YV viral protein 2 mRNA, partial cds |
| AF416620 | Infectious bursal disease virus strain BK912 viral protein 2 mRNA, partial cds |
| AF416621 | Infectious bursal disease virus strain CJ801 viral protein 2 mRNA, partial cds |
| AF416622 | Infectious bursal disease virus strain DMS viral protein 2 mRNA, partial cds |
| AF416623 | Infectious bursal disease virus strain NC viral protein 2 mRNA, partial cds |
| AF416624 | Infectious bursal disease virus strain LX viral protein 2 mRNA, partial cds |
| AF416625 | Infectious bursal disease virus strain LN viral protein 2 mRNA, partial cds |
| AF416626 | Infectious bursal disease virus strain QV viral protein 2 mRNA, partial cds |
| AF416627 | Infectious bursal disease virus strain GZ902 viral protein 2 mRNA, partial cds |
| AF426063 | Infectious bursal disease virus isolate I-1 polyprotein gene, partial cds |
| AF426064 | Infectious bursal disease virus isolate 01/00 polyprotein gene, partial cds |
| AF426065 | Infectious bursal disease virus isolate 01/01 polyprotein gene, partial cds |
| AF426066 | Infectious bursal disease virus isolate 03-4 polyprotein gene, partial cds |
| AF426067 | Infectious bursal disease virus isolate 02/00 polyprotein gene, partial cds |
| AF427103 | Infectious bursal disease virus VP2 protein gene, partial cds |
| AF443294 | Infectious bursal disease virus polyprotein gene, complete cds |
| AF454945 | Infectious bursal disease virus segment A, complete sequence |
| AF455136 | Infectious bursal disease virus segment B, complete sequence |
| AF457103 | Infectious bursal disease virus strain ABIC/MB71 VP2 protein gene, partial cds |
| AF457104 | Infectious bursal disease virus strain Int/228E VP2 protein gene, partial cds |
| AF457105 | Infectious bursal disease virus strain Sanofi/2512 IM/TW VP2 protein gene, partial cds |
| AF457106 | Infectious bursal disease virus strain Univax/G603/TW VP2 protein gene, partial cds |
| AF464901 | Infectious bursal disease virus VP2 gene, partial cds |
| AF487340 | Infectious bursal disease virus VP2 mRNA, partial cds |
| AF491865 | Infectious bursal disease virus strain SP3338 viral protein 2 (VP2) gene, partial cds |
| AF493979 | Infectious bursal disease virus strain HZ2 RNA-ependent RNA polymerase VP1 gene, complete cds |
| AF498618 | Infectious bursal disease virus viral protein 2 (VP2) gene, partial cds |
| AF498619 | Infectious bursal disease virus strain MX7502 viral protein 2 (VP2) gene, partial cds |
| AF498620 | Infectious bursal disease virus strain MX7504 viral protein 2 (VP2) gene, partial cds |
| AF498621 | Infectious bursal disease virus strain MX7506 viral protein 2 (VP2) gene, partial cds |
| AF498622 | Infectious bursal disease virus strain MX7997 viral protein 2 (VP2) gene, partial cds |
| AF498623 | Infectious bursal disease virus strain DR3237 viral protein 2 (VP2) gene, partial cds |
| AF498624 | Infectious bursal disease virus strain BR-5 viral protein 2 (VP2) gene, partial cds |
| AF498625 | Infectious bursal disease virus strain DR-1 viral protein 2 (VP2) gene, partial cds |
| AF498626 | Infectious bursal disease virus strain DR-2 viral protein 2 (VP2) gene, partial cds |
| AF498627 | Infectious bursal disease virus strain C-278 viral protein 2 (VP2) gene, partial cds |
| AF498628 | Infectious bursal disease virus strain 1084E viral protein 2 (VP2) gene, partial cds |
| AF498629 | Infectious bursal disease virus strain 89/03 viral protein 2 (VP2) gene, partial cds |
| AF498630 | Infectious bursal disease virus isolate 9865 viral protein 2 (VP2) gene, partial cds |
| AF498631 | Infectious bursal disease virus strain Bursine 2 viral protein 2 (VP2) gene, partial cds |
| AF498632 | Infectious bursal disease virus strain Bursine Plus viral protein 2 (VP2) gene, partial cds |
| AF498633 | Infectious bursal disease virus strain Bursavac viral protein 2 (VP2) gene, partial cds |
| AF498634 | Infectious bursal disease virus strain KR-1 viral protein 2 (VP2) gene, partial cds |
| AF498635 | Infectious bursal disease virus strain U-28 viral protein 2 (VP2) gene, partial cds |
| AF498636 | Infectious bursal disease virus strain BR-8 viral protein 2 (VP2) gene, partial cd |
| AF499929 | Infectious bursal disease virus VP5 (VP5) and polyprotein (pol) genes, complete cds |
| AF499930 | Infectious bursal disease virus RNA-dependent RNA polymerase (VP1) gene, complete cds |
| AF506494 | Synthetic construct clone CRAb3 immunoglobulin G single chain variable fragment mRNA, partial cds |
| AF506495 | Synthetic construct clone CRAb5 immunoglobulin G single chain variable fragment mRNA, partial cds |
| AF506496 | Synthetic construct clone CRAb7 immunoglobulin G single chain variable fragment mRNA, partial cds |
| AF506497 | Synthetic construct clone CRAb8 immunoglobulin G single chain variable fragment mRNA, partial cds |
| AF506498 | Synthetic construct clone CRAb11 immunoglobulin G single chain variable fragment mRNA, partial cds |
| AF506499 | Synthetic construct clone CRAb12 immunoglobulin G single chain variable fragment mRNA, partial cds |
| AF506500 | Synthetic construct clone CRAb15 immunoglobulin G single chain variable fragment mRNA, partial cds |
| AF506501 | Synthetic construct clone CRAb20 immunoglobulin G single chain variable fragment mRNA, partial cds |
| AF506502 | Synthetic construct clone CRAb21 immunoglobulin G single chain variable fragment mRNA, partial cds |
| AF506503 | Synthetic construct clone CRAb22 immunoglobulin G single chain variable fragment mRNA, partial cds |
| AF506504 | Synthetic construct clone CRAb23 immunoglobulin G single chain variable fragment mRNA, partial cds |
| AF506505 | Synthetic construct clone CRAb24 immunoglobulin G single chain variable fragment mRNA, partial cds |
| AF506506 | Synthetic construct clone CRAb33 immunoglobulin G single chain variable fragment mRNA, partial cds |
| AF506507 | Synthetic construct clone CRAb34 immunoglobulin G single chain variable fragment mRNA, partial cds |
| AF506508 | Synthetic construct clone CRAb52 immunoglobulin G single chain variable fragment mRNA, partial cds |
| AF506509 | Synthetic construct clone CRAb72 immunoglobulin G single chain variable fragment mRNA, partial cds |
| AF506510 | Synthetic construct clone CRAb83 immunoglobulin G single chain variable fragment mRNA, partial cds |
| AF506511 | Synthetic construct clone CRAb96 immunoglobulin G single chain variable fragment mRNA, partial cds |
| AF506512 | Synthetic construct clone CRAb0 immunoglobulin G single chain variable fragment mRNA, partial cds |
| AF508176 | Infectious bursal disease virus segment A, complete sequence |
| AF508177 | Infectious bursal disease virus VP2 gene, complete cds |
| AF508738 | Infectious bursal disease virus isolate Ido1 segment A VP2 protein mRNA, partial cds |
| AF508739 | Infectious bursal disease virus isolate Indo2 segment A VP2 protein mRNA, partial cds |
| AF508740 | Infectious bursal disease virus isolate Indo3 segment A VP2 protein mRNA, partial cds |
| AF508741 | Infectious bursal disease virus isolate Indo4 segment A VP2 protein mRNA, partial cds |
| AF508742 | Infectious bursal disease virus isolate Indo5 segment A VP2 protein mRNA, partial cds |
| AF508743 | Infectious bursal disease virus isolate Indo6 segment A VP2 protein mRNA, partial cds |
| AF508744 | Infectious bursal disease virus isolate Indo7 segment A VP2 protein mRNA, partial cds |
| AF508745 | Infectious bursal disease virus isolate Indo8 segment A VP2 protein mRNA, partial cds |
| AF508746 | Infectious bursal disease virus isolate Indo9 segment A VP2 protein mRNA, partial cds |
| AF508747 | Infectious bursal disease virus isolate Indo10 segment A VP2 protein mRNA, partial cds |
| AF508748 | Infectious bursal disease virus isolate Indo1I segment A VP2 protein mRNA, partial cds |
| AF508749 | Infectious bursal disease virus isolate Indo13 segment A VP2 protein mRNA, partial cds |
| AF508750 | Infectious bursal disease virus isolate Indo14 segment A VP2 protein mRNA, partial cds |
| AF508751 | Infectious bursal disease virus isolate Indo15 segment A VP2 protein mRNA, partial cds |
| AF508752 | Infectious bursal disease virus isolate Indol6 segment A VP2 protein mRNA, partial cds |
| AF508753 | Infectious bursal disease virus isolate Indol7 segment A VP2 protein mRNA, partial cds |
| AF527038 | Infectious bursal disease virus strain UPM94/273 RNA-dependent RNA polymerase gene, complete cds |
| AF527039 | Infectious bursal disease virus strain UPM94/273 VP5 and polyprotein genes, complete cds |
| AF527040 | Infectious bursal disease virus strain UPM97/61 RNA-dependent RNA polymerase gene, complete cds |
| AF533670 | Infectious bursal disease virus strain SH/92 polyprotein mRNA, complete cds |
| AF533671 | Infectious bursal disease virus strain 225 VP2 gene, partial cds |
| AF533672 | Infectious bursal disease virus strain 225V4 VP2 gene, partial cds |
| AF533673 | Infectious bursal disease virus strain 310 VP2 gene, partial cds |
| AF533674 | Infectious bursal disease virus strain 310V4 VP2 gene, partial cds |
| AF533675 | Infectious bursal disease virus strain 269 VP2 gene, partial cds |
| AF533676 | Infectious bursal disease virus strain 269V4 VP2 gene, partial cds |
| AF533677 | Infectious bursal disease virus strain K1 VP2 gene, partial cds |
| AF533678 | Infectious bursal disease virus strain K1 V4 VP2 gene, partial cds |
| AF533679 | Infectious bursal disease virus strain SH/92V4 VP2 gene, partial cds |
| AF537268 | Infectious bursal disease virus from chicken polyprotein mRNA, partial cds |
| AF537269 | Infectious bursal disease virus from duck polyprotein mRNA, partial cds |
| AF537270 | Infectious bursal disease virus from goose polyprotein mRNA, partial cds |
| AF537271 | Infectious bursal disease virus from sparrow polyprotein mRNA, partial cds |
| AF548653 | Infectious bursal disease virus strain P10 VP2 protein gene, partial cds |
| AF548654 | Infectious bursal disease virus strain P11 VP2 protein gene, partial cds |
| AF548655 | Infectious bursal disease virus strain P1 VP2 protein gene, partial cds |
| AF548656 | Infectious bursal disease virus strain P3 VP2 protein gene, partial cds |
| AF548657 | Infectious bursal disease virus strain P7 VP2 protein gene, partial cds |
| AF548658 | Infectious bursal disease virus strain P9 VP2 protein gene, partial cds |
| AF548659 | Infectious bursal disease virus strain MOH96 VP2 protein gene, partial cds |
| AF548660 | Infectious bursal disease virus strain 35.592 VP2 protein gene, partial cds |
| AJ001941 | Infectious bursal disease virus mRNA for capsid protein VP2, strain 88180, partial |
| AJ001942 | Infectious bursal disease virus mRNA for capsid protein VP2, strain 89224, partial |
| AJ001943 | Infectious bursal disease virus mRNA for capsid protein VP2, strain 91184, partial |
| AJ001944 | Infectious bursal disease virus mRNA for capsid protein VP2, strain 91247, partial |
| AJ001945 | Infectious bursal disease virus mRNA for capsid protein VP2, strain 92309, partial |
| AJ001946 | Infectious bursal disease virus mRNA for capsid protein VP2, strain 95072/2, partial |
| AJ001947 | Infectious bursal disease virus mRNA for capsid protein VP2, strain 95072/8, partial |
| AJ001948 | Infectious bursal disease virus mRNA for capsid protein VP2, strains 96108 and 96236, partial |
| AJ238647 | Infectious bursal disease virus mRNA for viral capsid protein 2, partial |
| AJ245883 | Infectious Bursal Disease Virus partial VP2 gene for structural protein, isolate KT1/98 |
| AJ245884 | Infectious Bursal Disease Virus partial VP2 gene for structural protein isolate AP1/93 |
| AJ245885 | Infectious Bursalase Virus partial VP2 gene for structural protein, isolate CH2/97 |
| AJ245886 | Infectious Bursal Disease Virus partial VP2 gene for structural protein, isolate CH1/97 |
| AJ249517 | Infectious Bursal Disease Virus partial VP2 gene for structural protein |
| AJ249518 | Infectious Bursal Disease Virus partial VP2 gene for structural protein, isolate HR1/96 |
| AJ249519 | Infectious Bursal Disease Virus partial VP2 gene for structural protein isolate Intermediate plus |
| AJ249520 | Infectious Bursal Disease Virus partial VP2 gene for structural protein, isolate JK1/97 |
| AJ249521 | Infectious Bursal Disease Virus partial VP2 gene for structural protein, isolate KT1/98 |
| AJ249522 | Infectious Bursal Disease Virus partial VP2 gene for structural protein, isolate TP1/96 |
| AJ249523 | Infectious Bursal Disease Virus partial VP2 gene for structural protein, isolate Tri-bio |
| AJ249524 | Infectious Bursal Disease Virus partial VP2 gene for structural protein, isolate UP2/97 |
| AJ277801 | Infectious bursal disease virus partial VP2 gene for structural protein VP2, isolate UP1/99, genomic RNA |
| AJ295021 | Infectious bursal disease virus partial vp1 gene, isolate RJ1/94, genomic RNA |
| AJ295022 | Infectious bursal disease virus partial vp1 gene, isolate KT1/98, genomic RNA |
| AJ295023 | Infectious bursal disease virus partial vp1 gene, isolate TN1/93, genomic RNA |
| AJ295024 | Infectious bursal disease virus partial vp1 gene, isolate POONA, genomic RNA |
| AJ295025 | Infectious bursal disease virus partial vp1 gene, isolate IM+, genomic RNA |
| AJ295026 | Infectious bursal disease virus partial vp1 gene, isolate AP1/93, genomic RNA |
| AJ295027 | Infectious bursal disease virus partial vp1 gene, isolate UP1/97, genomic RNA |
| AJ295028 | Infectious bursal disease virus partial vp1 gene, isolate LUKERT, genomic RNA |
| AJ295029 | Infectious bursal disease virus partial vp2 gene, isolate MH1/97, genomic RNA |
| AJ310185 | Infectious bursal disease virus genomic RNA for VP5 polyprotein genes |
| AJ310186 | Infectious bursal disease virus genomic RNA for RNA-dependent RNA-polymerase |
| AJ315026 | Infectious bursal disease virus partial genomic RNA for VP2 protein, isolate Hyd(SPF10 |
| AJ315027 | Infectious bursal disease virus partial genomic RNA for VP2 protein, isolate Hyd(BGM7) |
| AJ315028 | Infectious bursal disease virus partial genomic RNA for VP2 protein, isolate Hyd(C) |
| AJ318896 | Infectious bursal disease virus gene for polyprotein |
| AJ318897 | Infectious bursal disease virus gene for VP1 protein |
| AJ344251 | Gumboro virus proviral partial vp2 gene for VP2 variable region, genomic RNA |
| AJ404327 | Infectious Bursal Disease Virus partial RNA for structural protein VP2 (vp2 gene) |
| AJ416444 | Infectious bursal disease virus partial VP2 gene for host protective antigen, genomic RNA |
| AJ416445 | Infectious bursal disease virus partial VP2 gene for host protective antigen, genomic RNA |
| AJ427340 | Infectious bursal disease virus genomic RNA for polyprotein, isolate KT1/99 |
| AJ496637 | Infectious bursal disease virus VP1 gene for RNA polymerase, genomic RNA |
| AJ504473 | Infectious bursal disease virus partial mRNA for structural protein VP2 |
| AJ577092 | Infectious bursal disease virus proviral partial VP2 gene for VP2 structural protein, genomic RNA |
| AJ586916 | Infectious bursa disease virus partial VP2 gene, genomic RNA, isolate IBDV/Oyo.NIE/95/001/c |
| AJ586917 | Infectious bursa disease virus partial VP2 gene, genomic RNA, isolate IBDV/Ogun.NIE/95/007/c |
| AJ586918 | Infectious bursa disease virus partial VP2 gene, genomic RNA, isolate IBDV/Oyo.NIE/957003/c |
| AJ586919 | Infectious bursa disease virus partial VP2 gene, genomic RNA, isolate IBDV/Oyo.NIE/95/008/c |
| AJ586920 | Infectious bursa disease virus partial VP2 gene, genomic RNA, isolate IBDV/Oyo.NIE/95/016/c |
| AJ586921 | Infectious bursa disease virus partial VP2 gene, genomic RNA, isolate IBDV/Ogun.NIE/96/005/c |
| AJ586922 | Infectious bursa disease virus partial VP2 gene, genomic RNA, isolate IBDV/Ogun.NIE/96/086/c |
| AJ586923 | Infectious bursa disease virus partial VP2 gene, genomic RNA, isolate IBDV/Ogun.NIE/96/080/c |
| AJ586924 | Infectious bursa disease virus partial VP2 gene, genomic RNA, isolate IBDV/Osun.NIE/96/076/c |
| AJ586925 | Infectious bursa disease virus partial VP2 gene, genomic RNA, isolate IBDV/Osun.NIE/96/036/c |
| AJ586926 | Infectious bursa disease virus partial VP2 gene, genomic RNA, isolate IBDV/Oyo.NIE/96/090/c |
| AJ586927 | Infectious bursa disease virus partial VP2 gene, genomic RNA, isolate IBDV/Oyo.NIE/96/017/c |
| AJ586928 | Infectious bursa disease virus partial VP2 gene, genomic RNA, isolate IBDV/Oyo.NIE/96/077/c |
| AJ586929 | Infectious bursa disease virus partial VP2 gene, genomic RNA, isolate IBDV/Oyo.NIE/96/033/c |
| AJ586930 | Infectious bursa disease virus partial VP2 gene, genomic RNA, isolate IBDV/Lagos.NIE/97/011/c |
| AJ586931 | Infectious bursa disease virus partial VP2 gene, genomic RNA, isolate IBDV/Ogun.NIE/97/010/c |
| AJ586932 | Infectious bursa disease virus partial VP2 gene, genomic RNA, isolate IBDV/Ogun.NIE/97/014/c |
| AJ586933 | Infectious bursa disease virus partial VP2 gene, genomic RNA, isolate IBDV/Ogun.NIE/97/078/c |
| AJ586934 | Infectious bursa disease virus partial VP2 gene, genomic RNA, isolate IBDV/Ogun.NIE797/082/c |
| AJ586935 | Infectious bursa disease virus partial VP2 gene, genomic RNA, isolate IBDV/Osun.NIE/97/092/c |
| AJ586936 | Infectious bursa disease virus partial VP2 gene, genomic RNA, isolate IBDV/Oyo.NIE/97/057/c |
| AJ586937 | Infectious bursa disease virus partial VP2 gene, genomic RNA, isolate IBDV/Oyo.NIE/97/006/c |
| AJ586938 | Infectious bursa disease virus partial VP2 gene, genomic RNA, isolate IBDV/Oyo.NIE/97/013/c |
| AJ586939 | Infectious bursa disease virus partial VP2 gene, genomic RNA, isolate IBDV/Oyo.NIE/97/102/c |
| AJ586940 | Infectious bursa disease virus partial VP2 gene, genomic RNA, isolate IBDV/Oyo.NIE/97/222/c |
| AJ586941 | Infectious bursa disease virus partial VP2 gene, genomic RNA, isolate IBDV/Oyo.NIE/97/001/t |
| AJ586942 | Infectious bursa disease virus partial VP2 gene, genomic RNA, isolate IBDV/Oyo.NIE/97/004/t |
| AJ586943 | Infectious bursa disease virus partial VP2 gene, genomic RNA, isolate IBDV/Oyo.NIE/97/012/t |
| AJ586944 | Infectious bursa disease virus partial VP2 gene, genomic RNA, isolate IBDV/Ogun.NIE/98/120/c |
| AJ586945 | Infectious bursa disease virus partial VP2 gene, genomic RNA, isolate IBDV/Ogun.NIE/98/085/c |
| AJ586946 | Infectious bursa disease virus partial VP2 gene, genomic RNA, isolate IBDV/Osun.NIE/98/062/c |
| AJ586947 | Infectious bursa disease virus partial VP2 gene, genomic RNA, isolate IBDV/Oyo.NIE/98/084/c |
| AJ586948 | Infectious bursa disease virus partial VP2 gene, genomic RNA, isolate IBDV/Oyo.NIE/98/027/c |
| AJ586949 | Infectious bursa disease virus partial VP2 gene, genomic RNA, isolate IBDV/Oyo.NIE/98/227/c |
| AJ586950 | Infectious bursa disease virus partial VP2 gene, genomic RNA, isolate IBDV/Oyo.NIE/98/059/c |
| AJ586951 | Infectious bursa disease virus partial VP2 gene, genomic RNA, isolate IBDV/Oyo.NIE/98/058/c |
| AJ586952 | Infectious bursa disease virus partial VP2 gene, genomic RNA, isolate IBDV/Oyo.NIE/98/009/t |
| AJ586953 | Infectious bursa disease virus partial VP2 gene, genomic RNA, isolate IBDV/Ogun.NIE/99/034/c |
| AJ586954 | Infectious bursa disease virus partial VP2 gene, genomic RNA, isolate IBDV/Osun.NIE/99/030/c |
| AJ586955 | Infectious bursa disease virus partial VP2 gene, genomic RNA, isolate IBDV/Oyo.NIE/99/015/c |
| AJ586956 | Infectious bursa disease virus partial VP2 gene, genomic RNA, isolate IBDV/Oyo.NIE/99/050/c |
| AJ586957 | Infectious bursa disease virus partial VP2 gene, genomic RNA, isolate IBDV/Oyo.NIE/99/054/c |
| AJ586958 | Infectious bursa disease virus partial VP2 gene, genomic RNA, isolate IBDV/Osun.NIE/00/046/c |
| AJ586959 | Infectious bursa disease virus partial VP2 gene, genomic RNA, isolate IBDV/Oyo.NIE/00/042/c |
| AJ586960 | Infectious bursa disease virus partial VP2 gene, genomic RNA, isolate Ventri (vaccine) |
| AJ586961 | Infectious bursa disease virus partial VP2 gene, genomic RNA, isolate BURSINE Plus (vaccine) |
| AJ586962 | Infectious bursa disease virus partial VP2 gene, genomic RNA, isolate MB (vaccine) |
| AJ586963 | Infectious bursa disease virus partial VP2 gene, genomic RNA, isolate D78 |
| AJ586964 | Infectious bursa disease virus partial VP2 gene, genomic RNA, isolate NVRI-VOM (vaccine) |
| AJ586965 | Infectious bursa disease virus partial VP2 gene, genomic RNA, isolate IBA (vaccine) |
| AJ586966 | Infectious bursa disease virus partial VP2 gene, genomic RNA, isolate Nobilis Gumboro 228E (vaccine) |
| AJ586967 | infectious bursa disease virus partial VP2 gene, genomic RNA, isolate Bursaplex (vaccine) |
| AJ586968 | Infectious bursa disease virus partial VP2 gene, genomic RNA, isolate V877 (vaccine) |
| AJ586969 | Infectious bursa disease virus partial VP2 gene, genomic RNA, isolate 1084E |
| AR265314 | Sequence 1 from patent US 6492148 |
| AR265315 | Sequence 2 from patent US 6492148 |
| AR265316 | Sequence 3 from patent US 649214 |
| AR265317 | Sequence 4 from patent US 6492148 |
| AR265318 | Sequence 5 from patent US 6492148 |
| AR265319 | Sequence 6 from patent US 6492148 |
| AR265320 | Sequence 7 from patent US 6492148 |
| AR265321 | Sequence 8 from patent US 6492148 |
| AR265322 | Sequence 9 from patent US 6492148 |
| AR283491 | Sequence 1 from patent US 6528063 |
| AR337890 | Sequence 1 from patent US 6569422 |
| AR337891 | Sequence 2 from patent US 6569422 |
| AR337892 | Sequence 3 from patent US 6569422 |
| AR337893 | Sequence 4 from patent US 6569422 |
| AR337894 | Sequence 5 from patent US 6569422 |
| AR337895 | Sequence 6 from patent US 6569422 |
| AR337896 | Sequence 7 from patent US 6569422 |
| AR337897 | Sequence 8 from patent US 6569422 |
| AR337898 | Sequence 9 from patent US 6569422 |
| AX034695 | Sequence 1 from Patent EP1035203 |
| AX034696 | Sequence 2 from Patent EP1035203 |
| AX034697 | Sequence 3 from Patent EP1035203 |
| AX034698 | Sequence 4 from Patent EP1035203 |
| AX034699 | Sequence 5 from Patent EP1035203 |
| AX034700 | Sequence 6 from Patent EP1035203 |
| AX034701 | Sequence 7 from Patent EP1035203 |
| AX034702 | Sequence 8 from Patent EP1035203 |
| AX034703 | Sequence 9 from Patent EP1035203 |
| AX074441 | Sequence 1 from Patent WO0104319 |
| AX074442 | Sequence 2 from Patent WO0104319 |
| AX074443 | Sequence 3 from Patent WO0104319 |
| AX074444 | Sequence 4 from Patent WO0104319 |
| AX074445 | Sequence 5 from Patent WO0104319 |
| AX074446 | Sequence 6 from Patent WO0104319 |
| AX074447 | Sequence 7 from Patent WO0104319 |
| AX074448 | Sequence 8 from Patent WO0104319 |
| AX074449 | Sequence 9 from Patent WO0104319 |
| AX074450 | Sequence 10 from Patent WO0104319 |
| AX074451 | Sequence 11 from Patent WO0104319 |
| AX074452 | Sequence 12 from Patent WO0104319 |
| AX074453 | Sequence 13 from Patent WO0104319 |
| AX074454 | Sequence 14 from Patent WO0104319 |
| AX074455 | Sequence 15 from Patent WO0104319 |
| AX074456 | Sequence 16 from Patent WO0104319 |
| AX074457 | Sequence 17 from Patent WO0104319 |
| AX074458 | Sequence 18 from Patent WO0104319 |
| AX074459 | Sequence 19 from Patent WO0104319 |
| AX074460 | Sequence 20 from Patent WO0104319 |
| AX074461 | Sequence 21 from Patent WO0104319 |
| AX074462 | Sequence 22 from Patent WO0104319 |
| AX074463 | Sequence 23 from Patent WO0104319 |
| AX074464 | Sequence 24 from Patent WO0104319 |
| AX074465 | Sequence 25 from Patent WO0104319 |
| AX074466 | Sequence 26 from Patent WO0104319 |
| AX074467 | Sequence 27 from Patent WO0104319 |
| AX074468 | Sequence 28 from Patent WO0104319 |
| AX074469 | Sequence 29 from Patent WO0104319 |
| AX074470 | Sequence 30 from Patent WO0104319 |
| AX074471 | Sequence 31 from Patent WO0104319 |
| AX074472 | Sequence 32 from Patent WO0104319 |
| AX074473 | Sequence 33 from Patent WO0104319 |
| AX074474 | Sequence 34 from Patent WO0104319 |
| AX074475 | Sequence 35 from Patent WO0104319 |
| AX074476 | Sequence 36 from Patent WO0104319 |
| AX074477 | Sequence 37 from Patent WO0104319 |
| AX074478 | Sequence 38 from Patent WO0104319 |
| AX074479 | Sequence 39 from Patent WO0104319 |
| AX074480 | Sequence 40 from Patent WO0104319 |
| AX074481 | Sequence 41 from Patent WO0104319 |
| AX074482 | Sequence 42 from Patent WO0104319 |
| AX074483 | Sequence 43 from Patent WO0104319 |
| AX074484 | Sequence 44 from Patent WO0104319 |
| AX074485 | Sequence 45 from Patent WO0104319 |
| AX074486 | Sequence 46 from Patent WO0104319 |
| AX074487 | Sequence 47 from Patent WO0104319 |
| AX074488 | Sequence 48 from Patent WO0104319 |
| AX074489 | Sequence 49 from Patent W00104319 |
| AX074490 | Sequence 50 from Patent WO0104319 |
| AX074491 | Sequence 51 from Patent WO0104319 |
| AX074492 | Sequence 52 from Patent WO0104319 |
| AX074493 | Sequence 53 from Patent WO0104319 |
| AX074494 | Sequence 54 from Patent WO0104319 |
| AX074495 | Sequence 55 from Patent WO0104319 |
| AX074496 | Sequence 56 from Patent WO0104319 |
| AX074497 | Sequence 57 from Patent WO0104319 |
| AX074498 | Sequence 58 from Patent WO0104319 |
| AX074508 | Sequence 68 from Patent WO0104319 |
| AX074509 | Sequence 69 from Patent WO0104319 |
| AX074510 | Sequence 70 from Patent WO0104319 |
| AX074511 | Sequence 71 from Patent WO0104319 |
| AX074512 | Sequence 72 from Patent WO0104319 |
| AX074513 | Sequence 73 from Patent WO0104319 |
| AX074514 | Sequence 74 from Patent WO0104319 |
| AX138218 | Sequence 1 from Patent EP1069187 |
| AX138219 | Sequence 2 from Patent EP1069187 |
| AX138220 | Sequence 3 from Patent EP1069187 |
| AX138221 | Sequence 4 from Patent EP1069187 |
| AX138222 | Sequence 5 from Patent EP1069187 |
| AX138223 | Sequence 6 from Patent EP1069187 |
| AX138224 | Sequence 7 from Patent EP1069187 |
| AX138225 | Sequence 8 from Patent EP1069187 |
| AX138226 | Sequence 9 from Patent EP1069187 |
| AX138227 | Sequence 10 from Patent EP1069187 |
| AX138228 | Sequence 11 from Patent EP1069187 |
| AX138229 | Sequence 12 from Patent EP1069187 |
| AX138230 | Sequence 13 from Patent EP1069187 |
| AX138231 | Sequence 14 from Patent EP1069187 |
| AX138232 | Sequence 15 from Patent EP1069187 |
| AX138233 | Sequence 16 from Patent EP1069187 |
| AX138234 | Sequence 17 from Patent EP1069187 |
| AX138235 | Sequence 18 from Patent EP1069187 |
| AX138236 | Sequence 19 from Patent EP1069187 |
| AX138237 | Sequence 20 from Patent EP1069187 |
| AX138238 | Sequence 21 from Patent EP1069187 |
| AX138239 | Sequence 22 from Patent EP1069187 |
| AX138240 | Sequence 23 from Patent EP1069187 |
| AX138241 | Sequence 24 from Patent EP1069187 |
| AX138242 | Sequence 25 from Patent EP1069187 |
| AX138243 | Sequence 26 from Patent EP1069187 |
| AX138244 | Sequence 27 from Patent EP1069187 |
| AX138245 | Sequence 28 from Patent EP1069187 |
| AX138246 | Sequence 29 from Patent EP1069187 |
| AX138247 | Sequence 30 from Patent EP1069187 |
| AX138257 | Sequence 40 from Patent EP1069187 |
| AX138258 | Sequence 41 from Patent EP1069187 |
| AX138259 | Sequence 42 from Patent EP1069187 |
| AX138260 | Sequence 43 from Patent EP1069187 |
| AX138261 | Sequence 44 from Patent EP1069187 |
| AX138262 | Sequence 45 from Patent EP1069187 |
| AX138263 | Sequence 46 from Patent EP1069187 |
| AX323155 | Sequence 1 from Patent WO0192486 |
| AX323157 | Sequence 3 from Patent WO0192486 |
| AX323158 | Sequence 4 from Patent WO0192486 |
| AX343661 | Sequence 1 from Patent EF1170302 |
| AX343663 | Sequence 3 from Patent EP1170302 |
| AX709623 | Sequence 9 from Patent WO0209694 |
| AX709650 | Sequence 36 from Patent WO02096940 |
| AX709651 | Sequence 37 from Patent WO02096940 |
| AX709652 | Sequence 38 from Patent WO02096940 |
| AX709653 | Sequence 39 from Patent WO02096940 |
| AX709654 | Sequence 40 from Patent WO02096940 |
| AX709655 | Sequence 41 from Patent WO02096940 |
| AX709656 | Sequence 42 from Patent WO02096940 |
| AX709657 | Sequence 43 from Patent WO02096940 |
| AX709658 | Sequence 44 from Patent WO02096940 |
| AX709659 | Sequence 45 from Patent WO02096940 |
| AX709660 | Sequence 46 from Patent WO02096940 |
| AX709661 | Sequence 47 from Patent WO02096940 |
| AX709662 | Sequence 48 from Patent WO02096940 |
| AX709663 | Sequence 49 from Patent WO02096940 |
| AX709664 | Sequence 50 from Patent WO02096940 |
| AX709665 | Sequence 51 from Patent WO02096940 |
| AX709666 | Sequence 52 from Patent WO02096940 |
| AX709667 | Sequence 53 from Patent WO02096940 |
| AX709668 | Sequence 54 from Patent WO02096940 |
| AX709669 | Sequence 55 from Patent WO02096940 |
| AX709670 | Sequence 56 from Patent WO02096940 |
| AX709671 | Sequence 57 from Patent WO02096940 |
| AX709672 | Sequence 58 from Patent WO02096940 |
| AX709673 | Sequence 59 from Patent WO02096940 |
| AX709674 | Sequence 60 from Patent WO02096940 |
| AX709675 | Sequence 61 from Patent WO02096940 |
| AX709676 | Sequence 62 from Patent WO02096940 |
| AX709677 | Sequence 63 from Patent WO02096940 |
| AX709678 | Sequence 64 from Patent WO02096940 |
| AX721965 | Sequence 1 from Patent EP1298139 |
| AX721968 | Sequence 4 from Patent EP1298139 |
| AX721969 | Sequence 5 from Patent EP1298139 |
| AX721970 | Sequence 6 from Patent EP1298139 |
| AX721971 | Sequence 7 from Patent EP1298139 |
| AX721972 | Sequence 8 from Patent EP1298139 |
| AX721975 | Sequence 11 from Patent EP1298139 |
| AX721976 | Sequence 12 from Patent EP1298139 |
| AX721977 | Sequence 13 from Patent EP1298139 |
| AX721978 | Sequence 14 from Patent EP1298139 |
| AX721979 | Sequence 15 from Patent EP1298139 |
| AX721980 | Sequence 16 from Patent EP1298139 |
| AX721988 | Sequence 24 from Patent EP1298139 |
| AX721989 | Sequence 25 from Patent EP1298139 |
| AY012677 | Infectious bursal disease virus segment A isolate 619 viral protein 2 (VP2) mRNA, partial cds |
| AY012678 | Infectious bursal disease virus segment A isolate 625 viral protein 2 (VP2) mRNA, partial cds |
| AY012679 | Infectious bursal disease virus segment A isolate 849 viral protein 2 (VP2) mRNA, partial cds |
| AY012680 | Infectious bursal disease virus segment A isolate 850 viral protein 2 (VP2) mRNA, partial cds |
| AY012681 | Infectious bursal disease virus segment A isolate 853 viral protein 2 (VP2) mRNA, partial cds |
| AY012682 | Infectious bursal disease virus segment A isolate 11153 viral protein 2 (VP2) mRNA, partial cds |
| AY012683 | Infectious bursal disease virus segment A isolate 9109 viral protein 2 (VP2) mRNA, partial cds |
| AY029165 | Infectious bursal disease virus RNA-dependent RNA polymerase gene, complete cds |
| AY029166 | Infectious bursal disease virus 17-kDa nonstructural protein and 110-kDa polyprotein genes, complete cds |
| AY065630 | Infectious bursal disease virus isolate IBDTCN2001 VP2 (VP2) mRNA, partial cds |
| AY065631 | Infectious bursal disease virus isolate IBDTCL2001 VP2 (VP2) mRNA, partial cds |
| AY065632 | Infectious bursal disease virus isolate IBDTRP1999/2 VP2 (VP2) mRNA, partial cds |
| AY065633 | Infectious bursal disease virus isolate IBDTTN2000 nonfunctional VP2 protein, partial sequence |
| AY065634 | Infectious bursal disease virus isolate IBDTNK2001/1 nonfunctional VP2 protein mRNA, partial sequence |
| AY065635 | Infectious bursal disease virus isolate IBDTNK2001 VP2 (VP2) mRNA, partial cds |
| AY065636 | Infectious bursal disease virus isolate IBDTNK1999/1 nonfunctional VP2 protein mRNA, partial sequence |
| AY065637 | Infectious bursal disease virus isolate IBDTCB2001 VP2 (VP2) mRNA, partial cds |
| AY083925 | Infectious bursal disease virus strain VG-248 segment A viral protein 2 (VP2) gene, partial cds |
| AY083926 | Infectious bursal disease virus strain 5939 segment A viral protein 2 (VP2) gene, partial cds |
| AY083927 | Infectious bursal disease virus strain VG-311 segment A viral protein 2 (VP2) gene, partial cds |
| AY083928 | Infectious bursal disease virus strain VG-313 segment A viral protein 2 (VP2) gene, partial cds |
| AY083929 | Infectious bursal disease virus strain VG-262 segment A viral protein 2 (VP2) gene, partial cds |
| AY083930 | Infectious bursal disease virus strain 6145 segment A viral protein 2 (VP2) gene, partial cds |
| AY083931 | Infectious bursal disease virus strain VG-208 segment A viral protein 2 (VP2) gene, partial cds |
| AY083932 | Infectious bursal disease virus strain VG-276 segment A viral protein 2 (VP2) gene, partial cds |
| AY083933 | Infectious bursal disease virus strain 7333 segment A viral protein 2 (VP2) gene, partial cds |
| AY094618 | Infectious bursal disease virus polyprotein gene, partial cds |
| AY095229 | Infectious bursal disease virus strain Edgar viral protein 2 (VP2) gene, partial cds |
| AY095230 | Infectious bursal disease virus strain Lukert viral protein 2 (VP2) gene, partial cds |
| AY095231 | Infectious bursal disease virus strain Ohio viral protein 2 (VP2) gene, partial cds |
| AY095534 | Infectious bursal disease virus strain Mor viral protein 2 (VP2) gene, partial cds |
| AY099456 | Infectious bursal disease virus VP5 (VP5) and polyprotein genes, complete cds |
| AY099457 | Infectious bursal disease virus VP1 (VP1) gene, complete cds |
| AY099997 | Infectious bursal disease virus strain Ven-1 viral protein 2 (VP2) gene, partial cds |
| AY099998 | Infectious bursal disease virus strain Ven-2 viral protein 2 (VP2) gene, partial cds |
| AY099999 | Infectious bursal disease virus strain Ven-3 viral protein 2 (VP2) gene, partial cds |
| AY100000 | Infectious bursal disease virus strain Ven-4 viral protein 2 (VP2) gene, partial cds |
| AY100001 | Infectious bursal disease virus strain Ven-5 viral protein 2 (VP2) gene, partial cds |
| AY100319 | Infectious bursal disease virus strain EC-3 viral protein 2 (VP2) gene, partial cds |
| AY100320 | Infectious bursal disease virus strain MTA viral protein 2 (VP2) gene, partial cds |
| AY100321 | Infectious bursal disease virus strain PAD viral protein 2 (VP2) gene, partial cds |
| AY103464 | Infectious bursal disease virus strain JD1 RNA-dependent RNA polymerase VP1 gene, complete cds |
| AY115569 | Infectious bursal disease virus strain GDA VP2 protein gene, partial cds |
| AY115570 | Infectious bursal disease virus strain GHD VP2 protein gene, partial cds |
| AY115571 | Infectious bursal disease virus strain GPT VP2 protein gene, partial cds |
| AY134874 | Infectious bursal disease virus strain SH95 polyprotein gene, complete cds |
| AY134875 | Infectious bursal disease virus strain SH95 RNA-dependent RNA polymerase (VP1) gene, complete cds |
| AY245550 | Infectious bursal disease virus isolate UPM 93273 VP2 protein gene, partial cds |
| AY288047 | Infectious bursal disease virus isolate SD1-97 VP2 mRNA, partial cds |
| AY288048 | Infectious bursal disease virus isolate JS30-99 VP2 mRNA, partial cds |
| AY305386 | Infectious bursal disease virus isolate GX8/99 viral protein 2 (VP2) mRNA, partial cds |
| AY305387 | Infectious bursal disease virus isolate SD3-98 viral protein 2 (VP2) mRNA, partial cds |
| AY311479 | Infectious bursal disease virus structural protein VP2 (VP2) gene, partial cds |
| AY318758 | Infectious bursal disease virus VP2 protein hypervariable region (VP2) gene, partial cds |
| AY319768 | Infectious bursal disease virus strain NB VP5 and polyprotein genes, complete cds |
| AY321508 | Infectious bursal disease virus VP5 mRNA, complete cds |
| AY321509 | Infectious bursal disease virus VP2 mRNA, complete cds |
| AY321518 | Infectious bursal disease virus isolate CO/2003 VP2 gene, partial cds |
| AY321519 | Infectious bursal disease virus isolate DH/2003 nonfunctional VP2 gene, partial sequence |
| AY321520 | Infectious bursal disease virus isolate DHA/2003 nonfunctional VP2 gene, partial sequence |
| AY321521 | Infectious bursal disease virus isolate KAA/2003 nonfunctional VP2 gene, partial sequence |
| AY321522 | Infectious bursal disease virus isolate NAMI/2003 VP2 gene, partial cds |
| AY321523 | Infectious bursal disease virus isolate NAM2/2003 nonfunctional VP2 gene, partial sequence |
| AY321524 | Infectious bursal disease virus isolate NAM3/2003 VP2 gene, partial cds |
| AY321525 | Infectious bursal disease virus isolate NAMIV/2003 nonfunctional VP2 gene, partial sequence |
| AY321526 | Infectious bursal disease virus isolate TNU/2003 nonfunctional VP2 gene, partial sequence |
| AY321527 | Infectious bursal disease virus isolate VV/2003 VP2 gene, partial cds |
| AY321949 | Infectious bursal disease virus strain 849VB VP2 gene, partial cds |
| AY321950 | Infectious bursal disease virus strain 96108 VP2 gene, partial cds |
| AY321951 | Infectious bursal disease virus strain CJ801 VP2 gene, partial cds |
| AY321952 | Infectious bursal disease virus strain Cu-1wt VP2 gene, partial cds |
| AY321953 | Infectious bursal disease virus strain F52-70 VP2 gene, partial cds |
| AY321954 | Infectious bursal disease virus strain GX VP2 gene, partial cds |
| AY321955 | Infectious bursal disease virus strain Harbin VP2 gene, partial cds |
| AY321956 | Infectious bursal disease virus strain Henan1 VP2 gene, partial cds |
| AY321957 | Infectious bursal disease virus strain HK46 VP2 gene, partial cds |
| AY323952 | Infectious bursal disease virus segment A structural protein VP5 (VP5) and polyprotein genes, complete cds |
| AY327576 | Infectious bursal disease virus isolate RAP nonfunctional VP2 gene, partial sequence |
| AY327577 | Infectious bursal disease virus isolate RP nonfunctional VP2 gene, partial sequence |
| AY327578 | Infectious bursal disease virus isolate SAL nonfunctional VP2 gene, partial sequence |
| AY327579 | Infectious bursal disease virus isolate SALM nonfunctional VP2 gene, partial sequence |
| AY332560 | Infectious bursal disease virus isolate IBD BLEN VP2 protein (VP2) gene, partial cds |
| AY332561 | Infectious bursal disease virus isolate Bursine-2 VP2 protein (VP2) gene, partial cds |
| AY332562 | Infectious bursal disease virus isolate CEVAC IBD L VP2 protein (VP2) gene, partial cds |
| AY367560 | Infectious bursal disease virus isolate NP2K VP2 protein (VP2) gene, partial cds |
| AY423560 | Infectious bursal disease virus VP2 (VP2) gene, partial cds |
| BD000334 | Genetically engeneered cell culture adapted infectious bursal disease virus (IBDV) mutants |
| BD000335 | Genetically engeneered cell culture adapted infectious bursal disease virus (IBDV) mutants |
| BD000336 | Genetically engeneered cell culture adapted infectious bursal disease virus (IBDV) mutants |
| BD000337 | Genetically engeneered cell culture adapted infectious bursal disease virus (IBDV) mutants |
| BD000338 | Genetically engeneered cell culture adapted infectious bursal disease virus (IBDV) mutants |
| BD000339 | Genetically engeneered cell culture adapted infectious bursal disease virus (IBDV) mutants |
| BD000340 | Genetically engeneered cell culture adapted infectious bursal disease virus (IBDV) mutants |
| BD000341 | Genetically engeneered cell culture adapted infectious bursal disease virus (IBDV) mutants |
| BD000342 | Genetically engeneered cell culture adapted infectious bursal disease virus (IBDV) mutants |
| BD009825 | Avian polynucleotide vaccine formula |
| BD009826 | Avian polynucleotide vaccine formula |
| BD009827 | Avian polynucleotide vaccine formula |
| BD009829 | Avian polynucleotide vaccine formula |
| BD009830 | Avian polynucleotide vaccine formula |
| BD009832 | Avian polynucleotide vaccine formula |
| BD009833 | Avian polynucleotide vaccine formula |
| BD144646 | Broad-spectrum infectious bursal disease virus vaccine |
| BD144647 | Broad-spectrum infectious bursal disease virus vaccine |
| D00499 | Infectious bursal disease virus genomic RNA, segment A containing large ORF and small ORF, complete cds |
| D00867 | Infectious bursal disease virus gene for polyprotein (VP2a-VP4-VP3), complete cds, strain:Cu1 |
| D00868 | Infectious bursal disease virus gene for polyprotein (VP2-VP4-VP3), partial cds, strain:PBG-98 |
| D00869 | Infectious bursal disease virus gene for polyprotein (VP2-VP4-VP3), complete cds, strain:52/70 |
| D10065 | Infectious bursal disease virus genomic RNA for VP2, partial sequence |
| D12609 | Infectious bursal disease virus genomic RNA, 5'end of segment B |
| D12610 | Infectious bursal disease virus gene for VP1, partial sequence |
| D16630 | Infectious bursal disease virus RNA for polyprotein (hypervariable region), partial cds, strain: DV86 |
| D16675 | Infectious bursal disease virus RNA for polyprotein (hypervariable region), partial cds, strain: DV8 |
| D16677 | Infectious bursal disease virus RNA for polyprotein (hypervariable region), partial cds, strain: J1 |
| D16678 | Infectious bursal disease virus RNA for polyprotein (hypervariable region), partial cds, strain: K |
| D16679 | Infectious bursal disease virus RNA for polyprotein (hypervariable region), partial cds |
| D16828 | Infectious bursal disease virus RNA for polyprotein (hypervariable region), partial cds, strain: GBF-1 |
| D49706 | Infectious bursal disease virus RNA for polyprotein (hypervariable region), partial cds, strain: OKYM |
| D49707 | Infectious bursal disease virus VP1 gene for RNA polymerase, complete cds |
| D83985 | Infectious bursal disease virus RNA for polyprotein (hypervariable region), partial cds, strain: OKYMT |
| D84071 | Infectious bursal disease virus RNA for polyprotein (hypervariable region), partial cds, strain: TKSMT |
| D84072 | Infectious bursal disease virus RNA for polyprotein (hypervariable region), partial cds, strain: TKSM |
| D86860 | Infectious bursal disease virus gene for VP1, partial cds, strain:DV86 |
| D86861 | Infectious bursal disease virus RNA for VP1, partial cds, strain:GBF-1 |
| D87047 | Infectious bursal disease virus gene for VP1, partial cds, strain:GBF-1E |
| D87048 | Infectious bursal disease virus gene for VP1, partial cds, strain:LukertBP |
| D87049 | Infectious bursal disease virus gene for VP1, partial cds, strain:J1 |
| D87050 | Infectious bursal disease virus gene for VP1, partial cds, strain:Cu-1 |
| D87051 | Infectious bursal disease virus gene for VP1, partial cds, strain:2512 |
| D87052 | Infectious bursal disease virus gene for VP1, partial cds, strain:MO |
| E05277 | cDNA encoding split virus structural protein |
| E05442 | DNA sequence of IBDV SegA gene |
| E05443 | DNA sequence of IBDV SegA gene |
| E05444 | DNA sequence of IBDV SegA gene |
| E12060 | cDNA encoding VP2 protein of Infectious bursa of Fabricius Disease Virus(IBDV) |
| E12069 | SegA sequence of Infectious bursa of Fabricius Disease Virus(IBDV) |
| 134206 | Sequence 1 from patent US 5595912 |
| I34207 | Sequence 2 from patent US 5595912 |
| 134208 | Sequence 3 from patent US 5595912 |
| 134209 | Sequence 4 from patent US 5595912 |
| I34210 | Sequence 5 from patent US 5595913 |
| I34211 | Sequence 6 from patent US 5595912 |
| 134212 | Sequence 7 from patent US 5595912 |
| 134213 | Sequence 8 from patent US 5595912 |
| I34214 | Sequence 9 from patent US 5595912 |
| 134215 | Sequence 10 from patent US 5595912 |
| 134216 | Sequence 11 from patent US 5595912 |
| I34217 | Sequence 12 from patent US 5595912 |
| I34218 | Sequence 13 from patent US 5595912 |
| I34219 | Sequence 14 from patent US 5595912 |
| I34220 | Sequence 15 from patent US 5595912 |
| I34221 | Sequence 18 from patent US 5595912 |
| 134222 | Sequence 26 from patent US 5595912 |
| I34223 | Sequence 28 from patent US 5595912 |
| 134224 | Sequence 30 from patent US 5595912 |
| 134225 | Sequence 32 from patent US 5595912 |
| 143648 | Sequence 1 from patent US 5632989 |
| L19502 | Infectious bursal disease virus RNA polymerase (VP1) mRNA, complete cds |
| L32984 | Infectious bursal disease virus OH gene, 3' end of cds |
| L32985 | Infectious bursal disease virus OH gene, 3' end of cds |
| L32986 | Infectious bursal disease virus OH (OH) RNA, partial cds |
| L40429 | Gallid herpesvirus 2 ribonucleotide reductase large subunit (UL39), ribonucleotide reductase small subunit (UL40) and virion host shutoff protein (UL41) genes, three complete cds's |
| L42284 | Infectious bursal disease virus viral protein 2, viral protein 4 and viral protein 3 of segment A |
| M19336 | Infectious bursal disease virus of chickens, small ds-RNA genomic segment encoding a possible polymerase, complete cds |
| M64285 | Infectious bursal disease virus polyprotein (encoding VP2 and VP4) mRNA, 5'end |
| M66722 | Infectious bursal disease virus of chickens large RNA segment A VP2, VP3 and VP4 precursor mRNA, complete cds |
| M97346 | Infectious bursal disease virus VP2, VP3, VP4 genes, complete cds |
| NC_004178 | Infectious bursal disease virus segment A, complete sequence |
| NC_004179 | Infectious bursal disease virus segment B, complete sequence |
| S50730 | (strain STC genome homolog) [infectious bursal disease virus IBDV, E, Genomic RNA, 310 nt] |
| U20950 | Infectious bursal disease virus OH RNA polymerase (VP1) gene, complete cds |
| U30818 | Infectious bursal disease virus structural polyprotein gene, complete cds |
| U30819 | Infectious bursal disease virus RNA-directed RNA polymerase gene, complete cds |
| U62651 | infectious bursal disease virus VT1 RNA polymerase gene, complete cds |
| X03993 | Infectious bursal disease virus gene for polyprotein |
| X16107 | Infectious bursal disease virus (strain CU-1) VP5 gene for viral protein 5 and structural polyprotein |
| X54858 | Avian infectious bursal disease virus gene for polyprotein, genomic RNA |
| X79600 | Infectious bursal disease virus RNA for VP2 (IBDV 9064-16) |
| X79601 | Infectious bursal disease virus RNA for VP2 (IBDV 9064-17) |
| X79602 | Infectious bursal disease virus RNA for VP2 (IBDV 9147) |
| X84022 | Infectious bursal disease virus RNA for segment A, 3honcoding (23/82) |
| X84023 | Infectious bursal disease virus RNA for segment A, 5honcoding (23/82) |
| X84024 | Infectious bursal disease virus RNA for segment B, 3honcoding (23/82) |
| X84025 | Infectious bursal disease virus RNA for segment B, 5honcoding (23/82) |
| X84026 | Infectious bursal disease virus RNA for segment A, 3honcoding (Cu-1) |
| X84027 | Infectious bursal disease virus RNA for segment A, 5honcoding (23/82) |
| X84028 | Infectious bursal disease virus RNA for segment B, 3honcoding (Cu-1M) |
| X84029 | Infectious bursal disease virus RNA for segment B, 5honcoding (Cu-1M) |
| X84030 | Infectious bursal disease virus RNA for segment A, 3honcoding (Cu-1M) |
| X84031 | Infectious bursal disease virus RNA for segment A, 5honcoding (Cu-1) |
| X84032 | Infectious bursal disease virus RNA for segment B, 3honcoding (Cu-1) |
| X84033 | Infectious bursal disease virus RNA for segment B, 5honcoding (Cu-1) |
| X84034 | Infectious bursal disease virus RNA for small ORF (P2) |
| X84035 | Infectious bursal disease virus RNA for possible polymerase (P2) |
| X89570 | Infectious Bursal Disease Virus gene for VP2 protein, genomic RNA |
| X92760 | Infectious bursal disease virus gene for VP5 protein and VP2-4-3 polyprotein, genomic RNA |
| X92761 | Infectious bursal disease virus gene for VP1 RNA-dependent RNA polymerase, genomic RNA |
| X95883 | Infectious bursal disease virus VP2a gene |
| X96430 | Infectious bursal disease virus VP2 gene |
| X96472 | Infectious bursal disease virus mRNA for VP2 capsid protein |
| X96718 | Infectious bursal disease virus VP2 gene |
| Y14955 | Infectious bursal disease virus partial VP2 gene for structural protein VP2, genomic RNA, strain 94432 |
| Y14956 | Infectious bursal disease virus partial VP2 gene for structural protein VP2, genomic RNA, strain 89163 |
| Y14957 | Infectious bursal disease virus partial VP2 gene for structural protein VP2, genomic RNA, strain 91168 |
| Y14958 | Infectious bursal disease virus partial VP2 gene for structural protein VP2, genomic RNA, strain Faragher 52/70 |
| Y 14959 | Infectious bursal disease virus partial VP2 gene for structural protein VP2, genomic RNA, cell culture-adapted clone of variant A |
| Y14960 | Infectious bursal disease virus partial VP2 gene for structural protein VP2, genomic RNA, laboratory-selected cloned derivative of the Bursine2 strain |
| Y14961 | Infectious bursal disease virus partial VP2 gene for structural protein VP2, genomic RNA, laboratory-selected cloned derivative of the CT strain |
| Y14962 | Infectious bursal disease virus partial VP2 gene for structural protein VP2, genomic RNA, laboratory-selected cloned derivative of the D78 strain |
| Y14963 | Infectious bursal disease virus partial VP2 gene for structural protein VP2, genomic RNA, laboratory-selected IBDV strain EM3 |
| Y18612 | Infectious bursal disease virus VP2 gene, partial, isolate UP1/97 |
| Y18650 | Infectious bursal disease virus, VP2 gene, partial, isolate WB1/93 |
| Y18682 | Infectious bursal disease virus VP2 gene, partial, isolate RJ1/94 |
| 221971 | Infectious bursal disease virus ORF's |
| Z25480 | Infectious bursal disease virus VP2 protein, partial CDS |
| Z25481 | Infectious bursal disease virus VP2 protein, partial CDS |
| Z25482 | Infectious bursal disease virus VP2 protein, partial CDS |
| Z96993 | Infectious bursal disease virus partial VP2 gene for structural protein VP2, genomic RNA, isolate IBDVRF-12/96 |
| Z96994 | Infectious bursal disease virus partial VP2 gene for structural protein VP2, genomic RNA, isolate IBDVRF-1/93 |
| Z96995 | Infectious bursal disease virus partial VP2 gene for Structural protein VP2, genomic RNA, isolate IBDVRF-2/93 |
| Z96996 | Infectious bursal disease virus partial VP2 gene for structural protein VP2, genomic RNA, isolate IBDVRF-3/93 |
| Z96997 | Infectious bursal disease virus partial VP2 gene for structural protein VP2, genomic RNA, isolate IBDVRF-4/93 |
| Z96998 | Infectious bursal disease virus partial VP2 gene for structural protein VP2, genomic RNA, isolate IBDVRF-5/93 |
| Z96999 | Infectious bursal disease virus partial VP2 gene for structural protein VP2, genomic RNA, isolate IBDVRF-1/94 |
| Z97000 | Infectious bursal disease virus partial VP2 gene for structural protein VP2, genomic RNA, isolate IBDVRF-2/94 |
| Z97001 | Infectious bursal disease virus partial VP2 gene for structural protein VP2, genomic RNA, isolate IBDVRF-3/94 |
| Z97002 | Infectious bursal disease virus partial VP2 gene for structural protein VP2, genomic RNA, isolate IBDVRF-5/94 |
| Z97003 | Infectious bursal disease virus partial VP2 gene for structural protein VP2, genomic RNA, isolate IBDVRF-9/96 |
| Z97004 | Infectious bursal disease virus partial VP2 gene for structural protein VP2, genomic RNA, isolate IBDVRF-7/94 |
| Z97005 | Infectious bursal disease virus partial VP2 gene for structural protein VP2, genomic RNA, isolate IBDVRF-9/94 |
| Z97006 | Infectious bursal disease virus partial VP2 gene for structural protein VP2, genomic RNA, isolate IBDVRF-1/95 |
| Z97007 | Infectious bursal disease virus partial VP2 gene for structural protein VP2, genomic RNA. isolate IBDVRF-10/95 |
| Z97008 | Infectious bursal disease virus partial VP2 gene for structural protein VP2, genomic RNA, isolate IBDVRF-12/95 |
| Z97009 | Infectious bursal disease virus partial VP2 gene for structural protein VP2. genomic RNA, isolate IBDVRF-15/95 |
| Z97010 | Infections bursal disease virus partial VP2 gene for structural protein VP2, genomic RNA, isolate IBDVRF-1/96 |
| Z97011 | Infectious bursal disease virus partial VP2 gene for structural protein VP2, genomic RNA, isolate IBDVRF-2/96 |
| Z97012 | Infectious bursal disease virus partial VP2 gene for structural protein VP2, genomic RNA, isolate IBDVRF-6/96 |
| Z97013 | Infectious bursal disease virus partial VP2 gene for structural protein VP2, genomic RNA, isolate IBDVRF-8/96 |
| Z97014 | Infectious bursal disease virus partial VP2 gene for structural protein VP2, genomic RNA, isolate IBDVRF-14/96 |

**Table 3: Entrez Protein Accession Numbers of IBDV Amino Acid Sequences.**

| **Accession No.** | **Description** |
|---|---|
| A48546 | genome polyprotein - infectious bursal disease virus (strain A) (fragment) |
| AAA46237 | RNA polymerase |
| AAA46238 | polyprotein |
| AAA46239 | segment A polyprotein [Infections bursal disease virus] |
| AAA52086 | VP2, VP3, and VP4 |
| AAA58741 | OH |
| AAA58742 | OH |
| AAA80220 | RNA polymerase |
| AAA80556 | ribonucleotide reductase large subunit |
| AAA80557 | ribonucleotide reductase small subunit |
| AAA80558 | virion host shutoff protein |
| AAA89177 | possible polymerase |
| AAB22968 | VP2 [infectious bursal disease virus IBDV, serotype 2, Peptide Partial, 300 aa] |
| AAB46090 | Sequence 16 from patent US 5595912 |
| AAB46091 | Sequence 17 from patent US 5595912 |
| AAB46092 | Sequence 19 from patent US 5595912 |
| AAB46093 | Sequence 20 from patent US 5595912 |
| AAB46094 | Sequence 21 from patent US 5595912 |
| AAB46095 | Sequence 22 from patent US 5595912 |
| AAB46096 | Sequence 23 from patent US 5595912 |
| AAB46097 | Sequence 24 from patent US 5595912 |
| AAB46098 | Sequence 25 from patent US 5595912 |
| AAB46099 | Sequence 27 from patent US 5595912 |
| AAB46100 | Sequence 29 from patent US 5595912 |
| AAB46101 | Sequence 31 from patent US 5595912 |
| AAB46102 | Sequence 33 from patent US 5595912 |
| Lab46103 | Sequence 34 from patent US 5595912 |
| AAB63594 | structural protein Infectious bursal disease virus] |
| AAB68518 | VP1 RNA polymerase |
| AAB73033 | Sequence 2 from patent US 5632989 |
| AAC06016 | structural polyprotein VP2, VP4, VP3 [Infectious bursal disease virus] |
| AAC06017 | structural polyprotein VP2, VP4, VP3 [Infectious bursal disease virus] |
| AAC06018 | structural polyprotein VP2 precusor [Infectious bursal disease virus] |
| AAC36480 | viral protein 2 [Infectious bursal disease virus] |
| AAC36481 | viral protein 2 [Infectious bursal disease virus] |
| AAC36482 | viral protein 2 [Infectious bursal disease virus] |
| AAC55350 | orf |
| AAC55351 | structural polyprotein |
| AAC55352 | RNA-directed RNA polymerase |
| AAC72901 | VP2 protein [Infections bursal disease virus] |
| AAC72902 | VP2 protein [Infections bursal disease virus] |
| AAC72903 | VP2 protein [Infectious bursal disease virus] |
| AAC72904 | VP2 protein [Infectious bursal disease virus] |
| AAC72905 | VP2 protein [Infectious bursal disease virus] |
| AAC72906 | VP2 protein [Infectious bursal disease virus] |
| AAC72907 | VP2 protein [Infectious bursal disease virus] |
| AAC72908 | VP2 protein [Infectious bursal disease virus] |
| AAC72909 | VP2 protein [Infectious bursal disease virus] |
| AAC72910 | VP2 protein [Infectious bursal disease virus] |
| AAC72911 | VP2 protein [Infectious bursal disease virus] |
| AAC72912 | VP2 protein [Infectious bursal disease virus] |
| AAC72913 | VP2 protein [Infectious bursal disease virus] |
| AAC72914 | VP2 protein [Infectious bursal disease virus] |
| AAC78078 | VP5; nonstructural protein [Infectious bursal disease virus] |
| AAC78079 | polyprotein [Infectious bursal disease virus] |
| AAD23373 | structural polyprotein [Infectious bursal disease virus] |
| AAD23374 | VP5 [Infectious bursal disease virus] |
| AAD23375 | RNA-dependent RNA polymerase [Infectious bursal disease virus] |
| AAD25073 | major capsid protein VP2 [Infectious bursal disease virus] |
| AAD30136 | polyprotein [Infectious bursal disease virus] |
| AAD32617 | polyprotein VP0 [Infectious bursal disease virus] |
| AAD32618 | VP5 [Infectious bursal disease virus] |
| AAD32619 | double-stranded RNA-dependent RNA polymerase VP1 [Infectious bursal disease virus] |
| AAD36992 | polyprotein VP2 hypervariable region [Infectious bursal disease virus] |
| AAD36993 | polyprotein VP2 hypervariable region [Infectious bursal disease virus] |
| AAD36994 | polyprotein VP2 hypervariable region [Infectious bursal disease virus] |
| AAD43179 | polyprotein [Infectious bursal disease virus] |
| AAD44525 | structural polyprotein [Infectious bursal disease virus] |
| AAD48020 | VP5 [Infectious bursal disease virus] |
| AAD48021 | polyprotein [Infectious bursal disease virus] |
| AAD48022 | VP5 [Infectious bursal disease virus] |
| AAD48023 | polyprotein [Infectious bursal disease virus] |
| AAD48024 | VP5 [Infectious bursal disease virus] |
| AAD48025 | polyprotein [Infectious bursal disease virus] |
| AAD49775 | VP1 [Infectious bursal disease virus] |
| AAD49776 | VP1 [Infectious bursal disease virus] |
| AAD49777 | VP1 [Infectious bursal disease virus] |
| AAF07883 | VP2 [Infectious bursal disease virus] |
| AAF07884 | VP2 [Infectious bursal disease virus] |
| AAF07885 | VP2 [Infectious bursal disease virus] |
| AAF07886 | VP2 [Infectious bursal disease virus] |
| AAF07887 | VP2 [Infectious bursal disease virus] |
| AAF07888 | VP2 [Infectious bursal disease virus] |
| AAF07889 | VP2 [Infectious bursal disease virus] |
| AAF07890 | VP2 [Infectious bursal disease virus] |
| AAF07891 | VP2 [Infectious bursal disease virus] |
| AAF07892 | VP2 [Infectious bursal disease virus] |
| AAF07893 | VP2 [Infectious bursal disease virus] |
| AAF07894 | VP2 [Infectious bursal disease virus] |
| AAF07895 | VP2 [Infectious bursal disease virus] |
| AAF16081 | VP5 [Infectious bursal disease virus] |
| AAF16082 | polyprotein [Infectious bursal disease virus] |
| AAF16083 | VP1 [Infectious bursal disease virus] |
| AAF67802 | VP2 protein [Infectious bursal disease virus] |
| AAF67803 | VP2 protein [Infectious bursal disease virus] |
| AAF67804 | VP2 protein [Infectious bursal disease virus] |
| AAF67805 | VP2 protein [Infectious bursal disease virus] |
| AAF67806 | VP2 protein [Infectious bursal disease virus] |
| AAF67807 | VP2 protein [Infectious bursal disease virus] |
| AAF67808 | VP2 protein [Infectious bursal disease virus] |
| AAF67809 | VP2 protein [Infectious bursal disease virus] |
| AAF67810 | VP2 protein [Infectious bursal disease virus] |
| AAF85952 | viral protein 5 [Infectious bursal disease virus] |
| AAF85953 | polyprotein [Infectious bursal disease virus] |
| AAF85954 | viral protein 1 [Infectious bursal disease virus] |
| AAF86629 | structural protein [Infectious bursal disease virus] |
| AAF86630 | structural protein [Infectious bursal disease virus] |
| AAF86631 | structural protein [Infectious bursal disease virus] |
| AAF86632 | protein [Infectious bursal disease virus] |
| AAF86633 | structural protein [Infectious bursal disease virus] |
| AAF86634 | structural protein [Infectious bursal disease virus] |
| AAF86635 | structural protein [Infectious bursal disease virus] |
| AAF89210 | VP2 protein [Infectious bursal disease virus] |
| AAF91442 | viral protein 2; VP2 [Infectious bursal disease virus] |
| AAF91443 | viral protein 2; VP2 [Infectious bursal disease virus] |
| AAF98163 | VP2 protein [Infectious bursal disease virus] |
| AAF98164 | VP2 protein [Infectious bursal disease virus] |
| AAF98165 | VP2 protein [Infectious bursal disease virus] |
| AAF98166 | VP2 protein [Infectious bursal disease virus] |
| AAF98167 | VP2 protein [Infectious bursal disease virus] |
| AAF98168 | VP2 protein [Infectious bursal disease virus] |
| AAF98169 | VP2 protein [Infectious bursal disease virus] |
| AAF98170 | VP2 protein [Infectious bursal disease virus] |
| AAF98171 | VP2 protein [Infectious bursal disease virus] |
| AAF98172 | VP2 protein [Infectious bursal disease virus] |
| AAF98173 | VP2 protein [Infectious bursal disease virus] |
| AAG01809 | viral protein 2 variable region [Infectious bursal disease virus] |
| AAG01810 | viral protein 2 variable region [Infectious bursal disease virus] |
| AAG01811 | viral protein 2 variable region [Infectious bursal disease virus] |
| AAG01812 | viral protein 2 variable region [Infectious bursal disease virus] |
| AAG01813 | viral protein 2 variable region [Infectious bursal disease virus] |
| AAG01814 | viral protein 2 variable region [Infectious bursal disease virus] |
| AAG01815 | viral protein 2 variable region [Infectious bursal disease virus] |
| AAG01816 | viral protein 2 variable region [Infectious bursal disease virus] |
| AAG01817 | viral protein 2 variable region [Infectious bursal disease virus] |
| AAG01818 | viral protein 2 variable region [Infectious bursal disease virus] |
| AAG23824 | polyprotein [Infectious bursal disease virus] |
| AAG24574 | VP2 [Infectious bursal disease virus] |
| AAG24575 | VP2 [Infectious bursal disease virus] |
| AAG24576 | VP2 [Infectious bursal disease virus] |
| AAG24577 | VP2 [Infectious bursal disease virus] |
| AAG24578 | VP2 [Infectious bursal disease virus] |
| AAG24579 | VP2 [Infectious bursal disease virus] |
| AAG24580 | VP2 [Infectious bursal disease virus] |
| AAG24581 | VP2 [Infectious bursal disease virus] |
| AAG24582 | VP2 [Infectious bursal disease virus] |
| AAG24583 | VP2 [Infectious bursal disease virus] |
| AAG24584 | VP2 [Infectious bursal disease virus] |
| AAG24585 | VP2 [Infectious bursal disease virus] |
| AAG24586 | VP2 [Infectious bursal disease virus] |
| AAG24587 | VP2 [Infectious bursal disease virus] |
| AAG24588 | VP2 [Infectious bursal disease virus] |
| AAG24589 | VP2 [Infectious bursal disease virus] |
| AAG24590 | VP2 [Infectious bursal disease virus] |
| AAG24591 | VP2 [Infectious bursal disease virus] |
| AAG24592 | VP2 Infectious bursal disease virus] |
| AAG24593 | VP2 [Infectious bursal disease virus] |
| AAG24594 | VP2 [Infectious bursal disease virus] |
| AAG24595 | VP2 [Infectious bursal disease virus] |
| AAG24596 | VP2 [Infectious bursal disease virus] |
| AAG24597 | VP2 [Infectious bursal disease virus] |
| AAG24598 | VP2 [Infectious bursal disease virus] |
| AAG24599 | VP2 [Infectious bursal disease virus] |
| AAG24600 | VP2 [Infectious bursal disease virus] |
| AAG24601 | VP2 [Infectious bursal disease virus] |
| AAG24602 | VP2 [Infectious bursal disease virus] |
| AAG24603 | VP2 [Infectious bursal disease virus] |
| AAG24604 | VP2 [Infectious bursal disease virus] |
| AAG31694 | VP1 [Infectious bursal disease virus] |
| AAG40005 | VP5 [Infectious bursal disease virus] |
| AAG40006 | polyprotein [Infectious bursal disease virus] |
| AAG40007 | VP5 [Infectious bursal disease virus] |
| AAG40008 | polyprotein [Infectious bursal disease virus] |
| AAG40009 | VP5 [Infectious bursal disease virus] |
| AAG40010 | polyprotein [Infectious bursal disease virus] |
| AAG41195 | viral protein 2 [Infectious bursal disease virus] |
| AA041196 | viral protein [Infectious bursal disease virus] |
| AAG41197 | viral protein 2 [Infectious bursal disease virus] |
| AAG41198 | viral protein 2 [Infectious bursal disease virus] |
| AAG42305 | VP2 protein [Infectious bursal disease virus] |
| AAG42306 | VP2 protein [Infectious bursal disease virus] |
| AAG42307 | VP2 protein [Infectious bursal disease virus] |
| AAG42308 | VP2 protein [Infectious bursal disease virus] |
| AAG42309 | VP2 protein [Infectious bursal disease virus] |
| AAG42310 | VP2 protein [Infectious bursal disease virus] |
| AAG42311 | VP2 protein [Infectious bursal disease virus] |
| AAG42312 | VP2 protein [Infectious bursal disease virus] |
| AAG45001 | VP2 protein [Infectious bursal disease virus] |
| AAG45002 | VP2 protein [Infectious bursal disease virus] |
| AAG45238 | viral protein 2 [Infectious bursal disease virus] |
| AAG45239 | viral protein 2 [Infectious bursal disease virus] |
| AAG45240 | viral protein 2 [Infectious bursal disease virus] |
| AAG45241 | viral protein 2 [Infectious bursal disease virus] |
| AAG45242 | viral protein 2 [Infectious bursal disease virus] |
| AAG45243 | viral protein 2 [Infectious bursal disease virus] |
| AAG45244 | viral protein 2 [Infectious bursal disease virus] |
| AAG52759 | polyprotein VP2 [Infectious bursal disease virus] |
| AAG52760 | polyprotein VP2 [Infectious bursal disease virus] |
| AAG53939 | VP2 protein [Infectious bursal disease virus] |
| AAG60048 | polyprotein [Infectious bursal disease virus] |
| AAK12908 | VP2 protein [Infectious bursal disease virus] |
| AAK27323 | VP2 protein [Infectious bursal disease virus] |
| AAK30027 | RNA-dependent RNA polymerase [Infectious bursal disease virus] |
| AAK30028 | 17-kDa nonstructural protein [Infectious bursal disease virus] |
| AAK30029 | 110-kDa polyprotein [Infectious bursal disease virus] |
| AAK50615 | polyprotein [Infectious bursal disease virus] |
| AAK50616 | VP2 protein [Infectious bursal disease virus] |
| AAK51522 | polyprotein [Infectious bursal disease virus] |
| AAK51523 | RNA-dependent RNA-polymerase [Infectious bursal disease virus] |
| AAK69710 | RNA-dependent RNA polymerase VP1 [Infectious bursal disease virus] |
| AAK69711 | structural polyprotein [Infectious bursal disease virus] |
| AAK69712 | RNA-dependent RNA polymerase VP1 [Infectious bursal disease virus] |
| AAK69713 | structural polyprotein [Infectious bursal disease virus] |
| AAK69714 | RNA-dependent RNA polymerase VP1 [Infectious bursal disease virus] |
| AAK69715 | RNA-dependent RNA polymerase VP1 [Infectious bursal disease virus] |
| AAK69716 | structural polyprotein [Infectious bursal disease virus] |
| AAK72434 | VP5 [Infectious bursal disease virus] |
| AAK72435 | polyprotein [Infectious bursal disease virus] |
| AAL24821 | VP2 protein [Infectious bursal disease virus] |
| AAL35385 | polyprotein [Infectious bursal disease virus] |
| AAL46930 | VP2 protein [Infectious bursal disease virus] |
| AAL57864 | polyprotein [Infectious bursal disease virus] |
| AAL57865 | VP5 [Infectious bursal disease virus] |
| AAL57867 | RNA-dependent RNA polymerase VP1 [Infectious bursal disease virus] |
| AAL58578 | VP2 [Infectious bursal disease virus] |
| AAL58579 | VP2 [Infectious bursal disease virus] |
| AAL58580 | VP2 [Infectious bursal disease virus] |
| AAL58581 | VP2 [Infectious bursal disease virus] |
| AAL58582 | VP2 [Infectious bursal disease virus] |
| AAL75448 | VP2 [Infectious bursal disease virus] |
| AAL89461 | viral protein 2 [Infectious bursal disease virus] |
| AAL89462 | viral protein 2 [Infectious bursal disease virus] |
| AAL89463 | viral protein 2 [Infectious bursal disease virus] |
| AAL89464 | viral protein 2 [Infectious bursal disease virus] |
| AAL89465 | viral protein 2 [Infectious bursal disease virus] |
| AAL89466 | viral protein 2 [Infectious bursal disease virus] |
| AAL89467 | viral protein 2 [Infectious bursal disease virus] |
| AAL89468 | viral protein 2 [Infectious bursal disease virus] |
| AAL89469 | viral protein 2 [Infectious bursal disease virus] |
| AAL89624 | viral protein 2 [Infectious bursal disease virus] |
| AAL89625 | viral protein 2 [Infectious bursal disease virus] |
| AAL89626 | viral protein 2 [Infectious bursal disease virus] |
| AAL89627 | viral protein 2 [Infectious bursal disease virus] |
| AAL89628 | viral protein 2 [Infectious bursal disease virus] |
| AAL89629 | viral protein 2 [Infectious bursal disease virus] |
| AAL89630 | viral protein 2 [Infectious bursal disease virus] |
| AAL93206 | VP2 [Infectious bursal disease virus] |
| AAM09565 | viral protein 2 [Infectious bursal disease virus] |
| AAM11679 | RNA-dependent RNA polymerase VP1 [Infectious bursal disease virus] |
| AAM13411 | viral protein 2 [Infectious bursal disease virus] |
| AAM13412 | viral protein 2 [Infectious bursal disease virus] |
| AAM13413 | viral protein 2 [Infectious bursal disease virus] |
| AAM13414 | viral protein 2 [Infectious bursal disease virus] |
| AAM13415 | viral protein 2 [Infectious bursal disease virus] |
| AAM13416 | viral protein 2 [Infectious bursal disease virus] |
| AAM13417 | viral protein 2 [Infectious bursal disease virus] |
| AAM13418 | viral protein 2 [Infectious bursal disease virus] |
| AAM15645 | polyprotein [Infectious bursal disease virus] |
| AAM19248 | viral protein 2 [Infectious bursal disease virus] |
| AAM19336 | viral protein 2 [Infectious bursal disease virus] |
| AAM19337 | viral protein 2 [Infectious bursal disease virus] |
| AAM19338 | viral protein 2 [Infectious bursal disease virus] |
| AAM 19339 | viral protein 2 [Infectious bursal disease virus] |
| AAM19340 | viral protein 2 [Infectious bursal disease virus] |
| AAM21057 | viral protein 2 [Infectious bursal disease virus] |
| AAM21058 | viral protein 2 [Infectious bursal disease virus] |
| AAM21059 | viral protein 2 [Infectious bursal disease virus] |
| AAM21060 | viral protein 2 [Infectious bursal disease virus] |
| AAM21061 | viral protein 2 [Infectious bursal disease virus] |
| AAM21062 | viral protein 2 [Infectious bursal disease virus] |
| AAM21063 | viral protein 2 [Infectious bursal disease virus] |
| AAM21064 | viral protein 2 [Infectious bursal disease virus] |
| AAM21065 | viral protein 2 [Infectious bursal disease virus] |
| AAM21066 | viral protein 2 [Infectious bursal disease virus] |
| AAM21067 | viral protein 2 [Infectious bursal disease virus] |
| AAM21068 | viral protein 2 [Infectious bursal disease virus] |
| AAM21069 | viral protein 2 [Infectious bursal disease virus] |
| AAM21909 | viral protein 2 [Infectious bursal disease virus] |
| AAM21910 | viral protein 2 [Infectious bursal disease virus] |
| AAM21911 | viral protein 2 [Infectious bursal disease virus] |
| AAM21912 | viral protein 2 [Infectious bursal disease virus] |
| AAM21913 | viral protein 2 [Infectious bursal disease virus] |
| AAM21914 | viral protein 2 [Infectious bursal disease virus] |
| AAM21915 | viral protein 2 [Infectious bursal disease virus] |
| AAM21916 | viral protein 2 [Infectious bursal disease virus] |
| AAM21917 | viral protein 2 [Infectious bursal disease virus] |
| AAM21918 | viral protein 2 [Infectious bursal disease virus] |
| AAM21919 | viral protein 2 [Infectious bursal disease virus] |
| AAM21920 | viral protein 2 [Infectious bursal disease virus] |
| AAM28898 | polyprotein [Infectious bursal disease virus] |
| AAM28899 | viral protein 5 [Infectious bursal disease virus] |
| AAM28900 | VP2 [Infectious bursal disease virus] |
| AAM45383 | VP5 [Infectious bursal disease virus] |
| AAM45384 | polyprotein [Infectious bursal disease virus] |
| AAM45385 | VP1 [Infectious bursal disease virus] |
| AAM46155 | VP2 protein [Infectious bursal disease virus] |
| AAM46156 | VP2 protein [Infectious bursal disease virus] |
| AAM46157 | VP2 protein [Infectious bursal disease virus] |
| AAM46158 | VP2 protein [Infectious bursal disease virus] |
| AAM46159 | VP2 protein [Infectious bursal disease virus] |
| AAM46160 | VP2 protein [Infectious bursal disease virus] |
| AAM46161 | VP2 protein [Infectious bursal disease virus] |
| AAM46162 | VP2 protein [Infectious bursal disease virus] |
| AAM46163 | VP2 protein [Infectious bursal disease virus] |
| AAM46164 | VP2 protein [Infectious bursal disease virus] |
| AAM46165 | VP2 protein [Infectious bursal disease virus] |
| AAM46166 | VP2 protein [Infectious bursal disease virus] |
| AAM51641 | RNA-dependent RNA polymerase VP1 [Infectious bursal disease virus] |
| AAM76667 | VP2 protein [Infectious bursal disease virus] |
| AAM76668 | VP2 protein [Infectious bursal disease virus] |
| AAM76669 | VP2 protein [Infectious bursal disease virus] |
| AAM90732 | polyprotein [Infectious bursal disease virus] |
| AAM90733 | polyprotein [Infectious bursal disease virus] |
| AAM90734 | polyprotein [Infectious bursal disease virus] |
| AAM90735 | polyprotein [Infectious bursal disease virus] |
| AAM90736 | polyprotein [Infectious bursal disease virus] |
| AAM90791 | VP2 protein [Infectious bursal disease virus] |
| AAM90792 | VP2 protein [Infectious bursal disease virus] |
| AAM90793 | VP2 protein [Infectious bursal disease virus] |
| AAM90794 | VP2 protein [Infectious bursal disease virus] |
| AAM97561 | polyprotein [Infectious bursal disease virus] |
| AAM97562 | RNA-dependent RNA polymerase [Infectious bursal disease virus] |
| AAN04459 | RNA-dependent RNA polymerase [Infectious bursal disease virus] |
| AAN04460 | VP5 [Infectious bursal disease virus] |
| AAN04461 | polyprotein [Infectious bursal disease virus] |
| AAN04462 | RNA-dependent RNA polymerase [Infectious bursal disease virus] |
| AAN04902 | polyprotein [Infectious bursal disease virus] |
| AAN04903 | polyprotein [Infectious bursal disease virus] |
| AAN04904 | polyprotein [Infectious bursal disease virus] |
| AAN04905 | polyprotein [Infectious bursal disease virus] |
| AAN52491 | VP5 protein [Infectious bursal disease virus] |
| AAN52492 | polyprotein [Infectious bursal disease virus] |
| AAN52493 | VP1 protein [Infectious bursal disease virus] |
| AAN87130 | OH [Infectious bursal disease virus] |
| AAO15767 | VP5 [Infectious bursal disease virus] |
| AAO15768 | polyprotein [Infectious bursal disease virus] |
| AAO15847 | immunoglobulin G single chain variable fragment [synthetic construct] |
| AAO15848 | immunoglobulin G single chain variable fragment [synthetic construct] |
| AAO15849 | immunoglobulin G single chain variable fragment [synthetic construct] |
| AAO15850 | immunoglobulin G single chain variable fragment [synthetic construct] |
| AAO15851 | immunoglobulin G single chain variable fragment [synthetic construct] |
| AAO15852 | immunoglobulin G single chain variable fragment [synthetic construct] |
| AAO15853 | immunoglobulin G single chain variable fragment [synthetic construct] |
| AAO15854 | immunoglobulin G single chain variable fragment [synthetic construct] |
| AAO15855 | immunoglobulin G single chain variable fragment [synthetic construct] |
| AAO15856 | immunoglobulin G single chain variable fragment [synthetic construct] |
| AAO15857 | immunoglobulin G single chain variable fragment [synthetic construct] |
| AAO15858 | immunoglobulin G single chain variable fragment [synthetic construct] |
| AAO15859 | immunoglobulin G single chain variable fragment [synthetic construct] |
| AAO15860 | immunoglobulin G single chain variable fragment [synthetic construct] |
| AAO15861 | immunoglobulin G single chain variable fragment [synthetic construct] |
| AAO15862 | immunoglobulin G single chain variable fragment [synthetic construct] |
| AAO15863 | immunoglobulin G single chain variable fragment [synthetic construct] |
| AAO15864 | immunoglobulin G single chain variable fragment [synthetic construct] |
| AAO15865 | immunoglobulin G single chain variable fragment [synthetic construct] |
| AAO15883 | VP2 protein [Infectious bursal disease virus] |
| AAO15769 | RNA-dependent RNA polymerase [Infectious bursal disease virus] |
| AAO15884 | VP2 protein [Infectious bursal disease virus] |
| AAO15885 | VP2 protein [Infectious bursal disease virus] |
| AAO15886 | VP2 protein [Infectious bursal disease virus] |
| AAO15887 | VP2 protein [Infectious bursal disease virus] |
| AAO15888 | VP2 protein [Infectious bursal disease virus] |
| AAO15889 | VP2 protein [Infectious bursal disease virus] |
| AAO15890 | VP2 protein [Infectious bursal disease virus] |
| AAO15891 | VP2 protein [Infectious bursal disease virus] |
| AAO15892 | VP2 protein [Infectious bursal disease virus] |
| AAO15893 | VP2 protein [Infectious bursal disease virus] |
| AAO15894 | VP2 protein [Infectious bursal disease virus] |
| AAO15895 | VP2 protein [Infectious bursal disease virus] |
| AAO15896 | VP2 protein [Infectious bursal disease virus] |
| AAO15897 | VP2 protein [Infectious bursal disease virus] |
| AAO15898 | VP2 protein [Infectious bursal disease virus] |
| AAO16168 | VP2 [Infectious bursal disease virus] |
| AAO16169 | VP2 [Infectious bursal disease virus] |
| AAO16170 | VP2 [Infectious bursal disease virus] |
| AAO16171 | VP2 [Infectious bursal disease virus] |
| AAO16172 | VP2 [Infectious bursal disease virus] |
| AAO16173 | VP2 [Infectious bursal disease virus] |
| AAO16174 | VP2 [Infectious bursal disease virus] |
| AAO16175 | VP2 [Infectious bursal disease virus] |
| AAO16176 | VP2 [Infectious bursal disease virus] |
| AAO49501 | polyprotein [Infectious bursal disease virus] |
| AAO86512 | VP2 protein [Infectious bursal disease virus] |
| AAP29956 | VP2 protein [Infectious bursal disease virus] |
| AAP29957 | VP2 protein [Infectious bursal disease virus] |
| AAP29958 | VP2 protein [Infectious bursal disease virus] |
| AAP29959 | VP2 protein [Infectious bursal disease virus] |
| AAP29960 | VP2 protein [Infectious bursal disease virus] |
| AAP29961 | VP2 protein [Infectious bursal disease virus] |
| AAP29962 | VP2 protein [Infectious bursal disease virus] |
| AAP29963 | VP2 protein [Infectious bursal disease virus] |
| AAP46104 | VP2 [Infectious bursal disease virus] |
| AAP461105 | VP2 [Infectious bursal disease virus] |
| AAP68822 | viral protein 2 [Infectious bursal disease virus] |
| AAP68823 | viral protein 2 [Infectious bursal disease virus] |
| AAP75635 | structural protein VP2 [Infectious bursal disease virus] |
| AAP79442 | VP2 protein hypervariable region [Infectious bursal disease virus] |
| AAP83585 | polyprotein [Infectious bursal disease virus] |
| AAP83586 | VP5 [Infectious bursal disease virus] |
| AAP84061 | VP5 [Infectious bursal disease virus] |
| AAP84062 | VP2 [Infectious bursal disease virus] |
| AAP84365 | VP2 [Infectious bursal disease virus] |
| AAP84366 | VP2 [Infectious bursal disease virus] |
| AAP84367 | VP2 [Infectious bursal disease virus] |
| AAP84368 | VP2 [Infectious bursal disease virus] |
| AAP84369 | VP2 [Infectious bursal disease virus] |
| AAP84370 | VP2 [Infectious bursal disease virus] |
| AAP84371 | VP2 [Infectious bursal disease virus] |
| AAP84372 | VP2 [Infectious bursal disease virus] |
| AAP84373 | VP2 [Infectious bursal disease virus] |
| AAP85291 | structural protein VP5 [Infectious bursal disease virus] |
| AAP85292 | polyprotein [Infectious bursal disease virus] |
| AAP94894 | VP2 [Infectious bursal disease virus] |
| AAP94895 | VP2 [Infectious bursal disease virus] |
| AAP94896 | VP2 [Infectious bursal disease virus] |
| AAP94897 | VP2 [Infectious bursal disease virus] |
| AAQ00946 | VP2 protein [Infectious bursal disease virus] |
| AAQ00947 | VP2 protein [Infectious bursal disease virus] |
| AAQ00948 | VP2 protein [Infectious bursal disease virus] |
| AAQ75525 | VP2 protein [Infectious bursal disease virus] |
| AAQ99274 | VP2 [Infectious bursal disease virus] |
| BAA00390 | unnamed protein product [Infectious bursal disease virus] |
| BAA00391 | unnamed protein product [Infectious bursal disease virus] |
| BAA00740 | 110kD polyprotein [Infectious bursal disease virus] |
| BAA00741 | 110kD polyprotein [Infectious bursal disease virus] |
| BAA00742 | unnamed protein product [Infectious bursal disease virus] |
| BAA00743 | unnamed protein product [Infectious bursal disease virus] |
| BAA00744 | unnamed protein product [Infectious bursal disease virus] |
| BAA00745 | 110kD polyprotein [Infectious bursal disease virus] |
| BAA00954 | VP2 [Infectious bursal disease virus] |
| BAA02135 | VP1 [Infectious bursal disease virus] |
| BAA04056 | polyprotein [Infectious bursal disease virus] |
| BAA04083 | polyprotein [Infectious bursal disease virus] |
| BAA04108 | polyprotein [Infectious bursal disease virus] |
| BAA08555 | polyprotein [Infectious bursal disease virus] |
| BAA12175 | polyprotein [Infectious bursal disease virus] |
| BAA12211 | polyprotein [Infectious bursal disease virus] |
| BAA12212 | polyprotein [Infectious bursal disease virus] |
| BAA21001 | polyprotein [Infectious bursal disease virus] |
| BAA23207 | RNA polymerase [Infectious bursal disease virus] |
| BAA25201 | polyprotein [Infectious bursal disease virus] |
| BAA25202 | polyprotein [Infectious bursal disease virus] |
| BAA87931 | VP2-4-3 polyprotein [Infectious bursal disease virus] |
| BAA89953 | VP1 [Infectious bursal disease virus] |
| BAA89954 | VP1 [Infectious bursal disease virus] |
| BAA89955 | VP1 [Infectious bursal disease virus] |
| BAA89956 | VP1 [Infectious bursal disease virus] |
| BAA89957 | VP1 [Infectious bursal disease virus] |
| BAA89958 | VP1 [Infectious bursal disease virus] |
| BAA89959 | VP1 [Infectious bursal disease virus] |
| BAA89960 | VP1 [Infectious bursal disease virus] |
| CAA01045 | large RNA segment [Infectious bursal disease virus] |
| CAA02133 | segment A ORF 3 [Infectious bursal disease virus] |
| CAA02134 | segment A ORF 2 [Infectious bursal disease virus] |
| CAA02135 | segment A ORF 1 [Infectious bursal disease virus] |
| CAA02337 | unnamed protein product [Infectious bursal disease virus] |
| CAA05110 | capsid protein, VP2, variable domain [Infectious bursal disease virus] |
| CAA05111 | capsid protein, VP2, variable domain [Infectious bursal disease virus] |
| CAA05112 | capsid protein, VP2, variable domain [Infectious bursal disease virus] |
| CAA05113 | capsid protein, VP2, variable domain [Infectious bursal disease virus] |
| CAA05114 | capsid protein, VP2, variable domain [Infectious bursal disease virus] |
| CAA05115 | capsid protein, VP2, variable domain [Infectious bursal disease virus] |
| CAA05116 | capsid protein, VP2, variable domain [Infectious bursal disease virus] |
| CAA05117 | capsid protein, VP2, variable domain [Infectious bursal disease virus] |
| CAA27629 | polyprotein [Infectious bursal disease virus] |
| CAA34233 | viral protein 5 [Infectious bursal disease virus (strain CU-1)] |
| CAA34234 | structural polyprotein [Infectious bursal disease virus (strain CU-1)] |
| CAA38637 | polyprotein [Infectious bursal disease virus] |
| CAA58850 | unnamed protein product [Infectious bursal disease virus] |
| CAA58851 | unnamed protein product [Infectious bursal disease virus] |
| CAA58852 | unnamed protein product [Infectious bursal disease virus] |
| CAA61749 | VP2 structural protein hypervariable region [Infectious bursal disease virus] |
| CAA63415 | VP5 [Infectious bursal disease virus] |
| CAA63416 | VP2-4-3 polyprotein [Infectious bursal disease virus] |
| CAA63417 | VP1 RNA polymerase [Infectious bursal disease virus] |
| CAA65132 | VP2a protein [Infectious bursal disease virus] |
| CAA65290 | VP2 capsid protein [Infectious bursal disease virus] |
| CAA65326 | VP2 capsid protein [Infectious bursal disease virus] |
| CAA65479 | VP2 protein [Infectious bursal disease virus] |
| CAA75177 | structural protein VP2 [Infectious bursal disease virus] |
| CAA75178 | structural protein VP2 [Infectious bursal disease virus] |
| CAA75179 | structural protein VP2 [Infectious bursal disease virus] |
| CAA75180 | structural protein VP2 [Infectious bursal disease virus] |
| CAA75181 | structural protein VP2 [Infectious bursal disease virus] |
| CAA75182 | structural protein VP2 [Infectious bursal disease virus] |
| CAA75183 | structural protein VP2 [Infectious bursal disease virus] |
| CAA75184 | structural protein VP2 [Infectious bursal disease virus] |
| CAA75185 | structural protein VP2 [Infectious bursal disease virus] |
| CAA79982 | unnamed protein product [Infectious bursal disease virus] |
| CAA79983 | unnamed protein product [Infectious bursal disease virus] |
| CAA80968 | VP2 [Infectious bursal disease virus] |
| CAA80969 | VP2 [Infectious bursal disease virus] |
| CAA80970 | VP2 [Infectious bursal disease virus] |
| CAB09667 | structural protein VP2 [Infectious bursal disease virus] |
| CAB09668 | structural protein VP2 [Infectious bursal disease virus] |
| CAB09669 | structural protein VP2 [Infectious bursal disease virus] |
| CAB09670 | structural protein VP2 [Infectious bursal disease virus] |
| CAB09671 | structural protein VP2 [Infectious bursal disease virus] |
| CAB09672 | structural protein VP2 [Infectious bursal disease virus] |
| CAB09673 | structural protein VP2 [Infectious bursal disease virus] |
| CAB09674 | structural protein VP2 [Infectious bursal disease virus] |
| CAB09675 | structural protein VP2 [Infectious bursal disease virus] |
| CAB09676 | structural protein VP2 [Infectious bursal disease virus] |
| CAB09677 | structural protein VP2 [Infectious bursal disease virus] |
| CAB09678 | structural protein VP2 [Infectious bursal disease virus] |
| CAB09679 | structural protein VP2 [Infectious bursal disease virus] |
| CAB09680 | structural protein VP2 [Infectious bursal disease virus] |
| CAB09681 | structural protein VP2 [Infectious bursal disease virus] |
| CAB09682 | structural protein VP2 [Infectious bursal disease virus] |
| CAB09683 | structural protein VP2 [Infectious bursal disease virus] |
| CAB09684 | structural protein VP2 [Infectious bursal disease virus] |
| CAB09685 | structural protein VP2 [Infectious bursal disease virus] |
| CAB09686 | structural protein VP2 [Infectious bursal disease virus] |
| CAB09687 | structural protein VP2 [Infectious bursal disease virus] |
| CAB09688 | structural protein VP2 [Infectious bursal disease virus] |
| CAB41892 | VP2 protein [Infectious bursal disease virus] |
| CAB41893 | VP2 protein [Infectious bursal disease virus] |
| CAB41894 | VP2 protein [Infectious bursal disease virus] |
| CAB55814 | VP2 protein [Infectious bursal disease virus] |
| CAB55815 | VP2 protein [Infectious bursal disease virus] |
| CAB62394 | viral capsid protein 2 [Infectious bursal disease virus] |
| CAB65129 | structural protein (VP2) [Infectious bursal disease virus] |
| CAB65130 | structural protein (VP2) [Infectious bursal disease virus] |
| CAB65131 | structural protein (VP2) [Infectious bursal disease virus] |
| CAB65132 | structural protein (VP2) [Infectious bursal disease virus] |
| CAB65133 | VP2 protein [Infectious bursal disease virus] |
| CAB65134 | VP2 protein [Infectious bursal disease virus] |
| CAB65135 | VP2 protein [Infectious bursal disease virus] |
| CAB65136 | VP2 protein [Infectious bursal disease virus] |
| CAB65137 | VP2 protein [Infectious bursal disease virus] |
| CAB65138 | VP2 protein [Infectious bursal disease virus] |
| CAB90215 | structural protein VP2 [Infectious bursal disease virus] |
| CAB93983 | structural protein VP2 [Infectious bursal disease virus] |
| CAC13128 | structural protein VP2 [Infectious bursal disease virus] |
| CAC17775 | VP1 protein [Infectious bursal disease virus] |
| CAC17776 | VP1 protein [Infectious bursal disease virus] |
| CAC17777 | VP1 protein [Infectious bursal disease virus] |
| CAC17778 | VP1 protein [Infectious bursal disease virus] |
| CAC17779 | VP1 protein [Infectious bursal disease virus] |
| CAC17780 | VP1 protein [Infectious bursal disease virus] |
| CAC17781 | VP1 protein [Infectious bursal disease virus] |
| CAC17782 | VP1 protein [Infectious bursal disease virus] |
| CAC35320 | RNA-dependent RNA-polymerase [Infectious bursal disease virus] |
| CAC35469 | VP5 protein [Infectious bursal disease virus] |
| CAC35470 | polyprotein [Infectious bursal disease virus] |
| CAC59949 | VP2 protein [Infectious bursal disease virus] |
| CAC59950 | VP2 protein [Infectious bursal disease virus] |
| CAC59951 | VP2 protein [Infectious bursal disease virus] |
| CAC60254 | VP2 variable region [Infectious bursal disease virus] |
| CAC60256 | polyprotein [Infectious bursal disease virus] |
| CAC60257 | VP1 protein [Infectious bursal disease virus] |
| CAC94911 | host protective antigen [Infectious bursal disease virus] |
| CAC94912 | host protective antigen [Infectious bursal disease virus] |
| CAD20409 | unnamed protein product [Infectious bursal disease virus] |
| CAD22374 | unnamed protein product [Infectious bursal disease virus] |
| CAD22375 | unnamed protein product [Infectious bursal disease virus] |
| CAD24856 | polyprotein [Infectious bursal disease virus] |
| CAD24857 | VP5 protein [Infectious bursal disease virus] |
| CAD43216 | RNA polymerase [Infectious bursal disease virus] |
| CAD43217 | structural protein VP2 [Infectious bursal disease virus] |
| CAD90224 | unnamed protein product [Infectious bursal disease virus] |
| CAD90225 | unnamed protein product [Infectious bursal disease virus] |
| CAE11792 | VP2 structural protein [Infectious bursal disease virus] |
| CAE52917 | VP2 [Infectious bursal disease virus] |
| CAE52918 | VP2 [Infectious bursal disease virus] |
| CAE52919 | VP2 [Infectious bursal disease virus] |
| CAE52920 | VP2 [Infectious bursal disease virus] |
| CAE52921 | VP2 [Infectious bursal disease virus] |
| CAE52922 | VP2 [Infectious bursal disease virus] |
| CAE52923 | VP2 [Infectious bursal disease virus] |
| CAE52924 | VP2 [Infectious bursal disease virus] |
| CAE52925 | VP2 [Infectious bursal disease virus] |
| CAE52926 | VP2 [Infectious bursal disease virus] |
| CAE52927 | VP2 [Infectious bursal disease virus] |
| CAE52928 | VP2 [Infectious bursal disease virus] |
| CAE52929 | VP2 [Infectious bursal disease virus] |
| CAE52930 | VP2 [Infectious bursal disease virus] |
| CAE52931 | VP2 [Infectious bursal disease virus] |
| CAE52932 | VP2 [Infectious bursal disease virus] |
| CAE52933 | VP2 [Infectious bursal disease virus] |
| CAE52934 | VP2 [Infectious bursal disease virus] |
| CAE52935 | VP2 [Infectious bursal disease virus] |
| CAE52936 | VP2 [Infectious bursal disease virus] |
| CAE52937 | VP2 [Infectious bursal disease virus] |
| CAE52938 | VP2 [Infectious bursal disease virus] |
| CAE52939 | VP2 [Infectious bursal disease virus] |
| CAE52940 | VP2 [Infectious bursal disease virus] |
| CAE52941 | VP2 [Infectious bursal disease virus] |
| CAE52942 | VP2 [Infectious bursal disease virus] |
| CAE52943 | VP2 [Infectious bursal disease virus] |
| CAE52944 | VP2 [Infectious bursal disease virus] |
| CAE52945 | VP2 [Infectious bursal disease virus] |
| CAE52946 | VP2 [Infectious bursal disease virus] |
| CAE52947 | VP2 [Infectious bursal disease virus] |
| CAE52948 | VP2 [Infectious bursal disease virus] |
| CAE52949 | VP2 [Infectious bursal disease virus] |
| CAE52950 | VP2 [Infectious bursal disease virus] |
| CAE52951 | VP2 [Infectious bursal disease virus] |
| CAE52952 | VP2 [Infectious bursal disease virus] |
| CAE52953 | VP2 [Infectious bursal disease virus] |
| GAE52954 | VP2 [Infectious bursal disease virus] |
| CAE52955 | VP2 [Infectious bursal disease virus] |
| CAE52956 | VP2 [Infectious bursal disease virus] |
| CAE52957 | VP2 [Infectious bursal disease virus] |
| CAE52958 | VP2 [Infectious bursal disease virus] |
| CAE52959 | VP2 [Infectious bursal disease virus] |
| CAE52960 | VP2 [Infectious bursal disease virus] |
| CAE52961 | VP2 [Infectious bursal disease virus] |
| CAE52962 | VP2 [Infectious bursal disease virus] |
| CAE52963 | VP2 [Infectious bursal disease virus] |
| CAE52964 | VP2 [Infectious bursal disease virus] |
| CAE52965 | VP2 [Infectious bursal disease virus] |
| CAE52966 | VP2 [Infectious bursal disease virus] |
| CAE52967 | VP2 [Infectious bursal disease virus] |
| CAE52968 | VP2 [Infectious bursal disease virus] |
| CAE52969 | VP2 [Infectious bursal disease virus] |
| CAE52970 | VP2 [Infectious bursal disease virus] |
| GNXS52 | genome polyprotein - infectious bursal disease virus (strain 52/70) |
| GNXS98 | genome polyprotein infectious bursal disease virus (strain PBG98) |
| GNXSAU | genome polyprotein - infectious bursal disease virus (strain 002-73) |
| GNXSCU | genome polyprotein - infectious bursal disease virus (strain Cu-1) |
| GNXSIE | genome polyprotein - infectious bursal disease virus (strain E) (fragment) |
| GNXSIR | genome polyprotein - infectious bursal disease virus (strain STC) |
| GNXSOH | genome polyprotein - infectious bursal disease virus (strain OH) |
| JC1327 | protective antigen VP2 - infectious bursal disease virus |
| JQ0942 | hypothetical 17K protein - infectious bursal disease virus (strain 52/70) |
| JQ0943 | hypothetical 1.3K protein - infectious bursal disease virus (strain 52/70) |
| JQ2197 | major structural protein VP2 - infectious bursal disease virus (strain 23/82, serotype II) |
| JQ2198 | major structural protein VP2 - infectious bursal disease virus (strain Cu-1, serotype I) |
| JS0359 | hypothetical 16.6K protein - infectious bursal disease virus |
| NP_690837 | VP5 protein [Infectious bursal disease virus] |
| NP_690838 | VP2-4-3 polyprotein [Infectious bursal disease virus] |
| NP_690839 | VP1 RNA-dependent RNA polymerase [Infectious bursal disease virus] |
| P08364 | Structural polyprotein [Contains: Major structural protein VP2; Nonstructural protein VP4; Minor structural protein VP3] |
| P12918 | Putative RNA-directed RNA polymerase (VP1 protein) (RDRP) |
| P15480 | Structural polyprotein [Contains: Major structural protein VP2; Nonstructural protein VP4; Minor structural protein VP3] |
| P15481 | HYPOTHETICAL 16.6 KD PROTEIN |
| P22173 | Putative RNA-directed RNA polymerase (VP1 protein) (RDRP) |
| P22174 | Putative RNA-directed RNA polymerase (VP1 protein) (RDRP) |
| P22351 | Structural polyprotein [Contains: Major structural protein VP2; Nonstructural protein VP4; Minor structural protein VP3] |
| P22440 | HYPOTHETICAL 16.6 KD PROTEIN |
| P25219 | Structural polyprotein [Contains: Major structural protein VP2; Nonstructural protein VP4; Minor structural protein VP3] |
| P25220 | Structural polyprotein [Contains: Major structural protein VP2; Nonstructural protein VP4; Minor structural protein VP3] |
| P25221 | HYPOTHETICAL 16.6 KD PROTEIN |
| P25222 | HYPOTHETICAL 16.6 KD PROTEIN |
| P27276 | Structural polyprotein [Contains: Major structural protein VP2; Nonstructural protein VP4; Minor structural protein VP3] |
| P29802 | Structural polyprotein [Contains: Major structural protein VP2; Nonstructural protein VP4] |
| P31817 | Putative RNA-directed RNA polymerase (VP1 protein) (RDRP) |
| RRXSI5 | RNA-directed RNA polymerase (EC 2.7.7.48) - infectious bursal disease virus (strain 52/70) (fragment) |
| RRXSIB | RNA-directed RNA polymerase (EC 2.7.7.48) - infectious bursal disease virus |
| S06090 | hypothetical protein I - infectious bursal disease virus (strains Cu-1 and PBG-98) |
| S32213 | hypothetical protein - infectious bursal disease virus |
| S32214 | hypothetical protein - infectious bursal disease virus |
| S36415 | structural protein VP2 - infectious bursal disease virus (strain U. K.) (fragment) |
| S36418 | structural protein VP2 - infectious bursal disease virus (isolate Netherlands) (fragment) |
| S58005 | structural protein VP2 - infectious bursal disease virus (fragment) |
| S71934 | genome polyprotein - infectious bursal disease virus (strain E/DEL) (fragment) |

**Reference Example 7: Proventriculitis In Broiler Chickens And Its Relationship To IBDV**

ABSTRACT. **Proventriculitis** in broilers causes carcass condemnation because of contamination when swollen proventriculi tear during evisceration. Although the cause of proventriculitis is unknown, infectious bursal disease virus (IBDV) has been implicated. To study the role of IBDV in proventriculitis, Proventriculi and bursas were collected from chickens with naturally occurring proventriculitis, and from chickens experimentally infected with seven different IBDV strains. All tissues were examined for IBDV using light microscopy, immunohistochemistry (DHC), real time RT-PCR, and for apoptosis by TUNEL method. We concluded that proventriculitis can occur in the absence of IBDV, and that the IBDV strains tested do not directly produce proventriculitis.

Proventriculitis was studied by experimentally reproducing the disease in commercial and specific pathogen free (SPF) broilers. Differences in weight gain, organ weights, and the presence of lesions between these birds and controls were assessed. Bacteria were not identified in histological sections of proventriculi nor were they isolated from affected proventriculi. Attempted virus isolation from affected proventriculi caused stunting in inoculated embryos, and infectious bronchitis virus (IBV) was detected in allantoic fluid. Proventricular homogenates used to induce proventriculitis were positive for IBDV, IBV, adenovirus, and chicken anemia virus (CAV), but proventriculitis could also be produced in chickens in the absence of these viruses.

Immunosuppression was induced in broiler chickens using chemicals (cyclophosphamide and cyclosporin) or virus (IBDV) to study the effect of immunosuppression on proventriculitis. Cyclophosphamide and IBDV, both B cell suppressors, did not significantly affect the incidence or characteristics of the proventriculitis induced with a proventricular homogenate from a diseased bird. However, an increase in the size of the proventriculus was observed at 7 days post inoculation. Chickens immunosuppressed with cyclosporin, a T cell suppressor, developed more severe lesions and had a higher incidence of proventriculitis than immunocompetent controls. Although both, B and T cells, are involved in the immune response against proventriculitis, it appears that cell mediated immunity plays a more important role. This was also supported by the lymphocytic infiltrate observed in diseased proventricular glands. CD8+ T lymphocytes were the most common cell type and were widely distributed in the proventriculus, whereas CD4+ T cells and B cells tended to form aggregates in the chronic stages of the disease.

INTRODUCTION: Purpose of the Study. **Proventriculitis** is a naturally occurring disease that affects commercial chickens. Damaged proventriculi are enlarged, swollen and filled with fluid and feed and often rupture during routine evisceration causing contamination of the carcass (2, 14). The main economic impact of this disease is due to condemnation of these contaminated carcasses, although proventriculitis also has been associated with impaired growth, poor feed conversion, intestinal fragility, stunting syndrome and passage of undigested feed (1, 4, 14, 16, 21, 27). The poultry industry reports sporadic, thought economically important, outbreaks of proventriculitis in broilers (14), and the condition appears more common in younger birds, processed at 4 to 5 weeks of age (2).

Potential noninfectious causes of proventriculitis include oral exposure to biogenic amines (10, 20, 23), mycotoxins (5, 7, 11), lack of dietary fiber (20, 25), and excessive copper sulfate (3, 15). Possible infectious causes include adenovirus (16, 18), reovirus (17, 18, 21), infectious bronchitis virus (30), and megabacterium (12, 13, 19, 22). However, none of these noninfectious or infectious agents have been found in a majority of cases. Electron microscopy has detected viral particles in acute lesions but isolation of this virus from affected proventriculi has been unsuccessful (8, 9, 14).

Infectious Bursal Disease Virus (IBDV) has been implicated as the cause for this disease (2, 14) and IBDV vaccination decreases its incidence (6). Proventriculitis has been reproduced by orally inoculating broilers with homogenized proventriculi collected from affected birds (2, 14, 24). A filterable agent found in these homogenates causes lesions similar to those found in field cases (9, 14) and IBDV has been immunoprecipitated from these homogenates (14). Commercial broilers exposed to this IBDV developed increased proventricular lesion scores but had no increase in proventricular size, a characteristic feature produced by exposure to infectious proventricular homogenates (14).

Infectious Bursal Disease (IBD) is an acute, highly contagious viral disease in chickens which produces necrosis of lymphocytes in the bursa of Fabricius followed by immunosuppression (28). Classical strains of IBDV produced lesions in the proventriculus of specific pathogen free (SPF) leghorns (29). However, using *in situ* hybridization staining with riboprobes specific for the VP2 gene of IBDV, no virus was detected in the proventriculi of 3 week-old chickens experimentally exposed to IBDV strains Delaware A, D78 or Bursavac® and no histologicaly evident proventricular lesions were present (26).

The objective of this study was to determine the role of IBDV in proventriculitis, and better understand the pathogenesis and possible causes of proventriculitis.

Objectives and Originality. Recent investigations have implicated IBDV as a potential cause of proventriculitis. Variant strains of IBDV have been isolated from proventricular homogenates from diseased birds, and SPF leghorns and broilers inoculated with these homogenates, develop a disease typical of IBDV infection, as well as proventriculitis. Furthermore, vaccination against IBDV reduces the incidence of proventriculitis, but does not eliminate it. Although indirect evidence exists, the definite role of IBDV in proventriculitis has not been determined. It is possible that a new variant IBDV could be the direct cause of the disease, or it may be that IBDV, by its immunosuppressive effect, allows some other agent to produce the disease. This research was designed to investigate the role of IBDV as the causative agent of proventriculitis in chickens.

The first objective was to determine if IBDV either directly, or indirectly by inducing apoptosis, causes proventriculitis in chickens. To address this, the proventriculi and bursas of chickens with naturally occurring proventriculitis as well as those from SPF broilers experimentally infected with multiple strains of IBDV were examined. The presence of IBDV in these tissues was determined by RT-PCR and IHC for viral gene sequences and viral antigen, respectively. The presence of apoptosis was examined by a modified TUNEL method, and lesions induced by the virus were examined by histopathology.

The second objective was to reproduce proventriculitis and characterize the changes present in the proventriculus and other organs. To accomplish this proventriculi and other organs after experimental induction of proventriculitis in commercial and SPF broiler chickens were examined. In an attempt to identified possible causative agents involved in proventriculitis, including IBDV, molecular, bacteriological, serological, and histopathological methods, and electron microscopy were undertaken.

The purpose of the third study was to investigate if immunosuppression had an effect on the incidence, severity, or character of proventriculitis in broiler chickens. IBDV induces immunosuppression in chickens, which may play a role in the pathogenicity of proventriculitis. To address this objective, one-day-old commercial and SPF broilers were immunosuppressed with cyclophosphamide (B cell suppressor), cyclosporin (T cell suppressor), or IBDV. Subsequently these chickens were exposed to a proventricular homogenate from affected chickens, and the effect of immunosuppression on proventriculitis was determined.

The main histological finding in transmissible proventriculitis is a marked lymphocytic infiltration of the proventricular glands. The purpose of the fourth study was to characterize this lymphocytic infiltrate to gain insights into the identity of these cells and their potential role in generating a protective immune response in the proventriculus. To accomplish this objective commercial broiler chickens were experimentally infected with proventricular homogenates from affected broilers and studied the proventricular lesions using histopathology. Lymphocyte cell-surface markers were stained for, and the distribution of different lymphocyte subsets *in situ* were studied.

### References

1. Apple et al. Avian Dis. 35:422-425. 1991.
2. Bayyari et al. Poult Sci 74:1799-1809. 1995.
3. Bayyari et al. Poult Sci 74:1961-1969. 1995.
4. Bracewell et al. World's Poult Sci J 40. 1984.
5. Cullen et al. Am J Res 49:5. 1988.
6. Dormitorio et al. Proc. Southern Conference on Avian Diseases, Atlanta, GA. 40. 2001.
7. Dorner et al. Appl Environ Microbiol. 46:698-703. 1983.
8. Goodwin et al. Avian Pathol. 25:269-279. 1996.
9. Guy & Barnes. Proc.139th Meeting of the American Veterinary Medical Association, Nashville, TN. 2002.
10. Harryet al. Br Poult Sci 16:69-78. 1975.
11. Hayes & Wobeser. Can J Comp Med 47:180-187. 1983.
12. Hendersonet al. Vet Rec 123:492-494. 1988.
13. Huchzermeyeret al. Vet Rec 133:143-144. 1993.
14. Huff et al. Avian Dis. 45:828-843. 2001.
15. Jensen et al. Avian Dis- 35:969-973. 1991.
16. Kouwenhoven et al. Avian Pathol. 7:183-187. 1978.
17. Kouwenhoven et al. Avian Pathol. 17:879-892. 1988.
18. Lenz et al. J Vet Diagn Invest 10:145-151. 1998.
19. Mutlu et al. Tierarztl Prax Ausg G Grosstiere Nutztiere 25:460-462. 1997.
20. Newberne et al. J Am Vet Assoc 128:553-555. 1956.
21. Page et al. Avian Dis. 26:618-624.1982.
22. Phalen & Moore. Avian Dis. 47:254-260. 2003.
23. Poole. Proc. 43rd Western Poultry Disease Conference, Sacramento, CA. 40-42. 1994.
24. Reece. Canberra, Australia, RIRDC. 2002.
25. Riddell. Avian Dis. 20:442-445. 1976.
26. Sellers et al. Avian Dis. 45:26-33. 2001.
27. Shapiro & Nir. Poult Sci 74:33-44. 1995.
28. Sharma et al. Dev Comp Immunol 24:223-235. 2000.
29. Skeeles et al. Poult Sci. 77 (suppl.):133. 1998.
30. Yu et al. Avian Dis. 45:416-424. 2001.

LITERATURE REVIEW: The Proventriculus. The proventriculus or glandular stomach is a fusiform organ lying dorsal to the liver and between the esophagus and the gizzard. It is approximately 4-5 cm long and 2 cm in diameter in adult fowl. The lumen is narrow and the thick walls are composed mainly of masses of compound tubular glands (94). The primary function of the proventriculus is the production and release of the gastric secretions, pepsin, hydrochloric acid, and mucus. The food that passes through the proventriculus is held in the gizzard, where the gastric secretions act (222).

The wall of the proventriculus consists of four layers: the mucous membrane, submucosa, muscular tunic and serosa (154). The mucosal lining of the proventricular lumen forms folds termed plicae. Scattered over the mucosal surface are a number of papillae, through each of which passes a secretory duct of the proventricular glands opening at the apex of the papilla. The mucous membrane is lined by a single layer of columnar cells that secrete mucus. This mucous secretion acts as a protective lining for the surface of the epithelium (154). Underlying the surface epithelium and occupying the center of the mucosal folds is the tunica propria. Within this tunic lymphoid infiltrates are frequently found and large lymphoid foci often occur in association with mucosal papillae (151). Aggregates of lymphocytes are also found in the lamina propria of the esophageal-proventricular junction and these lymphoid accumulations have been named the esophageal tonsil (181).

The mass of proventricular glands makes up the greatest part of the thickness of the proventricular wall (94). The glands are composed of numerous rounded or polyhedral lobules which are arranged in small groups, each draining into the lumen through one of the mucosal papillae. Each lobule is composed of numerous straight alveoli radiating out from a central cavity. Groups of several alveoli join together to form first a short common tertiary duct, then a wider secondary duct, and finally a short primary duct passing up through the mucosal papilla and opening into the lumen. Surrounding each lobule are connective tissue septa of collagenous and elastic fibers, a few muscle fibers, and blood vessels and nerves (94).

The primary, secondary and tertiary ducts are all lined with columnar epithelium similar to that covering the mucosal surface. The glandular epithelium consists of a single layer of cuboidal to low columnar oxynticopeptic cells. These secrete both, hydrochloric acid and the enzyme precursor pepsinogen, hence combining the functions of mammalian zymogenic (chief) and parietal cells (222). The gastric juice is composed principally of hydrochloric acid, mucus and the proteolitic enzyme, pepsin. In addition to the oxynticopeptic cell, the epithelium of the tubular alveoli contains a number of glandular endocrine cells (154). As in mammals, stimulation of the vagus provokes the secretion of juice, and also a gastrin mechanism appears to exist (154). Gastrin cells have been described in the pyloric region of the fowl and would seem to confirm a role for gastrin in the proventricular secretion process (107). Other hormonal mechanisms may also be involved in the stimulation of proventricular secretion. Bombesin, present in proventricular endocrine cells, is secreted into the blood and carried to its target areas for stimulation of gastrin release, of pancreatic secretion, and enhancement of gut motility (107).

The submucosal connective tissue consists of a narrow band of white fibrous connective tissue and contains the submucosal nerve plexus. The muscularis externa consists of the inner circular and a much thinner, outer longitudinal layer of smooth muscle fibers. Between them lies a myenteric nerve plexus. Externally there is a thin layer of loose, adventitial connective tissue and a peritoneal coat (94). The celiac artery supplies both the proventriculus and the gizzard. Venous outflow occurs via the gastrointestinal vein which flows into the hepatic portal vein (222). The proventriculus is innervated by branches of the vagi and by perivascular nerve fibers from the celiac and mesenteric plexi (222).

The intermediate zone between the proventriculus and the gizzard is very short, being approximately 0.75 cm in the adult chicken. At the point where the proventriculus narrows to form this isthmus, the proventricular glands terminate abruptly and the plicae become shorter and gradually change over to the gizzard glands. The intermediate zone functions mainly when contracted as a barrier separating the proventriculus from the gizzard (154).

Matsumoto and Hashimoto (151) described the normal distribution and developmental changes of the lymphoid tissues in the chicken proventriculus. Development of lymphoid masses in the proventricular lamina propria occur underneath the surface epithelium and near the duct orifices, which suggests that the local mucosal immune mechanism develops primarily with a dominant participation of T lymphocytes in the early post-hatching period. Lymphocytes infiltrating the gland epithelium are γδ T lymphocytes, which play important roles both in recognition of antigenic substances invading the epithelium and in renovation of damaged epithelial cells. The development of B lymphocytes occurs following the invasion of antigens associated with food intake. No M cells could be detected in the proventriculus suggesting that routes for uptake of intraluminal antigens other than those traditionally attributed to M cells.

Transmissible proventriculitis: Definition and economic significance. Transmissible proventriculitis is an infectious disease of chickens of unknown etiology (73). It is characterized by an enlarged, atonic proventriculus that is filled with fluid and feed (11, 74, 79, 99, 122, 193). The gastric isthmus connecting the proventriculus and gizzard is enlarged, with dilation of the constriction present at this juncture.

The economic impact of proventriculitis is mainly due to condemnation of contaminated carcasses subsequent to the rupture of the proventriculus during routine evisceration (11, 99). An estimated 1% of processed birds must be reprocessed need because of gastro-intestinal tearing during mechanical evisceration (230). Contamination is the third most common cause of condemnation of broilers at processing after septicemia and airsacculitis in the us, accounting for about 0.05% of broilers processed in the united states (poultry slaughter, 2001).

Proventriculitis is more severe in younger birds (4-5 wks of age) and has been associated with impaired growth, poor feed conversion, intestinal fragility, stunting syndrome and passage of undigested feed (4, 21, 99, 130, 183, 193, 206). The poultry industry reports sporadic, thought economically important, outbreaks of proventriculitis in broilers (99). Although broiler chickens throughout the world are commonly plagued by outbreaks of disease characterized at least in part by proventricular enlargement, lesions consistent with transmissible proventriculitis have been described in detail only in the united states (74, 79, 99), holland (130), and australia (193). Definitive prevalence data regarding the global incidence and distribution of proventriculitis are lacking.

Transmission. The route of natural infection is unknown; however, chickens can be infected experimentally by oral inoculation with a homogenate prepared from proventriculi of chickens with proventriculitis (11, 79, 99, 193). Because the disease is reproduced with proventricular homogenate filtrates (0.2 µm), a virus is suspected as the etiologic agent (79, 99, 193). Consequently, the disease is also termed also transmissible viral proventriculitis (TVP)(74, 79). However, the severity of lesions and the effects on production are more severe in birds treated with unfiltered homogenates, suggesting an additive effect of others concomitant infectious agents (99).

Gross Lesions. Proventriculi of affected chickens are enlarged and the serosal surface of the proventriculus often appears mottled or has irregular white plaques. The proventricular wall is thickened, some glands are distended, and exude viscous white material when compressed (73). The gastric isthmus is distended and flaccid. Some affected commercial birds also have gizzard erosions, fragile and thin intestines, mild to moderate enteritis, and low uniformity in carcass weight (11).

Microscopic Lesions. There is necrosis of the alveolar (oxynticopeptic) pepsinogen- and hydrochloric acid-secreting cells. These cells have an amorphous, granular, or vacuolated cytoplasm and nuclear condensation, fragmentation or lysis (73). Fewer attached or sloughed cells have swollen nuclei with marginated chromatin and clear centers (11, 73, 79). Proliferating hyperplastic and hypertrophic columnar to low cuboidal cells line primary, secondary, and tertiary gland ducts. Cuboidal to low columnar, pale, basophilic, and distinctly vacuolated ductlike epithelium replaces the destroyed alveolar secretory cells (11, 74). Severely affected glands occasionally coalesce. There is a moderate to marked increase in number of lymphocytes infiltrating the connective tissue stroma (tunica propria). Lymphocyte infiltrates in the glandular interstitium develop in areas containing affected glandular epithelial cells. Marked lymphocyte infiltrates expand the glandular interstitium in the epithelium between the ductular and the glandular epitheliums (79).

Differential diagnosis. Several causes have been associated with proventricular enlargement and proventriculitis. A non-infectious proventriculitis can be produced by oral exposure to toxic chemicals such as biogenic amines (82, 172, 191, 221), and mycotoxins (39, 49, 84, 184, 185), which often contaminate poultry feed. A diet low in fiber has been shown to cause proventricular swelling and proventricular lesions (172, 197). Dietary copper sulfate within levels commonly fed to chickens as a growth stimulant, also causes proventricular hypertrophy (12, 120).

Some avian infectious agents can produce proventricular lesions. Velogenic strains of Newcastle disease virus (NDV) can produce hemorrhages in the proventricular mucosa (2), as can highly pathogenic avian influenza virus (HPAI)(224). Reticuloendotheliosis virus (REV) infection can cause stunting and neoplastic cellular infiltrates resembling nonpurulent inflammation are present in these proventriculi (168). In Marek's disease, diffuse lymphomatous involement and enlargement of the proventriculus occurs. Increased numbers of lymphoid follicles in the tunica muscularis of the proventriculus are pathognomonic for infection with avian encephalomyelitis (AE)(27).

Proventricular Dilation Syndrome (PDD), is a common chronic disease of psittacines birds characterized by dilation of the proventriculus, anorexia, regurgitation, passing of undigested seeds in feces, diarrhea, neurological signs, loss of weight, etc. The cause is not known, but is presumed to be a virus (75). In PPD there is accumulation of lymphocytes and plasma cells in the autonomic nervous system, especially the nerves that supply the muscles in the proventriculus and other digestive organs including crop, ventriculus and small intestine. Central nervous system signs associated with PDD, which may occur in addition to, or independent of, gastrointestinal signs, may include ataxia, abnormal head movements, seizures and proprioceptive or motor deficits. Dilated thin proventriculi are present in 70% of cases with lymphoplasmacytic ganglioneuritis of splanchnic nerves of crop/esophagus, proventriculus, gizzard, and intestine (75).

A very large, Gram-positive, rod-shaped microrganism has been found associated with proventriculitis in canaries, budgerigars, ostriches and recently in chickens (87, 97, 169, 187). This so-called "megabacterium" is a novel, anamorphic as comycetous yeast named *Macrohabdus ornithogaster* that colonizes the narrow zone (isthmus) between the proventriculus and gizzard. Proventricular trichomoniais has been reported in budgerigars (87), and filamentous bacteria (232) inhabit the upper gastrointestinal tract and are potential pathogens. Other bacterium, *Helicobacter pullorum,* is also found in the digestive tract of 60% of commercial poultry tested (5). *H. pullorum* belongs to the genus Helicobacter, the same as *H. pylori* which causes ulcerative gastritis in humans and some other mammals (64). The role of these bacteria in proventriculitis of chickens is unknown. They may have some pathogenic effects in the proventriculi, possibly potentiated by other infectious, chemical, or immunosuppressive agents.

Cases of marked necrotizing ulcerative mycotic proventriculitis in ostrich chicks have been associated with Zygomycetes and are accompanied by superficial microcolonies of yeasts (presumably *Candida* spp) (77, 119). Proventricular cryptosporidiosis is common among zoo and pet bird species (19, 233) and has been reported once in chickens (72). Mucosal colonization by *Cryptosporidium* is accompanied by inflammation and exfoliation of parasitized epithelial cells. Purulent necrotizing proventriculitis with intralesional *Toxoplasma gondii* was reported in one flock of chickens in Norway (52).

Outbreaks of *Dispharynx nasyta* have been reported in several avian species including chickens. Infected proventriculi are enlarged, and the mucosal surface is covered with parasites and necrotic debri (70). *Tetrameres americana, T. crami,* and *T. fissispina* also parasitize the proventriculus (176).The females reside deep within a proventricular gland and completely fill and distend its lumen. Heavy infections in chickens can cause emaciation and anemia (176).

Traumatic proventriculitis may also occur after foreign bodies have been ingested and retained (78) and secondary bacterial infection may occur.

History. Initially, transmissible proventriculitis was described as one of the lesions associated with malabsorption syndrome (21). Differing combinations of clinical manifestations resulted in a variety of names for this syndrome: infectious stunting syndrome (21, 194), runting-stunting syndrome (157), pale bird syndrome (71), and infectious proventriculitis (130). These conditions cause growth retardation and poor feed conversion in young broiler chickens. The causative agents of these syndromes have not been clearly identified, and proventriculitis may or may not be present as a lesion in these syndromes. For example, cases of malabsorption syndrome may or may not include proventricular lesions (219). Filterable agents isolated in the Netherlands were originally linked to proventriculitis, causing runting syndrome in broilers (130). These authors suggested the involment of both bacteria and viruses in the etiology of malabsorption syndrome (130, 131). Shapiro and Nir (206) reported both proventricular enlargement and decreased body weights in birds infected with crude homogenate of intestines from broiler chickens affected with stunting syndrome.

Reoviruses have been strongly implicated as a causative agent for concurrent proventricular lesions present in some flocks naturally affected with malabsorption syndrome (131). Proventriculitis was reproduced by inoculation of two reovirus isolates from the intestines of birds with malabsorption syndrome (183). A homogenate of proventricular and duodenal tissues from stunted birds raised in northwest Arkansas produced proventriculitis and decreased body weight when gavaged into specific-pathogen free birds. However a cell culture adapted reovirus isolated from this same homogenate produced proventriculitis without affecting the body weight (4). The addition of histamine to the feed of broiler chickens orally infected with an avian reovirus vaccine interacted to cause proventricular enlargement and decreased body weight (24).

A comparative study of the pathogenesis of five different malabsorption syndrome homogenates from the Netherlands and Germany distinguished the inoculated groups of chickens by their histopathologic lesions: proventriculitis, lesions in the intestine only, or combination of both (219). Lesions in the small intestine had more impact on weight gain depression than lesions in the proventriculus. Reovirus and enterovirus-like particles were detected in the inoculated groups. Also bacteriophages and bacteria (hemolytic *Escherichia coli, Pasteurella hemolytica,* and *Enterococcus durans* were isolated from inoculated chicks. The individual role that each of these pathogens plays in the pathogenesis of malabsorption syndrome is still unsolved (219).

Mild proventriculitis has also been reproduced experimentally in chickens infected with some isolates of adenovirus (130, 141) however, this virus hasn't been consistently isolated from diseased proventriculi. Infectious Bronchitis Virus (IBV) isolates from naturally occurring cases in China produced proventricular lesions in infected birds. Their proventriculi were enlarged and swollen and the mucosa was thickened and exuded white viscous fluid (255).

A filterable (0.2-µm) agent found in homogenized affected proventriculi can cause lesions similar to the proventriculitis seen in naturally-occurring cases but not to the same degree as the caused by unfiltered homogenate. This proventriculitis could be produced independently of an effect on growth, and only unfiltered homogenate caused stunting (11). The proventriculitis produced was best detected using histopathology, and was sufficiently severe to produce mural thinning with increased susceptibility to rupture during evisceration at processing.

Goodwin *et al.* (74) reported the presence of intralesional virions in proventriculi from chicks that failed to thrive and had proventriculitis, and suggested a causal relationship between the virus and the lesion in its host. Hexagonal intranuclear and intracytoplasmatic virus particles were described and resembled adenovirus or poliomavirus. However, DNA *in situ* hybridization failed to detect adenovirus or poliomavirus nucleic acids. Huff et al. (99) also reported the presence of similar virus-like particles in the nuclei of many epithelial cells of the proventriculus of chickens experimentally inoculated with homogenate prepared from the proventriculi of chickens with proventriculitis. The particles, nonenveloped spheres of about 100-200nm in diameter, appeared hexagonal and were arranged in semiparacrystalline arrays in the nuclei (99).

Guy and Barnes (79) reproduced proventriculitis by administration of a filtrate (0.2-µm) from a homogenate produced from the proventriculi of chickens with proventriculitis. However, affected chickens had no decrease in body weight. This inoculum was free of avian reovirus, avian group I adenovirus, infectious bursal disease virus (IBDV) and infectious bronchitis virus (IBV). Adenovirus-like particles, similar to those observed by Goodwin *et al* (74), were identified by thin-section electron microscopy in nuclei of affected glandular epithelium cells.

Reece (193) reported that flocks with proventriculitis and stunting syndrome were generally characterized by poor feed conversion, reduced growth rate and/or uneven weight at slaughter age. Proventricular homogenates prepared from these birds were highly infectious and transmissible for at least four passages in birds. Treatment of the inoculum with chloroform did not reduce infectivity, supporting the hypothesis that the putative etiological agent of infectious proventriculitis was a non-enveloped virus. This did not grow in any of a wide variety of primary and established cell culture systems. Chicken embryos were inoculated via various routes, embryo viscera were harvested, and these were inoculated into SPF chickens. No proventricultis was produced. The original inoculum contained chicken anemia virus (CAV), fowl adenovirus type 8, avian nephritis virus and Marek's disease virus (MDV) but did not contain avian leukosis virus (ALV), infectious bronchitis virus (IBV), reovirus, Newcastle disease virus (NDV) or infectious bursal disease virus (IBDV).

**Proventriculitis** and Infectious Bursal Disease Virus (IBDV). Proventriculitis was experimentally reproduced by oral infection of commercial broilers with a 0.2-µm filtrate of an infectious proventricular homogenate (11). Serologic tests for induced IBDV antibody were positive, whereas those for reovirus antibodies were negative, suggesting the possible involvement of IBDV. The presence of IBDV in this homogenate was later confirmed by isolation of immunoprecipitated IBDV virus in embryos, and visualization of IBDV-like particles in the livers of SPF embryos inoculated with a filtrate of this homogenate (99). A challenge study with the IBDV immunoprecipitated from this homogenate increased the proventricular lesion scores at 14 days post inoculation in commercial chickens that received the inoculum at one day of age. However, there was no proventricular enlargement due to IBDV inoculation. Huff *et al.* (99) reported the isolation of a unique bacterial culture (Clostridia sp.) from the same infected proventriculus homogenate suggesting bacterial involvement in this syndrome. Challenge studies in broiler chickens comparing the pathogenicity of this infectious proventricular homogenate, the monoclonal antibody precipitated IBDV and this bacteria isolate, alone or in combination, showed that only the combination of virus and bacteria reproduced proventriculitis similar to the proventricular homogenate. The homogenate, bacteria alone, and the combination of virus and bacteria each caused poorer feed conversion efficiency compared to the saline control, indicating that the *Clostridium sp.* isolate may be responsible for the poor feed conversion. The severity of lesions and the effects on production were more severe in birds treated with the homogenate, suggesting there were either additional factors involved, or dose-related effects on the pathogenesis. Huff *et al.* concluded that a viral infection, as well as various dietary factors, may facilitate bacterial invasion of the proventriculus, and more than one type of virus may act as facilitator in this disease syndrome.

IBDV produces hemorrhage, necrosis, and heterophilic infiltration, in the proventricular mucosa of SPF white leghorns (213). Proventriculitis experimentally produced by challenge of SPF leghorns with IBDV included gross and histopathological lesions but not the severe proventricular enlargement seen in naturally-occurring cases of this disease (173).

In the past several years, IBDV has been implicated as the cause of proventriculitis in broiler flocks from north Alabama (48). The disease resulted in poor feed conversion, weight reduction, and mortality. A small study using live IBDV vaccines was performed with two commercial IBDV vaccines. SPF birds were vaccinated with either a live intermediate vaccine containing an antigenic standard virus or a combination product, containing both standard and variant IBDV vaccine viruses. Vaccinated and nonvaccinated birds were exposed to a virulent Alabama IBDV isolate implicated in causing proventriculitis. Fifty percent of the nonvaccinated birds showed atrophy of the bursa and proventriculitis. In contrast, only 25% and 10% of the birds that received the combination or standard vaccine alone, respectively, had these lesions. Lesions still occurred suggesting that another agent or agents are involved in the production of proventriculitis. The authors suggested that a variant IBDV may play a role in proventriculitis and that vaccination of broiler progeny can be helpful in reducing the incidence and severity of the disease (48).

Proventriculitis cases were also reported in Arkansas (122). These birds were reovirus negative and variably positive for chicken anemia virus (CAV) by serologic tests. The proventriculus had a thickened wall with loss of glandular integrity and lesions in mucosal lamina propria. Homogenized proventriculi were gavaged into SPF chickens and they became antibody positive to IBDV and remained antibody negative to CAV and reovirus. Exposed chickens had bursal atrophy, enlarged proventriculi, swollen kidneys and spleens, and lesions at the junction of the esophagus and proventriculus.

Detection of IBDV by ISH-staining using riboprobes specific for the VP2 gene of IBDV failed to detect that virus in the proventriculi of 3 week old chickens experimentally exposed to Delaware A, D78, or Bursavac®. Also, no histologicaly evident proventricular lesions were present after these exposures (205). Combined with previous findings, these results indicate that IBDV probably has no direct effect on the proventriculus.

Infectious bursal disease virus (IBDV) is the etiological agent of Gumboro disease or infectious bursal disease (IBD). IBD is a highly contagious viral disease of young chickens, characterized by destruction of the lymphocytes in the bursa of Fabricius, producing severe immunosuppression. IBDV is endemic in most poultry producing areas of the world. The virus is highly stable in the environment and has a tendency to persist in the environment despite thorough cleaning and disinfection. There are two serotypes of IBDV: 1 and 2. All viruses capable of causing disease in chickens belong to serotype 1. Serotype 2 viruses may infect chickens and turkeys but are non-pathogenic for either species (109, 155). Chickens are the only avian species known to be susceptible to clinical disease and lesions produced by IBDV. Turkeys, ducks and ostriches are susceptible to infection with IBDV but are resistant to clinical disease (148, 156).

Despite widely used vaccination programs, IBD is one of the major economically important diseases of poultry worldwide. Most commercial chickens get exposed to IBDV early in life. In unprotected flocks, the virus causes mortality and immunosuppression. Although mortality can be quite significant, the major economic concern is the ability of IBDV to produce immunosuppression. Immunosuppressed flocks perform poorly and show reduced economic return (209).

The disease was first reported by Cosgrove in 1957. It was initially recognized as "avian nephrosis", and the syndrome became known as "Gumboro disease" because it occurred in the Gumboro district of Delaware, USA. The clinical features of the syndrome included whitish or watery diarrhea, followed by anorexia, depression, trembling, severe prostration, and death. At necropsy, the birds exhibited dehydration, hemorrhages in the leg and thigh muscles, urate deposits in kidneys and enlargement of the bursa of Fabricius (37).

The early consensus was that avian nephrosis or Gumboro disease was caused by the Gray strain of infectious bronchitis virus (IBV) because of gross changes in the kidney and because IBDV and IBV were concurrent in many cases. This misconception also arose because the two infections were concurrent in many cases and IBDV was difficult to isolate with the available methods (135). After subsequent studies, Winterfield *et al.* (249), succeeded in isolating the causative agent in embryonating eggs, and later Hitchner (93) proposed the term "infectious bursal disease" for the disease.

In 1972, Allan *et al.* (3) reported that IBDV infections at an early age were immunosuppressive. The recognition of the immunosuppressive capability of IBDV infections greatly increased the interest in the control of these infections. The existence of a second serotype was reported in 1980 (153).

In 1984 and 1985, the Delmarva peninsula broiler growing area experienced a significant increase in mortality. The clinical syndrome had significant variability, but often was respiratory in nature. Lesions ranged from moderate to severe, with death usually being attributed to *E. coli* infection (38). Using vaccinated sentinel birds, Rosenberger *et al.* (199) isolated four isolates designated as A, D, G, and E. These isolates differed from standard strains in that they produced a very rapid bursal atrophy associated with minimal inflammatory response. The available killed standard vaccines did not afford complete protection against these four new Delaware isolates. The Delaware isolates, A, D, G and E were designated as antigenic variants and killed vaccines were developed, tested and proven effective against them (199). Currently these and other similar variants are widely distributed in the United States (217, 218).

Since 1987, acute IBDV cases with up to 30% to 60% mortality in broiler and pullet flocks, respectively, became commonly reported in Europe. The first reports were made by Chettle *et al.* 1989 (30), and van den Berg *et al* in 1991 (242). Some of these acute outbreaks occurred in broiler flocks where appropriate hygienic and prophylactic measures had been taken. Although no antigenic drift was detected, these strains of increased virulence were identified as very virulent IBDV (vvIBDV) strains (242). The European situation has been dominated for a decade by the emergence of vvIBDV strains. These strains have now spread all over the world (57). In the Americas, acute IBDV outbreaks due to vvIBDV strains have already been reported in Brazil (41, 100), and the Dominican Republic (8).

Etiology. IBDV is a small, non-enveloped virus, belonging the *Birnaviridae* family, which is characterized by a bisegmented dsRNA genome (123). The *Birnaviridae* family includes three genera: Genus *Aquabirnavirus* (type species: infectious pancreatic necrosis virus or IPNV), Genus *Avibirnavirus* (type species: infectious bursal disease virus or IBDV), and Genus *Entomobirnavirus* (type species: *Drosophilla* X virus or DXV). (43). Other birnaviruses have been isolated from bivalve mollusks such as Tellina virus (236), and Oyster Virus (43, 129), and Japanese eels (139). To date, no Birnavirus capable of causing disease in mammals has been reported.

The virion has a single capsid shell of icosahedral symmetry composed of 32 capsomeres and a diameter of 60 to 70 nm (43, 81, 90, 174). By cryomicroscopy, the subunits forming the capsid are predominantly trimeric clusters. Due to the conformation of these subunits, the capsid acquires a nonspherical shape (20).

Viral genome structure and replication. The genome of IBDV is formed by two segments of double-stranded RNA (dsRNA) with the two segments detected by polyacrylamide gel electrophoresis (43, 113). Molecular weights of the two double stranded segments are 2.2 x 10⁶ and 1.9 x 10⁶ Da, respectively (162). The length of both segments is 3.2 kb and 2.8 kb respectively (98).

The larger segment A (approximately 3400 base pairs) contains two partially overlapping open reading frames. The first encodes a nonstructural polypeptide of 17 kDa known as VP5, which is dispensable for replication *in vitro* but important for virus-induced pathogenicity (165, 166). The second ORF encodes a 109-kDa polyprotein that is autoproteolytically cleaved into three polypeptides, VPX, VP3 and VP4. VPX is further processed to produce a polypeptide known as VP2 (6, 98, 161). VPX, VP2, and VP3 are the major structural proteins that form the virus capsid (20), while VP4 appears to be responsible for the proteolytic maturation of the polyprotein (118, 126, 140).

Segment B encodes VP1, a 95-kDa protein which is the RNA-dependent RNA polymerase (RdRp) responsible for the replication of the genome and synthesis of mRNAs (44, 220). VP1 shares a number of primary sequence features with RNA polymerases from diverse origins (23).

At the 5' and 3' ends in both genome segments of IBDV, there are direct terminal and inverted repeats that are likely to contain important signals for replication, transcription and packaging. It is not known whether virulence variations are due to mutations in these regions (170). The inverted adjacent repeats at the 3' terminus on segments A and 5' terminus on segment B have the potential to form stem and loop secondary structures (124), which are involved in the processes of RNA replication, translation and encapsidation like other RNA viruses such as poliovirus (211).

The mechanism of synthesis of both virus-specific ssRNA and dsRNA during infection with IBDV has not been clearly determined. An RNA-dependent RNA polymerase has been demonstrated in IBDV (220). Genome-linked proteins have been demonstrated in three different Bimaviruses, (162, 186, 195, 220), indicating that they replicate their nucleic acid by a strand displacement (semiconservative) mechanism (17, 158, 220).

Viral Proteins. Four mature viral structural proteins designated VP1, VP2, VP3 and VP4 are detected in infected cells (13, 42, 43, 174). A non-structural protein designated VP5 has been identified, the function of this protein is still unknown, but it is not essential for viral replication (165, 166).

During the processing of the polyprotein precursor into pVP2, VP3 and VP4, the existence of two sites, essential for the cleavage of the VPX-VP4 and VP4-VP3 precursors, respectively has been reported (202). These sequences are highly conserved among IBDV strains from both serotypes 1 and 2.

VP1, the RNA-dependent RNA polymerase of the virus, is present in small amounts in the virion, both as a free polypeptide and as a genome-linked protein (125, 163). It plays a key role in the encapsidation of the viral particles (146).

VP2 is the most abundant viral protein, accounting for 51% of the virus proteins of the serotype I IBDV's. This is the major protein component of the viral capsid, and is the host-protective antigen containing the antigenic region responsible for the induction of neutralizing antibodies and for serotype specificity (60). The transition from the precursor of VP2 (pVP2) to VP2 involves the cleavage of pVP2 near its C terminus (6). VP2 has also been identified as an inducer of apoptosis (62).

VP3 is also a structural protein, and accounts for 40% of the virion proteins (123). VP3 is found only on the inner surfaces of virus-like particles (150). This protein plays role in the assembly of viral particles, and packaging of the viral genome (146, 150, 225). VP3 is a group-specific antigen that is recognized by non-neutralizing antibodies, some of which cross-react with both serotypes 1 and 2 (14). It is likely that the outer subunits in the viral capsid consists of VP2, carrying the dominant neutralizing epitope, and that the inner trimers consist of protein VP3, (20).

VP4 is the viral protease involved in the processing of the precursor polyprotein(6). It is a proteolytic enzyme-like protein, which uses a Ser-Lys catalytic dyad to act on specific substrates and cleavage sites (18). The integrity of VP4 is essential for the proteolytic processing of the polyprotein (50, 118) and either itself, or through proteins under its control, plays a role in the activation of VP1 (18).

VP5 was the last IBDV protein identified (165). This protein is not essential for IBDV replication *in vitro* or *in vivo,* however, it plays an important role in viral pathogenesis (253). It has cytotoxic properties and it may play a role in the release of the IBDV progeny (147).

Host susceptibility. Domestic fowl are the natural host of IBDV (86). All breeds are affected. White Leghorns exhibit the most severe disease and have the highest mortality rate (148). Turkeys may be infected with serotypes 1 and 2 but do not show clinical signs of the disease (110, 156). There is, however, considerable potential for immunosuppression or interaction with other diseases under commercial conditions in turkeys (136). Serotype 2 was originally identified in clinically unaffected adult turkeys in Ireland (153). Ducks may develop IBDV infection and antibodies are detectable by serum virus neutralization, but neither gross nor microscopic lesions occur. Antibodies have been detected in wild birds. Five of 29 weavers (*Ploceus cucullatus*) and one of eight finches (*Uraegirathus bengalus*) (171) were seropositive. Surprisingly, seropositivity has also been detected in Antarctic adelie penguins, but the source of IBDV exposure has not been defined (66).

Transmission. IBDV is highly contagious and the disease may be spread by direct contact between infected and susceptible flocks. Infected chickens shed IBDV one day after infection and can transmit the disease for at least 14 days. There are neither experimental data nor naturally-occurring observations to suggest that IBDV is transmitted vertically by the transovarian route (148).

Indirect transmission of virus most probably occurs on fomites (feed, clothing and litter) or through airborne dissemination of virus-laden feathers and poultry house dust (15). IBDV is very persistent in the environment of a poultry house. Houses from which infected birds were removed, still had virus infective for other birds 54 and 122 days later (16). The lesser mealworms, *Alphitobius diaperinus* may be reservoir hosts (152, 214). IBDV has also been isolated from *Aedes vexans* mosquitoes (96), and antibodies against IBDV have been detected in rats found on poultry farms (180). No further evidence supports the conclusion that either mosquitoes or rats act as vectors or reservoirs of the virus.

Clinical forms of IBDV. The classical form, as described since the early 1960s, is caused by the classic moderately virulent strains of IBDV. The incubation period of IBD ranges from 2 to 4 days after exposure. One of the earliest signs of the classical infection in a flock is the tendency for some birds to pick at their own vents. The disease also produces acute onset of depression, reluctance to move, ruffled feathers, white or watery diarrhea, pericloacal staining of feathers with urates, trembling, and prostration. The feed intake is depressed but water consumption may be elevated. Severely affected birds become dehydrated and die (37).

The immunosuppressive form, principally described in the United States, is caused by low-pathogenicity strains of IBDV, as well as by variant strains, such as the Delaware variants or GLS strains, which partially resist neutralization by antibodies against the so-called "classic" or standard strains (217).

The acute and very virulent form, described initially in Europe, and then spread to Asia, Africa and some countries in Latin America, is caused by hypervirulent strains of IBDV, and it is characterized by an acute progressive clinical disease, leading to high mortality rates on affected farms. The initial outbreaks in Europe were characterized by high morbidity (80%) and mortality reaching 25% in broilers and 60% in pullets over a 7-day period (30, 177, 242).

Gross lesions. Chickens which die acutely of primary IBD infection show dehydration of the subcutaneous fascia and musculature of the thigh, inguinal and pectoral areas (37, 148). Hemorrhages occur in the mucosa of the proventriculus at its junction with the gizzard. Kidneys show enlargement and pallor with accumulation of crystalline urate in tubules (37). The renal lesions were more prominent in early outbreaks in the United States, perhaps due to co-infection with nephropathogenic strains of avian infectious bronchitis (148).

The bursa of Fabricius is the main organ in which lesions develop following exposure to IBDV (31). Chickens that die or are sacrificed at early stages after the infection show a doubling in size of the bursa due to edema. The bursa is pale yellow and has striations. By the 5^{th} day the bursa returns to normal weight, but it continues to atrophy, and from the 8^{th} day forward it is approximately one-third its original weight (148). Variant strains have been reported that do not induce an acute inflammatory response (199, 208). However, at least one variant strain was reportedly able to induce such acute inflammatory lesions (83).

Splenic enlargement has been documented, with small gray foci uniformly dispersed through the parenchyma (148, 160). The vvIBDV strains are able to cause greater decrease in thymic weight index and more severe lesions in cecal tonsils, thymi, spleens, and bone marrow, but the bursal lesions are similar (148). IBDV has been suggested to be part of an etiologic complex causing proventriculitis in broilers (99).

Histopathologic lesions. Infection with standard or variant strains results in death of bursal B lymphocytes. Necrosis of lymphocytes in the medullas of bursal follicles can be detected within one day of infection. By the third day an inflammatory response with edema, heterophil infiltration, congestion and hemorrhage is present in infections due to standard strains. At this time the follicles may be reduced to a necrotic center surrounded by heterophils. From the fourth day after infection the acute inflammatory reaction declines, and as necrotic debris are cleared by phagocytosis, cystic cavities are formed. Fibroplasia occurs in the surrounding connective tissue and the covering epithelium becomes infolded and irregular (31, 192). Sharma *et al.* (1989) observed that the infection with the variant A strain did not result in an acute inflammatory response, and follicular lymphoid necrosis was evident at three days after infection (208).

The development of lesions by IBDV in thymus depends on the pathotype of the virus (102,226). IBDV induced cortical thymic lymphocyte depletion is caused by apoptosis (102). The highly pathogenic vvIBDV strains from Europe and Japan are associated with severe thymic lymphocyte loss when compared to less pathogenic strains (226). Although the thymus undergoes marked atrophy and extensive apoptosis of thymic cells during the acute phase of virus infection, there is no evidence that the virus actually replicates in T cells (228). Gross and microscopic lesions in the thymus are quickly overcome and the thymus returns to its normal state within a few days of virus infection (209).

The spleen may have hyperplasia of reticuloendothelial cells around the adenoid sheath arteries in early stages of the infection, and lymphoid necrosis in the germinal follicles and the periarteriolar lymphoid sheath by the third day (148). The Harderian gland may also be affected. Normally this gland is infiltrated and populated with plasma cells as the chicken ages. Infection with IBDV prevents this infiltration (223). In cecal tonsils, there may be acute heterophil inflammation, destruction of lymphocytes, and regeneration on the fifth day after infection (86).

Histologic lesions in the kidney are nonspecific and probably occur because of severe dehydration of affected chickens. Lesions observed consisted of large casts of homogeneous material infiltrated with heterophils, and also glomerular hypercellularity (86).

Pathogenesis and Immunosuppression. The main target organ of IBDV is the mature bursa of Fabricius, which is the source for B lymphocytes in avian species. Bursectomized chickens did not develop clinical IBD despite the presence of infection (89). The severity of the disease is directly related to the number of susceptible cells present in the bursa of Fabricius; therefore the highest age susceptibility is between 3 and 6 weeks, when the bursa of Fabricius is at its maximum development. This age susceptibility is broader in the case of the vvIBDV strains (177).

After oral infection or inhalation, the virus replicates primarily in the lymphocytes and macrophages of the gut-associated tissues. From the gut, the virus is transported to other tissues by phagocytic cells, most likely resident macrophages (209, 240). By 13h post-inoculation (p.i.), most bursal follicles are positive for virus and by 16 h p.i. a second and pronounced viraemia occurs with secondary replication in other organs leading to disease and death (164). Similar kinetics are observed in vvIBDV but replication at each step is amplified (240).

Actively dividing, surface immunoglobulin M-bearing B-cells are lysed by infection (91, 92, 198), but cells of the monocyte-macrophage lineage can be infected in a persistent and productive manner, and play a crucial role in dissemination of the virus (25, 101) and in the onset of the disease (127, 133, 207). The exact cause of clinical disease and death is still unclear but does not seem to be related only to the severity of the lesions and the bursal damage. Prostration preceding death is very similar to what is observed in acute coccidiosis, and is reminiscent of a septic shock syndrome (240). The macrophage could play a specific role in this pathology by exacerbated release of cytokines such as tumor necrosis factor of interleukin 6 (127). As macrophages are known to be activated by interferon, this role could occur through an increased secretion of interferon as has been described *in vitro* after inflection of chicken embryo cultures or *in vivo* in chickens (67).

Clinical and subclinical infections with IBDV may cause suppression of both humoral and cellular immune responses (209). The first indication of damage in the immune system was reported by Helmboldt and Gardner in 1967 (86). In 1970, Cho demonstrated that white leghorn chickens exposed to IBDV at one day of age were consistently more likely to develop visceral tumors and nerve enlargement by Marek's disease virus (32). In 1972 Allan *et al.* reported that IBDV infection at an early age was immunosuppressive, and severely depressed the antibody response to Newcastle disease virus (3). IBDV replication in the bursa leads to extensive lymphoid cell destruction in the follicular medullas and cortices (227). The acute lytic phase of the virus is associated with a reduction in circulating IgM+ cells (92, 198). IBDV-exposed chickens produce suboptimal levels of antibodies against a number of infectious and noninfectious antigens (32, 61, 128, 250).

Only the primary antibody response is impaired, the secondary responses remain intact (68, 198, 208), and this humoral deficiency may be reversible (209). Although destruction of Ig-producing B cells may be one of the principal causes of humoral deficiency, other mechanisms are possible including the adverse effect of IBDV on antigen-presenting and helper T cell functions (208). A paradox associated with IBDV infections in chickens is that although there is immunosuppression against many antigens, the response against IBDV itself is normal, even in 1-day-old susceptible chickens (212). There appears to be a selective stimulation of the proliferation of B cells committed to anti-IBDV antibody production (148).

T-cells are resistant to infection with IBDV (91)(61), and there is no evidence that the virus actually replicates in thymic lymphocytes (208, 228). However, there is evidence that *in vitro* mitogenic proliferation from T cells of IBDV exposed birds is severely decreased. This mitogenic inhibition is likely mediated by macrophages, however how IBDV induces macrophages to exhibit this suppressor effect is not clear (209).

Sharma *et al.* (209) detected a dramatic infiltration of T-cells in the bursa during acute IBDV infection, accompanied by the precipitous drop in the number of IgM+ cells. By the seventh day of infection, the infiltrating cells were predominantly CD8+ lymphocytes. It was suggested that T-cells modulate the infection, limiting viral replication in the bursa in the early phase of the disease. They also promote bursal tissue damage and delay recovery, possibly through the release of cytokines and cytotoxic effects (135). Cytotoxic T cells may exascerbate virus-induced cellular destruction by lysing cells expressing viral antigens. T cells may also promote the production of pro-inflammatory factors, such as nitric oxide, increasing tissue destruction (209).

The effect of IBDV on innate immunity is centered in the modulatory effect of IBDV on macrophage functions. There is evidence that the *in vitro* phagocytic activity of these cells is compromised (209).

In addition to causing necrosis in the lymphoid cells of the bursa, IBDV also induces apoptosis (62, 132, 175, 228, 229, 245, 246). Apoptosis is characterized by cell shrinkage and chromatin condensation and does not generate a local inflammatory response. Induction of apoptosis in infected cells contributes to the pathogenesis of IBDV in the bursa (121, 179), chicken peripheral blood lymphocytes (245), and in the thymus (102, 228). Virally-induced apoptosis can occur in cells in the absence of detectable virus (121, 175, 228). A direct effect of viral proteins like VP2 and VP5 has been implicated in the induction of apoptosis (62, 254). Apoptotic cells have also been observed in viral antigen-negative bursal cells, underlining the possible role of immunological mediators in this process (175, 228). And finally, apoptosis has also been observed in the proventriculus of IBDV challenged SPF leghorn chickens (173).

Diagnosis of IBDV. Several diagnostic procedures can be applied in the diagnosis of IBD. Diagnosis of the clinical forms of IBD is based on typical signs of the disease and on the lesions of the bursa of Fabricius. Differential diagnosis should include velogenic viscerotropic Newcastle disease, chicken infectious anemia, and mycotoxicosis. In subclinical and immunosuppressive forms of IBD, Marek's disease, chicken anemia and mycotoxicosis should be considered (136, 148).

Since the lesion caused by IBDV infection is well characterized (31, 192), diagnosis by histopathology is frequently used. This approach has the advantage of giving valuable information about the virulence of the IBDV strain involved and the possible time when the infection occurred.

Current serological tests include serum-virus neutralization and ELISA (200). ELISA is widely used because is a sensitive and rapid method. With ELISA is easy to handle large number of samples. Using serological techniques it is possible to detect the immunologic response in an outbreak or evaluate vaccination programs (178).

The virus can be isolated in embryonated eggs, cell cultures or by inoculation of susceptible birds. Inoculation in birds is the best method, because the other methods may modify the original characteristics of the naturally-occurring IBDVstrains (200).

Viral antigens may be detected by direct or indirect fluorescent antibody techniques, immunohistochemistry, agar gel immunodiffusion and antigen-capture ELISA (AC-ELISA). The use of monoclonal antibodies in the capture detection allows for more precise antigenic characterization (216, 218).

The reverse transcription-polymerase chain reaction (RT-PCR) allows for the detection of viral RNA from infected clinical samples (114, 138, 252). Differentiation of the strains is possible if the RT-PCR amplicons are further analyzed using restriction enzymes (113, 115, 117) or sequencing (7, 9). Other molecular techniques include the use of DNA probes (111, 112).

Immunity. Natural exposure to the virus, or vaccination with either live or killed vaccines, stimulates active immunity. Antibody levels are normally very high after field exposure or vaccination. Immunization of chickens is the principal method used for the control of IBD in chickens. The immunization of breeder flocks is especially important to confer passive immunity to their progeny (148). Antibody transmitted from the dam via the yolk of the egg can protect chicks against early infections with IBDV, with resultant protection against the immunosuppressive effect of the virus (148). Because maternal immunity interferes with vaccination, the major problem with active immunization of young maternally immune chicks is determining the proper time of vaccination. This determination is aided by monitoring antibody levels in a breeder flock or its progeny (241).

Satisfactory protection against IBDV can be achieved by immunization with live or inactivated vaccines. Classical live vaccines achieve lifelong and broad protection, but posses residual pathogenicity and a proportional risk of reversion to virulence (240). Many choices of live vaccines are available based on virulence and antigenic diversity. According to virulence, vaccines are classified as mild, mild intermediate, intermediate, intermediate plus, or hot. Vaccines that contain Delaware variants are also available (148). Killed vaccines in oil emulsions to stimulate high levels of maternal immunity are extensively used in the field (148). Inactivated vaccines and live vaccines made from variant strains protect chickens from disease caused by either variant or standard strains, whereas inactivated vaccines made from standard strains do not protect, or only partially protect, against challenge with variant strains (105). Very virulent strains of IBDV can be controlled adequately under experimental conditions by vaccination with commercial vaccines prepared from classical attenuated strains (53, 182, 241).

*In ovo* vaccination may provide a way for vaccines to circumvent the effects of maternal antibody and initiate a primary immune response (65). Virus-antibody complex vaccines have also emerged and seem very promising (80). This new technology utilizes specific hyperimmune neutralizing antiserum with a vaccine virus under conditions that are not sufficient to neutralize the vaccine virus but which are sufficient for delaying the pathological effects of the vaccine alone. This allows chicks to be vaccinated more effectively in the presence of passive immunity even with a strain that would be to virulent for use *in ovo* or at hatching (80). IBDV proteins, expressed in yeast or via the baculovirus system, have been studied for the use as subunit vaccines (51, 149, 189, 239). An advantage of this technology is that a vaccine based on VP2 alone should allow monitoring of the field situation by the discrimination between antibody induced by vaccine (anti-VP2 only) and that induced by infection (anti-VP2 and VP3) (240). The use of a reverse genetics system could represent a basis for the genetic attenuation of strains and for the generation of new vaccines, although interference of passive immunity will still exist. Therefore, as they are less sensitive to neutralization by anti-IBDV maternally derived antibodies, recombinant viral vaccines expressing the VP2 protein, such as fowl pox virus (10), herpesvirus of turkey (HVT)(40, 234), or fowl adenovirus (210) might be able to prime an active immune response.

Antigenic variation. The high mutation rate due to the RNA polymerase of RNA viruses, generates a genetic diversification that could lead to natural emergence of viruses with new properties that allow them to persist in immune populations (240). In the case of IBDV, these mutations lead to antigenic variations and modifications in virulence *in vivo* and attenuation *in vitro* (240).

The capsid protein, VP2, is the major host protective immunogen. Immunization of susceptible chickens with purified VP2 elicits neutralizing antibodies and confers protection against homologous virulent virus challenge (14, 60). Monoclonal antibodies raised against VP2 have the ability to neutralize homologous virus (6, 14, 215, 218). Using one neutralizing monoclonal antibody, a specific antigenic region of VP2 between amino acids 206 and 350 was identified. Since this epitope was denaturated by SDS, it was determined that is a conformationaly-dependent epitope (6). Antigenic epitopes on VP3 protein have also been reported but these antibodies are not completely neutralizing (6, 59).

Antigenic diversity between IBDV serotypes has been recognized since 1980, when serotypes 1 and 2 were defined on the basis of their lack of *in vitro* cross neutralization (153). Based on studies with monoclonal antibodies, IBDV strains belonging to serotypes 1 and 2 have been found to not share major neutralizing epitopes (13, 203). Some researchers have developed polyvalent neutralizing antiserotype 1 monoclonal antibodies such as monoclonal antibodies 1, 6, 78 and 9 (55), monoclonal antibody 8 (218), and monoclonal antibodies 6F6 and 7C9 (243).

Antigenic differences have been demonstrated within serotype 1, and the study of different strains has led to dividing serotype 1 into six subtypes, differentiated by cross neutralization assays using polyclonal sera (108).

Studies with monoclonal antibodies demonstrated the presence of a number of modified neutralizing epitopes among antigenically variant strains detected in the United States. Based on this evidence, there may have been an antigenic shift in IBDV viruses in the US (216). There are a minimum of at least five neutralization epitopes on the standard IBDV strains as defined by the monoclonal antibodies 8, 179, R63, B69 and 10. Delaware viruses have lost the B69 site, GLS viruses lack the B69 and R63 sites, and the DS326 virus lacked the sites for monoclonal antibodies B69, R63 and 179 (216). Thus on the basis of the reactivities with various monoclonal antibodies, the IBDV viruses are antigenically grouped as classic or standard, GLS, DS326 and Delaware type variants (238).

In spite of their enhanced pathogenic properties, the vvIBDV strains were considered to be closely antigenically related to the standard strains such as the Faragher 52/70 strain, on the basis of high cross-neutralization indices (53). Using neutralizing monoclonal antibodies developed by Snyder to characterize US IBDV variants, van der Marel studied twelve European isolates of IBDV. He detected no important differences between the standard strain 52/70 and vvIBDV(244). Similar data was produced by Öppling *et al* (182). However, Etterradossi *et al.* (54) developed nine other monoclonal antibodies and using these he detected modified binding and neutralizing properties against French vvIBDV strains. All their monoclonal antibodies neutralized most mild or intermediate vaccines strains, whereas two monoclonal antibodies did not neutralize a French vvIBDV strain, as well as US variant A, and the European strain Faragher 52/70. Based on their results, they suggested a neutralizing epitope may be altered in the European vvIBDV strains, causing decreased antibody neutralization. This difference could be used to differentiate vvIBDV strains (54, 55).

Molecular basis of IBDV variability. Nucleic acid sequencing of genes coding for VP2 and subsequent deduction of their predicted amino acid sequences, lead to the identification of a hypervariable region. The amino acid changes between strains are not evenly distributed throughout the open reading frame but are clustered in certain regions. Most of the changes that occur in VP2 are located between amino acids 239 and 332 (9, 134, 238). This highly variable region falls entirely within those sequences of VP2 identified as the minimum region required for reaction with virus neutralization monoclonal antibody (6, 60, 251).

Hydrophilicity profiles of this region show that there are two hydrophilic peaks at either end of this region, the larger peak being from amino acids 212 to 224 and the other from 314 to 324. These hydrophilic regions have been shown to be important in binding of neutralizing antibodies and, hence, are presumed to be a main part of the neutralizing domain (85, 203). It is interesting that most of amino acid variations in this region fall within these two peaks (9, 134).

Variations in IBDV antigenicity depend on changes in hydrophilic peaks. The serotype 2 strain 23/82 (203), the North-American antigenic variants A, E, GLS and DS326 (85, 134, 238), and neutralization resistant escape mutants (203) all exhibit amino acid changes in these hydrophilic peaks. Only differences in the intervening hydrophobic domains are found between typical serotype 1 strains (238).

A nucleotide sequence comparison suggested that four amino acid alterations in the VP2 protein of the Delaware E strain allowed this variant to escape neutralizing antibodies. These amino acids were located at positions 213, 222, 318, and 323 (85). By restriction enzyme and amino acid sequence analysis, point mutations have been detected at residues 222, 254 and 323. Amino acid residues 222 and 254 are consistently mutated in the variant strains (47, 116). Glycine is present in the standard strains, amino acid residue number 254, whereas the variants have serine at this position (47, 116).

Vakharia *et al.* (238) used monoclonal antibodies to correlate antigenic variations with amino acid sequence substitutions in the hypervariable region of VP2. They found that the amino acid residue glutamine at position 249 might be involved in the binding of neutralizing antibody B69, which recognizes epitopes in standard strains. All the variant viruses have lysine instead of glutamine at this position, and they escape binding with antibody B69.

Using a baculovirus expression system to synthesize all the structural proteins coded for in segment A of the IBDV genome, Vakharia *et al.* (237), produced virus like particles. They mapped the antigenic sites by producing chimeric cDNA clones of IBDV using the variant GLS plasmid as a backbone and inserting fragments from the D78 and Delaware strains. At least two antigenic sites are present on the surface of IBDV, one resides between amino acid residues 222 and 249, and the other between 269 and 323.

The role of VP1 in the virulence of IBDV is not yet established. It is likely that the viral polymerase would influence the replication rate and, thus the pathogenic potential of a virus. The VP1 sequences of very virulent IBDV strains are genetically distinct from those of classical virulent or attenuated strains thus, VP1 of vvIBDV constitutes a genetic lineage distinct from that of classical virulent or attenuated strains and serotype 2 strains as well (104).

In highly virulent strains three specific amino acid residues in VP2 have been reported at position 222 (Ala), 256 (Ile), 294 (Ile) and 299 serine which differ from classical strains (22). These substitutions are also present in other strains isolated from other countries such as Germany (256), Bangladesh (103) China (29), Israel (190), Japan (143, 252), Taiwan (144), Malaysia (33, 95), Nigeria (231, 256), Vietnam (231), and Brazil (41, 100). Positions 222 - 223 and 318 - 324 may be critical for the vvIBDV (56). These positions have been identified as "hot spots" for mutations in several escape mutants resistant to selected neutralizing monoclonal antibodies (203, 243).

Immunosuppression in chickens. In poultry production, immunosuppressive diseases have been and remain economically important. Vaccination failure, increased condemnation and mortality, poor feed conversion, and increased morbidity and medication costs commonly result from immunosuppression. Immunosuppression has been defined as "a state of temporary or permanent dysfunction of the immune response resulting from damage to the immune system and leading to increased susceptibility to disease" (46). Numerous immunosuppressive agents affect avian and mammalian species (167) including viruses, prokaryotic and eukaryotic parasites, microbial toxins, chemicals, drugs, nutritional deficiencies (137) and various psychological or physical-environmental stressors (45). Infectious bursal disease virus (IBDV) is of major interest because of the widespread occurrence of the infection in commercial chickens. Infection with IBDV at an early age significantly compromises the humoral and local immune responses of the chickens (201). Chicken anemia virus is also an important pathogen in poultry and appears to target erythroid and lymphoid progenitor cells in the bone marrow and thymus respectively (1). Destruction of erythroid and myeloid progenitors in bone marrow results in severe anemia, and depletion of granulocytes and thrombocytes. Destruction of T cells result in depletion of mature cytotoxic and helper T cells with consequent immune suppression. In Marek's disease virus (MDV) infection, the degree of immunosuppression is determined by persistence of early cytolytic infection, atrophy of bursa of Fabricius and thymus, and histologic evidence of necrosis and atrophy in lymphoid organs (26, 28). Syndromes caused by dietary consumption of feed containing moderate to high levels of mycotoxins range from acute mortality to slow growth and reduced reproductive efficiency (188). Consumption of lower levels of fungal toxic metabolites may result in impaired immunity and decreased resistance to infectious disease. Mycotoxin-induced immunosuppression may be manifested as depressed T or B lymphocyte activity, suppressed immunoglobulin and antibody production, reduced complement activity, or impaired macrophage-effector cell function (35).

Treatments of chickens with cyclophosphamide or cyclosporin have been used as a means of inhibiting the humoral or cell-mediated immune responses in order to determine the role of T and B cells in protective responses to infectious pathogens of chickens (36, 58, 63, 106, 196,247).

Cyclophosphamide is an antineoplastic agent and immunomodulator used therapeutically in the treatment of tumors and autoimmune disorders. The parent compound, cyclophosphamide, *in vitro* is neither alkylating, cytotoxic, nor immunosuppressive (76). *In vivo,* cyclophosphamide is converted by hepatic microsomal enzymes to 4-hydroxycyclophosphamide (4-OHCP) that is reversibly altered to aldophosphamide (AP) (34). Then the 4-OHCP/AP compound is either enzymatically detoxified or undergoes spontaneous degradation to phospharamide mustard (PM) and acrolein within cells (34). This alkylating agent induces DNA cross-links - an important step in causing the development of point mutations and chromosome aberrations (34). Newly hatched chickens treated with cyclophosphamide are rendered irreversibly B cell deficient (142). Furthermore, selective B-lymphocyte cytotoxicity is most dramatically achieved when cyclophosphamide exposure occurs during embryogenesis (248). T cells can be killed or their proliferation slowed by single or multiple, high dose CP treatment in neonatal chicks, but the numbers of T cells in thymus can recover in two weeks (69). The selective toxicity of cyclophosphamide is primarily due to its differential lymphocyte sensitivity, and not due to differential compound distribution, uptake by immune tissues, or to site-specific activation and detoxification (159). Structure-activity studies in the chick embryo revealed induction of selective B lymphocyte toxicity that was induced by cyclophosphamide analogs capable of forming DNA interstrand cross-links (248).

Cyclosporin, a selective T-cell immunosuppressant drug, depresses cell-mediated immunity in chickens, causing prolonged skin graft survival, depressed proliferative responses in mitogen-stimulated lymphocytes and decreased wattle T-lymphocyte responses to injected antigen (88). Cyclosporin prevents the synthesis of cytokines by T cells by blocking a late stage in the signaling pathway initiated by the T-cell receptor. This especially affects the production of interleukin-2 (IL-2), hence T cell proliferation is reduced. As a consequence, IL-2 dependent functions which include T-helper activities, cytotoxicity, natural killer cell activity and antibody dependent cell cytotoxicity would be depressed after cyclosporin treatment (88).

### References:

1. Adair. Dev Comp Immunol 24:247-255. 2000.
2. Alexander. Diseases of Poultry 11 ed. Saif, Y. M. Iowa State Press Ames, IA. 63-87. 2003.
3. Allan et al. Vet Rec 90:511-512. 1972.
4. Apple et al. Avian Dis 35:422-425. 1991.
5. Atabay et al. J Appl Microbiol. 84:1017-1024. 1998.
6. Azad et al. Virology 161:145-152. 1987.
7. Banda et al. Avian Dis 45:620-630. 2001.
8. Banda et al. Avian Dis. 47:87-95. 2003.
9. Bayliss et al. J Gen Virol 71 (Pt 6):1303-1312. 1990.
10. Bayliss et al. Arch Virol 120:193-205. 1991.
11. Bayyari et al. Poult Sci 74:1799-1809. 1995.
12. Bayyari et al. Poult Sci 74:1961-1969. 1995.
13. Becht. Curr Top Microbiol Immunol 90:107-121. 1980.
14. Becht et al. J Gen Virol 69 (Pt 3):631-640. 1988.
15. Benton et al. Avian Dis 11:430-438. 1967.
16. Benton et al. Avian Dis 11:438-445. 1967.
17. Bernard et al. J Virol. 33:717-723. 1980.
18. Birghan et al. Embo J 19:114-123.2000.
19. Blagburn et al. J Protozool 38:25S-28S. 1991.
20. Bottcher et al. J Virol 71:325-330.1997.
21. Bracewell et al. World's Poult Sci J 40. 1984.
22. Brown et al. J Gen Virol 75 (Pt 3):675-680. 1994.
23. Bruenn. Nucleic Acids Res 19:217-226. 1991.
24. Brugh. Avian Dis 30:199-203. 1986.
25. Burkhardt & Muller. Arch Virol 94:297-303. 1987.
26. Buscaglia et al. J Gen Virol 69:1067-1077. 1988.
27. Calnek. Diseases of Poultry 11th ed. Saif, Y. M. Iowa State Press Ames, IA. 271-282. 2003.
28. Calnek et al. Avian Dis 42:124-132. 1998.
29. Cao et al. Avian Dis 42:340-351. 1998.
30. Chettle et al. Vet Rec 125:271-272. 1989.
31. Cheville. Am J Pathol 51:527-551. 1967.
32. Cho. Avian Dis 14:665-675. 1970.
33. Chong et al. Acta Virol 45:217-226. 2001.
34. Colvin & Hilton. Cancer Treat Rep 65:89-95. 1981.
35. Corrier. Vet Immunol Immunopathol 30:73-87. 1991.
36. Corrier et al. Avian Dis 35:40-45. 1991.
37. Cosgrove. Avian Dis 6:385-389. 1962.
38. Craig. Proc 20th National Meeting on Poultry Health and Condemnations, Ocean City, MD. 35-37. 1985.
39. Cullen et al. Am J Res 49:5. 1988.
40. Darteil et al. Virology 211:481-490. 1995.
41. Di Fabio et al. Vet Rec 145:203-204. 1999.
42. Dobos. J Virol 32:1047-1050. 1979.
43. Dobos et al. J Virol 32:593-605. 1979.
44. Dobos & Roberts. Can J Microbiol 29:377-384. 1983.
45. Dohms & Metz. Vet Immunol Immunopathol 30:89-109. 1991.
46. Dohms & Saif. Avian Dis. 28:305-310. 1984.
47. Dormitorio et al. Avian Dis 41:36-44. 1997.
48. Dormitorio et al. Southern Conference on Avian Diseases, Atlanta, GA. 40. 2001.
49. Dorner et al. Appl Environ Microbiol 46:698-703. 1983.
50. Duncan et al. J. Virol. 61:3655-3664. 1987.
51. Dybing & Jackwood. Avian Dis 42:80-91. 1998.
52. Erichsen & Harboe. Acta Pathol Microbiol Scand 33:56-71. 1953.
53. Eterradossi et al. Zentralbl Veterinarmed [B] 39:683-691. 1992.
54. Eterradossi et al. Arch Virol 142:2079-2087. 1997.
55. Eterradossi et al. Arch Virol 142:255-270. 1997.
56. Eterradossi et al. Arch Virol 143:1627-1636. 1998.
57. Eterradossi et al. Avian Pathol. 28:36-46. 1999.
58. Fadly et al. Avian Dis 29:12-25. 1985.
59. Fahey et al. J Gen Virol 66 (Pt 12):2693-2702. 1985.
60. Fahey et al. J Gen Virol 70 (Pt 6):1473-1481. 1989.
61. Faragher et al. Vet Rec 95:385-388. 1974.
62. Fernandez-Arias et al. J Virol 71:8014-8018. 1997.
63. Fitzgerald et al. Avian Dis 43:476-483. 1999.
64. Fox & Lee. Lab Anim Sci 47:222-225. 1997.
65. Gagic et al. Avian Dis 43:293-301. 1999.
66. Gardner et al. Nature 387:245. 1997.
67. Gelb et al. Avian Dis 23:634-645. 1979.
68. Giambrone et al. Am J Vet Res 38:581-583.1977.
69. Glick. Transplantation 11:4334-4439. 1971.
70. Goble & Kutz. J Parasitology 31:323-331. 1945.
71. Good. Poult Dig 41:298. 1982.
72. Goodwin. Avian Dis 39:643-645. 1995.
73. Goodwin & Hafner. Diseases of Poultry. 10th ed. Calnek, B. W. Iowa State University Press Ames, IA. 1034-1038. 1997.
74. Goodwin et al. Avian Pathol 25:269-279. 1996.
75. Gregory, C. R., B. W. Ritchie, K. S. Latimer, W. L. Steffens, D. Pesti, R. Campagnoli, and P. D. Lukert. Progress in understanding proventricular dilatation disease. International Aviculturists Society, Orlando, FL. 1998
76. Grochow, L. B., and M. Colvin. Clinical pharmacokinetics of cyclophosphamide. Clin Pharmacokinet. 4:380-394. 1979.
77. Gulbahar et al. Aust Vet J 78:247-249. 2000.
78. Gupta & Trapp. Avian Dis 15:408-412. 1971.
79. Guy & Barnes. 139th Meeting of the American Veterinary Medical Association, Nashville, TN. 2002.
80. Haddad et al. Avian Dis 41:882-889. 1997
81. Harkness et al. Arch Virol 48:63-73. 1975.
82. Harry et al. Br Poult Sci 16:69-78. 1975.
83. Hassan et al. Avian Dis 40:567-571. 1996.
84. Hayes & Wobeser. Can J Comp Med 47:180-187. 1983.
85. Heine et al. J Gen Virol 72 (Pt 8):1835-1843. 1991.
86. Hemboldt & Garner. Avian Dis 8:561-575.1964.
87. Henderson et al. Vet Rec 123:492-494. 1988.
88. Hill et al. Avian Dis 33:86-92. 1989.
89. Hiraga et al. J Vet Med Sci 56:1057-1063. 1994
90. Hirai et al. Infect Immun 10:1235-1240. 1974.
91. Hirai & Calnek. Infect Immun 25:964-970. 1979.
92. Hirai et al. Avian Dis 25:484-496. 1981.
93. Hitchner. Poult Sci 49:511-516. 1970.
94. Hodges. The histology of the fowl. Academic Press. London. 1974.
95. Hoque et al. Avian Dis 30:369-380. 2001.
96. Howie & Thorsen. Can J Comp Med 45:315-320. 1981.
97. Huchzermeyer et al. Vet Rec 133:143-144. 1993.
98. Hudson et al. Nucleic Acids Res 14:5001-5012. 1986.
99. Huff et al. Avian Dis 45:828-843. 2001.
100. Ikuta et al. Avian Dis 45:297-306. 2001.
101. Inoue et al. J Vet Med Sci 54:575-577. 1992.
102. Inoue et al. Avian Dis 38:839-846. 1994.
103. Islam et al. J Vet Med B Infect Dis Vet Public Health 48:211-221. 2001.
104. Islam et al. Arch Virol 146:2481-2492. 2001.
105. Ismail & Saif. Avian Dis 35:460-469. 1991.
106. Isobe et al. Dev Comp Immunol 24:433-441. 2000.
107. Hill. Physiology and Biochemistry of the Domestic Fowl. Ed. B. M. Freeman. Academic Press. London. 1983.
108. Jackwood & Saif. Avian Dis 31:766-770. 1987.
109. Jackwood et al. Avian Dis 26:871-882. 1982.
110. Jackwood et al. Avian Dis 29:1184-1194. 1985.
111. Jackwood. Vet Microbiol 24:253-260. 1990
112. Jackwood et al. Avian Dis 36:154-157. 1992.
113. Jackwood & Jackwood. Avian Dis 38:531-537. 1994.
114. Jackwood et al. Avian Dis 40:457-460. 1996.
115. Jackwood & Jackwood. Molecular identification of infectious bursal disease virus strains. Avian Dis 41:97-104.1997.
116. Jackwood et al. Virus Res 49:131-137. 1997.
117. Jackwood & Sommer. Avian Dis 42:321-339. 1998.
118. Jagadish et al. J Virol 62:1084-1087. 1988.
119. Jeffrey et al. Avian Dis 38:630-634. 1994.
120. Jensen et al. Avian Dis 35:969-973. 1991.
121. Jungmann et al. J Gen Virol 82:1107-1115. 2001.
122. Kelly et al. 138th American Veterinary Medical Association, Boston, MA. 2001.
123. Kibenge, F. S., A. S. Dhillon and R. G. Russell. Biochemistry and immunology of infectious bursal disease virus. J Gen Virol 69 (Pt 8):1757-1775. 1988.
124. Kibenge et al. Arch Virol 141:1133-1141. 1996.
125. Kibenge & Dhama. Arch Virol 142:1227-1236. 1997.
126. Kibenge et al. Arch Virol 142:2401-2419. 1997
127. Kim et al. Vet Immunol Immunopathol 61:331-341. 1998.
128. Kim et al. Avian Dis 43:401-413. 1999.
129. Kitamura et al. Arch Virol 145:2003-2014. 2000.
130. Kouwenhoven et al. Avian Pathol 7:183-187. 1978.
131. Kouwenhoven et al. Avian Pathol 17:879-892. 1988.
132. Lam. J Comp Pathol 116:367-377. 1997.
133. Lam. Microb Pathog 25:147-155. 1998.
134. Lana et al. Virus Genes 6:247-259. 1992.
135. Lasher & Davis. Avian Dis 41:11-19. 1997.
136. Lasher & Davis. World's Poult Sci J 50:134-166. 1994.
137. Latshaw. Vet Immunol Immunopathol 30:111-120. 1991.
138. Lee et al. J Virol Methods 40:243-253. 1992.
139. Lee et al. Dis Aquat Organ 37:13-21. 1999.
140. Lejal et al. J Gen Virol 81:983-992. 2000.
141. Lenz et al. J Vet Diagn Invest 10:145-151. 1998.
142. Lerman & Weidanz. J Immunol. 105:614-619. 1970.
143. Lin et al. Avian Dis 37:315-323. 1993.
144. Liu et al. Res Vet Sci 70:139-147. 2001.
145. Liu et al. Avian Dis 44:161-169. 2000.
146. Lombardo et al.. J Virol 73:6973-6983. 1999.
147. Lombardo et al. Virology 277:345-357. 2000.
148. Lukert & Saif. Diseases of Poultry. 10th B. W. Calnek, H. J. B., C. W. Beard, L. R. McDougald, Y. M. Saif. Iowa State University Press Arnes, IA. 721-738- 1997.
149. Macreadie et al. Vaccine 8:549-552. 1990.
150. Martinez-Torrecuadrada et al. Virology 278:322-331. 2000.
151. Matsumoto, R., and Y. Hashimoto. Distribution and developmental change of lymphoid tissues in the chicken proventriculus. J Vet Med Sci 62:161-167. 2000.
152. McAllister et al. Poult Sci 74:45-49. 1995.
153. McFerran et al. Avian Pathol 9:395-404. 1980.
154. McLelland. Form and function in birds. Vol 1. A. S. King, a. J. M. Academic Press. London. 103-132. 1979.
155. McNulty & Saif. Avian Dis 32:374-375. 1988.
156. McNulty et al. Avian Pathol 8:205-212. 1979.
157. McNulty et al. Avian Pathol 13:429-439. 1984.
158. Mertens et al. J Gen Virol 59:47-56. 1982.
159. Misra & Bloom. Toxicology 66:239-256. 1991.
160. Morales & Boclair. 42nd. Western Poultry Disease Conference, Sacramento, CA. 91-92. 1993.
161. Muller & Becht. J Virol 44:384-392. 1982.
162. Muller & Nitschke. Virology 159:174-177. 1987.
163. Muller & Nitschke. Med Microbiol Immunol (Berl) 176:113-121. 1987.
164. Muller et al. Zentralbl Veterinarmed [B] 26:345-352. 1979.
165. Mundt et al. J Gen Virol 76 (Pt 2):437-443. 1995.
166. Mundt et al. J Virol 71:5647-5651. 1997.
167. Muneer et al. Br Vet J 144:288-301. 1988.
168. Mussman & Twiehaus. Avian Dis 15:483-502. 1971.
169. Mutlu et al. Tierarztl Prax Ausg G Grosstiere Nutztiere 25:460-462. 1997.
170. Nagarajan & Kibenge. Can J Vet Res 61:81-88. 1997.
171. Nawathe et al. Vet Rec 102:444. 1978.
172. Newberne et al. J Am Vet Assoc 128:553-555. 1956.
173. Newberry. Ph.D. Dissertation. Fayetteville, AR, University of Arkansas. 1996.
174. Nick et al. J Virol 18:227-234. 1976.
175. Nieper et al. Avian Pathol. 28:279-285. 1999.
176. Norton & Ruff. Diseases of Poultry 11th ed. Saif, Y. M. Iowa State Press Ames, IA. 937-938. 2003.
177. Nunoya et al. Avian Dis 36:597-609. 1992.
178. Odor, E. M. Elisa serology: Application to ibd in broiler production. International Poultry Symposium-Summit on Infectious Bursal Disease, Athens, GA. 104-105. 1995.
179. Ojeda et al. Avian Dis 41:312-316. 1997.
180. Okoye & Uche. Acta Vet Brno 58:91-96. 1986.
181. Olah et al. Poult Sci 82:767-770. 2003.
182. Oppling et al. Arch Virol 119:211-223. 1991.
183. Page et al. Avian Dis 26:618-624. 1982.
184. Pegram et al. Avian Dis 26:47-59. 1982.
185. Pegram & Wyatt. Poult Sci 60:2429-2440. 1981.
186. Persson et al. J Virol 44:437-443. 1982.
187. Phalen & Moore. Avian Dis 47:254-260. 2003.
188. Pier et al. J Am Vet Med Assoc. 179:719-724. 1980.
189. Pitcovski et al. Avian Dis 40:753-761. 1996.
190. Pitcovski et al. Avian Dis 42:497-506. 1998.
191. Poole. 43rd Western Poultry Disease Conference, Sacramento, CA. 40-42. 1994.
192. Pope. Avian histopathology. Ridell, C. American Association of Avian Pathologists, Kennett Square. PA 17-44. 1996.
193. Reece. Infectious proventriculitis and stunting syndrome of broiler chickens. Canberra, Australia, RIRDC. 2002.
194. Reece &. Frazier. Avian Pathol. 19:723-758. 1990.
195. Revet & Delain. Virology 123:29-44. 1982.
196. Reynolds & Maraqa. Avian Dis 44:145-154. 2000.
197. Riddell. Avian Dis 20:442-445. 1976.
198. Rodenberg et al. Avian Dis 38:16-21. 1994.
199. Rosenberger &Cloud. J Vet Med Assoc 189:357. 1986.
200. Rosenberger et al. A laboratory manual for the isolation and identification of avian pathogens 4th edition. D. E. Swayne, J. R. G., M. W. Jackwood, J. E. Pearson, W. M. Reed. American Association of Avian Pathologists Kennet Square, PA. 215-218. 1998.
201. Saif. Vet Immunol Immunopathol 30:45-50. 1991.
202. Sanchez & Rodriguez. Virology 262:190-199. 1999.
203. Schnitzler et al. J Gen Virol 74 (Pt 8):1563-1571. 1993.
204. Schulze & Heidrich. Dtsch Tierarztl Wochenschr 108:264-266. 2001.
205. Sellers et al. Avian Dis 45:26-33. 2001.
206. Shapiro & Nir. Poult Sci 74:33-44. 1995.
207. Sharma & Lee. Infect Immun 42:747-754. 1983.
208. Sharma et al. Avian Dis 33:112-124. 1989.
209. Sharma et al. Dev Comp Immunol 24:223-235. 2000.
210. Sheppard et al. Arch Virol 143:915-930. 1998.
211. Simoes & Sarnow. J Gen Virol 74:661-668. 1993.
212. Skeeles et al. Avian Dis 23:107-117. 1979.
213. Skeeles et al. Poult Sci 77 (suppl.):133. 1998.
214. Snedeker et al. Avian Dis 11:519-528. 1967.
215. Snyder et al. Avian Dis 32:527-534. 1988.
216. Snyder et al. Avian Dis 32:535-539. 1988.
217. Snyder. Avian Pathol. 19:419-423. 1990.
218. Snyder et al. Arch Virol 127:89-101. 1992.
219. Songserm et al. Avian Dis 44:556-567. 2000.
220. Spies et al. Virus Res 8:127-140. 1987.
221. Stuart et al. J Environ Pathol Toxicol Oncol 6:369-386. 1986.
222. Sturkie. Avian Physiology. Fourth edition, Springer-Verlag. New York. 1986.
223. Survashe et al. Avian Pathol. 8:77-93. 1979.
224. Swayne & Halvorson. Diseases of Poultry 11th ed. Saif, Y. M. Iowa State Press Ames, IA. 135-160. 2003.
225. Tacken et al. J Gen Virol 81:209-218. 2000.
226. Tanimura et al. Avian Dis 39:9-20. 1995.
227. Tanimura & Sharma. Avian Dis 41:638-645. 1997.
228. Tanimura & Sharma. J Comp Pathol 118:15-27. 1998.
229. Tham & Moon. Avian Dis 40:109-113. 1996.
230. Thayer & Walsh. Poult Sci 72:741-746. 1993.
231. To et al. J Vet Med Sci 61:429-432. 1999.
232. Tsai, S. S., J. H. Park, K. Hirai and C. Itakura. Catarrhal proventriculitis associated with a filamentous organism in pet birds. Jpn J Vet Res 40:143-148. 1992.
233. Tsai et al. Chin J Microbiol Immunol 16:307-311. 1983.
234. Tsukamoto et al. Virology 257:352-362. 1999.
235. Turpin. Master Thesis. Athens, GA, University of Georgia. 1998.
236. Underwood et al. J Gen Virol 36:93-109. 1977.
237. Vakharia. Biotech Ann Rev 3:15-168. 1997.
238. Vakharia et al. Virus Res 31:265-273. 1994.
239. Vakharia et al. Vaccine 12:452-456. 1994.
240. van den Berg. Avian Pathol 29:174-194. 2000.
241. Van den Berg & Meulemans. Avian Pathol. 20:409-421. 1991.
242. Van den Berg et al. Avian Pathol. 20:133-143. 1991.
243. Van der Berg et al. Avian Pathol. 25:751-768. 1996.
244. Van der Marel et al. Dtsch TierartI Wochenschr 97:81-83. 1990.
245. Vasconcelos & Lam. J Gen Virol 75 (Pt 7):1803-1806. 1994.
246. Vasconcelos & Lam. J Comp Pathol 112:327-338. 1995.
247. Weinack et al. Avian Dis 28:416-425.1984.
248. Wilmer & Bloom. Mutat Res 253:161-172. 1991.
249. Winterfield et al. L&M News Views 3:103. 1962.
250. Wyeth. Vet Rec 96:238-243. 1975.
251. Yamaguchi et al. Arch Virol 141:1493-1507. 1996.
252. Yamaguchi et al. Arch Virol 142:1441-1458. 1997.
253. Yao et al. J Virol 72:2647-2654. 1998.
254. Yao & Vakharia. Virology 285:50-58. 2001.
255. Yu et al. Avian Dis 45:416-424. 2001.
256. Zierenberg et al. Arch Virol 145:113-125. 2000.

### INFECTIOUS BURSAL DISEASE VIRUS AND PROVENTRICULTIS IN BROILER CHICKENS (See, Pantin-Jackwood & Brown. 2003. Avian Diseases.47:681-690)

Table 4. Naturally occurring cases of proventriculitis. Histopathology, RT-PCR for IBDV, IHC for IBDV, and apoptosis staining (TUNEL) on formalin-fixed, paraffin-embedded tissue sections of bursas and proventriculi from broiler chickens with proventriculitis.

| | Bursa | | | | Proventriculus | | | |
|---|---|---|---|---|---|---|---|---|
| Bird | LS^{A} | RT-PCR | IHC^{C} | TUNEL^{D} | LS | RT-PCR | IHC | TUNEL |
| 1 | 2 | - | - | + | 4 | - | - | + |
| 2 | 3 | + | + | ++ | 4 | - | - | + |
| 3 | 2 | - | - | + | 4 | - | - | + |
| 4 | 3 | + | + | ++ | 4 | - | - | + |

| | | | | | | | | |
|---|---|---|---|---|---|---|---|---|
| ^{A}LS = lesion score. 1 = no lesions. For bursal sections, 2 = mild variation in follicle size; 3 = moderate variation in size of follicles; 4 = either necrosis or follicle atrophy. For Proventricular sections, 2 = mild glandular lumenal ectasia; 3 = ectasia plus lymphoid infiltrates in the interglandular interstitium; 4 = either acute glandular necrosis or severe fibrosis with lymphoid infiltrates. ^{B}Reverse transcriptase polymerase chain reaction; - = negative; + = positive. ^{C}Immunohistochemistry; - = no staining; + = minimal staining; ++ = moderate staining; +++ = intense staining. ^{D}TUNEL= terminal deoxynucleotidyl transferase mediated dUTP nick end labeling; - = no staining; + = minimal staining; ++ = moderate staining; +++ = intense staining. | | | | | | | | |

**Table 5. Experimental chickens. 4 dpi. Histopathology, RT-PCR for IBDV, IHC for IBDV, and apoptosis staining (TUNEL) on formalin-fixed, paraffin-embedded tissue sections of proventriculi, bursas, and thymuses from broiler chickens challenged with different strains of IBDV. Data for individual birds.**

| | Proventriculus | | | | Bursa | | | | Thymus | | | |
|---|---|---|---|---|---|---|---|---|---|---|---|---|
| Strain^{A} | LS^{B} | RT-PCR^{C} | IHC^{D} | TUNEL^{E} | LS | RT-PCR | IHC | TUNEL | LS | RT-PCR | IHC | TUNEL |
| Control | 1 | - | - | - | 1 | - | - | + | 1 | - | - | + |
| | 1 | - | - | - | 1 | - | - | + | 1 | - | - | + |
| STC | 1 | - | - | + | 4 | + | +++ | +++ | 4 | + | - | ++ |
| | 1 | + | + | + | 4 | + | +++ | +++ | 3 | + | - | ++ |
| GLS | 1 | - | - | - | 2 | + | + | ++ | 1 | - | - | ++ |
| | 1 | + | - | - | 4 | + | +++ | +++ | 1 | + | - | ++ |
| Var.E | 1 | + | - | + | 4 | + | ++ | +++ | 1 | + | + | ++ |
| | 1 | - | - | - | 4 | + | ++ | +++ | 1 | + | - | ++ |
| Var.A | 1 | - | - | - | 4 | + | ++ | +++ | 1 | + | - | ++ |
| | 1 | - | - | - | 4 | + | +++ | +++ | 1 | - | - | ++ |
| D78 | 1 | - | - | - | 2 | + | ++ | ++ | 1 | - | - | ++ |
| | 1 | - | - | - | 2 | + | + | ++ | 2 | + | - | ++ |
| Bursine | 1 | - | - | - | 4 | + | + | ++ | 1 | - | - | ++ |
| | 1 | - | - | - | 4 | + | ++ | ++ | 1 | - | - | ++ |
| Lukert | 1 | - | - | - | 1 | - | - | + | 1 | - | - | + |
| | 1 | - | - | - | 1 | - | - | + | 1 | - | - | + |

| | | | | | | | | | | | | |
|---|---|---|---|---|---|---|---|---|---|---|---|---|
| ^{A}IBDV strain used for challenging chickens ^{B}LS = lesion score. 1 = no lesions. For bursal sections, 2 = mild variation in follicle size; 3 = moderate variation in size of follicles; 4 = either necrosis or follicle atrophy. For proventricular sections, 2 = mild glandular lumenal ectasia; 3 = ectasia plus lymphoid infiltrates in the interglandular interstitium; 4, either acute glandular necrosis or severe fibrosis with lymphoid infiltrates. ^{C}Reverse transcriptase polymerase chain reaction; - = negative; + = positive. ^{D}Immunohistochemistry; - = no staining; + = minimal staining; ++ = moderate staining; +++ = intense staining. ^{E}TUNEL= terminal deoxynucleotidyl transferase mediated dUTP nick end labeling; - = no staining; + = minimal staining; ++ = moderate staining; +++ = intense staining. | | | | | | | | | | | | |

**Table 6. Experimental chickens 6 dpi. Histopathology, RT-PCR for IBDV, IHC for IBDV, and apoptosis staining (TUNEL) on formalin-fixed, paraffin-embedded tissue sections of proventriculi, bursas, and thymuses from broiler chickens challenged with different strains of IBDV. Data for individual birds.**

| | Proventriculus | | | | Bursa | | | | Thymus | | | |
|---|---|---|---|---|---|---|---|---|---|---|---|---|
| Strain^{A} | LS^{B} | RT-PCR^{C} | IHC^{D} | TUNEL^{E} | LS | RT-PCR | IHC | TUNEL | LS | RT- PCR | IHC | TUNEL |
| Control | 1 | - | - | - | 1 | - | - | + | 1 | - | - | + |
| | 1 | - | - | - | 1 | - | - | + | 1 | - | - | + |
| STC | 1 | - | + | + | 4 | + | +++ | +++ | 4 | + | + | ++ |
| | 3 | + | + | + | 4 | + | +++ | +++ | 3 | + | - | ++ |
| GLS | 1 | - | - | + | 4 | + | + | +++ | 1 | - | - | ++ |
| | 2 | + | + | + | 4 | + | + | +++ | 1 | + | - | ++ |
| Var.E | 1 | - | - | - | 4 | + | ++ | +++ | 1 | + | ++ | ++ |
| | 1 | + | - | + | 4 | + | ++ | +++ | 1 | + | + | ++ |
| Var.A | 1 | - | - | - | 4 | + | ++ | +++ | 1 | + | - | ++ |
| | 2 | + | - | + | 4 | + | ++ | +++ | 1 | - | - | ++ |
| D78 | 1 | - | - | - | 4 | + | +++ | ++ | 1 | - | - | ++ |
| | 1 | + | - | + | 4 | + | +++ | ++ | 2 | + | - | ++ |
| Bursine | 1 | - | - | - | 4 | + | + | ++ | 1 | - | - | ++ |
| | 1 | + | - | - | 4 | + | ++ | ++ | 1 | - | - | ++ |
| Lukert | 1 | - | - | - | 1 | - | - | + | 1 | - | - | + |
| | 1 | - | - | - | 1 | - | - | + | 1 | - | - | + |

| | | | | | | | | | | | | |
|---|---|---|---|---|---|---|---|---|---|---|---|---|
| ^{A}IBDV strain used for challenging chickens ^{B}LS = lesion score. 1 = no lesions. For bursal sections, 2 = mild variation in follicle size; 3 = moderate variation in size of follicles; 4 = either necrosis or follicle atrophy. For proventricular sections, 2 = mild glandular lumenal ectasia; 3 = ectasia plus lymphoid infiltrates in the interglandular interstitium; 4, either acute glandular necrosis or severe fibrosis with lymphoid infiltrates. ^{C}Reverse transcriptase polymerase chain reaction; - = negative; + = positive. ^{D}Immunohistochemistry; - = no staining; + = minimal staining; ++ = moderate staining; +++ = intense staining. ^{E}TUNEL= terminal deoxynucleotidyl transferase mediated dUTP nick end labeling; - = no staining; + = minimal staining; ++ = moderate staining; +++ = intense staining. | | | | | | | | | | | | |

### REPRODUCTION OF PROVENTRICULITS IN COMMERCIAL AND SPF BROILER CHICKENS (Pantin-Jackwood et al. Avian Diseases, in press)

SUMMARY. Proventriculitis was studied by experimentally reproducing the disease in broiler chickens. One-day-old commercial and SPF broilers were orally gavaged with a proventricular homogenate produced from the proventriculi of broilers with proventriculitis. At 7 and 14 days post-inoculation differences in weight gain, organ/body weight ratios, and the presence of macro and microscopic lesions between these birds and controls were assessed. Both, commercial and SPF broilers had enlargement of the proventriculus with necrosis of the glandular epithelium and lymphocytic infiltrates in the proventricular gland. SPF broilers exposed to the proventricular homogenates developed Infectious Bursal Disease, and infectious bursal disease virus (IBDV) was detected by reverse transcriptase polymerase chain reaction (RT-PCR) and immunohistochemistry (IHC) in bursal and proventricular tissues. They also were positive by RT-PCR to infectious bronchitis virus (IBV) and developed nephritis. Commercial broilers developed mild nephritis but not bursal disease, and were negative for IBDV and IBV by RT-PCR. Both, commercial and SPF chickens, were negative for reovirus, and Newcastle disease virus (NDV), and positive for chicken anemia virus (CAV) and adenovirus by molecular techniques. Bacteria were not identified in histological sections nor were they isolated from affected proventriculi. Filtrates from the proventricular homogenates passed in embryos for virus isolation caused stunting but identification of the cause by electron microscopy (EM) was unsuccessful. However, allantoic fluid from the eggs was positive for IBV by RT-PCR. Thin sectioning EM on proventriculi from affected birds failed to identify a causative agent. In conclusion, the original proventricular homogenates had IBDV, IBV, adenovirus and CAV, but their role in producing proventriculitis was not proven. Keywords: Chicken; Proventriculitis. Abbreviations: CAV = chicken anemia virus; Dpi = days post-inoculation; EM = electron microscopy; H&E =hematoxylin and eosin; IBDV = infectious bursal disease virus; IBV = infectious bronchitis virus; IHC = immunohistochemistry; NDV = Newcastle disease virus; PBS = phosphate buffer saline; RT = reverse transcripatase; PCR = polymerase chain reaction; SPF = specific-pathogen free.

INTRODUCTION. Proventriculitis is an infectious disease of chickens of unknown etiology (7). It is characterized by an enlarged, atonic proventriculus that is filled with fluid and feed (2, 8, 9, 13, 17, 28). The gastric isthmus connecting the proventriculus and gizzard is enlarged, with dilation of the constriction present at this juncture.

The economic impact of proventriculitis is mainly due to condemnation of contaminated carcasses subsequent to the rupture of the proventriculus during routine evisceration (2, 13). Proventriculitis is more severe in younger birds (4-5 wks of age) and has been associated with impaired growth, poor feed conversion, intestinal fragility, stunting syndrome and passage of undigested feed (1, 3, 13, 19, 24, 28, 30). The poultry industry reports sporadic, thought economically important, outbreaks of proventriculitis in broilers (13). Although broiler chickens throughout the world are commonly plagued by outbreaks of disease characterized at least in part by proventricular enlargement, lesions consistent with transmissible proventriculitis have been described in detail only in the United States (8, 9, 13), Holland (19), and Australia (28).

Routes of natural infection of proventriculitis are not known; however, chickens can be infected experimentally by oral inoculation with a homogenate prepared from proventriculi of chickens with proventriculitis (2, 9, 13, 28). Because the disease is reproduced with proventricular homogenate filtrates (0.2 µm), a virus is suspected as the etiologic agent (9, 13, 28). Consequently, the disease is also termed transmissible viral proventriculitis (TVP)(8, 9). However, the severity of lesions and the effects on production are more severe in birds treated with unfiltered homogenates, suggesting an additive effect of other infectious agents (13).

Potential infectious causes of proventriculitis include adenovirus (19, 21), reovirus (20, 21, 24), infectious bronchitis virus (IBV)(35), infectious bursal disease virus (IBDV) (2, 13, 14, 17, 23, 31) and megabacterium (11, 12, 22, 26). However, none of these agents have been found in a majority of cases. Electron microscopy has detected adenovirus-like viral particles in acute lesions but isolation of this virus from affected proventriculi has been unsuccessful (8, 9, 13).

Our objective in this study was to reproduce proventriculitis in broiler chickens, characterize the changes present in the proventriculus and other organs, and examine the affected proventriculus for the presence of virus or bacteria by histological, bacteriological, virological, and molecular methods.

MATERIALS AND METHODS. Chickens. One-day-old unvaccinated broiler chicks were obtained from a commercial hatchery. Also, Fertile White Plymouth Rock chicken eggs (SEPRL, USDA, Athens, GA, USA) were obtained from a breeder flock maintained under SPF conditions and hatched, the parent flock and all progeny were free of common poultry diseases, specifically IBDV, MDV, IBV, reovirus and CAV. All chicks were wing-banded, weighed, separated into groups and maintained in positive pressure Horsfal isolation units. Feed and water were provided *ad libitum.*

Proventricular homogenates. Two different proventricular homogenates were used. Homogenate 1 (Hom.1) was prepared from proventriculi from 4-wk old chickens with proventriculitis, obtained from a commercial Cornish hen processing plant in northwest Alabama (2). Homogenate 2 (Hom.2) was prepared from proventriculi of broiler chickens that presented proventriculitis after being challenged at day of age with Hom.1 (13).

Experimental design. 18 one-day-old commercial broilers, and 18 one-day-old SPF broilers were divided into 3 groups each. The first group was inoculated by oral gavage with 1ml of sterile saline solution (negative control). The second group received 1 ml of proventricular homogenate 1 (Hom. 1). The third group received 1 ml of proventricular homogenate 2 (Hom. 2).

Sample collection and processing. At 7 and 14 days of age, 3 birds from each group were examined, weighed, bled, killed by cervical dislocation, and necropsied. Bursa, proventriculus, spleen, and right side of thymus were weighed and sections of these organs and of liver, kidney, duodenum, pancreas, heart, gizzard and bone were collected from each bird and fixed immediately by immersion in 10% neutral buffered formalin for 24 hours. Tissues were then processed and embedded in paraffin using routine histologic techniques. A section of proventriculi was also collected in a solution of 2% glutaraldehyde, 2% paraformaldehyde, 0.2% picric acid, and 0.1M cacodylate buffer at pH 7.2- 7.3 for thin sectioning and electron microscopic examination. The remaining proventriculi were pooled per group and collected in sterile plastic tubes over ice, homogenates prepared (2, 13). Briefly, proventriculi were washed in sterile phosphate buffer saline (PBS) three times on a magnetic stirrer to remove feed residues and toxins. Washed proventriculi were then diluted 1:1 wt/vol in sterile PBS and homogenized with a commercial blender (Waring, New Hartford, Connecticut). The homogenates were then frozen at -80 C. Relative organ weights were obtained using the formula [Relative organ weight = (organ weight / body weight) x 100].

Histopathology. Paraffin-embedded tissues were sectioned, mounted, stained using hematoxylin and eosin (H&E), and examined blinded as to treatment for lesions using light microscopy. Tissue sections from proventriculus, bursa, thymus and spleen were assigned a lesion severity score. A lesion score of 1 represented no lesions. For bursal sections, 2 was defined as mild variation in follicle size, 3 as moderate variation in size of follicles, and 4 as either necrosis or follicle atrophy. For proventricular sections, 2 was defined as mild glandular lumenal ectasia, 3 as ectasia, mild glandular necrosis, plus lymphoid infiltrates in the interglandular interstitium, and 4 as either acute glandular necrosis or severe fibrosis with lymphoid infiltrates. For thymus sections, 2 was defined as mild cortical thinning, 3 as moderate cortical thinning, and 4 as absence of cortical lymphocytes. For spleen sections 2 was defined as mild lymphocyte depletion, 3 as moderate lymphocyte depletion, and 4 as severe lymphocyte depletion.

For identification of bacteria by light microscopy, tissue sections of proventriculi were stained by the Warthin-Starry technique (4), and a modified *Helicobacter pylori* and gastric stain (6).

Serology. Serum samples obtained at 7 and 14 days of age, from both commercial and SPF broilers, were examined for antibody to IBDV, IBV, NDV, CAV, reovirus, MS and MG, using commercially available ELISA tests (IDEXX Laboratories, Inc. Westbrook, Maine).

Bacteriology. Pooled proventricular homogenates from experimentally infected birds from each group were diluted in sterile saline and plated on Campy blood agar (Remel, Lenexa, TX). Inoculated plates were placed into GasPak pouches (BD Diagnostic Systems; Sparks, MD) and incubated at 42C for 48 hrs. To check for anaerobic growth, blood agar and PEA (phenylethyl alcohol) agar plates were streaked and incubated overnight at 37C in a GasPak pouch. The proventricular homogenates were also plated on Sabouraud dextrose agar plates and incubated at 37C overnight, and then maintained at room temperature and examined daily for mold growth. Isolation of Salmonella sp. was attempted by standard protocol using tetrathionate enrichment broth (10).

Virus isolation. A portion of proventricular homogenate 2 (Hom.2), proventricular homogenate made from pooled proventriculi obtained from commercial chickens challenged with Hom. 2 (Hom.2 com.) at 7 dpi, and negative proventricular homogenate from control group (-PV), was frozen and thawed three times. Sediment was removed from the homogenates by centrifugation at 2,500 X g for 30 min at 4 C. The supernatants were forced through a series of glass fiber filters with a final membrane pore size of 0.2 µm. Four groups of five SPF leghorn chicken embryos were inoculated at 9 days of age via chorioallantoic membrane and allantoic cavity routes (29), with 0.2 ml of one of the following: Hom.2 filtrate, Hom.2 com. filtrate, -PV filtrate, and sterile saline. Eggs were examined daily for embryo death. At 7 days post inoculation chorioallantoic membranes (CAMs) and allantoic fluid were aseptically collected and placed in sterile microfuge tubes and frozen at -80C. A portion of the CAMs was collected in 10% buffered formalin and processed for histopathology. Five blind passes were done and at each, allantoic fluid and CAM's were diluted 1:10 in antibiotic diluent prior to reinoculation.

RNA extraction. RNA was extracted from formalin fixed paraffin-embedded bursas and proventriculus and from Hom.1, Hom. 2, pooled proventricular homogenates from experimental groups at 7 dpi, and from allantoic fluid from eggs inoculated with homogenate filtrates (fifth passage). Sections totaling fifty µm in thickness were cut from each formalin-fixed paraffin-embedded tissue block with a microtome and a new blade for each block. Sections were then deparaffinized (HemoDe and 100% ethanol; Fisher Scientific, Pittsburgh, PA). All tissues were digested with 10% proteinase K (Sigma Chemical Co., St. Louis, MO) for 3 hr at 50 C. RNA was extracted with Trizol (Life Technologies, Inc. Gaithersburg, MD) according to the manufacturer's recommendations, diluted in 90% dimethyl sulfoxide (DMSO), and frozen at -80 C until assayed.

DNA extraction. DNA was extracted from Hom.1, Hom.2, pooled proventricular homogenates from experimental groups at 7 dpi, and from allantoic fluid from eggs inoculated with homogenate filtrates (fifth passage) using the QIAamp DNA Mini Kit (Qiagen Inc., Valencia, CA) according to manufacturers recommendations. Extracted DNA was frozen at - 80C until assayed.

Real time reverse transcriptase-polymerase reaction (RT-PCR). For detection of IBDV, IBV, NDV, and reovirus this protocol was followed: Extracted RNA was denatured at 95 C for 5 min and put on ice (only for IBDV and reovirus reactions). Real time RT-PCR was performed separately for each sample with reagents from the Light Cycler-RNA Amplification SYBR^{®} Green I Kit (ROCHE Molecular Biochemicals, Indianapolis, IN). The primers used were specific for amplification of each of the viruses (Table 7). Amplification and detection of specific products was performed with a Light Cycler according to the manufacturer's recommendations (ROCHE Light Cycler version 3.0; ROCHE Molecular Biochemicals). Briefly, reverse transcription was done at 55 C for 10 minutes, followed by denaturation at 95 C for 30 sec. Forty PCR cycles were done consisting of denaturation (95 C for 1 sec), hybridization (55 C for 10 sec), and extension (72 C for 13 sec). A melting curve analysis was done after an initial denaturation at 95 C. The melting curve was established with an initial temperature of 65 C for 10 sec and then gradual temperature increase of 0.1 C per second until reaching 95 C. The melting temperature was used to confirm the identity of viral specific products obtained using real time RT-PCR. Additional confirmation of specific amplification was done by gel electrophoresis of 8 µl of the PCR products on 2% agarose (Sigma Chemical Co., St. Louis, MO) followed by ethidium bromide staining. PCR products from IBDV positive samples were purified using the QIAgen purification kit and sequenced (Molecular Genetics Instrumentation Facility; University of Georgia, GA). Sequence data was then analyzed by DNASTAR and sequences compared to that of known IBDV. Samples positive for IBV were analyzed by RT-PCR RFLP for molecular grouping (15).

PCR. Detection of adenovirus and CAV was done as follows. Primers used for these reactions are specified in Table 7. PCR for adenovirus was performed using 'Ready to go' PCR Beads (Pharmacia Biotech) and following the protocol from Raue *et al.* (27). PCR for chicken anemia virus was performed following the same protocol used by Todd *et al.* (33). A 8 µl aliquot of each reaction was separated by electrophoresis in an 2% agarose gel (Sigma Chemical Co.) followed by ethidium bromide staining and examination with a U.V. transiluminator.

IBDV Immunohistochemistry (IHC) and Immunofluorecence Assay (IFA). All procedures were done at room temperature. Tissue sections were cut (4 µm) from paraffin-embedded bursas and proventriculi of inoculated chickens and mounted on positively charged glass slides (Superfrost/Plus; Fisher Scientific). Paraffin was melted from the slides (10 min at 65 C) and removed by immersion in Hemo-De three times (5 min each). Slides were then air dried and digested with 10% proteinase K (DAKO, Carpinteria, CA) for 5 min to expose antigenic target sites. Staining for IHC was performed on bursas and proventriculi with an automated stainer (Leica ST 5050, Nussloch, Germany) with a non-biotin peroxidase kit (DAKO Envision System; DAKO) according to the manufacturer's recommendations. The primary antibody used was a monoclonal antibody specific to and cross reactive for all IBDVs (ATCC No.HB9490). After IHC staining, sections were counterstained with hematoxylin, air dried, coverslipped, and examined by light microscopy. Staining for IBDV was recorded as positive or negative staining. IFA was performed on proventriculus sections using as primary antibody a convalescent sera obtained from SPF chickens at 14 dpi and diluted 1:100 in sterile PBS. Slides were incubated for 20 min followed by three washes with PBS of 5 min each. For secondary antibody FITC monoclonal anti-chicken IgG (Accu-Specs) was used at a 1:40 dilution in PBS. Slides were incubated for 20 min then washed three times with PBS. Slide coverslips were mounted using 1:1 glycerol/PBS and the sections were examined using a fluorescent microscope (Leitz).

Transmission electron microscopy. Sections of proventriculus collected from inoculated chickens that presented proventriculitis, and allantoic fluid collected from the fifth pass in eggs of Hom. 2, Hom. 2 com. and -PV were sent for direct examination with a JEOL JEM 1210 transmission electron microscope.

Statistical analysis. The body weight gain, relative organ weight, and lesion scores were analyzed using ANOVA and means comparisons for all pairs using Tukey-Kramer HSD (JMP) Significance was assumed at the 0.05 level of probability.

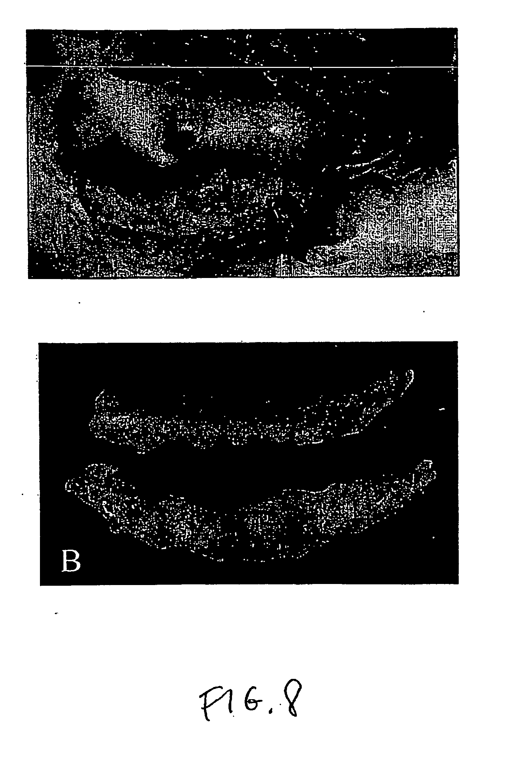

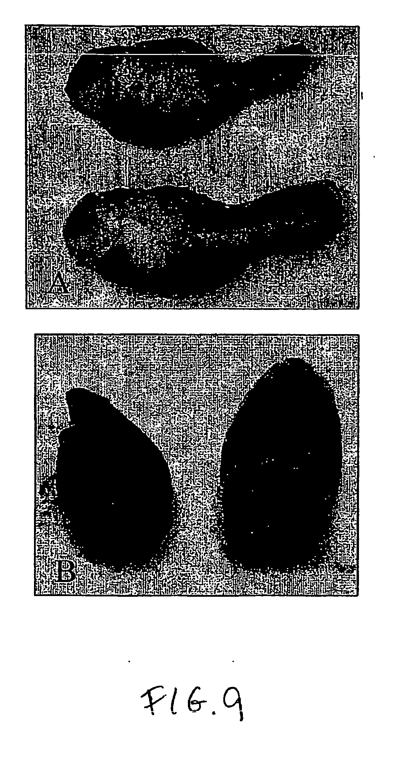

RESULTS. Clinical signs and macroscopic lesions. No clinical signs were observed in the saline control groups or the commercial chickens that received the proventricular homogenates. SPF broilers that received the homogenates had mild depression. Gross lesions were observed in all proventriculi from homogenate-inoculated commercial and SPF chickens. At both, 7 and 14 days post-inoculation, the proventriculi were enlarged, mottled, and had a distended gastric isthmus (Fig 8, 9). The proventricular wall was thickened, with a white lobular pattern observed when sectioned. These lesions were more evident in the commercial broilers than in SPF broilers, and at 14 days post inoculation for both groups. No macroscopic lesions were observed in any other organ of experimentally infected birds.

Body weight gain. Commercial broilers inoculated with proventricular homogenate had no significant suppression of weight gain compared to age matched control birds. Weight gain in SPF broilers was affected by both proventricular homogenate treatments (Table 8 and 9).

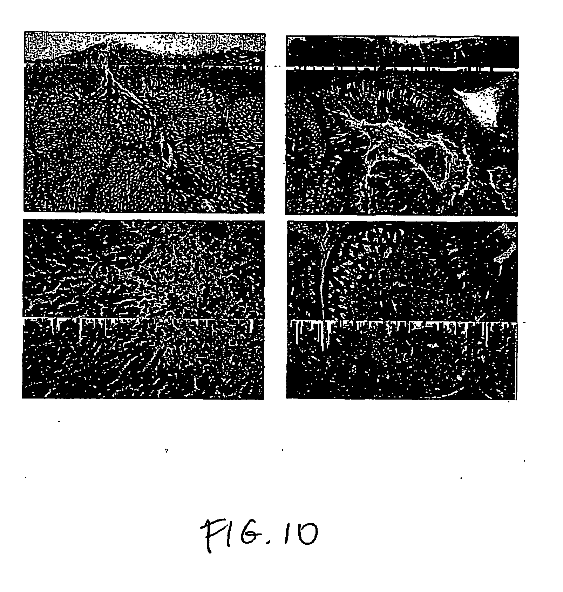

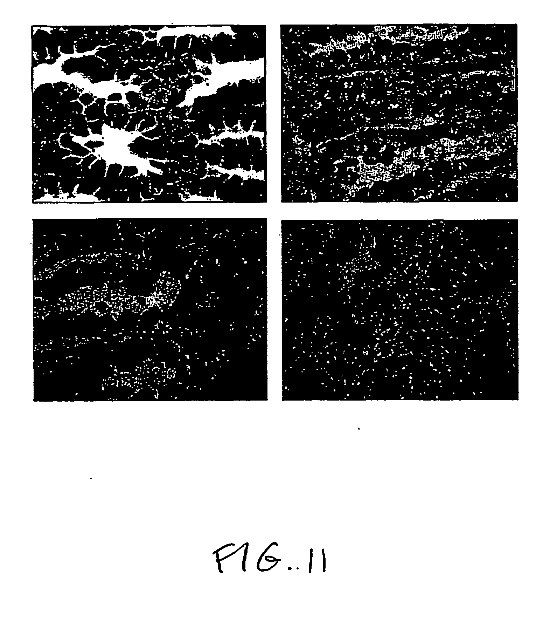

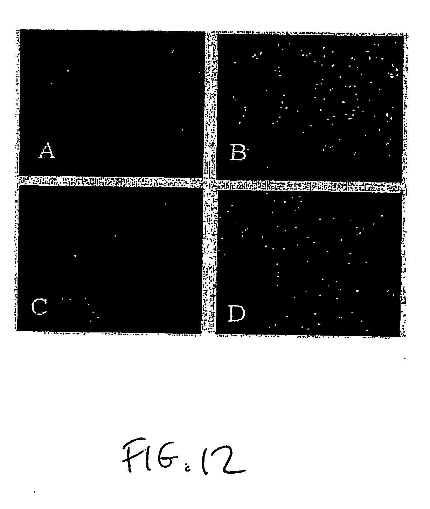

Organ weights and microscopic lesions. Commercial and SPF chickens that received the positive proventricular homogenates had increased proventricular organ/weight ratio, and microscopic lesions in the proventriculus at 7 and 14 dpi. Bursa and thymus organ/weight ratio was not affected in commercial broilers, but their spleen organ weight increased with the homogenate treatment (Tables 8 and 9). SPF broilers that received proventricular homogenate had smaller bursas, thymuses and spleens compared with controls at 14 dpi (Table 9). Microscopically, at 7 dpi, acute necrosis of the proventricular glandular epithelium was present in both, commercial and SPF chickens (Fig. 10, 11, 12). Collecting sinuses of the glands were dilated and contained desquamated epithelium. Severely affected glands coalesced. Nuclei of the glandular epithelial cells were enlarged and pale, with marginated chromatin. Lymphocytic infiltration was present in the lamina propria of the mucosa and in the glandular interstitium in areas containing affected glandular epithelial cells. At 14 dpi, proliferating hyperplastic and hypertrophic columnar cells lined primary, secondary, and tertiary gland ducts. Cuboidal to low columnar, pale, basophilic, and distinctly vacuolated ductlike epithelium replaced the destroyed alveolar secretory cells. Germinal center formation was present in the glands and mucosa. No difference in lesion scores were present in bursa, thymus and spleen between commercial chickens and controls. Bursa and thymus of SPF chickens that received the homogenates had increased lesions scores when compared to controls (Table 8 and 9).

Mild lymphocytic infiltration was present in the intestine, pancreas and liver of commercial and SPF chickens inoculated with the proventricular homogenates, at both 7 and 14 dpi (Tables 10 and 11). All homogenate-inoculated SPF broilers also had moderate to severe lymphocytic infiltration in the kidneys at both 7 and 14 dpi. No other lesions were present in these or the remaining organs examined from both commercial an SPF chickens.

Serology. Commercial broilers that received the proventricular homogenate were negative for reovirus, NDV, MG, and MS at 7 and 14 dpi. These birds were positive for IBDV and IBV at both time points and also for CAV at 14 dpi. (Table 12). SPF broilers that received the proventricular homogenates were negative for IBDV, IBV, reovirus, NDV, MG, MS, and CAV at 7 dpi, but at 14 dpi seroconverted to IBDV, IBV, and CAV (Table 13).

Bacteriology. No bacteria were isolated from proventricular homogenates from birds experimentally infected by the methods described above. No bacteria were observed by direct light microscopy in routine or special stained sections.

Virus isolation. Embryo inoculated with proventricular homogenate 2 (Hom. 2) and proventricular homogenate from commercial chickens inoculated with Hom. 2 (Hom. 2 com.) were stunted from the second passage on. Chorrioallantoic membranes (CAMs) harvested from these eggs did not have plaque formation and no lesions were observed histopathologically.

RT-PCR and PCR results. *IBDV RT-PCR on paraffin-embedded tissues.* Bursas and proventriculi of commercial broilers were all negative for IBDV (Table 12). All bursas and some of the proventriculi of SPF broilers that received either proventricular homogenate were positive for IBDV (Table 13). Amplicons were sent for sequencing and were most similar to variant A IBDV (data not shown).

*RT-PCRs and PCRs on proventricular homogenates and allantoic fluids.* All samples were negative for reovirus and NDV (Table 14). Hom. 1 was positive for IBDV, IBV. adenovirus and CAV. Hom. 2 was positive for IBDV, IBV, and CAV and negative for adenovirus. Proventricular homogenates from commercial broilers inoculated with the Hom. 1, and collected at 7 dpi, were negative for all virus examined except adenovirus. Commercial broilers inoculated with Hom. 2 were negative for all viruses examined. SPF broilers inoculated with Hom.1 were positive for IBDV, IBV, and adenovirus and negative for the rest. SPF broilers inoculated with Hom. 2 were positive for IBDV and IBV and negative for the rest. Allantoic fluids from embryos inoculated with Hom. 2 or Hom. 2 com. were only positive for IBV.

*Molecular characterization of detected IBDV and IBV.* Analysis of the sequence data obtained from the amplified IBDV revealed that this virus is a IBDV variant strain and is most similar to Variant A. RFLP analysis of the amplified IBV determined that this virus strain was Connecticut (data not shown).

IBDV Immunohistochemistry. Positive staining for viral antigen was detected in all bursas and some of the proventriculi of SPF chickens inoculated with the proventricular homogenates. None of the bursas or proventriculi of the commercial broilers were positive.

Immunoflourescence Assay (IFA). Positive fluorescent staining was present in glandular epithelial cells in the proventriculi from homogenate-inoculated chickens when examined at 7 dpi. The specific reaction was seen localized within the cytoplasma of the glandular epithelial cells. Fluorescent staining was also present on the outer surface of what seemed to be lymphocytes. No fluorescent staining was observed in proventriculi of saline-inoculated chickens.

Electron microscopy. No viruses were detected in the samples sent for examination.

DISCUSSION. **Proventriculitis** was successfully reproduced by oral inoculation of commercial and SPF broilers with proventricular homogenates obtained from chickens with proventriculitis. Inoculated chickens had enlargement of the proventriculus and a distended gastric isthmus. The proventricular walls were thickened with a white lobular pattern observed when sectioned. Microscopic lesions consisted of degeneration and necrosis of the glandular epithelium, severe lymphocytic infiltration, and ductal epithelial hyperplasia. This loss of glandular tissue and ductal hyperplasia may result in loss of function of the proventriculus (10). This would explain the poor feed conversion and reduced growth rates reported in some naturally affected chickens with proventriculitis (28), and also the reduced weight gain observed in our homogenate-inoculated SPF chickens. However, the body weight gain in our commercial chickens was not affected. Bayyari *et al.* (2) found that proventriculitis was produced independently of an effect on growth, and a common field observation is that proventriculitis can occur in the best performing flocks when processed at 4-5 wk of age (13). This leads us to believe that proventriculitis may or may nor be associated with stunting in broilers, and that several agents or conditions most likely modify the severity of proventriculitis and its effect on weight gain. In fact, proventriculitis has been associated with infectious stunting or malabsorption syndrome in chickens (3), but cases of malabsorption syndrome may or may not include proventricular lesions (32). Filterable agents isolated in the Netherlands were originally linked to proventriculitis, causing runting syndrome in broilers (19). These authors suggested the involment of both bacteria and viruses in the etiology of malabsorption syndrome (19, 20). A comparative study of the pathogenesis of five different malabsorption syndrome homogenates from the Netherlands and Germany distinguished the inoculated groups of chickens by their histopathologic lesions: proventriculitis, lesions in the intestine only, or combination of both (32). Lesions in the small intestine had more impact on weight gain depression than lesions in the proventriculus. In our study no intestinal lesions were observed in chickens inoculated with the proventricular homogenates.

Reoviruses have been implicated as a causative agent for concurrent proventricular lesions present in some flocks naturally affected with malabsorption syndrome (20), and proventriculitis was reproduced by inoculation of two reovirus isolates from the intestines of birds with malabsorption syndrome (24). In our study however, no reovirus were isolated from the homogenates, and no reovirus was detected by RT-PCR in any of the inoculated groups. Also none of the chickens seroconverted to this virus, which indicates that proventriculitis can occur in the absence of reovirus.

Mild proventriculitis has also been reproduced experimentally in chickens infected with some isolates of adenovirus (19, 21), though this virus hasn't been consistently isolated from diseased proventriculi. One of the proventricular homogenates used in our study was positive for adenovirus, and also the proventriculi of the chickens that were inoculated with this homogenate. However, the role of this virus in proventriculitis is not clear because the disease still occurred in its absence, and visualization of viral particles in affected proventricular glands by EM was unsuccessful. Goodwin *et al.* (8) reported the presence of intralesional virions in proventriculi from chicks that had proventriculitis, and suggested a causal relationship between the virus and the lesion in its host. Hexagonal intranuclear virus particles were described and resembled adenovirus or poliomavirus. However, DNA *in situ* hybridization failed to detect adenovirus or poliomavirus nucleic acids. Huff *et al.* (13) also reported the presence of similar virus-like particles in the nuclei of many epithelial cells of the proventriculus of chickens experimentally inoculated with homogenate prepared from the proventriculi of chickens with proventriculitis. The particles, nonenveloped spheres of about 100-200nm in diameter, appeared hexagonal and were arranged in semiparacristalline arrays in the nuclei (13). These adenovirus-like particles have not been isolated so its role as causative agent in proventriculitis has not been corroborated.

IBDV has also been associated with proventriculitis (2, 13, 23) but its role in this disease is not clear. Both gross and microscopic lesions of the proventriculus have been produced by IBDV challenge in leghorn chickens (24) and vaccination against IBDV has been reported to decrease the incidence of proventriculitis (7,15). However, proventriculitis was not produced by inoculation of SPF broilers with different strains of IBDV (25). Both proventricular homogenates used in our study to induce proventriculitis were positive for IBDV by RT-PCR. Proventriculi of commercial broilers inoculated with these homogenates were negative for the virus by RT-PCR and IHC, and these birds did not present lesions or virus in the bursa. These chickens had antibody titers against IBDV at 7 and 14 dpi and were probably protected against the virus. On the other hand, SPF broilers had lesions in the bursa characteristic of IBDV infection, the virus was detected by RT-PCR and IHC in all bursas and some of the proventriculi, and some of the birds seroconverted at 14 dpi. Because proventriculitis was produced in commercial broilers independently of the presence of IBDV, this virus probably is not directly involved in the disease.

Both of the proventricular homogenates used in our study to induce proventriculitis were also positive for IBV by RT-PCR, and homogenates produced from the proventriculi of inoculated SPF broilers were also positive by RT-PCR and seroconverted to IBV at 14 dpi. These birds also had moderate to severe nephritis, a lesion associated with infection with IBV (5). Commercial broilers inoculated with the proventricular homogenates were negative by RT-PCR for IBV but presented mild interstitial nephritis, and IBV was isolated in embryos when inoculated with a filtrate produced from the homogenate prepared from the pooled proventriculi of these birds. These commercial broilers had antibodies against IBV, most likely of maternal origin, detected at both 7 and 14 dpi, which probably offered some protection against the effect of the virus. Infectious bronchitis virus (IBV) isolates from naturally occurring cases in China have been reported to produce proventricular lesions in infected birds (35). The strain of IBV isolated in our study was determined to be Connecticut by RFLP, a strain that has been isolated also from cecal tonsils and intestine in chickens (16). The role of this strain of IBV in proventriculitis needs to be further explored.

Guy and Barnes (9) reproduced proventriculitis by administration of a filtrate (0.2-µm) from a homogenate produced from the proventriculi of chickens with proventriculitis. This inoculum was free of avian reovirus, avian group I adenovirus, infectious bursal disease virus (IBDV) and infectious bronchitis virus (IBV). Adenovirus-like particles, similar to those observed by Goodwin *et al* (8), were identified by thin-section electron microscopy in nuclei of affected glandular epithelium cells. These authors also detected intranuclear staining by IFA using as primary antibody hyperimmune sera from birds inoculated with infectious proventricular filtrates. The results of our immunofluorescent assays did not corroborate these findings. Although immunofluorescence was also observed in the affected glandular epithelial cells, it was localized in the cytoplasma, not the nucleous. Also staining of the surface of lymphocytes was observed, which was probably antigen attached to them.

Reece (28) reported that proventricular homogenates prepared from chickens with proventriculitis were highly infectious and transmissible for at least four passages in birds. Treatment of the inoculum with chloroform did not reduce infectivity supporting the hypothesis that the putative etiological agent of infectious proventriculitis was a non-enveloped virus. This virus did not grow in any of a wide variety of primary and established cell culture systems and viral isolation in embryos was unsuccessful. The original inoculum contained chicken anemia virus (CAV), fowl adenovirus type 8, avian nephritis virus and Marek's disease virus (MDV) but did not contain avian leucosis virus (ALV), infectious bronchitis virus (IBV), reovirus, Newcastle disease virus (NDV) or infectious bursal disease virus (IBDV). The proventricular homogenates used in our study were also positive for CAV and all birds treated with these homogenates seroconverted at 14 dpi. The role of this virus in proventriculitis also needs to be studied.

Huff *et al.* (13) reported the isolation of a unique bacterial agent (*Clostridia sp.*) from a proventriculus homogenate that caused proventriculitis, suggesting bacterial involvement in this syndrome. These authors conclude that a viral infection, as well as various dietary factors, may facilitate bacterial invasion of the proventriculus, and more than one type of virus may act as facilitator in this disease syndrome. In our study, no bacteria was isolated or identified by histopathology and special staining in the proventriculus of affected chickens, however the role of bacteria should be taken into consideration when studying proventriculitis.

In conclusion, proventriculitis can be transmitted by oral inoculation with homogenates produced from proventriculi of birds with proventriculitis. The causative agent(s) was not identified, although most likely is a virus. The severity of proventriculitis and its effect on weight gain is probably affected by other factors such as concomitant infection with other agents, viral or bacterial, and nutritional factors. Viral candidates that seem to be involved in proventriculitis are IBV, IBDV, adenovirus and reovirus, however it has been demonstrated that none of them is found in every case of proventriculitis or can reproduce the disease when inoculated in chickens. This leads us to believe that another, non identified virus is the primary causative agent of proventriculitis.

**Table 7. Primers used for RT-PCR AND PCR analysis.**

| Virus | Primer Sequence | Product size | Reference |
|---|---|---|---|
| IBDV | B4 5'TCTTGGGTATGTGAGGCTTG (SEQ ID NO: 9) | 400 bp | Pantin *et al*(25) |
| | B4 3'GGATGTGATTGGCTGGGTTA (SEQ ID NO: 10) | | |
| Reovirus | MK87:GGTGCGACTGCTGTATTTGGTAAC (SEQ ID NO: 22) | 532 bp | Xie *et al.* (33) |
| | MK88:AATGGAACGATAGCGTGTGGG (SEQ ID NO: 23) | | |
| IBV | New S1 oligo 5':TGAAACTGGAACAAAAGAC (SEQ ID NO: 24) | 1720 bp | Jackwood *et al.* (15) |
| | S1 oligo 3': CATAACTAACATAAGGGCAA (SEQ ID NO: 25) | | |
| NDV | FOP1:TACACCTCATCCCAGACAGGGTC (SEQ ID NO: 26) | 532 bp | *Kho et al.* (18) |
| | FOP2:AGGCAGGGGAAGTGATTTGTGGC (SEQ ID NO: 27) | | |
| CAV | F:CTAAGATCTGCAACTGCGGA (SEQ ID NO: 28) | 675 bp | Todd. *et al.* (32) |
| | R:CCTTGGAAGCGGATAGTCAT (SEQ ID NO: 29) | | |
| Adenovirus | H1:TGGAC ATGGGGGCGACCTA (SEQ ID NO: 30) | 1219 | Raue *et al.* (27) |
| | H2:AGGG ATTGACGTTGTCCA (SEQ ID NO: 31) | bp | |

**Table 8. Body weight gain (g), relative organ weights (% body weight) and organ lesions scores of commercial broilers orally challenged at day of age with sterile saline, proventricular homogenate 1 (Hom. 1) or proventricular homogente 2 (Hom. 2), and necropsied at 7 or 14 days postinoculation (dpi).**

| Dpi | Treatment | Body weight gain | PV relative weight | PV lesion score | Bursa relative weight | Bursa lesion score | Thymus relative weight | Thymus lesion score | Spleen relative weight | Spleen lesion score |
|---|---|---|---|---|---|---|---|---|---|---|
| 7 | Saline | 120 ± 10^{a} | 81 ± .09^{a} | 1.33±.57^{a} | .10 ±.005^{a} | 2.00^{a} | .10± .02^{a} | 1.00^{a} | .02 ±.005^{a} | 2.00^{a} |
| | Hom. 1 | 122 ± 6^{a} | 1.20±.01^{ab} | 3.00±1.0^{b} | .20± .04^{a} | 3.00^{a} | .23 ± .04^{b} | 1.00^{a} | .12 ± .03^{b} | 2.00^{a} |
| | Hom. 2 | 98 ± 16^{a} | 1.48±.33^{b} | 3.66±57^{b} | .17 ± .06^{a} | 3.0±1.0^{a} | .16 ± .65^{ab} | 1.00^{a} | .17± .02^{b} | 2.00^{a} |
| 14 | Saline | 399 ±50^{a} | .53 ±.05^{a} | 1.33±.57^{a} | .15± .03^{a} | 1.33±5^{ab} | .20 ± .03^{a} | 1.00^{a} | .05 ±.005^{a} | 1.66±.57^{a} |
| | Hom. 1 | 336 ± 2^{a} | 1.06±.37^{b} | 3.00±1° | .23± .03^{a} | 1.00^{a} | .23 ± .03^{a} | 1.00^{a} | .12± .03^{b} | 1.66±.57^{a} |
| | Hom. 2 | 402 ±47^{a} | .97 ±.17^{b} | 4.00^{b} | .15± .03^{a} | 2.00^{b} | .26 ± .07^{a} | 1.00^{a} | .08 ±.08^{ab} | 1.00^{a} |

**Table 9. Body weight gain (g), relative organ weights (% body weight) and organ lesions scores of SPF broilers orally challenged at day of age with sterile saline, proventricular homogenate 1 (Hom. 1) or proventricular homogente 2 (Hom. 2), and necropsied at 7 or 14 days postinoculation (dpi).**

| Dpi | Treatment | Body weight gain | PV relative weight | PV lesion score | Bursa relative weight | Bursa lesion score | Thymus relative weight | Thymus lesion score | Spleen relative weight | Spleen lesion score |
|---|---|---|---|---|---|---|---|---|---|---|
| 7 | Saline | 41 ± 3 ^{a} | .93 ± .12 ^{a} | 1.00^{a} | .22± .04^{a} | 2.66±.57^{a} | .20± .05^{a} | 1.00^{a} | .55± .64^{a} | 2.00^{a} |
| | Hom. 1 | 24±7 b | 1.29 ±.18^{a} | 1.33 ± .5^{a} | .08 ± .02^{b} | 4.00^{b} | .13± .005^{a} | 2.66 ± .57 ^{a} | .20 ± .06^{a} | 2.00^{a} |
| | Hom. 2 | 22±8 b | 1.47 ±.46^{a} | 3.00±1.73^{a} | .21 ± .02^{a} | 3.33±.5^{ab} | .18± .007^{a} | 2.00±1.73 ^{a} | .21 ± .06^{a} | 2.00^{a} |
| 14 | Saline | 126 ±19^{a} | .72±.06 ^{a} | 1.00^{a} | .36± .12^{a} | 1.33±.57^{a} | .35± .19^{a} | 1.00^{a} | .27 ± .08^{a} | 1.66±.57^{a} |
| | Hom. 1 | 88 ±18^{ab} | .98±.4 ^{ab} | 2.00±1^{ab} | .11± .03^{b} | 4.00^{b} | .17± .07^{b} | 3.00±1.7 ^{ab} | .17± .06^{a} | 2.00^{a} |
| | Hom. 2 | 53±9 b | 1.55±.49 b | 3.33±1.1^{b} | .16 ±.04^{ab} | 3.33±1.5^{b} | .07± .03^{a} | 4.00^{b} | .14 ± .08^{a} | 2.00^{a} |

**Table 10. Lymphocytic infiltration in organs from commercial broilers inoculated with infectious proventricular homogenates (Hom.1 or 2) or saline, at 7 and 14 days post inoculation (dpi).**

| Dpi | Treatment | Intestine | Pancreas | Liver | Kidney |
|---|---|---|---|---|---|
| 7 | Saline | 0/3^{a} | 0/3 | 0/3 | 0/3 |
| | Hom 1 | 2/3 | 2/3 | 1/3 | 0/3 |
| | Hom 2 | 3/3 | 2/3 | 0/3 | 0/3 |
| 14 | Saline | 0/3 | 0/3 | 0/3 | 0/3 |
| | Hom. 1 | 2/3 | 1/3 | 2/3 | 1/3 |
| | Hom.2 | 2/3 | 3/3 | 3/3 | 1/3 |

| | | | | | |
|---|---|---|---|---|---|
| ^{a} = number of chickens positive/number of chickens inoculated | | | | | |

**Table 11. Lymphocytic infiltration in organs from SPF broilers inoculated with infectious proventricular homogenates (Hom. 1 or 2) or saline, at 7 and 14 days post inoculation (dpi)**

| Dpi | Treatment | Intestine | Pancreas | Liver | Kidney |
|---|---|---|---|---|---|
| 7 | Saline | 0/3^{a} | 0/3 | 0/3 | 0/3 |
| | Hom 1 | 1/3 | 1/3 | 2/3 | 3/3 |
| | Hom 2 | 1/3 | 2/3 | 2/3 | 3/3 |
| 14 | Saline | 0/3 | 0/3 | 0/3 | 0/3 |
| | Hom. 1 | 3/3 | 2/3 | 3/3 | 3/3 |
| | Hom.2 | 1/3 | 2/3 | 1/3 | 3/3 |

| | | | | | |
|---|---|---|---|---|---|
| ^{a}= number of chickens positive/number of chickens inoculated | | | | | |

**Table 12. Seropositivity by ELISA of commercial broilers inoculated with infectious proventricular homogenates (Hom.1 or 2) or saline, at 7 and 14 days post inoculation (dpi).**

| Dpi | Treatment | IBDV | Reovir. | IBV | NDV | CAV | MS | MG |
|---|---|---|---|---|---|---|---|---|
| 7 | Saline | 3/3^{a} | 0/3 | 3/3 | 0/3 | 0/3 | 0/3 | 0/3 |
| | Hom 1 | 3/3 | 0/3 | 3/3 | 0/3 | 0/3 | 0/3 | 0/3 |
| | Hom 2 | 3/3 | 0/3 | 3/3 | 1/3 | 0/3 | 0/3 | 0/3 |
| 14 | Saline | 2/3 | 0/3 | 2/3 | 0/3 | 0/3 | 0/3 | 0/3 |
| | Hom. 1 | 2/3 | 0/3 | 2/3 | 0/3 | 1/3 | 0/3 | 0/3 |
| | Hom.2 | 1/3 | 0/3 | 0/3 | 0/3 | 1/3 | 0/3 | 0/3 |

| | | | | | | | | |
|---|---|---|---|---|---|---|---|---|
| ^{a} = number of chickens positive/number of chickens inoculated | | | | | | | | |

**Table 13. Seropositivity by ELISA of SPF broilers inoculated with infectious proventricular homogenates (Hom.1 or 2) or saline, at 7 and 14 days post inoculation (dpi).**

| Dpi | Treatment | IBDV | Reovir. | IBV | NDV | CAV | MS | MG |
|---|---|---|---|---|---|---|---|---|
| 7 | Saline | 0/3^{a} | 0/3 | 0/3 | 0/3 | 0/3 | 0/3 | 0/3 |
| | Hom 1 | 0/3 | 0/3 | 0/3 | 0/3 | 0/3 | 0/3 | 0/3 |
| | Hom 2 | 1/3 | 0/3 | 0/3 | 0/3 | 3/3 | 0/3 | 0/3 |
| 14 | Saline | 0/3 | 0/3 | 0/3 | 0/3 | 0/3 | 0/3 | 0/3 |
| | Hom. 1 | 1/3 | 0/3 | 2/3 | 0/3 | 1/3 | 0/3 | 0/3 |
| | Hom.2 | 1/3 | 0/3 | 1/3 | 0/3 | 3/3 | 0/3 | 0/3 |

| | | | | | | | | |
|---|---|---|---|---|---|---|---|---|
| ^{a}= number of chickens positive/number of chickens inoculated. | | | | | | | | |

**Table 14. IBDV RT-PCR and IHC results from formalin fixed, paraffin embedded bursa and proventriculus tissues from commercial broilers inoculated with infectious proventricular homogenates (Hom.1 or 2) or saline, at 7 or 14 days post inoculation (dpi).**

| Dpi | Treatment | Bursa RT-PCR | Bursa IHC | PV RT-PCR | PV IHC |
|---|---|---|---|---|---|
| 7 | Saline | 0/3^{a} | 0/3 | 0/3 | 0/3 |
| | Hom 1 | 0/3 | 0/3 | 0/3 | 0/3 |
| | Hom 2 | 0/3 | 0/3 | 0/3 | 0/3 |
| 14 | Saline | 0/3 | 0/3 | 0/3 | 0/3 |
| | Hom. 1 | 0/3 | 0/3 | 0/3 | 0/3 |
| | Hom.2 | 0/3 | 0/3 | 0/3 | 0/3 |

| | | | | | |
|---|---|---|---|---|---|
| ^{a}= number of chickens positive/number of chickens inoculated | | | | | |

**Table 15. IBDV RT-PCR and IHC results from formalin fixed, paraffin embedded bursa and proventriculus tissues from SPF broilers inoculated with infectious proventricular homogenates (Hom.1 or 2) or saline, at 7 or 14 days post inoculation (dpi).**

| Dpi | Treatment | Bursa RT-PCR | Bursa IHC | PV RT-PCR | PV IHC |
|---|---|---|---|---|---|
| 7 | Saline | 0/3^{a} | 0/3 | 0/3 | 0/3 |
| | Hom 1 | 3/3 | 3/3 | 2/3 | 0/3 |
| | Hom 2 | 1/3 | 3/3 | 0/3 | 0/3 |
| 14 | Saline | 0/3 | 0/3 | 0/3 | 0/3 |
| | Hom. 1 | 3/3 | 1/3 | 2/3 | 0/3 |
| | Hom.2 | 1/3 | 0/3 | 1/3 | 0/3 |

| | | | | | |
|---|---|---|---|---|---|
| ^{a}= number of chickens positive/number of chickens inoculated | | | | | |

**Table 16. RT-PCR and PCR results from proventricular homogenate and allantoic fluid (AF) samples. Proventricular homogenates used for inoculation of chickens (Hom. 1 and 2), proventricular homogenates obtained from chickens inoculated with saline, Hom.1 or Hom.2; from commercial (Com.) and SPF broilers at 7dpi; allantoic fluid (AF) from embryos inoculated with Hom. 2. filtrate, proventricular homogenate filtrate obtained from the proventriculi of commercial broilers inoculated with Hom. 2, and from proventricular homogente filtrate made from the proventriculi of control chickens (-PV), harvested at the 5^{th} pass.**

| Sample | IBDV | Reovirus | NDV | IBV | Adenovirus | CAV |
|---|---|---|---|---|---|---|
| Hom. 1 | + | - | - | - | + | + |
| Hom. 2 | - | - | - | + | - | + |
| Saline Com. 7dpi | - | - | - | - | - | ND^{a} |
| Hom.1 Com 7dpi | - | - | - | - | + | ND |
| Hom.2 Com. 7dpi | - | - | - | - | - | ND |
| Saline SPF 7dpi | - | - | - | - | - | ND |
| Hom.1 SPF 7dpi | + | - | - | + | + | ND |
| Hom 2 SPF 7 dpi | + | - | - | + | - | ND |
| AF Hom.2 5^{th} pass | - | - | - | + | - | ND |
| AF Hom.2 5^{th} pass | - | - | - | + | - | ND |
| AF -PV 5^{th} pass | - | - | - | - | - | ND |

| | | | | | | |
|---|---|---|---|---|---|---|
| ^{a} = ND, not done | | | | | | |

### References:

1. Apple et al. Avian Dis 35:422-425. 1991.
2. Bayyari et al. Poult Sci 74:1799-1809. 1995.
3. Bracewell et al. World's Poult Sci J 40. 1984.
4. Carson. Histotechnology. A self instructional text. Second edition. Chicago, American Society of Clinical Pathologists. 1997.
5. Cavanagh & Naqi. Diseases of Poultry. 10th ed. B. W. Calnek, H. J. B., C. W. Beard, L. R. McDougald, Y. M. Saif. Iowa State University Press Ames, IA. 1997.
6. Genta et al. Human Path. J. 25:221-226. 1994.
7. Goodwin & Hafner. Diseases of Poultry. 10th ed. Calnek, B. W. Iowa State University Press Arnes, IA. 1034-1038. 1997.
8. Goodwin et al. Avian Pathol 25:269-279. 1996.
9. Guy & Barnes. 139th Meeting of the American Veterinary Medical Association, Nashville, TN. 2002.
10. Hajna & Damon. Appl Microbiol 4:341-345. 1956.
11. Henderson et al. Vet Rec 123:492-494. 1988.
12. Huchzermeyer et al. Vet Rec 133:143-144. 1993.
13. Huff et al. Avian Dis 45:828-843. 2001.
14. Huff et al. Poult Sci 73:1352-1356. 1994.
15. Jackwood et al. Avian Dis 41:105-110. 1997.
16. Karaca et al. Avian Dis 34:899-904. 1990.
17. Kelly et al. 138th American Veterinary Medical Association, Boston, MA. 2001.
18. Kho et al. J Virol Methods 80:71-83. 2000.
19. Kouwenhoven et al. Avian Pathol. 7:183-187. 1978.
20. Kouwenhoven et al.. Avian Pathology 17:879-892. 1988.
21. Lenz et al. J Vet Diagn Invest 10:145-151. 1998.
22. Mutlu et al. Tierarztl Prax Ausg G Grosstiere Nutztiere 25:460-462. 1997.
23. Newberry. Ph.D. Dissertation. Fayetteville, AR, University of Arkansas. 1996.
24. Page et al. Avian Diseases 26:618-624. 1982.
25. Pantin-Jackwood & Brown. Avian Dis 47:681-690. 2003.
26. Phalen & Moore. Avian Dis 47:254-260. 2003.
27. Raue & Hess. J Virol Methods 73:211-217. 1998.
28. Reece. Infectious proventriculitis and stunting syndrome of broiler chickens. Canberra, Australia, RIRDC. 2002.
29. Senne. A laboratory manual for the isolation and identification of avian pathogens. 4th ed. D. E. Swayne, J. R. G., M. W. Jackwood, J. E. Pearson, W. M. Reed. American Association of Avian Pathologists. 235-247. 1998.
30. Shapiro & Nir. Poult Sci 74:33-44. 1995.
31. Skeeles et al. Poult Sci 77 (suppl.):133. 1998.
32. Songserm et al. Avian Dis 44:556-567. 2000.
33. Todd et al. J Clin Microbiol 30:1661-1666. 1992.
34. Xie et al. Avian Dis 41:654-660. 1997.
35. Yu et al. Avian Dis 45:416-424.2001.

### PROVENTRICULITIS IN BROILER CHICKENS: EFFECTS OF IMMUNOSUPPRESSION (Pantin-Jackwood et al. Avian Diseases, in press)

SUMMARY Proventriculitis in broilers causes carcass condemnation when swollen proventriculi tear during evisceration. The cause of this proventriculitis is unknown but several infectious agents have been associated with it. One such agent, infectious bursal disease virus (IBDV), has been implicated as a cause of proventriculitis, but a direct effect of this virus on the proventriculus has not been proven. The role of IBDV in proventriculitis may be indirect due to its ability to cause immunosuppression. The objective of this study was to understand how immunosuppression affects the incidence of proventriculitis in broiler chickens. Immunosuppression was induced in commercial and SPF broiler chickens using chemicals (cyclophosphamide and cyclosporin) or virus (IBDV). All groups were then exposed to a proventricular homogenate produced from diseased birds. At 7 and 14 days post inoculation, the incidence of proventriculitis in these groups was compared to that produced by homogenate exposure in immunocompetent broilers. All birds exposed to the proventricular homogenate from diseased birds developed proventriculitis. Cyclophosphamide and IBDV, both B cell suppressors, did not significantly affect the incidence or characteristics of the proventriculitis observed, although they did have an effect on the size of the proventriculus at 7 days post inoculation. Chickens immunosuppressed with cyclosporin, a T cell suppressor, developed more severe lesions and had a higher incidence of proventriculitis. These findings indicate that both, B and T cells, are involved in the immune response against proventriculitis, but cell mediated immunity appears to have a more important role in controlling the disease. IBDV affects both humoral and cellular immunity in the chicken so although under experimental conditions it didn't have a major effect on proventriculitis, it may explain why control of IBDV in the field seems to reduce the incidence of proventriculitis. Key words: Proventriculitis, immunosuppression, IBDV. Abbreviations: CBH = cutaneous basophil hypersensitivity; CMI = cell-mediated immunity; CP = cyclophosphamide; CP = cyclosporin; IBDV = infectious bursal disease virus; RT-PCR = reverse transcriptase polymerase chain reaction; SPF = specific-pathogen free.

INTRODUCTION. Proventriculitis is a clinical condition that affects broiler chickens. It is characterized by enlargement of the proventriculus and weakness of the gastric isthmus. During routine evisceration at processing, affected proventriculi rupture causing spillage of the proventricular contents into the body cavity, which results in condemnation of affected carcasses for contamination. The disease has also been associated with impaired growth, and poor feed conversion (16, 13). Microscopically, degeneration and necrosis of proventricular glands is observed accompanied by marked intraglandular interstitial lymphocytic infiltration (4, 9,10).

Several agents have been implicated as potential causes of proventricular lesions. Noninfectious causes include oral exposure to biogenic amines (2,27), mycotoxins (26), lack of dietary fiber (29), and excessive copper sulfate (3,14,41). Infectious causes include adenovirus (19), reovirus (17,38), infectious bronchitis virus (39), and megabacterium (23,35). However, none of these noninfectious or infectious agents have been found consistently in a majority of cases. Electron microscopy has detected viral particles in acute lesions but isolation of a virus from affected proventriculi has been unsuccessful (9,10, 13).

Infectious Bursal Disease Virus (IBDV) has been implicated as the cause for this disease (4,13,24), and IBDV vaccination has been reported to decrease its incidence (7,15). Proventriculitis can be reproduced by orally inoculating broilers with homogenized proventriculi collected from affected birds (16,4). A filterable agent found in these homogenates causes lesions similar to those found in field cases (4), and IBDV has been immunoprecipitated from these homogenates (13). Commercial broilers exposed to this IBDV developed increased proventricular lesion scores but had no increase in proventricular size, a characteristic feature produced by exposure to proventricular homogenates (13). These findings suggest other agents or conditions may be required to produce proventriculitis.

IBDV induces immunosuppression in chickens (21,34,40). Immunosuppressed flocks may have an increased incidence of secondary infections, poor feed conversion, and reduced protective response to commonly used vaccines (34). IBDV causes lytic destruction of IgM+ B lymphocytes that results in suboptimal levels of antibodies against a number of infectious and noninfectious antigens (8,30,34). Although the immunosuppression caused by IBDV is principally due to B lymphocyte damage, an effect on cell-mediated immunity (CMI) has also been demonstrated (5,18,33,34).

SPF broilers exposed to different strains of IBDV did not develop proventricular lesions or enlargement at 4 or 6 days post-inoculation (25). The virus was detected in large quantities in the bursa of these birds by RT-PCR and immunohistochemical techniques, but not in the proventriculus, indicating it is not a target organ for IBDV. However, the immunosuppressive effect of IBDV could explain its reported relationship to proventriculitis. The purpose of our study was to see if immunosuppression had any effect on the incidence, severity, or character of proventriculitis in broiler chickens. To address this, commercial and SPF one-day old broilers were immunosuppressed with cyclophosphamide (B cell suppressor), cyclosporin (T cell suppressor), or IBDV. Subsequently these chickens were exposed to a proventricular homogenate from affected chickens, and the effect of immunosuppression on proventriculitis was determined.

MATERIALS AND METHODS Animal housing. One-day-old chickens were divided into groups and housed in positive pressure Horsfal units. Unmedicated feed and water were provided *ad libidum.*

Experimental design. Trials 1 and 2. A total of 88 unvaccinated commercial broiler chicks, obtained from a local hatchery, were divided into 9 groups of 8 or 12 birds, and chicks in each group received either an immunosuppressive treatment or no treatment (Table 17). Chickens subsequently received as described below, either positive (+PV) or negative (-PV) proventricular homogenate, saline, or no homogenate. Group 1 had 12 birds, which were inoculated *per os* with 1 ml of sterile saline at 7 days of age. Group 2 had 8 birds, which were inoculated *per os* with 1 ml of -PV produced from normal commercial broilers at 7 days of age. Group 3 had 8 birds, which were inoculated *per os* with 1 ml of +PV produced from broilers that had proventriculitis at 7 days of age. Group 4 had 12 birds, which were immunosuppressed with IBDV administered at one day of age. Group 5 had 12 birds, which were immunosuppressed with cyclophosphamide (CP) starting at 1 day of age. Group 6 had 12 birds, which were immunosuppressed with cyclosporin (CS) starting at 1 day of age. Group 7 had 8 birds, which were immunosuppressed with IBDV administered at 1 day of age and treated with +PV at 7 days of age. Group 8 had 8 birds, which were immunosuppressed with CP starting at 1 day of age, and treated with +PV at 7 days of age. Group 9 had 8 birds, which were immunosuppressed with CS starting at 1 day of age, and treated with +PV at 7 days of age.

Trial 3. This trial was conducted as trials 1 and 2 with the following modifications.

*Chickens.* Fertile White Plymouth Rock chicken eggs (SEPRL, USDA, Athens, GA) from a breeder flock maintained under SPF conditions were obtained, hatched, and maintained in positive pressure Horsfal isolation units. The parent flock and all progeny used in this experiment were free of common poultry diseases, specifically IBDV, MDV, IBV, reovirus and CAV. The same experimental design and protocol as trials 1 and 2 was followed. Additional animals were included to allow a third sacrifice at 21 days post inoculation.

Immunosuppressive treatment groups. Chickens were immunosuppressed with either, IBDV, CP, or CS as described bellow.

*IBDV Treatment.* Birds in trial 1, (groups 4 and 7) were challenged with IBDV Variant E strain (Intervet, Inc. Millsboro, DE). In trials 2 and 3 chickens in groups 4 and 7 were treated with the STC challenge strain 124-ADV of IBDV (National Veterinary Services Laboratory, Ames, Iowa). Inoculations were given *per os* and by eye drop, and consisted of 100 µl containing at least 10³ mean tissue culture infective dose of virus diluted in phosphate-buffered saline (PBS).

*Cyclophosphamide (CP) treatment.* B lymphocyte immunosuppression was induced using an established protocol (32). Briefly, groups 5 and 8 in all three trials received one intraperitoneal injection of 4 mg CP (Cyclophosphamide monohydrate; Sigma Chemical Co., St.Louis, MO) (0.1 ml) daily for 4 days starting the first day after hatch. For injection, CP was obtained in a dry form, and an aqueous solution was prepared by reconstituting 1.6 g in 40 ml of calcium- and magnesium-free phosphate buffered sterile saline (CMF-PBS) and filtering this through a 0.22 µm syringe filter. The resulting solution contained 40 mg of CP/ml.

*Cyclosporin (CS) treatment.* T lymphocyte immunosuppression was induced using an established protocol (31). Briefly, chickens from groups 6 and 9 in all three trials received one intramuscular injection of CS, 100mg/kg body weight, every 3 days from the first day after hatch until the experiment ended. CS was prepared by diluting a stock solution (Sandimmune, 100mg/ml, Novartis Pharma AG, Basle, Switzerland) 1:1 in olive oil. Dilutions of the drug were adjusted as body weights increased.

Immunosuppression in IBDV, CP, and CS treated groups was assessed by histopathologic examination of immune organs (bursa, thymus and spleen), cutaneous hypersensitivity response testing (CBH), and humoral response to NDV vaccination.

Challenge with proventricular homogenates. At 7 days of age birds from groups 3, 7, 8, and 9 in trial 1 were inoculated by oral gavage with 1 ml of a positive proventricular homogenate (+PV) consisting of proventriculi obtained from commercial broilers with proventriculitis (13). Proventriculi from chickens in group 3 (+PV treated) of trial 1 were homogenized as previously described (4) and used to expose +PV groups in trial 2 and trial 3. Briefly, proventriculi collected from birds that developed proventriculitis were washed in sterile normal saline (PBS) three times on a magnetic stirrer to remove feed residues and toxins. Washed proventriculi were then diluted 1:1 wt/vol in PBS and homogenized with a commercial blender (Waring, New Hartford, Connecticut). The homogenates were frozen at -80 C and thawed at room temperature immediately before inoculation. The same procedure was used with proventriculi from normal broiler chickens without proventriculitis to produce a negative proventricular homogenate (-PV). This was used to inoculate birds from group 2 in all three trials. Birds of group 1 in all trials were sham inoculated with 1 ml of sterile saline.

Cutaneous basophil hypersensitivity (CBH) response. This test was performed to evaluate T-cell function in the immunosuppression treatment groups at 2 weeks of age as previously described (6). Four chickens from groups 1 (saline), 4 (IBDV), 5 (CP), and 6 (CS) were injected intradermally in the skin between the third and fourth digits of the left foot with 200 µg of Phytohemmagglutinin-P (PHA-P, Sigma, St. Louis, MO) in 100 µl of sterile physiological saline solution (PSS). The right foot of each chicken was similarly injected with 100 µl of PSS without PHA-P to serve as a control. The CBH response to PHA-P was evaluated by determining the thickness of the interdigital skin before injection, and at 12 and 24 hours after injection with a constant-tension, digital micrometer (Mitotuyo Co., Kanagawa, Japan). The CBH response was calculated by two methods: 1) CBH-1 or increased skin thickness = (post-injection skin thickness, left foot)-(pre-injection skin thickness, left foot); and 2) CBH-2 response = (PHA-P response, left foot)-(PSS response, right foot).

NDV vaccination. To asses humoral immune function 4 birds from groups 1 (saline), 4 (IBDV), 5 (CP), and 6 (CS) were vaccinated at 21 days old with killed Newcastle Disease vaccine (Vineland Laboratories, Vineland, NJ). Each bird was given one dose of 0.5 ml of vaccine intramuscularly as recommended by the manufacturer. Two weeks later birds were bled to obtain sera, and antibodies to NDV were quantified by ELISA (IDEXX Laboratories, Inc. Westbrook, Maine), and HI test using the diluted serum-constant virus procedure (37).

Sample collection and processing. All birds were wing banded and weighed at one day of age. At 14 and 21 days of age, 4 birds were randomly selected from each group and examined, weighed, bled, killed by cervical dislocation, and necropsied. Bursa, proventriculus, spleen, and the right half of the thymus were collected from each bird, weighed, and a portion of each fixed immediately by immersion in 10% neutral buffered formalin for 24 hours. Tissues were then processed and embedded in paraffin using routine histologic techniques. The remaining proventriculi were collected in sterile plastic tubes over ice for subsequent preparation of homogenate as explained previously. Relative organ weights were obtained using the formula [Relative organ weight = (organ weight / body weight) x 100].

Histopathology. Paraffin-embedded tissues samples from bursa, proventriculus, spleen and thymus from each bird were sectioned, mounted, stained using hematoxylin and eosin (HE), and examined in a blinded fashion as to treatment for lesions using light microscopy. All sections of bursa and proventriculus were assigned a lesion severity score. A lesion score of 1 represented no lesions. For bursal sections, 2 was defined as mild variation in follicle size, 3 as moderate variation in size of follicles, and 4 as either necrosis or follicle atrophy. For proventricular sections, 2 was defined as mild glandular lumenal ectasia, 3 as ectasia plus lymphoid infiltrates in the interglandular interstitium and 4 as either acute glandular necrosis or severe fibrosis with lymphoid infiltrates. Also spleen and thymus were examined for the presence of lesions.

Serology. Serum samples obtained at days 14 and 21 of age were examined for antibody to IBDV, IBV, NDV, CAV, and reovirus using commercially available ELISA tests (IDEXX Laboratories, Inc. Westbrook, Maine).

Real time RT-PCR. RNA was extracted from formalin-fixed paraffin-embedded bursas and proventriculi and examined for IBDV nucleic acid by real time RT-PCR (25). Sections totaling fifty microns in thickness were cut from each formalin-fixed paraffin-embedded tissue block, deparaffinized in HemoDe (Fisher Scientific, Pittsburgh, PA), washed with 100% ethanol, and digested with 25µg/ml proteinase K (Sigma Chemical Co., St. Louis, MO) for 1 hour at 50 C. RNA was extracted using Trizol (Life Technologies, Inc. Gaithersburg, MD) according to the manufacturer's recommendations, diluted in 25µl of 90% dimethyl sulfoxide (DMSO), and frozen at -80 C until assayed. Extracted RNA was denatured at 95 C for 5 minutes and put on ice. A reverse transcriptase polymerase chain reaction (RT-PCR) was performed using reagents from the Light Cycler-RNA Amplification SYBR^{®} Green I Kit (ROCHE Molecular Biochemicals, Indianapolis, IN). The primers used were designed to amplify a 400 bp segment of the IBDV genome shared by all strains, which flanks a hypervariable region of the VP2 gene. Primer sequences were B4 5' TCTTGGGTATGTGAGGCTTG (SEQ ID NO: 9) and B4 3' GGATGTGATTGGCTGGGTTA (SEQ ID NO: 10). Amplification and detection of specific products was also performed using a Light Cycler (ROCHE Molecular Biochemicals, Indianapolis, IN) according to the manufacturer's recommendations (ROCHE Light Cycler version 3.0, ROCHE Molecular Biochemicals, Indianapolis, IN). Briefly, reverse transcription was done at 55 C for 10 minutes, followed by denaturation at 95 C for 30 seconds. Forty PCR cycles were done consisting of denaturation (95 C for 1 second), hybridization (55 C for 10 sec), and extension (72 C for 13 sec). A melting curve analysis was done with an initial denaturation at 95 C. DNA melting was accomplished with an initial temperature of 65 C for 10 seconds and a gradual temperature increase of 0.1 degree C per second until reaching 95 C. The melting temperature of the expected 400 bp amplicon was between 82 C and 84 C. This estimated melting temperature was used to confirm the identity of IBDV specific products obtained using real time RT-PCR. Additional confirmation of specific amplification was done using routine gel electrophoretic techniques of the PCR products on 2% agarose (Sigma Chemical Co., St. Louis, MO) followed by ethidium bromide staining.

Statistical analysis. The body weight gain, relative bursal and proventricular weights, and bursal and proventricular lesion scores were analyzed using ANOVA and means comparisons for all pairs using Tukey-Kramer HSD. Significance was assumed at the 0.05 level of probability.

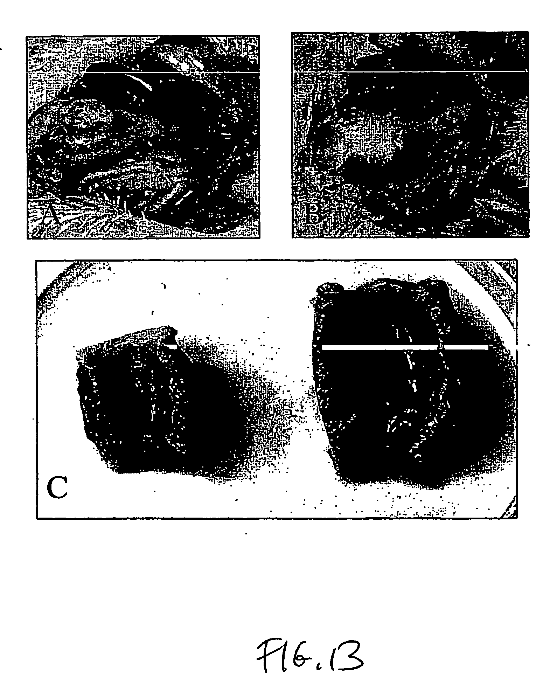

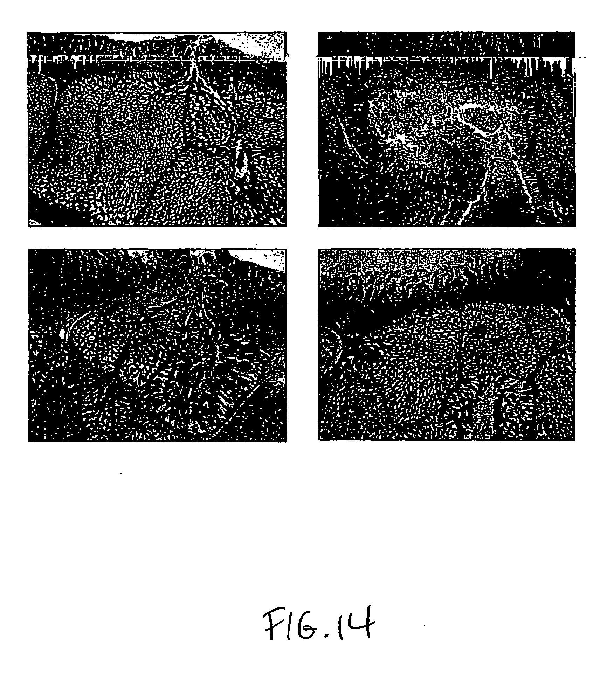

RESULTS: Control Groups. *Proventricular homogenate controls.* Chickens inoculated only with saline or negative proventricular homogenate (-PV) did not develop proventriculitis in any of the three trials. Macroscopic lesions were not observed when examined at necropsy (Fig. 13). Mean body weight gain and relative proventriculus weight for these two groups was very similar (Tables 18 and 19 respectively). Mild microscopic lesions consisting mainly of mild lumenal ectasia of the proventricular glands were present in some of these birds (Table 20). Chickens that were inoculated only with positive proventricular homogenate (+PV) had no significant suppression of weight gain compared to saline and -PV groups in all three trials (Table 18). There was a trend toward enlargement of the proventriculus in chickens that received the positive Proventricular homogenate. Increased microscopic lesions were present in the proventriculus of chickens that received positive proventricular homogenate in trials 1 and 2 at 7 and 14 dpi, and in trial 3 at 14 dpi. At necropsy, the proventriculus of these chickens were enlarged and swollen, with plaques or mottling visible on the serosal surface, dilation of the gastric isthmus, and mucosal lesions (flattened papillae, with secretion of white fluid) (Fig. 13). Microscopically, at 7 dpi, acute necrosis of the glandular epithelium was present. Collecting sinuses of the glands were dilated and contained desquamated epithelium. Nuclei of the glandular epithelial cells were enlarged and pale, with marginated chromatin. Lymphocytic infiltrates were present as sheets in the lamina propria of the mucosa and expanded the glandular epithelium between the epithelium of the ducts and the glands (Fig. 14). At 14 dpi, glandular epithelium was replaced by ductal epithelium. Lymphocyte infiltrates and germinal center formation were present in the glands and mucosa (Fig. 14). In trial 3 chickens that were inoculated with +PV, showed similar mild to moderate lesions in the proventriculus at 21 dpi, but no significant increase in size of the proventriculus compared to saline or -PV controls. Small germinal centers were present in the glands (Fig. 14) of +PV-dosed chickens but not in those given saline or -PV.

No lesions or differences in relative organ weight of the bursa were observed between chickens that received saline, -PV, or +PV (Tables 21 and 22).

*Immuno suppression controls.* Commercial broilers (group 4) in trials 1 and 2, treated with IBDV strains Variant E and STC respectively, had no signs of IBDV infection at 7 and 14 dpi. Their bursas had no significant microscopic lesions, no difference in relative organ weight when compared to controls (Tables 21 and 22), and were negative for IBDV by RT-PCR. CBH response, and humoral response to NDV vaccination was similar to the saline control group (Tables 23 and 24), all indicating that challenge with IBDV in these birds did not produce IBDV infection. However, SPF broiler chickens in trial 3 exposed to IBDV strain STC did have signs of depression at 7 days post inoculation and their bursas were significantly smaller than saline control chickens at 7, 14 and 21 dpi (Table 21). Severe microscopic lesions were also observed (Table 22), and bursas were positive for IBDV by RT-PCR. Humoral immune response to NDV vaccination was significantly lower than saline controls (Table 24).

Cyclophosphamide (CP) control chickens (group 5) in all three trials tended to be smaller than chickens from the other groups, due to a reduction in their weight gain. This reduction was significant in the SPF broilers in trial 3 (Table 18). These chickens also had decreased feathering and appeared weak. The bursas of these chickens were significantly smaller in all three trials (Table 21), and marked lymphocytic depletion and atrophy of the bursa was observed (Table 22). A small reduction of CBH response, was observed in these birds (Table 23), and humoral response to NDV vaccination was significantly reduced (Table 24).

Cyclosporin (CS) control chickens (group 6) in trials 1 and 2 appeared normal (similar to saline controls). Although their weight gain was reduced it was not significantly different from that of the saline controls (Table 18). Weight gain in chickens in trial 3 was reduced at 7 and 21 dpi. Bursas of birds treated with CS had no lesions and there was no difference in size compared to saline controls (Tables 21. and 22). Thymuses did not have any significant lesions, but the CMI immune capacity was significantly reduced (Table 23). The CBH-1 and CBH-2 responses were decreased (p < 0.05) compared to the saline control group. The humoral immune response, measured by antibody production after NDV vaccination, was not affected (Table 24).

The effect of the immunosuppressive treatments (IBDV, CP, CS) on the proventriculus relative weight or presence of lesions was very mild and not significantly different than that observed in saline or -PV controls (Tables 19 and 20).

Experimental groups. *Body weight gain.* Chickens treated with CP and +PV had a significant reduction in body weight gain compared to the control groups (saline, -PV and +PV), including those given CP only, in trials 1 at 7 and 14 dpi and trial 2 at 7 dpi. (Table 18). The combination of CS and +PV had a detrimental effect on weight gain in trail 2 at 14 dpi and trial 3 at 7 and 21 dpi, but the difference from chickens given CS only was not significant in any instance.

*Organ relative weights and lesions.* No significant difference was observed between control and experimental groups for spleen and thymus in any of the trials. (Data not shown). The exceptions were the chickens treated with CP where at 7dpi their thymuses were smaller than the rest of the groups, but by 14 dpi they were the same as controls. In all three trials, birds treated with CP and +PV had a significant decrease in bursal size and developed high lesion scores but these were no different than those in CP controls (Tables 21 and 22). In trial 3, lesions and a significant decrease in size of the bursa occurred in chickens that were challenged with IBDV and exposed to +PV, similar to that observed in the IBDV controls (Tables 21 and 22). These bursas were also IBDV positive by RT-PCR.

Relative proventricular weight of chickens that were immunosuppressed and treated with +PV was increased at 7 and 14 dpi when compared to the control chickens (saline and -PV), but in most cases there was no significant difference when compared to the +PV controls. Chickens in trial 1 and 2 at 7 dpi treated with the combination of CP/+PV, had a significant increase in relative proventricular weight relative to the +PV controls (Table 19). The lesion score of the proventriculi from all immunosuppressed birds treated with +PV was also similar to those observed in the +PV control groups at 7 and 14 dpi (Table 20), although there was an increase in the incidence of proventriculitis and a difference in the appearance and severity of the lesions observed in the birds treated with CS. This was more evident in the SPF broilers where all birds treated with the combination of CS and +PV had moderate to severe proventriculitis. CS/+PV scores were significantly higher than all other treatments at 21 dpi in trial 3. In all three trials, the incidence and severity of proventriculitis was highest at 14 dpi than 7dpi. In trial 3 at 21 dpi the relative weight and lesion score of the proventriculi of all birds that received +PV was similar to the negative controls, with the exception of the chickens treated with CP/+PV which scoring and weight remained significantly higher than birds in the other groups (Tables 19 and 20).

Chickens treated with +PV in all three trials, regardless of the immunosuppression treatment, had acute necrosis of the proventricular glands at 7 dpi with some lymphocyte infiltrates, mostly in the mucosa. In some cases lymphocyte infiltrates also were present in the glands in the form of sheets. Hemorrhage and congestion were also sometimes present. Birds treated with CS had more severe lesions, with destruction and coalescence of the glands.

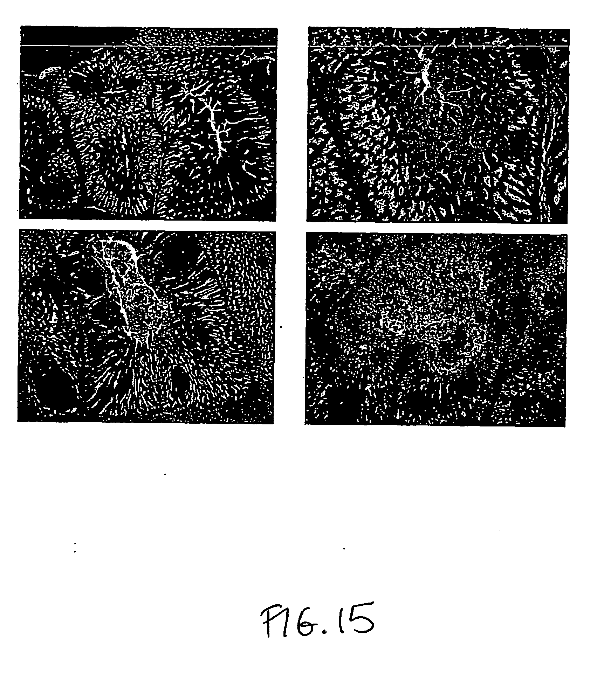

At 14 dpi, chickens treated with IBDV and +PV, or CP and +PV, had metaplastic replacement of proventricular glandular secretory epithelium by ductal epithelium, and lymphocyte infiltrates as observed in the +PV only-treated chickens. Proventricular lymphoid germinal centers were smaller, or not present, in birds treated with CP (in all three trials) or IBDV (in trial 3). Chickens treated with CS and +PV in trials 1 and 2 still had acute necrosis at 14 dpi, reduced lymphocyte infiltration and variable germinal center formation, and minimal metaplasia (Fig 15).

At 21 dpi, SPF broilers treated with IBD and +PV, or CP and +PV, had mild to moderate lesions, with very little lymphocyte infiltration. These were mostly in the form of small germinal centers. Chickens treated with CS and +PV had severe lesions consisting of acute necrosis of the glandular epithelium, coalescing of glands, and small and multiple germinal centers.

Serology. Chickens from all groups in trial 1 had ELISA titers against IBDV and IBV at 14 days of age (7 dpi), and had no titers against NDV, reovirus or CAV. These IBDV and IBV titers decreased but were still present at 21 days of age (14 dpi). Chickens in trial 2 had titers for IBDV, IBV, and NDV at 14 days of age (7 dpi), but not against reovirus. In both trials, chickens that received -PV or +PV (with the exception of birds treated with CP) developed titers against reovirus at 21 days of age (14 dpi).

SPF broiler chickens (trial 3) at 14 days of age (7 dpi) were seronegative for NDV, IBV, reovirus, and CAV. They also were negative for IBDV with the exception of those challenged with IBDV, which developed and had seroconversion at 14, 21 and 30 days of age (7, 14, and 21 dpi). At 21 and 30 days of age (14 and 21 dpi) birds that received +PV, but were not treated with CP, had titers against IBV, NDV, and reovirus. All birds were negative for CAV at all time points.

IBDV RT-PCR. IBDV was not detected in paraffin-embedded bursas or proventriculi from any of the birds in Trials 1 or 2. In Trial 3, IBDV was detected at 7, 14 and 21 dpi in paraffin-embedded bursas from all IBDV challenged birds. It was not detected in any of the proventriculi from these birds, or in bursas or proventriculi from chickens in the other groups in trial 3.

DISCUSSION. The relationship between IBDV and proventriculitis is not clear. Both gross and microscopic lesions of the proventriculus have been produced by IBDV challenge in leghorn chickens (24) and vaccination against IBDV has been reported to decrease the incidence of proventriculitis (7,15). However, proventriculitis was not produced by inoculation of SPF broilers with different strains of IBDV (25). Commercial chickens get exposed to IBDV early in life, and although mortality in unprotected flocks can be quite significant, the major concern for the poultry industry is IBDV's ability to cause immunosuppression. Immunosuppressed birds often fail to respond adequately to vaccination and are susceptible to secondary infections. The mechanisms of IBDV-induced immunosuppression are not fully understood. Both humoral and cellular immune responses are compromised (34). Inhibition of humoral immunity is more severe and is attributed to the destruction of immunoglobulin-producing B cells by the virus. IBDV-exposed chickens produce suboptimal levels of antibodies against a number of infectious and non-infectious antigens (34). Although T cells do not serve as targets for IBDV replication, cell-mediated immune responses of virus-exposed chickens seem to be compromised (5,18,33,34).

Protection against IBDV is achieved by the induction of neutralizing antibodies, as well as by passive transfer of maternal antibodies to young chickens. These maternal antibodies may interfere with IBDV vaccination schedules. In our study, commercial broiler chickens (Trials 1 and 2) inoculated with an infecting dose of IBDV did not develop disease. No lesions were observed in their bursas, and RT-PCR did not detect any virus. Consequently, these birds were not immunosuppressed by IBDV as intended, and had a normal response to NDV vaccination. On the other hand, SPF broiler chickens were successfully infected with IBDV when intentionally challenged at one day of age. Their bursas were significantly smaller than controls, had lesions typical of IBDV infection, and were positive for the virus by RT-PCR. They also developed antibodies against IBDV, and were immunosuppressed as measured by their low seroconversion to NDV. However, infection with this particular strain of IBDV (STC) produced no proventriculitis.

CP treatment has been used to inhibit humoral immunity in order to determine its role in the pathogenesis of infectious pathogens of chickens (1,31). Chickens treated with CP had bursas that were significantly smaller and depleted of lymphocytes, and they did not develop specific antibody after NDV vaccination, demonstrating their humoral immunosuppression. Both CP and IBDV have minor effects on CMI (32,34). There was a mild depression of the CBH response in birds treated with IBDV (trial 3) or CP, but this was not significant when compared to controls.

As expected, chickens from all three trials treated with CS exhibited a significantly decreased CBH response (6). CS prevents cytokine synthesis in T cells by blocking a later stage of T cell receptor initiated signaling, reducing production of interleukin-2 (IL-2), and hence T cell proliferation (12,28). As a consequence, IL-2 dependent functions, which include T-helper activities, cytotoxicity, natural killer cell activity, and antibody dependent cytotoxicity, are decreased (11). As expected, humoral immune response of birds treated with CS was not affected, and they developed anti-NDV antibodies following NDV vaccination.

The homogenate used to induce proventriculitis in trial 1 was known to contain IBDV (13). In an attempt to reproduce a proventriculitis as close to that observed in naturally occurring cases, commercial broilers with maternal antibodies to IBDV were used in trials 1 and 2. Inoculation of these chickens in trial 1 with the IBDV-bearing homogenate produced proventriculitis but no IBDV infection since their anti-IBDV antibody was protective. Since proventriculitis still occurred, this suggests that proventriculitis was not directly produced by infection with the IBDV present in that homogenate, but does not exclude IBDV as a potential contributing factor. In trials 2 and 3, proventriculitis was produced by inoculating birds with positive proventricular homogenate produced from birds with proventriculitis in trial 1. Excluding those challenged with IBDV intentionally, chickens given this homogenate in trials 2 and 3 developed proventriculitis but no IBDV infection. These data suggest our serial passage of the original proventricular homogenate through antibody positive broiler chickens cleared it of IBDV and propagated the causative agent responsible for proventriculitis.

The proventriculitis produced in trial 1 was more severe than that in trials 2 and 3. This may be due to reduction in titer of the causative pathogen by *in vivo* passage in the presence of antibody, or clearance of the IBDV as described above. Even so, the incidences of proventriculitis within groups and the effects of immunosuppression on proventriculitis were similar across all three trials.

Immunosuppression induced by cyclophosphamide (CP) in all three trials, and by IBDV in trial 3, did not affect the incidence or lesion severity of the proventriculitis observed. Proventricular lesions observed in chickens that received CP/+PV were similar to that observed in the +PV controls. There was acute glandular necrosis and some lymphocyte infiltrate at 7 dpi, and glandular metaplasia with severe lymphocyte infiltrates at 14 dpi. Both, sheets and follicles of lymphocytes, were present, representing T and B cells respectively (22,25), however in these birds less follicle formation was observed. Helper and cytotoxic T cells are both present in normal proventriculi (22) and their numbers increase dramatically in proventriculitis (25). Lower numbers of B cells are present in normal proventriculi, and in proventriculitis their numbers also increase. Although the lesions observed in the proventriculi of birds treated with CP/+PV were similar to that of the controls, at 7 dpi chickens from these groups in trials 1 and 2, had significantly higher proventriculus weights than the +PV controls. This suggests a role of B cells in the early stages of proventriculitis, where compromised production of antibodies could exacerbate the severity of the condition.

All chickens with T-cell suppression due to cyclosporin (CS) and treated with +PV had equal or higher incidence and lesion scores of proventriculitis than +PV controls. The proventricular relative weights also tended to be higher than +PV controls, being more evident in the SPF birds in Trial 3 where this difference was significant at 21 dpi. Cell mediated immune (CMI) responses have been suggested to play a key role in the elimination of avian enteric pathogens (1,20,36), and our data indicate that T cell functions play a role in controlling proventriculitis. The high incidence of lesions in the proventriculi of birds in Trail 3 at 21 dpi that were immunosuppressed with CP and treated with +PV indicates the importance of T lymphocytes in the clearing and resolution of proventriculitis. It is well known that IBDV can affect the CMI response (5,18,33,34) and although we saw little effect of IBDV-induced immunosuppression on the severity of proventriculitis in this study, it is possible that preventing severe immunosuppression in the field through vaccination against IBDV, could diminish the severity of proventriculitis.

Serologic results infer that the original positive proventricular homogenate used in this study contained IBDV, IBV, NDV and reovirus because some dosed experimental chickens seroconverted to these agents. Passage of this homogenate in commercial broilers seemed to have eliminated IBDV because SPF's challenged with the subsequent proventricular homogenate did not seroconvert to this virus or develop bursal disease. The objectives and experimental design of the present study were not designed to determine the role(s) of these other agents in proventriculitis, so no conclusions should be drawn from their presence here.

In conclusion, B cell immunosuppression, by CP or IBDV, did not have an effect on the incidence of proventriculitis, and the lesions observed were similar to those produced by +PV alone. However, proventricular enlargement was more evident in these birds at 7dpi, indicating that humoral response might be important in the early stages of the disease probably by controlling the causative agent by production of antibodies. T cell suppression by CS, on the other hand, did have an effect on the incidence of proventriculitis, and the lesions observed were more severe and lasted longer than in +PV controls. T cells are more abundant in the proventriculus than B cells, which suggests their importance in immune responses to infectious agents in this organ. In this study, by affecting T cell function, the severity of proventriculitis was increased and resolution of the disease was prolonged.

### References:

1. Arnold & Holt. Poult Sci.74. 656-665. 1995.
2. Barnes et al. Poultry Sci. 80: 906-911. 2001.
3. Bayyari et al. Poult Sci. 74:1961-1969. 1995.
4. Bayyari et al. Poult Sci, 74: 799-1809. 1995.
5. Confer et al. Am J Vet Res 42:2109-2113. 1981.
6. Corrier & De Loach. Poult Sci. 69: 403-408.1990.
7. Dormitorio et al. International Poultry Scientific Forum. January 15-16. Atlanta, Georgia. 2001.
8. Giambrone et al. Am J Vet Res 38:581-583. 1977.
9. Goodwin et al. Avian Pathol. 25:369-379. 1996.
10. Guy & Barnes. Proceedings of the 139th AVMA Annual Convention. July. Nashville. TN. 2002.
11. Hill et al. Avian Dis. 33: 86-92. 1989.
12. Ho et al. Clinical Immunol Immnunopathol. 80: S40-45. 1996.
13. Huff et al. Avian Dis. 45:828-843. 2001.
14. Jensen et al. Avian Dis. 35:969-973. 1991.
15. Kelly et al. Proceedings of the 138th AVMA Annual Convention. July 14-18. Boston, MA. 2001.
16. Kouwenhoven et al. Avian Pathol. 7:183-187. 1978.
17. Kouwenhoven et al. Avian Pathol. 17:879-892. 1988.
18. Lam. Avian Pathol. 20:867-876. 1991.
19. Lenz et al. J Vet Diagn Invest. 10:145-151. 1998.
20. Lillehoj & Bacon. Avian Dis. 35:294-301. 1991.
21. Lukert & Saif. Disease of Poultry, 11th edition. B.W. Calnek, H.J. Barnes, C.W. Beard, L. R. McDougald, and Y.M. Saif, eds. Iowa State University Press, Ames. IA. P 161-179.2003.
22. Matsumoto & Hashimoto. J Vet Med Sci 62:161-167. 2000.
23. Mutlu et al. Tierarztl Prax Ausg G Grosstiere Nutztiere. 25:460-462. 1997.
24. Newberry. Ph. D. Dissertation. University of Arkansas, Fayetteville, AR. 1996.
25. Pantin & Brown. Abstracts of the 52nd Annual Meeting of the American College of Veterinary Pathologists. Vet Pathol. 38:579. 2001.
26. Pegram & Wyatt. Poultry Sci. 60:2429-2440.1981.
27. Poole. Proceedings of the 43rd Western Poultry Disease Conference, Sacramento CA. Pp. 40-42. 1994.
28. Resch & Szamel. Int J Immunopharmacology. 19:579-585. 1997.
29. Riddell. Avian Dis. 20:442-445. 1976.
30. Rodenberg et al. Avian Dis. 38:16-21. 1994.
31. Russell et al. Vet Immunol Immunopathol. 60:171-185. 1997.
32. Sharma & Lee. Infection and Immunity. 227-230. 1977.
33. Sharma et al. Avian Dis. 33: 112-124. 1989.
34. Sharma et al. Dev Comp Immunology. 24: 223-235. 2000.
35. Schulze & Heidrich. Dtsch Tierarztl Wochenschr 108:264-266.2001.
36. Songserm et al. Vet Immunol Immunopathol 85:51-62. 2002.
37. Thayer & Beard. A laboratory manual for the isolation and identification of avian pathogens. Fourth edition. Published by The American Association of Avian Pathologists. 1998.
38. Turpin. Ms. Thesis. UGA .1998.
39. Yu et al. Avian Dis. 45:416-424. 2001.
40. Van der Berg. Avian Pathol. 29:175-194. 2000.
41. Wideman et al. J Appl Poult Res 5:219-230. 1996.

### PROVENTRICULITIS IN BROILER CHICKENS: EFFECTS OF IMMUNOSUPPRESSION (Pantin-Jackwood et al. Veterinary Pathology, in press).

SUMMARY. Proventriculitis in broilers causes carcass condemnation when swollen proventriculi tear during evisceration. The cause of this proventriculitis is unknown but several infectious agents have been associated with it. One such agent, infectious bursal disease virus (IBDV), has been implicated as a cause of proventriculitis, but a direct effect of this virus on the proventriculus has not been proven. The role of IBDV in proventriculitis may be indirect due to its ability to cause immunosuppression. The objective of this study was to understand how immunosuppression affects the incidence of proventriculitis in broiler chickens. Immunosuppression was induced in commercial and SPF broiler chickens using chemicals (cyclophosphamide and cyclosporin) or virus (IBDV). All groups were then exposed to a proventricular homogenate produced from diseased birds. At 7 and 14 days post inoculation, the incidence of proventriculitis in these groups was compared to that produced by homogenate exposure in immunocompetent broilers. All birds exposed to the proventricular homogenate from diseased birds developed proventriculitis. Cyclophosphamide and IBDV, both B cell suppressors, did not significantly affect the incidence or characteristics of the proventriculitis observed, although they did have an effect on the size of the proventriculus at 7 days post inoculation. Chickens immunosuppressed with cyclosporin, a T cell suppressor, developed more severe lesions and had a higher incidence of proventriculitis. These findings indicate that both, B and T cells, are involved in the immune response against proventriculitis, but cell mediated immunity appears to have a more important role in controlling the disease. IBDV affects both humoral and cellular immunity in the chicken so although under experimental conditions it didn't have a major effect on proventriculitis, it may explain why control of IBDV in the field seems to reduce the incidence of proventriculitis. Key words: Proventriculitis, immunosuppression, IBDV. Abbreviations: CBH = cutaneous basophil hypersensitivity; CMI = cell-mediated immunity; CP = cyclophosphamide; CP = cyclosporin; IBDV = infectious bursal disease virus; RT-PCR = reverse transcriptase polymerase chain reaction; SPF = specific-pathogen free.

INTRODUCTION. Proventriculitis is a clinical condition that affects broiler chickens. It is characterized by enlargement of the proventriculus and weakness of the gastric isthmus. During routine evisceration at processing, affected proventriculi rupture causing spillage of the proventricular contents into the body cavity, which results in condemnation of affected carcasses for contamination. The disease has also been associated with impaired growth, and poor feed conversion (16, 13). Microscopically, degeneration and necrosis of proventricular glands is observed accompanied by marked intraglandular interstitial lymphocytic infiltration (4, 9,10).

Several agents have been implicated as potential causes of proventricular lesions. Noninfectious causes include oral exposure to biogenic amines (2,27), mycotoxins (26), lack of dietary fiber (29), and excessive copper sulfate (3,14,41). Infectious causes include adenovirus (19), reovirus (17,38), infectious bronchitis virus (39), and megabacterium (23,35). However, none of these noninfectious or infectious agents have been found consistently in a majority of cases. Electron microscopy has detected viral particles in acute lesions but isolation of a virus from affected proventriculi has been unsuccessful (9,10, 13).

Infectious Bursal Disease Virus (IBDV) has been implicated as the cause for this disease (4,13,24), and IBDV vaccination has been reported to decrease its incidence (7,15). Proventriculitis can be reproduced by orally inoculating broilers with homogenized proventriculi collected from affected birds (16,4). A filterable agent found in these homogenates causes lesions similar to those found in field cases (4), and IBDV has been immunoprecipitated from these homogenates (13). Commercial broilers exposed to this IBDV developed increased proventricular lesion scores but had no increase in proventricular size, a characteristic feature produced by exposure to proventricular homogenates (13). These findings suggest other agents or conditions may be required to produce proventriculitis.

IBDV induces immunosuppression in chickens (21,34,40). Immunosuppressed flocks may have an increased incidence of secondary infections, poor feed conversion, and reduced protective response to commonly used vaccines (34). IBDV causes lytic destruction of IgM+ B lymphocytes that results in suboptimal levels of antibodies against a number of infectious and noninfectious antigens (8,30,34). Although the immunosuppression caused by IBDV is principally due to B lymphocyte damage, an effect on cell-mediated immunity (CMI) has also been demonstrated (5,18,33,34).

SPF broilers exposed to different strains of IBDV did not develop proventricular lesions or enlargement at 4 or 6 days post-inoculation (25). The virus was detected in large quantities in the bursa of these birds by RT-PCR and immunohistochemical techniques, but not in the proventriculus, indicating it is not a target organ for IBDV. However, the immunosuppressive effect of IBDV could explain its reported relationship to proventriculitis. The purpose of our study was to see if immunosuppression had any effect on the incidence, severity, or character of proventriculitis in broiler chickens. To address this, commercial and SPF one-day old broilers were immunosuppressed with cyclophosphamide (B cell suppressor), cyclosporin (T cell suppressor), or IBDV. Subsequently these chickens were exposed to a proventricular homogenate from affected chickens, and the effect of immunosuppression on proventriculitis was determined.

MATERIALS AND METHODS. Animal housing. One-day-old chickens were divided into groups and housed in positive pressure Horsfal units. Unmedicated feed and water were provided *ad libidum.*

Experimental design. Trials 1 and 2. A total of 88 unvaccinated commercial broiler chicks, obtained from a local hatchery, were divided into 9 groups of 8 or 12 birds, and chicks in each group received either an immunosuppressive treatment or no treatment (Table 17). Chickens subsequently received as described below, either positive (+PV) or negative (-PV) proventricular homogenate, saline, or no homogenate. Group 1 had 12 birds, which were inoculated *per os* with 1 ml of sterile saline at 7 days of age. Group 2 had 8 birds, which were inoculated *per os* with 1 ml of -PV produced from normal commercial broilers at 7 days of age. Group 3 had 8 birds, which were inoculated *per os* with 1 ml of +PV produced from broilers that had proventriculitis at 7 days of age. Group 4 had 12 birds, which were immunosuppressed with IBDV administered at one day of age. Group 5 had 12 birds, which were immunosuppressed with cyclophosphamide (CP) starting at 1 day of age. Group 6 had 12 birds, which were immunosuppressed with cyclosporin (CS) starting at 1 day of age. Group 7 had 8 birds, which were immunosuppressed with IBDV administered at 1 day of age and treated with +PV at 7 days of age. Group 8 had 8 birds, which were immunosuppressed with CP starting at 1 day of age, and treated with +PV at 7 days of age. Group 9 had 8 birds, which were immunosuppressed with CS starting at 1 day of age, and treated with +PV at 7 days of age.

Trial 3. This trial was conducted as trials 1 and 2 with the following modifications. *Chickens.* Fertile White Plymouth Rock chicken eggs (SEPRL, USDA, Athens, GA) from a breeder flock maintained under SPF conditions were obtained, hatched, and maintained in positive pressure Horsfal isolation units. The parent flock and all progeny used in this experiment were free of common poultry diseases, specifically IBDV, MDV, IBV, reovirus and CAV. The same experimental design and protocol as trials 1 and 2 was followed. Additional animals were included to allow a third sacrifice at 21 days post inoculation.

Immunosuppressive treatment groups. Chickens were immunosuppressed with either, IBDV, CP, or CS as described bellow.

*IBDV Treatment.* Birds in trial 1, (groups 4 and 7) were challenged with IBDV Variant E strain (Intervet, Inc. Millsboro, DE). In trials 2 and 3 chickens in groups 4 and 7 were treated with the STC challenge strain 124-ADV of IBDV (National Veterinary Services Laboratory, Ames, Iowa). Inoculations were given *per os* and by eye drop, and consisted of 100 µl containing at least 10³ mean tissue culture infective dose of virus diluted in phosphate-buffered saline (PBS).

*Cyclophosphamide (CP) treatment.* B lymphocyte immunosuppression was induced using an established protocol (32). Briefly, groups 5 and 8 in all three trials received one intraperitoneal injection of 4 mg CP (Cyclophosphamide monohydrate; Sigma Chemical Co., St.Louis, MO) (0.1ml) daily for 4 days starting the first day after hatch. For injection, CP was obtained in a dry form, and an aqueous solution was prepared by reconstituting 1.6 g in 40 ml of calcium- and magnesium-free phosphate buffered sterile saline (CMF-PBS) and filtering this through a 0.22 µm syringe filter. The resulting solution contained 40 mg of CP/ml.

*Cyclosporin (CS) treatment.* T lymphocyte immunosuppression was induced using an established protocol (31). Briefly, chickens from groups 6 and 9 in all three trials received one intramuscular injection of CS, 100mg/kg body weight, every 3 days from the first day after hatch until the experiment ended. CS was prepared by diluting a stock solution (Sandimmune, 100mg/ml, Novartis Pharma AG, Basle, Switzerland) 1:1 in olive oil. Dilutions of the drug were adjusted as body weights increased.

Immunosuppression in IBDV, CP, and CS treated groups was assessed by histopathologic examination of immune organs (bursa, thymus and spleen), cutaneous hypersensitivity response testing (CBH), and humoral response to NDV vaccination.

Challenge with proventricular homogenates. At 7 days of age birds from groups 3, 7, 8, and 9 in trial 1 were inoculated by oral gavage with 1 ml of a positive proventricular homogenate (+PV) consisting of proventriculi obtained from commercial broilers with proventriculitis (13). Proventriculi from chickens in group 3 (+PV treated) of trial 1 were homogenized as previously described (4) and used to expose +PV groups in trial 2 and trial 3. Briefly, proventriculi collected from birds that developed proventriculitis were washed in sterile normal saline (PBS) three times on a magnetic stirrer to remove feed residues and toxins. Washed proventriculi were then diluted 1:1 wt/vol in PBS and homogenized with a commercial blender (Waring, New Hartford, Connecticut). The homogenates were frozen at -80 C and thawed at room temperature immediately before inoculation. The same procedure was used with proventriculi from normal broiler chickens without proventriculitis to produce a negative proventricular homogenate (-PV). This was used to inoculate birds from group 2 in all three trials. Birds of group 1 in all trials were sham inoculated with 1 ml of sterile saline.

Cutaneous basophil hypersensitivity (CBH) response. This test was performed to evaluate T-cell function in the immunosuppression treatment groups at 2 weeks of age as previously described (6). Four chickens from groups 1 (saline), 4 (IBDV), 5 (CP), and 6 (CS) were injected intradermally in the skin between the third and fourth digits of the left foot with 200 µg of Phytohemmagglutinin-P (PHA-P, Sigma, St. Louis, MO) in 100 µl of sterile physiological saline solution (PSS). The right foot of each chicken was similarly injected with 100 µl of PSS without PHA-P to serve as a control. The CBH response to PHA-P was evaluated by determining the thickness of the interdigital skin before injection, and at 12 and 24 hours after injection with a constant-tension, digital micrometer (Mitotuyo Co., Kanagawa, Japan). The CBH response was calculated by two methods: 1) CBH-1 or increased skin thickness = (post-injection skin thickness, left foot)-(pre-injection skin thickness, left foot); and 2) CBH-2 response = (PHA-P response, left foot)-(PSS response, right foot).

NDV vaccination. To asses humoral immune function 4 birds from groups 1 (saline), 4 (IBDV), 5 (CP), and 6 (CS) were vaccinated at 21 days old with killed Newcastle Disease vaccine (Vineland Laboratories, Vineland, NJ). Each bird was given one dose of 0.5 ml of vaccine intramuscularly as recommended by the manufacturer. Two weeks later birds were bled to obtain sera, and antibodies to NDV were quantified by ELISA (IDEXX Laboratories, Inc. Westbrook, Maine), and HI test using the diluted serum-constant virus procedure (37).

Sample collection and processing. All birds were wing banded and weighed at one day of age. At 14 and 21 days of age, 4 birds were randomly selected from each group and examined, weighed, bled, killed by cervical dislocation, and necropsied. Bursa, proventriculus, spleen, and the right half of the thymus were collected from each bird, weighed, and a portion of each fixed immediately by immersion in 10% neutral buffered formalin for 24 hours. Tissues were then processed and embedded in paraffin using routine histologic techniques. The remaining proventriculi were collected in sterile plastic tubes over ice for subsequent preparation of homogenate as explained previously. Relative organ weights were obtained using the formula [Relative organ weight = (organ weight / body weight) x 100].

Histopathology. Paraffin-embedded tissues samples from bursa, proventriculus, spleen and thymus from each bird were sectioned, mounted, stained using hematoxylin and eosin (HE), and examined in a blinded fashion as to treatment for lesions using light microscopy. All sections of bursa and proventriculus were assigned a lesion severity score. A lesion score of 1 represented no lesions. For bursal sections, 2 was defined as mild variation in follicle size, 3 as moderate variation in size of follicles, and 4 as either necrosis or follicle atrophy. For proventricular sections, 2 was defined as mild glandular lumenal ectasia, 3 as ectasia plus lymphoid infiltrates in the interglandular interstitium and 4 as either acute glandular necrosis or severe fibrosis with lymphoid infiltrates. Also spleen and thymus were examined for the presence of lesions.

Serology. Serum samples obtained at days 14 and 21 of age were examined for antibody to IBDV, IBV, NDV, CAV, and reovirus using commercially available ELISA tests (IDEXX Laboratories, Inc. Westbrook, Maine).

Real time RT-PCR. RNA was extracted from formalin-fixed paraffin-embedded bursas and proventriculi and examined for IBDV nucleic acid by real time RT-PCR (25). Sections totaling fifty microns in thickness were cut from each formalin-fixed paraffin-embedded tissue block, deparaffinized in HemoDe (Fisher Scientific, Pittsburgh, PA), washed with 100% ethanol, and digested with 25µg/ml proteinase K (Sigma Chemical Co., St. Louis, MO) for 1 hour at 50 C. RNA was extracted using Trizol (Life Technologies, Inc. Gaithersburg, MD) according to the manufacturer's recommendations, diluted in 25µl of 90% dimethyl sulfoxide (DMSO), and frozen at -80 C until assayed. Extracted RNA was denatured at 95 C for 5 minutes and put on ice. A reverse transcriptase polymerase chain reaction (RT-PCR) was performed using reagents from the Light Cycler-RNA Amplification SYBR^{®} Green I Kit (ROCHE Molecular Biochemicals, Indianapolis, IN). The primers used were designed to amplify a 400 bp segment of the IBDV genome shared by all strains, which flanks a hypervariable region of the VP2 gene. Primer sequences were B4 5' TCTTGGGTATGTGAGGCTTG (SEQ ID NO: 9) and B4 3' GGATGTGATTGGCTGGGTTA (SEQ ID NO: 10). Amplification and detection of specific products was also performed using a Light Cycler (ROCHE Molecular Biochemicals, Indianapolis, IN) according to the manufacturer's recommendations (ROCHE Light Cycler version 3.0, ROCHE Molecular Biochemicals, Indianapolis, IN). Briefly, reverse transcription was done at 55 C for 10 minutes, followed by denaturation at 95 C for 30 seconds. Forty PCR cycles were done consisting of denaturation (95 C for 1 second), hybridization (55 C for 10 sec), and extension (72 C for 13 sec). A melting curve analysis was done with an initial denaturation at 95 C. DNA melting was accomplished with an initial temperature of 65 C for 10 seconds and a gradual temperature increase of 0.1 degree C per second until reaching 95 C. The melting temperature of the expected 400 bp amplicon was between 82 C and 84 C. This estimated melting temperature was used to confirm the identity of IBDV specific products obtained using real time RT-PCR. Additional confirmation of specific amplification was done using routine gel electrophoretic techniques of the PCR products on 2% agarose (Sigma Chemical Co., St. Louis, MO) followed by ethidium bromide staining.

Statistical analysis. The body weight gain, relative bursal and proventricular weights, and bursal and proventricular lesion scores were analyzed using ANOVA and means comparisons for all pairs using Tukey-Kramer HSD. Significance was assumed at the 0.05 level of probability.

RESULTS. Control Groups. *Proventricular homogenate controls.* Chickens inoculated only with saline or negative proventricular homogenate (-PV) did not develop proventriculitis in any of the three trials. Macroscopic lesions were not observed when examined at necropsy (Fig. 13). Mean body weight gain and relative proventriculus weight for these two groups was very similar (Tables 18 and 19 respectively). Mild microscopic lesions consisting mainly of mild lumenal ectasia of the proventricular glands were present in some of these birds (Table 20). Chickens that were inoculated only with positive proventricular homogenate (+PV) had no significant suppression of weight gain compared to saline and -PV groups in all three trials (Table 18). There was a trend toward enlargement of the proventriculus in chickens that received the positive proventricular homogenate. Increased microscopic lesions were present in the proventriculus of chickens that received positive proventricular homogenate in trials 1 and 2 at 7 and 14 dpi, and in trial 3 at 14 dpi (Table 4). At necropsy, the proventriculus of these chickens were enlarged and swollen, with plaques or mottling visible on the serosal surface, dilation of the gastric isthmus, and mucosal lesions (flattened papillae, with secretion of white fluid) (Fig. 13). Microscopically, at 7 dpi, acute necrosis of the glandular epithelium was present. Collecting sinuses of the glands were dilated and contained desquamated epithelium. Nuclei of the glandular epithelial cells were enlarged and pale, with marginated chromatin. Lymphocytic infiltrates were present as sheets in the lamina propria of the mucosa and expanded the glandular epithelium between the epithelium of the ducts and the glands (Fig. 14). At 14 dpi, glandular epithelium was replaced by ductal epithelium. Lymphocyte infiltrates and germinal center formation were present in the glands and mucosa (Fig. 14). In trial 3 chickens that were inoculated with +PV, showed similar mild to moderate lesions in the proventriculus at 21 dpi, but no significant increase in size of the proventriculus compared to saline or -PV controls. Small germinal centers were present in the glands (Fig. 14) of +PV-dosed chickens but not in those given saline or -PV.

No lesions or differences in relative organ weight of the bursa were observed between chickens that received saline, -PV, or +PV (Tables 21 and 22).

*Immunosuppression controls.* Commercial broilers (group 4) in trials 1 and 2, treated with IBDV strains Variant E and STC respectively, had no signs of IBDV infection at 7 and 14 dpi. Their bursas had no significant microscopic lesions, no difference in relative organ weight when compared to controls (Tables 21 and 22), and were negative for IBDV by RT-PCR. CBH response, and humoral response to NDV vaccination was similar to the saline control group (Tables 23 and 24), all indicating that challenge with IBDV in these birds did not produce IBDV infection. However, SPF broiler chickens in trial 3 exposed to IBDV strain STC did have signs of depression at 7 days post inoculation and their bursas were significantly smaller than saline control chickens at 7, 14 and 21 dpi (Table 21). Severe microscopic lesions were also observed (Table 22), and bursas were positive for IBDV by RT-PCR. Humoral immune response to NDV vaccination was significantly lower than saline controls (Table 24).

Cyclophosphamide (CP) control chickens (group 5) in all three trials tended to be smaller than chickens from the other groups, due to a reduction in their weight gain. This reduction was significant in the SPF broilers in trial 3 (Table 18). These chickens also had decreased feathering and appeared weak. The bursas of these chickens were significantly smaller in all three trials (Table 21), and marked lymphocytic depletion and atrophy of the bursa was observed (Table 22). A small reduction of CBH response, was observed in these birds (Table 23), and humoral response to NDV vaccination was significantly reduced (Table 24).

Cyclosporin (CS) control chickens (group 6) in trials 1 and 2 appeared normal (similar to saline controls). Although their weight gain was reduced it was not significantly different from that of the saline controls (Table 18). Weight gain in chickens in trial 3 was reduced at 7 and 21 dpi. Bursas of birds treated with CS had no lesions and there was no difference in size compared to saline controls (Tables 21. and 22). Thymuses did not have any significant lesions, but the CMI immune capacity was significantly reduced (Table 23). The CBH-1 and CBH-2 responses were decreased (p < 0.05) compared to the saline control group. The humoral immune response, measured by antibody production after NDV vaccination, was not affected (Table 24).

The effect of the immunosuppressive treatments (IBDV, CP, CS) on the proventriculus relative weight or presence of lesions was very mild and not significantly different than that observed in saline or -PV controls (Tables 19 and 20).

Experimental groups. *Body weight gain.* Chickens treated with CP and +PV had a significant reduction in body weight gain compared to the control groups (saline, -PV and +PV), including those given CP only, in trials 1 at 7 and 14 dpi and trial 2 at 7 dpi. (Table 18). The combination of CS and +PV had a detrimental effect on weight gain in trail 2 at 14 dpi and trial 3 at 7 and 21 dpi, but the difference from chickens given CS only was not significant in any instance.

*Organ relative weights and lesions.* No significant difference was observed between control and experimental groups for spleen and thymus in any of the trials. (Data not shown). The exceptions were the chickens treated with CP where at 7dpi their thymuses were smaller than the rest of the groups, but by 14 dpi they were the same as controls. In all three trials, birds treated with CP and +PV had a significant decrease in bursal size and developed high lesion scores but these were no different than those in CP controls (Tables 21 and 22). In trial 3, lesions and a significant decrease in size of the bursa occurred in chickens that were challenged with IBDV and exposed to +PV, similar to that observed in the IBDV controls (Tables 21 and 22). These bursas were also IBDV positive by RT-PCR.

Relative proventricular weight of chickens that were immunosuppressed and treated with +PV was increased at 7 and 14 dpi when compared to the control chickens (saline and -PV), but in most cases there was no significant difference when compared to the +PV controls. Chickens in trial 1 and 2 at 7 dpi treated with the combination of CP/+PV, had a significant increase in relative proventricular weight relative to the +PV controls (Table 19). The lesion score of the proventriculi from all immunosuppressed birds treated with +PV was also similar to those observed in the +PV control groups at 7 and 14 dpi (Table 20), although there was an increase in the incidence of proventriculitis and a difference in the appearance and severity of the lesions observed in the birds treated with CS. This was more evident in the SPF broilers where all birds treated with the combination of CS and +PV had moderate to severe proventriculitis. CS/+PV scores were significantly higher than all other treatments at 21 dpi in trial 3. In all three trials, the incidence and severity of proventriculitis was highest at 14 dpi than 7dpi. In trial 3 at 21 dpi the relative weight and lesion score of the proventriculi of all birds that received +PV was similar to the negative controls, with the exception of the chickens treated with CP/+PV which scoring and weight remained significantly higher than birds in the other groups (Tables 19 and 20).

Chickens treated with +PV in all three trials, regardless of the immunosuppression treatment, had acute necrosis of the proventricular glands at 7 dpi with some lymphocyte infiltrates, mostly in the mucosa. In some cases lymphocyte infiltrates also were present in the glands in the form of sheets. Hemorrhage and congestion were also sometimes present. Birds treated with CS had more severe lesions, with destruction and coalescence of the glands.

At 14 dpi, chickens treated with IBDV and +PV, or CP and +PV, had metaplastic replacement of proventricular glandular secretory epithelium by ductal epithelium, and lymphocyte infiltrates as observed in the +PV only-treated chickens. Proventricular lymphoid germinal centers were smaller, or not present, in birds treated with CP (in all three trials) or IBDV (in trial 3). Chickens treated with CS and +PV in trials 1 and 2 still had acute necrosis at 14 dpi, reduced lymphocyte infiltration and variable germinal center formation, and minimal metaplasia (Fig 15).

At 21 dpi, SPF broilers treated with IBD and +PV, or CP and +PV, had mild to moderate lesions, with very little lymphocyte infiltration. These were mostly in the form of small germinal centers. Chickens treated with CS and +PV had severe lesions consisting of acute necrosis of the glandular epithelium, coalescing of glands, and small and multiple germinal centers.

Serology. Chickens from all groups in trial 1 had ELISA titers against IBDV and IBV at 14 days of age (7 dpi), and had no titers against NDV, reovirus or CAV. These IBDV and IBV titers decreased but were still present at 21 days of age (14 dpi). Chickens in trial 2 had titers for IBV, IBV, and NDV at 14 days of age (7 dpi), but not against reovirus. In both trials, chickens that received -PV or +PV (with the exception of birds treated with CP) developed titers against reovirus at 21 days of age (14 dpi).

SPF broiler chickens (trial 3) at 14 days of age (7 dpi) were seronegative for NDV, IBV, reovirus, and CAV. They also were negative for IBDV with the exception of those challenged with IBDV, which developed and had seroconversion at 14, 21 and 30 days of age (7, 14, and 21 dpi). At 21 and 30 days of age (14 and 21 dpi) birds that received +PV, but were not treated with CP, had titers against IBV, NDV, and reovirus. All birds were negative for CAV at all time points.

IBDV RT-PCR. IBDV was not detected in paraffin-embedded bursas or proventriculi from any of the birds in Trials 1 or 2. In Trial 3, IBDV was detected at 7, 14 and 21 dpi in paraffin-embedded bursas from all IBDV challenged birds. It was not detected in any of the proventriculi from these birds, or in bursas or proventriculi from chickens in the other groups in trial 3.

DISCUSSION. The relationship between IBDV and proventriculitis is not clear. Both gross and microscopic lesions of the proventriculus have been produced by IBDV challenge in leghorn chickens (24) and vaccination against IBDV has been reported to decrease the incidence of proventriculitis (7,15). However, proventriculitis was not produced by inoculation of SPF broilers with different strains of IBDV (25). Commercial chickens get exposed to IBDV early in life, and although mortality in unprotected flocks can be quite significant, the major concern for the poultry industry is IBDV's ability to cause immunosuppression. Immunosuppressed birds often fail to respond adequately to vaccination and are susceptible to secondary infections. The mechanisms of IBDV-induced immunosuppression are not fully understood. Both humoral and cellular immune responses are compromised (34). Inhibition of humoral immunity is more severe and is attributed to the destruction of immunoglobulin-producing B cells by the virus. IBDV-exposed chickens produce suboptimal levels of antibodies against a number of infectious and non-infectious antigens (34). Although T cells do not serve as targets for IBDV replication, cell-mediated immune responses of virus-exposed chickens seem to be compromised (5,18,33,34).

Protection against IBDV is achieved by the induction of neutralizing antibodies, as well as by passive transfer of maternal antibodies to young chickens. These maternal antibodies may interfere with IBDV vaccination schedules. In our study, commercial broiler chickens (Trials 1 and 2) inoculated with an infecting dose of IBDV did not develop disease. No lesions were observed in their bursas, and RT-PCR did not detect any virus. Consequently, these birds were not immunosuppressed by IBDV as intended, and had a normal response to NDV vaccination. On the other hand, SPF broiler chickens were successfully infected with IBDV when intentionally challenged at one day of age. Their bursas were significantly smaller than controls, had lesions typical of IBDV infection, and were positive for the virus by RT-PCR. They also developed antibodies against IBDV, and were immunosuppressed as measured by their low seroconversion to NDV. However, infection with this particular strain of IBDV (STC) produced no proventriculitis.

CP treatment has been used to inhibit humoral immunity in order to determine its role in the pathogenesis of infectious pathogens of chickens (1,31). Chickens treated with CP had bursas that were significantly smaller and depleted of lymphocytes, and they did not develop specific antibody after NDV vaccination, demonstrating their humoral immunosuppression. Both CP and IBDV have minor effects on CMI (32,34). There was a mild depression of the CBH response in birds treated with IBDV (trial 3) or CP, but this was not significant when compared to controls.

As expected, chickens from all three trials treated with CS exhibited a significantly decreased CBH response (6). CS prevents cytokine synthesis in T cells by blocking a later stage of T cell receptor initiated signaling, reducing production of interleukin-2 (IL-2), and hence T cell proliferation (12,28). As a consequence, IL-2 dependent functions, which include T-helper activities, cytotoxicity, natural killer cell activity, and antibody dependent cytotoxicity, are decreased (11). As expected, humoral immune response of birds treated with CS was not affected, and they developed anti-NDV antibodies following NDV vaccination.

The homogenate used to induce proventriculitis in trial 1 was known to contain IBDV (13). In an attempt to reproduce a proventriculitis as close to that observed in naturally occurring cases, commercial broilers with maternal antibodies to IBDV were used in trials 1 and 2. Inoculation of these chickens in trial 1 with the IBDV-bearing homogenate produced proventriculitis but no IBDV infection since their anti-IBDV antibody was protective. Since proventriculitis still occurred, this suggests that proventriculitis was not directly produced by infection with the IBDV present in that homogenate, but does not exclude IBDV as a potential contributing factor. In trials 2 and 3, proventriculitis was produced by inoculating birds with positive proventricular homogenate produced from birds with proventriculitis in trial 1. Excluding those challenged with IBDV intentionally, chickens given this homogenate in trials 2 and 3 developed proventriculitis but no IBDV infection. These data suggest our serial passage of the original proventricular homogenate through antibody positive broiler chickens cleared it of IBDV and propagated the causative agent responsible for proventriculitis.

The proventriculitis produced in trial 1 was more severe than that in trials 2 and 3. This may be due to reduction in titer of the causative pathogen by *in vivo* passage in the presence of antibody, or clearance of the IBDV as described above. Even so, the incidences of proventriculitis within groups and the effects of immunosuppression on proventriculitis were similar across all three trials.

Immunosuppression induced by cyclophosphamide (CP) in all three trials, and by IBDV in trial 3, did not affect the incidence or lesion severity of the proventriculitis observed. Proventricular lesions observed in chickens that received CP/+PV were similar to that observed in the +PV controls. There was acute glandular necrosis and some lymphocyte infiltrate at 7 dpi, and glandular metaplasia with severe lymphocyte infiltrates at 14 dpi. Both, sheets and follicles of lymphocytes, were present, representing T and B cells respectively (22,25), however in these birds less follicle formation was observed. Helper and cytotoxic T cells are both present in normal proventriculi (22) and their numbers increase dramatically in proventriculitis (25). Lower numbers of B cells are present in normal proventriculi, and in proventriculitis their numbers also increase. Although the lesions observed in the proventriculi of birds treated with CP/+PV were similar to that of the controls, at 7 dpi chickens from these groups in trials 1 and 2, had significantly higher proventriculus weights than the +PV controls. This suggests a role of B cells in the early stages of proventriculitis, where compromised production of antibodies could exacerbate the severity of the condition.

All chickens with T-cell suppression due to cyclosporin (CS) and treated with +PV had equal or higher incidence and lesion scores of proventriculitis than +PV controls. The proventricular relative weights also tended to be higher than +PV controls, being more evident in the SPF birds in Trial 3 where this difference was significant at 21 dpi. Cell mediated immune (CMI) responses have been suggested to play a key role in the elimination of avian enteric pathogens (1,20,36), and our data indicate that T cell functions play a role in controlling proventriculitis. The high incidence of lesions in the proventriculi of birds in Trail 3 at 21 dpi that were immunosuppressed with CP and treated with +PV indicates the importance of T lymphocytes in the clearing and resolution of proventriculitis. It is well known that IBDV can affect the CMI response (5,18,33,34) and although we saw little effect of IBDV-induced immunosuppression on the severity of proventriculitis in this study, it is possible that preventing severe immunosuppression in the field through vaccination against IBDV, could diminish the severity of proventriculitis.

Serologic results infer that the original positive proventricular homogenate used in this study contained IBDV, IBV, NDV and reovirus because some dosed experimental chickens seroconverted to these agents. Passage of this homogenate in commercial broilers seemed to have eliminated IBDV because SPF's challenged with the subsequent proventricular homogenate did not seroconvert to this virus or develop bursal disease. The objectives and experimental design of the present study were not designed to determine the role(s) of these other agents in proventriculitis, so no conclusions should be drawn from their presence here.

In conclusion, B cell immunosuppression, by CP or IBDV, did not have an effect on the incidence of proventriculitis, and the lesions observed were similar to those produced by +PV alone. However, proventricular enlargement was more evident in these birds at 7dpi, indicating that humoral response might be important in the early stages of the disease probably by controlling the causative agent by production of antibodies. T cell suppression by CS, on the other hand, did have an effect on the incidence of proventriculitis, and the lesions observed were more severe and lasted longer than in +PV controls. T cells are more abundant in the proventriculus than B cells, which suggests their importance in immune responses to infectious agents in this organ. In this study, by affecting T cell function, the severity of proventriculitis was increased and resolution of the disease was prolonged.

**Table 17. Experimental protocol for trials 1 and 2 (commercial broilers), and trial 3 (SPF broilers). Four birds were necropsied per group on day 14 (7dpi) and 21 (14dpi) in all three trials, and also on day 28 (21dpi) in trial 3.**

| GROUPS | ONE DAY OF AGE IMMUNOSUPPRESSION TREATMENT¹ | SEVEN DAYS OF AGE HOMOGENATE TREATMENT² |
|---|---|---|
| 1. Saline | - | Saline |
| 2. -PV | - | -PV |
| 3. +PV | - | +PV |
| 4. IBDV | IBDV | - |
| 5. CP | CP | - |
| 6. CS | CS | - |
| 7. IBDV/+PV | IBDV | +PV |
| 8. CP/+PV | CP | +PV |
| 9.CS/+PV | CS | +PV |

| | | |
|---|---|---|
| ¹ IBDV treatment: 10³ CID₅₀ *per os* strains Variant E (trial 1) or STC (trials 2 and 3). Cyclophosphamide (CP) treatment: 4 mg intraperitoneally for 4 days starting at one day of age. Cyclosporin (CS) treatment: intramuscular injection of 50 mg/Kg body weight every third day, starting on one day of age. ² Saline: 1ml sterile saline *per os*; -PV= proventricular homogenate from normal chickens, 1ml *per os*; +PV= proventricular homogenate from chickens with proventriculitis, 1ml *per os.* | | |

**Table 18. Body weight gain (g) of commercial broilers (trials 1 and 2), and SPF broilers (trial 3), orally challenged at 7 days of age with sterile saline, negative proventricular homogenate (-PV), or positive proventricular homogenate (+PV), and necropsied at 7, 14, and 21 days post inoculation (mean ± standard deviation)¹.**

| Groups | Trial 1 | Trial 2 | Trial 3 |
|---|---|---|---|
| Day 14 (7dpi) | | | |
| 1. Saline | 360.5 ± 34.3 ^{a} | 403.5 ± 14.5 ^{a} | 146.2 ± 10.2 ^{a} |
| 2. -PV | 392.0 ± 7.16 ^{a} | 411.2 ± 31.8 ^{a} | 160.0 ± 16.7 ^{a} |
| 3. +PV | 349.0 ± 24.9 ^{a} | 356.6 ± 45.0 ^{ab} | 147.5 ± 10.1 ^{a} |
| 4. IBDV | 406.0 ± 42.6 ^{a} | 400.8 ± 26.1 ^{a} | 131.2 ± 13.2 ^{ab} |
| 5. CP | 329.2 ± 95.9 ^{a} | 326.6 ± 50.8 ^{ab} | 76.7 ± 21.6 ^{c} |
| 6. CS | 332.0 ± 83.7 ^{a} | 360.7 ± 54.7 ^{ab} | 128.7 ± 7.5 ^{ab} |
| 7. IBDV/+PV | 340.0 ± 25.9 ^{a} | 361.8 ± 28.4 ^{ab} | 130.7 ± 2.7 ^{ab} |
| 8. CP/+PV | 174.7 ± 40.5 ^{b} | 220.1 ± 43.0 ^{b} | 84.7 ± 8.1 ^{c} |
| 9. CS/+PV | 370.7 ± 29.9 ^{a} | 290.3 ± 76.2 ^{ab} | 114.7 ± 8.13 ^{b} |

| Day 21 (14 Dpi) | | | |
|---|---|---|---|
| 1. Saline | 800.2 ± 26.1 ^{a} | 807.7 ± 39.1 ^{a} | 258.7 ± 18.7 ^{ab} |
| 2. -PV | 831.7 ± 67.5 ^{a} | 714.4 ± 52.5 ^{a} | 294.0 ± 19.3 ^{a} |
| 3. +PV | 807.7 ± 45.9 ^{a} | 689.0 ± 24.3 ^{ab} | 285.2 ± 24.2 ^{a} |
| 4. IBDV | 741.2 ±104.5 ^{a} | 773.4 ± 8.1 ^{a} | 254.2 ± 32.8 ^{ab} |
| 5. CP | 733.2 ± 65.8 ^{a} | 549.0 ± 80.0 ^{ab} | 144.2 ± 39.1 ^{c} |
| 6. CS | 816.2 ± 43.8 ^{a} | 506.2 ± 75.8 ^{ab} | 229.5 ± 256 ^{ab} |
| 7. IBDV/+PV | 729.2 ± 123.9 ^{a} | 712.5 ± 81.9 ^{ab} | 249.5 ± 43.2 ^{ab} |
| 8. CP/+PV | 539.2 ± 77.5 ^{b} | 392.2 ±148.7 ^{b} | 169.5 ± 36.5 ^{c} |
| 9. CS/+PV | 658.0 ± 72.0 ^{ab} | 528.4 ± 157.6 ^{ab} | 213.5 ± 11.3 ^{b} |

| Day 28 (21 dpi) | | | |
|---|---|---|---|
| 1. Saline | | | 561.3 ± 73.0 ^{a} |
| 2. -PV | | | 561.0 ± 109.9 ^{a} |
| 3. +PV | | | 532.0 ± 97.5 ^{a} |
| 4. IBDV | | | 518.6 ± 92.6 ^{ab} |
| 5. CP | | | 316.0 ± 67.2 ^{b} |
| 6. CS | | | 484.0 ± 68.9 ^{ab} |
| 7. IBDV/+PV | | | 553.0 ± 92.9 ^{a} |
| 8. CP/+PV | | | 393.0 ± 95.3 ^{ab} |
| 9. CS/+PV | | | 422.0 ± 74.1 ^{ab} |

| | | | |
|---|---|---|---|
| ¹ Means within a column and time point with no common lowercase superscript are significantly different (P<0.05). Means calculated from four birds in each group. | | | |

**Table 19. Relative proventriculus weight (% body weight) of commercial broilers (trials 1 and 2), and SPF broilers (trial 3), orally challenged at 7 days of age with sterile saline, negative proventricular homogenate (-PV), or positive proventricular homogenate (+PV), and necropsied at 7, 14, and 21 days post inoculation (mean ± standard deviation) ¹.**

| Groups | Trial 1 | Trial 2 | Trial 3 |
|---|---|---|---|
| Day 14 (7dpi) | | | |
| 1. Saline | 0.602 ± .051 ^{a} | 0.582 ± .047 ^{a} | 0.677 ± .097 ^{a} |
| 2. -PV | 0.654 ± .042 ^{ab} | 0.562 ±.040 ^{a} | 0.707 ± .058 ^{ab} |
| 3. +PV | 0.932 ± .023 ^{ab} | 0.812 ± .250 ^{a} | 0.925 ± .750 ^{abc} |
| 4. IBDV | 0.550 ± .045 ^{a} | 0.670 ± .083 ^{a} | 0.685 ± .120 ^{a} |
| 5. CP | 0.754 ± .098 ^{ab} | 0.685 ± .023 ^{a} | 0.965 ± .054 ^{abc} |
| 6. CS | 0.670 ± .080 ^{ab} | 0.696 ± .064 ^{a} | 0.892 ± .180 ^{abc} |
| 7. IBDV/+PV | 0.962 ± .220 ^{b} | 0.770 ± .153 ^{a} | 1.010 ± .212 ^{c} |
| 8. CP/+PV | 1.406 ± .330 ^{c} | 1.002 ± .208 ^{b} | 0.985 ± .105 ^{bc} |
| 9. CS/+PV | 0.930 ± .095 ^{ab} | 0.895 ± .175 ^{a} | 1.020 ± .099 ^{c} |

| Day 21 (14 dpi) | | | |
|---|---|---|---|
| 1. Saline | 0.540 ± .075 ^{a} | 0.473 ± .030 ^{a} | 0.552 ± .061 ^{a} |
| 2. -PV | 0.510 ± .060 ^{a} | 0.610 ± .140 ^{a} | 0.582 ± .022 ^{a} |
| 3. +PV | 0.922 ± .194 ^{ab} | 0.743 ± .089 ^{a} | 0.745 ± .140 ^{abcd} |
| 4. IBDV | 0.532 ± .072 ^{a} | 0.480 ± .036 ^{a} | 0.650 ± .083 ^{abc} |
| 5. CP | 0.490 ± .057 ^{a} | 0.540 ± .045 ^{a} | 0.852 ±.140 ^{bcd} |
| 6. CS | 0.535 ± .050 ^{a} | 0.580 ± .060 ^{a} | 0.685 ± .100 ^{abc} |
| 7. IBDV/+PV | 0.900 ± .204 ^{ab} | 0.723 ± .130 ^{a} | 0.820 ± .110 ^{abcd} |
| 8. CP/+PV | 0.950 ± .154 ^{ab} | 0.706 ± .210 ^{a} | 1.020 ± .152 ^{d} |
| 9. CS/+PV | 1.202 ± .470 ^{b} | 0.886 ± .370 ^{a} | 0.927 ± .170 ^{bcd} |

| Day 28 (21 dpi) | | | |
|---|---|---|---|
| 1. Saline | | | 0.463 ± .083 ^{a} |
| 2. -PV | | | 0.436 ± .073 ^{a} |
| 3.+PV | | | 0.580 ± .111 ^{a} |
| 4. IBDV | | | 0.483 ± .149 ^{a} |
| 5. CP | | | 0.676 ± .005 ^{a} |
| 6. CS | | | 0.546 ± .096 ^{a} |
| 7. IBDV/+PV | | | 0.506 ± .046 ^{a} |
| 8. CP/+PV | | | 0.640 ± .103 ^{a} |
| 9. CS/+PV | | | 0.970 ± .261 ^{b} |

| | | | |
|---|---|---|---|
| ¹ Means within a column and time point with no common lowercase superscript are significantly different (P<0.05). Means calculated from four birds in each group. | | | |

**Table 20. Incidence and scoring of the severity of proventricular lesions in commercial broilers (trials 1 and 2), and SPF broilers (trial 3), orally challenged at 7 days of age with sterile saline, negative proventricular homogenate (-PV), or positive proventricular homogenate (+PV), and necropsied at 7, 14, and 21 days post inoculation ¹.**

| Groups | Trial 1 | | Trial 2 | | Trial 3 | |
|---|---|---|---|---|---|---|
| Day 14 (7dpi) | | | | | | |
| 1. Saline | 1.00 ^{a2} | 0/4³ | 1.00 ^{a} | 0/4 | 1.50 ^{a} | 2/4 |
| 2. -PV | 1.00 ^{a} | 0/4 | 1.00 ^{a} | 0/4 | 1.75 ^{a} | 2/4 |
| 3. +PV | 3.00 ^{b} | 3/4 | 2.50 ^{b} | 2/4 | 2.50 ^{ab} | 3/4 |
| 4. IBDV | 1.00 ^{a} | 0/4 | 1.25 ^{a} | 1/4 | 1.50 ^{a} | 2/4 |
| 5. CP | 1.25 ^{a} | 1/4 | 1.25 ^{a} | 1/4 | 1.00 ^{a} | 0/4 |
| 6. CS | 2.00 ^{a} | 2/4 | 1.00 ^{a} | 0/4 | 1.50 ^{a} | 2/4 |
| 7. IBDV/+PV | 3.00 ^{b} | 3/4 | 2.00 ^{ab} | 2/4 | 2.50 ^{ab} | 3/4 |
| 8. CP/+PV | 2.50 ^{ab} | 3/4 | 2.50 ^{b} | 3/4 | 1.25 ^{a} | 1/4 |
| 9. CS/+PV | 3.50 ^{b} | 4/4 | 2.75 ^{b} | 3/4 | 3.25 ^{b} | 4/4 |

| Day 21 (14 dpi) | | | | | | |
|---|---|---|---|---|---|---|
| 1. Saline | 1.25 ^{a} | 1/4 | 1.00 ^{a} | 0/4 | 1.25 ^{a} | 2/4 |
| 2. -PV | 1.00 ^{a} | 0/4 | 1.50 ^{a} | 2/4 | 1.50 ^{a} | 2/4 |
| 3. +PV | 3.75 ^{b} | 4/4 | 3.50 ^{b} | 4/4 | 3.25 ^{b} | 4/4 |
| 4. IBDV | 1.50 ^{a} | 2/4 | 1.25 ^{a} | 1/4 | 1.00 ^{a} | 0/4 |
| 5. CP | 1.25 ^{a} | 1/4 | 1.25 ^{a} | 1/4 | 1.25 ^{a} | 1/4 |
| 6. CS | 1.25 ^{a} | 1/4 | 1.00 ^{a} | 0/4 | 1.50 ^{a} | 2/4 |
| 7. IBDV/+PV | 3.25 ^{b} | 4/4 | 3.50 ^{b} | 4/4 | 2.50 ^{ab} | 2/4 |
| 8. CP/+PV | 3.00 ^{b} | 4/4 | 2.50 ^{ab} | 3/4 | 2.75 ^{ab} | 3/4 |
| 9. CS/+PV | 4.00 ^{b} | 4/4 | 4.00 ^{b} | 4/4 | 3.25 ^{b} | 4/4 |

| Day 28 (21 dpi) | | | | | | |
|---|---|---|---|---|---|---|
| 1. Saline | | | | | 1.25 ^{a} | 1/4 |
| 2. -PV | | | | | 1.50 ^{a} | 2/4 |
| 3. +PV | | | | | 1.50 ^{a} | 2/4 |
| 4. IBDV | | | | | 1.25 ^{a} | 1/4 |
| 5. CP | | | | | 1.25 ^{a} | 1/4 |
| 6. CS | | | | | 1.50 ^{a} | 2/4 |
| 7. IBDV/+PV | | | | | 1.50 ^{a} | 2/4 |
| 8. CP/+PV | | | | | 1.50 ^{a} | 2/4 |
| 9. CS/+PV | | | | | 3.50 ^{b} | 4/4 |

| | | | | | | |
|---|---|---|---|---|---|---|
| ¹ Means within a column and trial with no common lowercase superscript are significantly different (P<0.05). Means calculated from four birds in each group ² Proventriculus score: 1: no lesions; 2: mild glandular lumenal ectasia; 3: ectasia plus lymphoid infiltrates in the interglandular interstitium; and 4: either acute glandular necrosis or severe fibrosis with lymphoid infiltrates. ³ Number of birds with mild, moderate or severe lesions in the proventriculus/ number of birds necropsied. | | | | | | |

**Table 21. Bursa relative weights (% body weight) of commercial broilers (trials 1 and 2), and SPF broilers (trial 3), orally challenged at 7 days of age with sterile saline, negative proventricular homogenate (-PV), or positive proventricular homogenate (+PV), and necropsied at 7, 14, and 21 days post inoculation (mean ± standard deviation) ¹.**

| Groups | Trial 1 | Trial 2 | Trial 3 |
|---|---|---|---|
| Day 14 (7dpi) | | | |
| 1. Saline | 0.127 ± .075 ^{a} | 0.220 ± .034 ^{a} | 0.310 ± .045 ^{a} |
| 2. -PV | 0.170 ± .027 ^{a} | 0.160 ± .039 ^{a} | 0.320 ± .029 ^{a} |
| 3. +PV | 0.195 ± .020 ^{a} | 0.205 ± .035 ^{a} | 0.365 ± .090 ^{a} |
| 4. IBDV | 0.160 ± .010 ^{a} | 0.165 ± .052 ^{a} | 0.065 ± .010 ^{b} |
| 5. CP | 0.032 ± .009 ^{b} | 0.060 ± .008 ^{b} | 0.017 ± .015 ^{b} |
| 6. CS | 0.150 ± .017 ^{a} | 0.213 ± .020 ^{a} | 0.370 ± .067 ^{a} |
| 7. IBDV/+PV | 0.200 ± .026 ^{a} | 0.152 ± .035 ^{a} | 0.172 ± .009 ^{b} |
| 8. CP/+PV | 0.055 ± .012 ^{b} | 0.060 ± .024 ^{b} | 0.085 ± .005 ^{b} |
| 9. CS/+PV | 0.202 ± .022 ^{a} | 0.182 ± .088 ^{a} | 0.325 ± .098 ^{a} |

| Day 21 (14 dpi) | | | |
|---|---|---|---|
| 1. Saline | 0.250 ± .098 ^{a} | 0.230 ± .081 ^{a} | 0.340 ± .065 ^{a} |
| 2. -PV | 0.272 ± .090 ^{a} | 0.230 ± .095 ^{a} | 0.304 ± .045 ^{a} |
| 3. +PV | 0.225 ± .036 ^{a} | 0.196 ± .075 ^{a} | 0.305 ± .084 ^{a} |
| 4. IBDV | 0.190 ± .060 ^{a} | 0.193 ± .068 ^{a} | 0.112 ± .009 ^{b} |
| 5. CP | 0.032 ± .009 ^{b} | 0.050 ± .017 ^{b} | 0.055 ± .012 ^{b} |
| 6. CS | 0.232 ± .033 ^{a} | 0.153 ± .055 ^{a} | 0.387 ± .098 ^{a} |
| 7. IBDV/+PV | 0.225 ± .042 ^{a} | 0.193 ± .064 ^{a} | 0.080 ± .049 ^{b} |
| 8. CP/+PV | 0.070 ± .018 ^{b} | 0.063 ± .020 ^{b} | 0.070 ± .040 ^{b} |
| 9. CS/+PV | 0.282 ± .052 ^{a} | 0.190 ± .066 ^{a} | 0.377 ± .054 ^{a} |

| Day 28 (21 dpi) | | | |
|---|---|---|---|
| 1. Saline | | | 0.233 ± .023 ^{a} |
| 2. -PV | | | 0.243 ± .015 ^{a} |
| 3. +PV | | | 0.316 ± .047 ^{a} |
| 4. IBDV | | | 0.073 ± .036 ^{b} |
| 5. CP | | | 0.066 ± .005 ^{b} |
| 6. CS | | | 0.325 ± .051 ^{a} |
| 7. IBDV/+PV | | | 0.060 ± .010 ^{b} |
| 8. CP/+PV | | | 0.060 ± .036 ^{b} |
| 9. CS/+PV | | | 0.463 ± .027 ^{a} |

| | | | |
|---|---|---|---|
| ¹ Means within a column and time point with no common lowercase superscript are significantly different (P<0.05). Means calculated from four birds in each group. | | | |

**Table 22. Incidence and scoring of the severity in bursal lesions of commercial broilers (trials 1 and 2), and SPF broilers (trial 3), orally challenged at 7 days of age with sterile saline, negative proventricular homogenate (-PV), or positive proventricular homogenate (+PV), and necropsied at 7, 14, and 21 days post inoculation ¹.**

| Groups | Trial 1 | | Trial 2 | | Trial 3 | |
|---|---|---|---|---|---|---|
| Day 14 (7dpi) | | | | | | |
| 1. Saline | 1.00 ^{a 2} | 0/4³ | 1.50 ^{a} | 2/4 | 1.25 ^{a} | 1/4 |
| 2. -PV | 1.00 ^{a} | 0/4 | 2.00 ^{a} | 4/4 | 1.25 ^{a} | 1/4 |
| 3. +PV | 1.75 ^{a} | 2/4 | 2.25 ^{a} | 4/4 | 2.50 ^{b} | 4/4 |
| 4. IBDV | 1.25 ^{a} | 1/4 | 1.75 ^{a} | 3/4 | 4.00 ^{c} | 4/4 |
| 5. CP | 4.00 ^{b} | 4/4 | 4.00 ^{b} | 4/4 | 4.00 ^{c} | 4/4 |
| 6. CS | 1.75 ^{a} | 3/4 | 1.25 ^{a} | 1/4 | 1.75 ^{a b} | 3/4 |
| 7. IBDV/+PV | 1.75 ^{a} | 3/4. | 1.25 ^{a} | 1/4 | 4.00 ^{c} | 4/4 |
| 8. CP/+PV | 4.00 ^{b} | 4/4 | 4.00 ^{b} | 4/4 | 4.00 ^{c} | 4/4 |
| 9. CS/+PV | 1.50^{a} | 2/4 | 1.50 ^{a} | 3/4 | 2.00 ^{a b} | 4/4 |
| Day 21 (14 dpi) | | | | | | |
| 1. Saline | 1.25 ^{a} | 1/4 | 1.25 ^{a} | 1/4 | 1.25 ^{a} | 1/4 |
| 2. -PV | 1.25 ^{a} | 1/4 | 1.00 ^{a} | 0/4 | 1.50 ^{a} | 2/4 |
| 3. +PV | 1.25 ^{a} | 1/4 | 1.25 ^{a} | 1/4 | 1.75 ^{a} | 2/4 |
| 4. IBDV | 1.00 ^{a} | 0/4 | 1.25 ^{a} | 1/4 | 4.00 ^{b} | 4/4 |
| 5. CP | 4.00 ^{b} | 4/4 | 4.00 ^{b} | 4/4 | 4.00 ^{b} | 4/4 |
| 6. CS | 1.50 ^{a} | 2/4 | 1.25 ^{a} | 1/4 | 1.50 ^{a} | 2/4 |
| 7. IBDV/+PV | 1.00 ^{a} | 0/4 | 2.00 ^{a} | 4/4 | 4.00 ^{b} | 4/4 |
| 8. CP/+PV | 4.00 ^{b} | 4/4 | 4.00 ^{b} | 4/4 | 4.00 ^{b} | 4/4 |
| 9. CS/+PV | 1.75 ^{a} | 2/4 | 1.00 ^{a} | 0/4 | 1.00 ^{a} | 0/4 |
| Day 28 (21 dpi) | | | | | | |
| 1. Saline | | | | | 1.00 ^{a} | 0/4 |
| 2. -PV | | | | | 1.25 ^{a} | 1/4 |
| 3. +PV | | | | | 1.00 ^{a} | 0/4 |
| 4. IBDV | | | | | 4.00 ^{b} | 4/4 |
| 5. CP | | | | | 4.00 ^{b} | 4/4 |
| 6. CS | | | | | 1.00 ^{a} | 0/4 |
| 7. IBDV/+PV | | | | | 4.00 ^{b} | 4/4 |
| 8. CP/+PV | | | | | 4.00 ^{b} | 4/4 |
| 9. CS/+PV | | | | | 1.00 ^{a} | 0/4 |

| | | | | | | |
|---|---|---|---|---|---|---|
| ¹ Means within a column and trial with no common lowercase superscript are significantly different (P<0.05). Means calculated from four birds in each group. ² Bursa score: 1: no lesions; 2: mild variation in follicle size; 3: moderate variation in size of follicles; and 4: either necrosis or follicle atrophy. ³ Number of birds with mild, moderate or severe lesions in the bursa/ number of birds necropsied | | | | | | |

**Table 23. Effect of immunosuppression treatments¹ on the cutaneous basophil hypersensitivity (CBH) response² induced by injection of phytohemagglutinin P (PHA-P) and physiological saline solution (PSS) in 2 week-old chickens.**

| Treatment | Trial 1 | | Trial 2 | | Trial 3 | |
|---|---|---|---|---|---|---|
| | CBH-1 | CBH-2 | CBH-1 | CBH-2 | CBH-1 | CBH-2 |
| Saline | .74 ± .14 | .87 ± .10 | .72 ± .21 | .74 ± .18 | .58 ±.18 | .54 ± .17 |
| IBDV | .75 ± .25 | .80 ± .24 | .82 ± .27 | .80 ± .24 | .45 ± .22 | .30 ± .08 |
| CP | .67 ± .07 | .73 ± .01 | .69 ± .18 | .69 ± .2 | .34 ± .06 | .31 ± .07 |
| CS | .42 ± .14* | .49 ± .15* | .33 ± .21* | .27 ± .14* | .19 ± .03* | .14 ± .02* |

| | | | | | | |
|---|---|---|---|---|---|---|
| ¹ IBDV treatment: 10³ CID₅₀ *per os* strains Variant E (trial 1) or STC (trials 2 and 3). CP treatment: 4mg intraperitoneally for 4 days starting at one day of age. CS treatment: intramuscular injection of 50 mg/Kg body weight every third day, starting on one day of age. ² Data expressed as mean ± standard deviation; n= 4. ³ CBH-1 = (skin thickness at 12 h post-injection, left foot) - (pre-injection skin thickness, left foot). ⁴ CBH-2 = (skin thickness, PHA-P injected foot) - (skin thickness, PSS injected foot). * Significantly different from groups in the same column (P<0.05). | | | | | | |

**Table 24. Immune responses to killed Newcastle Disease (ND) vaccine in chickens inoculated with either sterile saline, Infectious Bursal Disease Virus (IBDV), cyclophosphamide (CP), or cyclosporine (CS). 14 days postinoculation.¹**

| Trial 1 | Saline² | IBDV | CP⁴ | CS⁵ |
|---|---|---|---|---|
| Mean ELISA titer | 3,966^{a} | 3,433^{a} | 1.0^{b} | 3,034^{a} |
| Mean HI titer | 200^{a} | 160^{a} | 0^{b} | 160^{a} |

| Trial 2 | Saline² | IBDV³ | CP⁴ | CS⁵ |
|---|---|---|---|---|
| Mean ELISA titer | 5,277^{a} | 5,240^{a} | 36.0^{b} | 4,801^{a} |
| Mean HI titer | 200^{a} | 200^{a} | 0^{b} | 200^{a} |

| Trial 3 | Saline² | IBDV³ | CP⁴ | CS⁵ |
|---|---|---|---|---|
| Mean ELISA titer | 11,784^{a} | 4,202^{bc} | 1.0^{c} | 11,140^{a} |
| Mean HI titer | 480^{a} | 160^{bc} | 0^{c} | 360^{ab} |

| | | | | |
|---|---|---|---|---|
| ¹ Means within a row with no common lowercase superscript are significantly different (P< 0.05). Means calculated from four birds. ² Saline: 1 ml sterile saline *per os* ³ IBDV treatment: 10³ CID₅₀ *per os* strains Variant E (trial 1) or STC (trials 2 and 3). ⁴ CP treatment: 4mg intraperitoneal for 4 days starting at one day of age. ⁵ CS treatment: intramuscular injection of 50 mg/Kg body weight every third day, starting on one day of age. | | | | |

### References:

1. Arnold & Holt. Poult Sci.74. 656-665. 1995.
2. Barnes et al. Poultry Sci. 80: 906-911. 2001.
3. Bayyari et al. Poult Sci. 74:1961-1969. 1995.
4. Bayyari et al. Poult Sci, 74:1799-1809. 1995.
5. Confer et al. Am J Vet Res 42:2109-2113. 1981.
6. Corrier & De Loach. Poult Sci. 69: 403-408.1990.
7. Dormitorio et al. International Poultry Scientific Forum. January 15-16. Atlanta, Georgia. 2001.
8. Giambrone et al. Am J Vet Res 38:581-583. 1977.
9. Goodwin et al. Viral proventriculitis in chickens. Avian Pathol. 25:369-379. 1996.
10. Guy & Barnes. Proceedings of the 139th AVMA Annual Convention. July. Nashville. TN. 2002.
11. Hill et al. Avian Dis. 33: 86-92. 1989.
12. Ho et al. Clinical Immunol Immnunopathol. 80: S40-45. 1996.
13. Huff et al. Avian Dis. 45:828-843. 2001.
14. Jensen et al. Avian Dis. 35:969-973. 1991.
15. Kelly et al. Proceedings of the 138th AVMA Annual Convention. July 14-18. Boston, MA. 2001.
16. Kouwenhoven et al. Avian Pathol. 7:183-187. 1978.
17. Kouwenhoven et al. Avian Pathol. 17:879-892. 1988.
18. Lam. Avian Pathol. 20:867-876. 1991.
19. Lenz et al. J Vet Diagn Invest. 10:145-151. 1998.
20. Lillehoj & Bacon. Avian Dis. 35:294-301. 1991.
21. Lukert & Saif. Disease of Poultry, 11th edition. B.W. Calnek, H.J. Barnes, C.W. Beard, L. R. McDougald, and Y.M. Saif, eds. Iowa State University Press, Ames. IA. P 161-179. 2003.
22. Matsumoto & Hashimoto. J Vet Med Sci 62:161-167. 2000.
23. Mutlu et al. Tierarztl Prax Ausg G Grosstiere Nutztiere. 25:460-462. 1997.
24. Newberry. Ph. D. Dissertation. University of Arkansas, Fayetteville, AR. 1996.
25. Pantin & Brown. Abstracts of the 52nd Annual Meeting of the American College of Veterinary Pathologists. Vet Pathol. 38:579. 2001.
26. Pegram & Wyatt. Poultry Sci. 60:2429-2440.1981.
27. Poole. Proceedings of the 43rd Western Poultry Disease Conference, Sacramento CA. Pp. 40-42. 1994.
28. Resch & Szamel. Int J Immunopharmacology. 19:579-585. 1997.
29. Riddell. Avian Dis. 20:442-445. 1976.
30. Rodenberg et al. Avian Dis. 38:16-21. 1994.
31. Russell et al. Vet Immunol Immunopathol. 60:171-185. 1997.
32. Sharma & Lee. Infection and Immunity. 227-230.1977.
33. Sharma et al. Avian Dis. 33: 112-124. 1989.
34. Sharma et al. Dev Comp Immunology. 24: 223-235. 2000.
35. Schulze et al. Dtsch Tierarztl Wochenschr 108:264-266. 2001.
36. Songserm et al. Vet Immunol Immunopathol 85:51-62. 2002.
37. Thayer & Beard. A laboratory manual for the isolation and identification of avian pathogens. Fourth edition. Published by The American Association of Avian Pathologists.1998.
38. Turpin. Ms. Thesis. UGA .1998.
39. Yu et al. Avian Dis. 45:416-424. 2001.
40. Van der Berg. Avian Pathol. 29:175-194. 2000.
41. Wideman et al. J Appl Poult Res 5:219-230. 1996.

### PROVENTRICULITIS IN THE BROILER CHICKENS: CHARACTERIZATION OF THE LYMPHOCYTIC INFILTRATION IN THE PROVENTRICULAR GLANDS (Pantin-Jackwood et al.)

SUMMARY. Broiler chickens with transmissible proventriculitis have severe lymphocytic infiltration in the proventricular glands and the mucosa. The distribution of T cells (CD3+, CD4+, and CD8+) and B cells in the proventriculus of affected chicken was studied immunohistochemically and histopathologically. One-day-old commercial boilers were orally gavaged with a proventricular homogenate produced from broilers with proventriculitis to reproduce this disease. Resulting proventricular lesions were studied at 7, 14 and 21 days post-inoculation (dpi). Lymphocytic infiltrates in the proventricular glands and the lamina propria of the mucosa were observed at all time points, and were most prominent at 14 days post-inoculation with well-developed lymphoid aggregates present. Both T and B Lymphocytes were present during acute and chronic proventriculitis, but their distribution varied within the glands. Lymphocytic infiltrates in both the proventricular glands and in the lamina propria were mainly T cells (CD3+), and were predominantly CD8+ T lymphocytes. CD4+ T cells and B cells tended to form aggregates as the proventriculitis became chronic. These findings show that both cell mediated and humoral immune responses are induced during transmissible proventriculitis, and that the cell mediated immune response is morphologically greater. Keywords: Chicken; Proventriculitis; T and B Lymphocytes; Immunohistochemistry. Abbreviations: Dpi, days post-inoculation; IEL, intraepithelial lymphocytes; MALT, mucosa associated lymphoid tissue; -PV, negative proventricular homogenate; +PV, positive proventricular homogenate.

Introduction. Proventriculitis is a transmissible disease that occurs in commercial broiler chickens. It is characterized by enlargement of the proventriculus and weakness of the gastric isthmus. During routine evisceration at processing, affected proventriculi rupture causing spillage of the retained ingesta into the body cavity, which results in condemnation of affected carcasses for contamination. The disease has also been associated with impaired growth and poor feed conversion (10, 12). Microscopically, degeneration and necrosis of the proventricular glandular epithelium is accompanied by marked lymphocytic infiltration (4, 10, 11, 12).

The etiology of proventriculitis is not clear. Several agents have been implicated as potential causes of proventriculitis. Noninfectious causes include oral exposure to biogenic amines (3), mycotoxins (24), lack of dietary fiber (25), and excessive copper sulfate (5,13). Infectious causes include adenovirus (17), reovirus (16,29), infectious bronchitis virus (33), infectious bursal disease virus (4,10,12,20) and megabacterium (27). However, none of these noninfectious or infectious agents have been found consistently in a majority of cases. Electron microscopy has detected viral particles in acute lesions but isolation of a virus from affected proventriculi has been unsuccessful (10,11,12). Proventriculitis has been successfully reproduced by inoculation with proventricular homogenates produced from diseased chickens (10,11,12). Filtrates from these homogenates also produced lesions in the proventriculus suggesting that a virus is the cause of the disease (10,11,12). However, proventriculitis is more severe when birds are inoculated with the unfiltered homogenate suggesting that infectious proventriculitis has a complex etiology involving both viral and bacterial agents (12).

The main histologic finding in transmissible proventriculitis is a marked lymphocytic infiltration of the proventricular glands (22). The purpose of this study was to characterize this lymphocytic infiltrate to gain insights into the identity of these cells and their functional role in generating a protective immune response in the proventriculus. To accomplish this we experimentally infected commercial broiler chickens with proventricular homogenates from affected broilers and studied the proventricular lesions using histopathology, staining for lymphocyte cell-surface markers, and by identifying the distribution of these different lymphocyte subsets.

MATERIALS AND METHODS. Chickens. One-day-old unvaccinated broiler chicks were obtained from a commercial hatchery. All chicks were wing-banded, weighed, separated into groups and maintained in positive pressure Horsfal isolation units. Feed and water were provided *ad libitum.*

Proventricular homogenates. A proventricular homogenate (+PV) was prepared from proventriculi from 2 to 4-week old chickens with proventriculitis (12). A second proventricular homogenate (-PV) was similarly prepared from proventriculi of normal healthy broiler chickens without proventriculitis and was used as a control inoculum. Both +PV and -PV were prepared as previously described (4) and frozen at -70C, and thawed immediately prior to use.

Experimental design. We divided 24 one-day-old commercial broilers into 2 groups. The first group was inoculated by oral gavage with 1ml of -PV. The second group received 1 ml of the +PV. At 7, 14 and 21 days post inoculation (dpi) four birds from each group were weighed and killed by cervical dislocation. Bursa, proventriculus, and the right side of the thymus were weighed, and sections from these organs were collected from each bird and fixed immediately by immersion in 10% neutral buffered formalin for 24 hours for histopathology. Sections of proventriculus, bursa and thymus were also placed in Cryo-Gel embedding medium (Instrumedics, Inc.,Hackensack, NJ) and immediately frozen in liquid nitrogen and kept at -70C until immunohistological studies were performed. Tissues in formalin were later processed using routine histologic techniques and embedded in paraffin. Also, a part of the proventriculus from each bird was washed several times in sterile saline, homogenized, and frozen at -70C.

Histopathology. Paraffin-embedded tissues were sectioned, mounted, stained using hematoxylin and eosin (HE), and examined, blinded as for treatment, for lesions using light microscopy. All sections were assigned a lesion severity score. For all tissues a lesion score of 1 represented no lesions. For bursal sections, 2 was defined as mild variation in follicle size, 3 as moderate variation in follicle size, and 4 as either necrosis or follicle atrophy. For thymic sections 2 was defined as mild cortical thinning, 3 as moderate cortical thinning, and 4 as absence of cortical lymphocytes. For proventricular sections, 2 was defined as mild glandular lumenal ectasia, 3 as ectasia, necrosis of the glandular epithelium, plus lymphoid infiltrates in the interglandular interstitium, and 4 as either acute glandular necrosis or severe fibrosis with lymphoid infiltrates. The wall thickness of the sections of proventriculi mounted on the slides was measured with a millimeter ruler on the thickest part.

Monoclonal antibodies. Monoclonal antibodies for T lymphocytes (Southern Biotechnology Associates Inc., Birmingham, Alabama) were: mouse anti-chicken CT-3 (anti-CD3), CT-4 (anti-CD-4), and CT-8 (anti-CD8). HisCl antibody (Cedi Diagnostics BV, Lelystad, The Netherlands) was used for B lymphocytes.

Immunohistochemistry. Optimal conditions for immunohistochemical staining with each monoclonal antibody were determined using bursa and thymus tissues from normal chickens. All monoclonals stained cell populations in positive-control tissues with equal intensity. These tissues were included as controls during the staining of each group of slides. Frozen tissue blocks were cut in a cryostat into 5 µm sections, and placed on Superfrost Plus slides (Fisher Scientific, Pittsburgh, PA). They were fixed immediately in acetone for 10 minutes and stored at -70 C until stained. Immunostaining was accomplished using a nonbiotin peroxidase kit (DAKO Envision System, DAKO, Carpinteria, CA) according to the manufacturer's recommendations. Briefly, the sections on slides were placed in a moist chamber and washed for 5 min. in 0.1 M phosphate buffered saline (PBS), followed by incubation for 5 min. in peroxidase blocking reagent (DAKO Envision System). Sections were then washed in PBS for 5 min. and incubated with monoclonal antibodies at 4C overnight (CD-3, CD-4, and CD-8 were used at a dilution of 1:100; His-C1 at a dilution of 1:50). Following primary antibody incubation, sections were washed in PBS for 5 min. and incubated with the secondary antibody (peroxidase labeled polymer conjugated to goat anti-mouse immunoglobulins, DAKO Envision System) at room temperature for 45 min. After washing in PBS for 5 min., bound antibody was detected by a 5-10 min. incubation with 3,3'-diaminobenzidine substrate- chromogen (DAB, DAKO Envision System). After IHC staining, sections were counter-stained with hematoxylin, air dried, cover slipped, and examined using light microscopy.

Statistical analysis. The relative organ weights and lesion scores were analyzed using ANOVA and means comparisons for all pairs using Tukey-Kramer HSD. Significance was set at a 0.05 level of probability.

RESULTS. Clinical signs and macroscopic lesions. No clinical signs were observed in any of the chickens in control or experimental groups. Gross lesions were observed in all proventriculi from +PV-inoculated chickens. At 7 and 14 days post inoculation (dpi) proventriculi were enlarged with a mottled appearance and a distended gastric isthmus. At 21 dpi the enlargement still was present but was less severe. No enlargement of the proventriculi was observed in the chickens given -PV. The proventricular wall of chickens inoculated with the +PV was thickened, with a white lobular pattern observed when sectioned. No macroscopic lesions were observed in any other organ of experimentally infected birds.

Body weight gain. At 7 and 14 dpi, chickens inoculated with positive homogenate had no significant suppression of weight gain compared to age matched chickens given -PV. At 21 dpi there was a decrease in body weight gain in birds that received +PV (Table 25).

Organ weights. Chickens that received +PV had increased proventricular organ/weight ratio at 7 and 14 dpi, which was statistically significant when compared to chickens that received -PV (Table 25). There was no difference in the bursa and thymus organ/weight ratio between birds inoculated with +PV and those given -PV (data not shown).

Macroscopic lesions. Proventricular lesion scores were significantly higher in birds that received +PV compared to compared to those given -PV. No lesions were present in the proventricular glands of the -PV treated birds at 7dpi. At 14 and 21 dpi, 2 of 4 birds in this group had mild lumenar ectasia of the glandular lumen. Lymphocytes were observed at all time points in the lamina propria of the mucosa especially in areas surrounding the orifices of the excretory ducts of the deep proventricular glands (Fig. 16-A). By 21 dpi these lymphocytes formed small aggregates in the proventricular glands (Fig. 16-B).

Proventriculi of chickens challenged with +PV presented necrosis of the glandular epithelium at 7 dpi (Fig. 16-C). Collecting sinuses of the glands were dilated and contained desquamated epithelium and debris. Nuclei of glandular epithelium were enlarged and pale, with marginated chromatin. At 7 and 14 dpi lymphocytic infiltrates were present in large numbers in the lamina propria of the mucosa and also in affected glands expanding the glandular interstitium (Fig. 16-C and 16-D). At 14 and 21 dpi, the glandular epithelium in some of the glands was replaced by ductal epithelium (Fig. 16-E). At 21 dpi there was less necrosis of the glandular epithelium, but there was regeneration or metaplasia to ductal epithelium. At that time, lymphocytes were still present, mostly forming aggregates or germinal centers (Fig. 16-F).

Proventricular wall thickness. There was a significant difference in thickness of the proventricular wall between chickens that were inoculated with -PV and those inoculated with +PV at all time points (Table 25).

Localization of CD3+, CD4+, CD8+ and B cells. Both T and B cells were present in the lamina propria of the proventricular mucosa of chickens treated with -PV. Most lymphocytes in the proventricular glands were T cells, and were localized to the interstitium between the glands, and intraepithelially as individual lymphocytes (Fig. 16-B). Small lymphoid aggregates were present in the glands at 14 and 21 dpi, and were mostly composed of B cells.

In chickens that received +PV, T cells predominated at all time points and were dispersed within the lamina propria of the mucosa and in deeper areas of proventricular glands. B cells were also present, but their distribution varied depending on the stage of the proventriculitis. Initially, B cells were localized similar to the T cells but in lower numbers (Fig. 17-C). As the proventriculitis progressed, B cells formed aggregates (germinal centers) in deeper portions of proventricular glands and less frequently in the lamina propria of the mucosa (Fig. 17-E and 17-G). T cells surrounded these germinal centers and infiltrated the proventricular glands and the mucosa (Fig 17-D, 17-F, and 17-H).

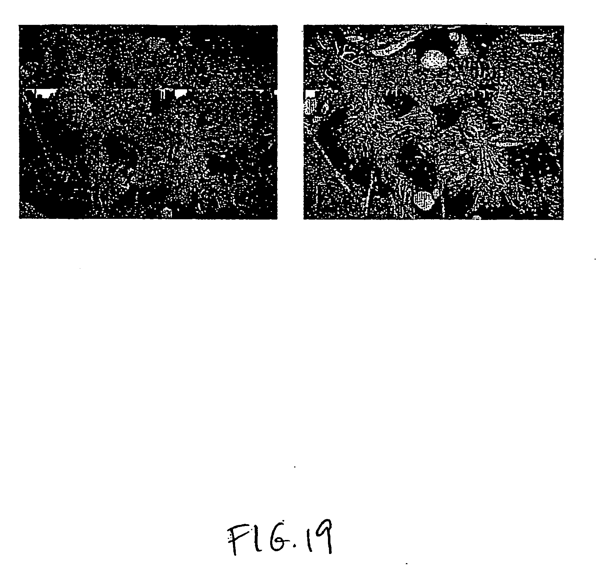

The two subsets of T lymphocytes studied (CD4+ and CD8+) were distributed differently in affected proventriculi (Fig 19. A to H). Both subsets were found at all time points in large quantities in the lamina propria of the mucosa, but CD4+'s predominated at 7 dpi. At 14 and 21 dpi, CD4+ positive cells were found mostly surrounding the B cell germinal centers and forming aggregates that by HE stain were germinal centers. Also, CD4+ cells infiltrated these B cell germinal centers. The CD8+ cells were more widely distributed, were surrounding the germinal centers, and also infiltrated the proventricular glands in the intraepithelial spaces. In glands with acute necrosis of the epithelium, CD8+ lymphocytes were the predominant cells infiltrating the gland. In the chronic lesions this subset was still observed in large numbers throughout the gland, while CD4+ and B cells formed aggregates located in deeper portions of proventricular glands (Fig 19-A and 19-B).

DISCUSSION. In the present study, lymphocyte subpopulation changes during proventriculitis were investigated. Proventriculitis was successfully reproduced by inoculation with a proventricular homogenate derived from proventriculi collected from broiler chickens affected with proventriculitis (+PV). Microscopic changes in these proventriculi included necrosis of the glandular epithelium and replacement of this epithelium with ductal epithelium. This loss of glandular tissue and ductal hyperplasia may result in loss of function of the proventriculus (10). This would explain the poor feed conversion and reduced growth rates observed in naturally affected chickens with proventriculitis, and the reduced body weight observed in our experimental chickens at 21 days post inoculation.

Severe lymphocytic infiltration was observed in all experimentally infected chickens. The distribution of these lymphocytes in the proventriculus varied. In the acute or early stages lymphocytes were present as sheets in large numbers in the lamina propria of the mucosa and infiltrating affected glands. In the later stages the lymphocytes formed aggregates in both the lamina propria of the mucosa and deep in the proventricular glands. These chronic changes were accompanied by less necrosis and ductal hyperplasia. Staining of these lymphocytes showed that both B and T cells are increased in number during proventriculitis but occupied different histologic locations within the proventriculus depending on the stage of the disease.

Lymphocytes are present in the mucosa of normal chicken organs as the mucosal associated lymphoid tissue (MALT). This complex immune apparatus has developed in the chicken in response to antigens entering the body through mucosal surfaces lining the respiratory, digestive and genitourinary tracts, and provides the first line of defense against these antigens (2). Matsumoto and Hashimoto (19) described the normal distribution and developmental changes of the lymphoid tissues in the chicken proventriculus. They observed the development of lymphoid masses in the proventricular lamina propria underneath the surface epithelium and near the duct orifice, which suggested that the local mucosal immune mechanism develops primarily with a dominant participation of T lymphocytes in the early post-hatching period. The development of B lymphocytes occurs following the invasion of the antigens associated with food intake, owing to immunological information from the prerequisite T lymphocytes. In our study, the response to a non-defined infectious agent present in the positive proventricular homogenate induced proliferation of the lymphoid tissue present in the proventriculus. This immune response was similar to that observed in the mucosa of other organs in response to different pathogens (2,6,8,11,18,21,26,28,31,32). Intrapithelial lymphocytes (EEL) could be observed in the deep proventricular gland and Matsumoto and Hashimoto (19) identified them as γδ T lymphocytes, similar to those found in the chicken intestine. These authors could not demonstrate the presence of M cells in the proventriculus suggesting that there are alternative routes for uptake of intraluminal antigens.

The cause of proventriculitis is not known but it seems most likely that a virus is the primary agent involved (11,12). T cell mediated immune responses to viral pathogens are well established, and occur by a number of different mechanisms, including induction of cytotoxic activity, recognition of target antigens in conjunction with the major histocompatibility complex (MHC), and production of lymphokines such as interferon-γ, interleukin-2 and tumor necrosis factor-β. Cells mediating these different activities can be identified by cell surface antigens, CD4+ for helper T cells, CD8+ for cytotoxic and suppressor T cells, and CDS3+ as a common T cell antigen (30). Most virus specific cytotoxic T lymphocyte (CTL) activity identified is MHC class I restricted and mediated by CD8+ T cells. The CD4+ subset has an important role in virus infections as it provides the helper T cell necessary to promote the clonal expansion and differentiation of virus-specific B cells (1). The activation of B cells and their differentiation into antibody secreting plasma cells is triggered by antigen and usually requires helper T cells. In our study, CD4+ T cells were the most abundant lymphocyte subset found in the lamina propria of the mucosa in the early stages of proventriculitis. These lymphocytes were later found surrounding what appeared to be B-cell germinal centers.

The CD8+ T cells found in the affected proventricular glands, formed sheets infiltrating the glandular epithelium. The influx of CD8+.T cells suggests cytotoxic activity associated with pathogen clearance. The CD8+ CTL response has been shown to be critical for the control of primary, persistent, and reactivated virus infections (7). The antiviral action of CTL is mediated by direct lysis of infected cells (e.g. by perforin/ granzyme release), the induction of apoptosis (e.g. by Fas/ Fas ligand interaction) and the production of antiviral cytokines (7). Kotani *et al* (15) studied the lymphocytic subsets in the trachea of chickens inoculated with infectious bronchitis virus (IBV) and concluded that the chicken's immune system may utilize specific CTL to eliminate IBV at the early stage of infection, and in the later stage may depend on humoral immunity to control viral infection. In an earlier study it was found that cellular immunosuppression increased the severity and duration of proventriculitis, underlining the importance of T cells in the immune response against proventriculitis (23).

Songserm *el al* (28) also observed an increase of CD8+ cells in the intestine of chickens inoculated with malabsorption syndrome homogenate. They found that an increase of cytotoxic activity was associated with the intestinal lesions and weight gain depression. In our study the influx of CD8+ cells in the proventriculus appeared to occur after the onset of the necrosis of the glandular epithelium. However, the increase of lymphocytes in the glands exacerbated the lesions present in the proventriculus increasing the loss glandular epithelium. Lesions in the proventricular glands did not occur simultaneously. The necrosis and influx of T lymphocytes appeared to start in the area surrounding the mucosal papillae and spread to the glands that drained through these papillae. Microscopically, some glands present lesions and other appear normal, and depending on the severity of the proventriculitis, more glands would be affected.

In addition to CD4+ and CD8+ cells, natural killer cells (NK) may play a role in the defense against gut pathogens. NK cells are phenotypically defined as CD8+ lacking T (CD3+) or B lineage specific markers. Gobel *et al* (9) demonstrated by these criteria that approximately 30% of CD8+ intestinal intraepithelial lymphocytes (IEL) were NK cells. The physiological role of the intestinal NK cells is not known but might constitute the first line of defense once epithelial cells get infected serving similar functions as T cells (9). In our study, because we didn't have a marker for NK cells, this specific subset was not analyzed and we cannot draw conclusions about the role of NK cells in proventriculitis. Most of the lymphocytes observed in the affected proventriculi that stained with the cytotoxic T cell marker (CD8+) also stained with the pan T cell marker (CD3+) with a low percentage not staining for the CD3+ marker. The presence of NK cells in the proventriculus, and their role in proventriculitis needs to be further investigated.

In conclusion, the influx of CD4+ cells during proventriculitis suggests that these cells are involved in the induction of the immune response, whereas the CD8+ cells most likely act as effector cells. The influx of B cells and formation of highly organized germinal centers, indicates that antibody-mediated mechanisms are also involved in the control of proventriculitis in chickens. Germinal center formation occurred in the later stages of the disease when the lesions in the proventriculus were less severe. In this study, staining of B cell immunoglobulins (IgG, IgM, and IgA) was not performed. IgM and IgA in the intestinal secretions prevent environmental antigen influx into internal body compartments, neutralization of viruses and microbial toxins, and prevention of adherence and colonization of mucosal surfaces by pathogens (18). The role of these immunoglobulins is not clear for some poultry infections and further study of the their importance in proventriculitis ought to be done.

**Table. 25. Body weight gain (g), relative proventriculus weight (% body weight), proventriculus lesion score and incidence of proventriculitis, and proventriculus wall thickness (mm) of commercial broilers orally challenged at day of age with a negative proventricular homogenate (-PV), or a positive proventricular homogenate (+PV), and necropsied at 7, 14, and 21 days post-inoculation (mean ± standard deviation).¹**

| Days post-inoculation | Treatment | Body weight gain | PV relative weight | PV lesion score and incidence | | PV wall thickeness |
|---|---|---|---|---|---|---|
| 7 | -PV | 153.7 ± 29.6 ^{a} | 0.85 ± .11 ^{a} | 1 ^{a} | 0/4 | 3.62 ^{a} |
| | +PV | 144.0 ± 10.6 ^{a} | 1.26 ± .22 ^{b} | 3 ^{b} | 3/4 | 4.62 ^{b} |
| 14 | -PV | 370.0 ± 44.2 ^{a} | 0.69 ± 0.08 ^{a} | 1.5 ^{a} | 2/4 | 4.25 ^{a} |
| | +PV | 331.0 ± 62.8 ^{a} | 0.95 ± 0.09 ^{b} | 4 ^{b} | 4/4 | 5.10 ^{b} |
| 21 | -PV | 882.0 ± 35.7 ^{a} | 0.54 ± .005 ^{a} | 1.5 ^{a} | 2/4 | 5.00 ^{a} |
| | +PV | 749.0 ± 20.8 ^{b} | 0.78 ± 0.15 ^{a} | 3 ^{b} | 4/4 | 7.75 ^{b} |

| | | | | | | |
|---|---|---|---|---|---|---|
| ¹Means within a column and time point with different lowercase superscript are significantly different (P<0.05). Means calculated from four birds in each group. | | | | | | |

### References:

1. Ahmed et al. J Virol. 62:2101-2106. 1988.
2. Bar-Shira et al. Dev Comp Immunol. 27:147-157. 2003
3. Barnes et al. Poult Sci 80: 906-911. 2001.
4. Bayyari et al. Poult Sci, 74:1799-1809. 1995.
5. Bayyari et al. Poult Sci 74:1961-1969. 1996.
6. Collisson et al. Dev Comp Immunol 24:187-200. 2000.
7. Farrel et al. Cell Dev Biol. 9:369-378. 1998.
8. Gaunson et al. Microbiology, 146: 1223-1229. 2002.
9. Gobel et al. Int Immunology 13:757-762. 2001.
10. Goodwin et al. Avian Pathol. 25:369-379. 1996.
11. Guy & Barnes. Proceedings of the 139th AVMA annual Convention. July 13-17. Nashville, Tennessee. 2002.
12. Huff et al. Avian Dis. 45: 828-843, 2001.
13. Jensen et al. Avian Dis. 35:969-973. 1991.
14. Kelly et al. Proceedings of the 1381h AVMA Annual Convention. July 14-18. Boston, MA. 2001.
15. Kotani et al. J Vet Med Sci 62:397-401. 2000.
16. Kouwenhoven et al. Avian Pathol. 7:183-187. 1978.
17. Lenz et al. J Vet Diagn Invest 10:145-151. 1998.
18. Lillejoh & Trout. 1996. Clin Microbiol Rev 9, 349-360.
19. Matsumoto & Hashimoto. J Vet Med Sci 62: 161-167. 2000.
20. Newberry. Ph. D. Dissertation. University of Arkansas, Fayetteville, AR. 1996.
21. Ohshima & Hiramatsu. Histol Histopathol 15: 713-720. 2000.
22. Pantin-Jack-wood & Brown. Annual Meeting of the American College of Veterinary Pathologists. New Orleans, LA. Dec. 8-11, 2002.
23. Pantin-Jackwood & Brown. Proceedings of the 139th AVMA annual Convention. July 13-17. Nashville, Tennessee. 2002.
24. Pegram & Wyatt. Poultry Sci. 60:2429-2440.1981.
25. Riddell. Avian Dis. 20:442-445. 1976.
26. Rothwell et al. Parasite Immunol. 17:525-533. 1995
27. Schulze & Heidrich. Dtsch Tierarztl Wochenschr 108:264-266. 2001.
28. Songserm et al. Vet Immunol Immunopathol. 85: 51-62. 2002.
29. Turpin. Thesis. University of Georgia. 1998.
30. Vainio & Lassila. Crit Rev Poult Biol 2: 97-102. 1989.
31. Vervelde et al. Parasite Immunol. 18: 247-256. 1996.
32. Withanage et al. Vet Immunol Immunopathol. 66: 173-184. 1998.
33. Yu et al. Avian Dis. 45:416-424. 2001.

DISCUSSION AND CONCLUSIONS. SPF broilers experimentally infected with different strains of IBDV did not develop proventriculitis, and chickens with naturally occurring cases of proventriculitis did not have IBDV in their proventriculi. Although the strains chosen for this study belong to five of the six molecular groups used to classify IBDV strains, it is possible that other untested strains may produce proventriculitis directly, or that proventriculitis is due to an undetermined cause and could be exacerbated by immunosuppression produced by IBDV infection.

Proventriculitis was studied by experimentally reproducing the disease in broiler chickens. One-day-old commercial and SPF broilers were orally gavaged with a proventricular homogenate produced from the proventriculi of broilers with proventriculitis. Both, commercial and SPF broilers presented enlargement of the proventriculus with necrosis of the glandular epithelium and lymphocytic infiltrates in the proventricular gland. SPF broilers exposed to the proventricular homogenates developed Infectious Bursal Disease, and infectious bursal disease virus (IBDV) was detected by reverse transcriptase polymerase chain reaction (RT-PCR) and immunohistochemistry (IHC) in bursal and proventricular tissues. They also were positive by RT-PCR to infectious bronchitis virus (IBV) and developed nephritis. Commercial broilers developed mild nephritis but not bursal disease, and were negative for IBDV and IBV by RT-PCR. Both, commercial and SPF chickens, were negative for reovirus, and Newcastle disease virus (NDV), and positive for chicken anemia virus (CAV) and adenovirus by molecular techniques. Bacteria were not identified in histological sections nor were they isolated from affected proventriculi. Filtrates from the proventricular homogenates passed in embryos for virus isolation caused stunting but identification of the cause by electron microscopy was unsuccessful. However, allantoic fluid from the eggs was positive for IBV by RT-PCR. Thin sectioning EM on proventriculi from affected birds failed to identify a causative agent. In conclusion, the original proventricular homogenates had IBDV, IBV, adenovirus and CAV, but the role of each in producing proventriculitis was not proven.

B cell immunosuppression, by CP or IBDV, did not have an effect on the incidence of proventriculitis, and the lesions observed were similar to those produced by positive proventricular homogenate (+PV) alone. However, proventricular enlargement was more evident in birds immunosuppressed with these agents at 7dpi, indicating that a humoral response might play a role in the early stages of the disease probably by controlling the causative agent by production of antibodies. T cell suppression by CS, on the other hand, did have an effect on the incidence of proventriculitis, and the lesions observed were more severe and lasted longer than in +PV controls. T cells are more abundant in the proventriculus than B cells, underlining their importance in immune responses to infectious agents in this organ. In this study, by affecting T cell function, the severity of proventriculitis was increased and resolution of the disease was prolonged.

The lymphocytic infiltrates present during proventriculitis in both the proventricular gland and the lamina propria, were mainly T cells. The influx of CD4+ cells suggests that these cells are involved in the induction of the immune response, whereas the CD8+ cells most likely act as effector cells. The influx of B cells and formation of highly organized germinal centers, indicates that antibody-mediated mechanisms are also involved in the control of proventriculitis in chickens.

In conclusion, proventriculitis can be reproduced by oral inoculation of chickens with homogenates produced from proventriculi of birds with proventriculitis. The causative agent(s) was not identified, although most likely it is a virus. The severity of proventriculitis and its effect on weight gain is probably affected by other factors such as concomitant infection with more than one agent, viral or bacterial, and nutritional factors. Proventriculitis was reproduced in the absence of IBDV and IBDV did not cause proventriculitis when susceptible chickens were inoculated with the virus. IBDV affects both humoral and cellular immunity in the chicken, so although under experimental conditions it didn't have a major effect on proventriculitis, it may explain why control of IBDV under commercial conditions reduces the incidence of proventriculitis.

The description is further described by the following numbered paragraphs:
1. A method of characterizing a strain of IBDV comprising: generating and sequencing an IBDV cDNA from a sample suspected of having a strain of IBDV, aligning the sequenced IBDV with one or more IBDV sequences, and comparing relatedness of aligned IBDV sequences, thereby characterizing a strain of IBDV.
2. The method of paragraph 1 wherein the sample is a paraffin-embedded tissue sample.
3. The method of paragraph 1 or 2 wherein generating an IBV cDNAs comprises extracting RNA from the sample and RT-PCR amplification of the IBDV cDNA with IBDV-specific primers.
4. The method of paragraph 3 wherein the IBDV-specific primers amplify a hypervariable portion of IBDV.
5. The method of paragraph 4 wherein the hypervariable portion of IBDV is VP1, VP3, VP4 or VP5.
6. The method of paragraph 4 wherein the hypervariable portion of IBDV is VP2.
7. The method of paragraphs 1 to 6 wherein the comparing is with a dendritogram.
8. The method of paragraphs 1 to 7 wherein the one or more IBDV sequences are nucleic acid sequences.
9. The method of paragraphs 1 to 7 wherein an amino acid sequence is deduced from the IBDV cDNA and the one or more IBDV sequences are amino acid sequences.
10. The method of paragraphs 1 to 9 further comprising identifying a novel strain of IBDV wherein the IBDV sequence does not align to any of the one or more IBDV sequences with about 95%, advantageously about 98% to about 99.8%, most advantageously about 99.3% to about 99.6%, homology.
11. The method of paragraph 10 further comprising isolating the novel strain of IBDV.
12. The method of paragraphs 1 to 9 further comprising selecting a vaccine to protect an avian against the strain of IBDV, wherein the vaccine has an IBDV sequence most closely matched with about about 95%, advantageously about 98% to about 99.8%, most advantageously about 99.3% to about 99.6%, homology to the IBDV cDNA.
13. The method of paragraph 12 wherein an amino acid sequence is deduced from the IBDV cDNA and the one or more IBDV sequences are amino acid sequences.
14. The method of paragraph 12 wherein the avian is selected from the group consisting of a chicken, duck, goose, pheasant, quail and turkey.
15. A method of identifying a vaccine for a strain of IBDV comprising: (a) generating an IBDV cDNA from the strain of IBDV, (b) aligning the IBDV cDNA with a plurality of IBDV sequences, (c) comparing relatedness of aligned IBDV sequences, and (d) identifying a vaccine for a strain of IBDV if the IBDV cDNA is at least about about 95%, advantageously about 98% to about 99.8%, most advantageously about 99.3% to about 99.6%, homologous to any one of the plurality of IBDV sequences.
16. A method of identifying a novel strain of IBDV comprising: (a) generating an IBDV cDNA from the strain of IBDV, (b) aligning the IBDV cDNA with a plurality of IBDV sequences, (c) comparing relatedness of aligned IBDV sequences, and (d) identifying a novel strain of IBDV if the IBDV cDNA is less than about about 95%, advantageously about 98% to about 99.8%, most advantageously about 99.3% to about 99.6%, homologous to any one of the plurality of IBDV sequences.
17. The method of paragraph 15 or 16 wherein the strain of IBDV is from a sample suspected of having a strain of IBDV.
18. The method of paragraph 17 wherein the sample is a paraffin-embedded tissue sample.
19. The method of paragraph 17 or 18 wherein generating an IBDV cDNAs comprises extracting RNA from the sample and RT-PCR amplification of the IBDV cDNA with IBDV-specific primers.
20. The method of paragraph 19 wherein the IBDV-specific primers amplify a hypervariable portion of IBDV.
21. The method of paragraph 20 wherein the hypervariable portion of IBDV is VP1, VP3, VP4 or VP5.
22. The method of paragraph 20 wherein the hypervariable portion of IBDV is VP2.
23. The method of paragraphs 16 to 22 wherein the comparing is with a dendritogram.
24. The method of paragraphs 16 to 22 wherein the plurality of IBDV sequences are nucleic acid sequences.
25. The method of paragraphs 16 to 22 wherein an amino acid sequence is deduced from the IBDV cDNA and the plurality of IBDV sequences are amino acid sequences.
26. A computer-assisted method for characterizing a strain of IBDV comprising: using a computer system, e.g., a programmed computer comprising a processor, a data storage system, an input device, and an output device, the steps of: (a) inputting into the programmed computer through the input device data comprising sequences of IBDV generated from a sample suspected of having a strain of IBDV, thereby generating a data set; (b) comparing, using the processor, the data set to a computer database of IBDV sequences stored in the computer data storage system; (c) selecting from the database, using computer methods, IBDV sequences stored in the computer data storage system having a portion that is about about 95%, advantageously about 98% to about 99.8%, most advantageously about 99.3% to about 99.6%, homologous to the data set; (d) and outputting to the output device the selected IBDV sequences having a portion that is at least about about 95%, advantageously about 98% to about 99.8%, most advantageously about 99.3% to about 99.6%, homologous to the data set, or optionally outputting to the output device indicating the absence of IBDV sequences having a portion that is at least about about 95%, advantageously about 98% to about 99.8%, most advantageously about 99.3% to about 99.6%, homologous to the data set if no IBDV sequences have a portion that is at least about 95%, advantageously about 98% to about 99.8%, most advantageously about 99.3% to about 99.6%, homologous to the data set, thereby characterizing a strain of IBDV.
27. The method of paragraph 26 wherein the sample is a paraffin-embedded tissue sample.
28. The method of paragraph 26 or 27 wherein the IBDV sequences correspond to one or more hypervariable portions of IBDV.
29. The method of paragraph 28 wherein the hypervariable portion of IBDV is VP1, VP3, VP4 or VP5.
30. The method of paragraph 28 wherein the hypervariable portion of IBDV is VP2.
31. The method of paragraphs 26 to 30 wherein the IBDV sequences in the storage system are nucleic acid sequences.
32. The method of paragraphs 26 to 30 wherein the IBDV sequences in the storage system are amino acid sequences.
33. The method of paragraph 32 further comprising generating a data set of amino acid sequences.
34. The method of paragraphs 26 to 33 comprising identifying a vaccine for the strain of IBDV wherein the vaccine is identified by identifying IBDV strains with one or more IBDV sequences having a portion that is at least about about 95%, advantageously about 98% to about 99.8%, most advantageously about 99.3% to about 99.6%, homologous to the data set.
35. The method of paragraphs 26 to 33 comprising identifying a novel strain of IBDV wherein the novel strain is identified if no IBDV sequences have a portion that is at least about about 95%, advantageously about 98% to about 99.8%, most advantageously about 99.3% to about 99.6%, homologous to the data set.
36. A method of transmitting data comprising transmission of information from such methods herein discussed or steps thereof, e.g., via telecommunication, telephone, video conference, mass communication, e.g., presentation such as a computer presentation (e.g. POWERPOINT), internet, email, documentary communication such as a computer program (e.g. WORD) document and the like.
37. A computer system for characterizing a strain of IBDV, the system containing either: IBV nucleotide sequences according to Table 2 and/or FIG. 1 or IBDV amino acid sequences of Table 3 or IBDV amino acid sequences derived from the nucleotide sequences according to Table 2 and/or FIG. 1.
38. A computer readable media containing either: IBDV nucleotide sequences according to Table 2 and/or FIG. 1 or IBDV amino acid sequences of Table 3 or IBDV amino acid sequences derived from the nucleotide sequences according to Table 2 and/or FIG. 1.
39. A method of doing business comprising providing to a user the computer system of paragraph 37 or the media of paragraph 38 or either: IBDV nucleotide sequences according to Table 2 and/or FIG. 1 or IBDV amino acid sequences of Table 3 or IBDV amino acid sequences derived from the nucleotide sequences according to Table 2 and/or FIG. 1.
40. A method for obtaining an epitope, antigen, or immunogen of a novel strain of IBDV comprising isolating the epitope, antigen, or immunogen from a novel strain of IBDV identified by the methods of paragraphs 10, 16 or 35.
41. The method of paragraph 40 wherein the epitope, antigen, or immunogen is an expression product of a nucleic acid molecule that is heterologous to the virus. ,
42. A method for eliciting an immune response comprising administering the epitope, antigen, or immunogen from the method of paragraphs 40 or 41 in an effective amount to elicit the immune response to an animal.
43. A method of eliciting an immune response comprising: administering virus from a novel strain of IBDV identified by the methods of paragraphs 10, 16 or 35 in an effective amount for eliciting an immune response to an animal.
44. The method of paragraph 42 or 43 further comprising administering an adjuvant.
45. The method of paragraphs 42 to 4-4 further comprising administrating a cytokine.
46. The method of paragraph 45 wherein the cytokine is expressed by the virus.
47. The method of paragraphs 40 to 46 wherein said animal is an avian.
48. The method of paragraph 47 wherein the avian is selected from the group consisting of a chicken, duck, goose, pheasant, quail and turkey.
49. The method of paragraphs 40 to 48 wherein the virus is inactivated or attenuated.
50. A method for inducing an immunological or protective response comprising administering an effective amount of a virus from the method of paragraphs 43 to 49, or an immunogen, antigen, or epitope thereof, to induce the response in an avian.
51. A method for inducing an immunological or protective response comprising administering an effective amount of an immunogen, antigen, or epitope from the method of paragraphs 40 or 41 to induce the response in an avian.
52. An isolated IBDV that consists essentially of Sequence No. 1631 having the sequence of SEQ ID NO: 1.
53. An isolated IBDV that consists essentially of Sequence No. 087 having the sequence of SEQ ID NO: 3.
54. An isolated IBDV that consists essentially of Sequence No. 077 having the sequence of SEQ ID NO: 5.
55. An isolated IBDV polypeptide that consists essentially of the amino acid residues of Sequence No. 1631 having the sequence of SEQ ID NO: 2.
56. An isolated IBDV polypeptide that consists essentially of the amino acid residues of Sequence No. 087 having the sequence of SEQ ID NO: 4.
57. An isolated IBDV polypeptide that consists essentially of the amino acid residues of Sequence No. 077 having the sequence of SEQ ID NO: 6.
58. An isolated IBDV polynucleotide, or an antisense strand that is fully complementary thereto, that consists essentially of Sequence No. 1631, having the sequence of SEQ I17 NO: 1.
59. An isolated IBDV polynucleotide, or an antisense strand that is fully complementary thereto, that consists essentially of Sequence No. 087, having the sequence of SEQ ID NO: 3.
60. An isolated IBDV polynucleotide, or an antisense strand that is fully complementary thereto, that consists essentially of Sequence No. 077, having the sequence of SEQ ID NO: 5.
61. The polynucleotide of paragraphs 58-60, wherein the polynucleotide is a DNA molecule.
62. The polynucleotide of paragraphs 58-60, wherein the polynucleotide is an RNA molecule.
63. A method for obtaining an epitope, antigen, or immunogen of a novel strain of IBDV comprising isolating the epitope, antigen, or immunogen from the IBDV of paragraphs 52-54.
64. The method of paragraph 63 wherein the epitope, antigen, or immunogen is an expression product of a nucleic acid molecule that is heterologous to the virus.
65. A method for eliciting an immune response comprising administering the epitope, antigen, or immunogen from the method of paragraphs 63-64 in an effective amount to elicit the immune response to an animal.
66. A method of eliciting an immune response comprising: administering virus from the IBDV of paragraphs 52-54 in an effective amount for eliciting an immune response to an animal.
67. The method of paragraphs 65-66 further comprising administering an adjuvant.
68. The method of paragraphs 65-67 further comprising administrating a cytokine.
69. The method of claim 57 wherein the cytokine is expressed by the virus.
70. The method of paragraphs 65-69 wherein said animal is an avian.
71. The method of paragraph 70 wherein the avian is selected from the group consisting of a chicken, duck, goose, pheasant, quail and turkey.
72. The method of paragraphs 65-71 wherein the virus is inactivated or attenuated.
73. A method for inducing an immunological or protective response comprising administering an effective amount of IBV of paragraphs 52-54, or an immunogen, antigen, or epitope thereof, to induce the response in an avian.
74. A method for inducing an immunological or protective response comprising administering an effective amount of an immunogen, antigen, or epitope from the IBDV of paragraphs 52-54 to induce the response in an avian.

## Claims

1. An isolated IBDV polypeptide sequence that consists of the amino acid residues of SEQ ID NO: 4 or SEQ ID NO: 6.

2. An isolated IBDV polynucleotide sequence that consists of the sequence of SEQ ID NO: 3 or SEQ ID NO: 5, or an antisense strand that is fully complementary thereto.

3. The polynucleotide sequence of claim 2, wherein the polynucleotide sequence is a DNA molecule.

4. The polynucleotide sequence of claim 2, wherein the polynucleotide sequence is an RNA molecule.

5. An expression vector comprising a polynucleotide sequence according to any one of claims 2 to 4.

6. A preparation comprising one or more expression vectors according to claim 5.

7. The preparation according to claim 6 wherein said preparation is an immunogenic composition.

8. The preparation according to claim 6 or 7 wherein said preparation comprises a pharmaceutically or veterinarily acceptable carrier, excipient or vehicle.

9. Use of an isolated IBDV polypeptide sequence according to claim 1, or an isolated IBDV polynucleotide sequence according to any one of claims 2 to 4, or an expression vector according to claim 5, for the preparation of an immunogenic composition.

10. An an isolated IBDV polypeptide sequence according to claim 1, or an isolated IBDV polynucleotide sequence according to any one of claims 2 to 4, or an expression vector according to claim 5, or a preparation according to any one of claims 6 to 8, for use in inducing an immunological response to IBDV in an avian.

11. Use of an isolated IBDV polypeptide sequence according to claim 1, or an isolated IBDV polynucleotide sequence according to any one of claims 2 to 4, or an expression vector according to claim 5, or a preparation according to any one of claims 6 to 8, for the preparation of a medicament for inducing an immunological response to IBDV in an avian.

## Patentansprüche

1. Isolierte IBDV-Polypeptidsequenz, die aus den Aminosäureresten der SEQ ID NO:4 oder der SEQ ID NO:6 besteht.

2. Isolierte IBDV-Polynucleotidsequenz, die aus der Sequenz SEQ ID NO:3 oder SEQ ID NO:5 besteht, oder Antisense-Strang, der hierzu vollständig komplementär ist.

3. Polynucleotidsequenz nach Anspruch 2, wobei die Polynucleotidsequenz ein DNA-Molekül ist.

4. Polynucleotidsequenz nach Anspruch 2, wobei die Polynucleotidsequenz ein RNA-Molekül ist.

5. Expressionsvektor, der eine Polynucleotidsequenz nach einem der Ansprüche 2 bis 4 umfasst.

6. Zubereitung, die einen oder mehrere Expressionsvektoren nach Anspruch 5 umfasst.

7. Zubereitung nach Anspruch 6, wobei die Zubereitung eine immunogene Zusammensetzung ist.

8. Zubereitung nach Anspruch 6 oder 7, wobei die Zubereitung einen pharmazeutisch oder veterinärmedizinisch verträglichen Träger, Exzipienten oder ein pharmazeutisch oder veterinärmedizinisch verträgliches Vehikel umfasst.

9. Verwendung einer isolierten IBDV-Polypeptidsequenz nach Anspruch 1 oder einer isolierten IBDV-Polynucleotidsequenz nach einem der Ansprüche 2 bis 4 oder eines Expressionsvektors nach Anspruch 5 zur Herstellung einer immunogenen Zusammensetzung.

10. Isolierte IBDV-Polypeptidsequenz nach Anspruch 1 oder isolierte IBDV-Polynucleotidsequenz nach einem der Ansprüche 2 bis 4 oder Expressionsvektor nach Anspruch 5 oder Zubereitung nach einem der Ansprüche 6 bis 8 zur Verwendung bei der Induzierung einer Immunantwort gegen IBDV bei einem Vogel.

11. Verwendung einer isolierten IBDV-Polypeptidsequenz nach Anspruch 1 oder einer isolierten IBDV-Polynucleotidsequenz nach einem der Ansprüche 2 bis 4 oder eines Expressionsvektors nach Anspruch 5 oder einer Zubereitung nach einem der Ansprüche 6 bis 8 zur Herstellung eines Medikaments für die Induzierung einer Immunantwort gegen IBDV bei einem Vogel.

## Revendications

1. Séquence de polypeptide du virus de la bursite infectieuse (VBI) isolée, qui est constituée des résidus d'aminoacides de SEQ ID NO: 4 ou SEQ ID NO: 6.

2. Séquence de polynucléotide du VBI isolée, qui est constituée de la séquence de SEQ ID NO: 3 ou SEQ ID NO: 5, ou brin antisens qui en est entièrement complémentaire.

3. Séquence de polynucléotide selon la revendication 2, la séquence de polynucléotide étant une molécule d'ADN.

4. Séquence de polynucléotide selon la revendication 2, la séquence de polynucléotide étant une molécule d'ARN.

5. Vecteur d'expression comprenant une séquence de polynucléotide selon l'une quelconque des revendications 2 à 4.

6. Préparation comprenant un ou plusieurs vecteurs d'expression selon la revendication 5.

7. Préparation selon la revendication 6, ladite préparation étant une composition immunogène.

8. Préparation selon la revendication 6 ou 7, ladite préparation comprenant un support, excipient ou véhicule acceptable sur le plan pharmaceutique ou vétérinaire.

9. Utilisation d'une séquence de polypeptide du VBI isolée selon la revendication 1, ou d'une séquence de polynucléotide du VBI isolée selon l'une quelconque des revendications 2 à 4, ou d'un vecteur d'expression selon la revendication 5, pour la préparation d'une composition immunogène.

10. Séquence de polypeptide du VBI isolée selon la revendication 1, ou séquence de polynucléotide du VBI isolée selon l'une quelconque des revendications 2 à 4, ou vecteur d'expression selon la revendication 5, ou préparation selon l'une quelconque des revendications 6 à 8, pour une utilisation dans l'induction d'une réponse immunologique au VBI chez un oiseau.

11. Utilisation d'une séquence de polypeptide du VBI isolée selon la revendication 1, ou d'une séquence de polynucléotide du VBI isolée selon l'une quelconque des revendications 2 à 4, ou d'un vecteur d'expression selon la revendication 5, ou d'une préparation selon l'une quelconque des revendications 6 à 8, pour la préparation d'un médicament destiné à l'induction d'une réponse immunologique au VBI chez un oiseau.


### SEQUENCE LISTING

<110> UNIVERSITY OF GEORGIA RESEARCH FOUNDATION, INC.
<120> METHODS OF CHARACTERIZING INFECTIOUS BURSAL DISEASE VIRUS
<130> 574313-3195.1
<140> PCT/US2004/037255
   <141> 2004-11-08
<150> 60/519,571
   <151> 2003-11-13
<160> 61
<170> PatentIn ver. 3.3
<210> 1
   <211> 276
   <212> DNA
   <213> Infectious bursal disease virus
<400> 1
   cagccgatga ttaccagttc tcatcacagt accaagcagg tggggtaaca atcacactgt 60
   tctcagctaa tatcgatgcc atcacaagcc tcagcatcgg gggagaactc gtgtttcaaa 120
   caagcgtcca aggccttata ctgggtgcta ccatctacct tataggcttt gatgggactg 180
   cggtaatcac cagagctgtg gccgcagaca atgggctaac ggccggcact gacaacctta 240
   tgccattcaa tattgtgatt ccaaccaacg agataa                           276
<210> 2
   <211> 91
   <212> PRT
   <213> Infectious bursal disease virus
<400> 2
   Ala Asp Asp Tyr Gln Phe Ser Ser Gln Tyr Gln Ala Gly Gly Val Thr
   1 5 10 15
   Ile Thr Leu Phe Ser Ala Asn Ile Asp Ala Ile Thr Ser Leu Ser Ile
   20 25 30
   Gly Gly Glu Leu Val Phe Gln Thr Ser Val Gln Gly Leu Ile Leu Gly
   35 40 45
   Ala Thr Ile Tyr Leu Ile Gly Phe Asp Gly Thr Ala Val Ile Thr Arg
   50 55 60
   Ala Val Ala Ala Asp Asn Gly Leu Thr Ala Gly Thr Asp Asn Leu Met
   65 70 75 80
   Pro Phe Asn Ile Val Ile Pro Thr Asn Glu Ile
   85 90
<210> 3
   <211> 213
   <212> DNA
   <213> Infectious bursal disease virus
<400> 3
   cagccaacat tgatgccatc acaagcctca gcgttggggg agagcttttg tttaaaacaa 60
   gcgtccaaag ccttgtactg ggcgctacca tctaccttat aggctttgat gggactgcgg 120
   taatcactag agctgtagcc gcaaacaatg ggctgatgac cggcatcgac aatcttatgc 180
   cattcaatct tgtgattcca accaacgaga taa 213
<210> 4
   <211> 70
   <212> PRT
   <213> Infectious bursal disease virus
<400> 4
   Ala Asn Ile Asp Ala Ile Thr Ser Leu Ser Val Gly Gly Glu Leu Leu
   1 5 10 15
   Phe Lys Thr Ser Val Gln Ser Leu Val Leu Gly Ala Thr Ile Tyr Leu
   20 25 30
   Ile Gly Phe Asp Gly Thr Ala Val Ile Thr Arg Ala Val Ala Ala Asn
   35 40 45
   Asn Gly Leu Met Thr Gly Ile Asp Asn Leu Met Pro Phe Asn Leu Val
   50 55 60
   Ile Pro Thr Asn Glu Ile
   65 70
<210> 5
   <211> 276
   <212> DNA
   <213> Infectious bursal disease virus
<400> 5
   cagccgatga ttaccaattc tcatctcagt accaatcagg tggagtaaca atcacactgt 60
   tctcagccaa cattgatgcc atcacaagcc tcagcgttgg gggagagctt gtgttccaaa 120
   caagcgtcca agaccttata ctgggcgcca ctatctacct tataggcttt gatgggacta 180
   cggtgaccac cagagctgtg gccgcaaaca atgggctgac ggccggcacc gacaatccta 240
   ttccattcaa tcttgtgttc ccgaccaacg agataa                           276
<210> 6
   <211> 91
   <212> PRT
   <213> Infectious bursal disease virus
<400> 6
   Ala Asp Asp Tyr Gln Phe Ser Ser Gln Tyr Gln Ser Gly Gly Val Thr
   1               5                   10                  15
   Ile Thr Leu Phe Ser Ala Asn Ile Asp Ala Ile Thr Ser Leu Ser Val
               20                  25                  30
   Gly Gly Glu Leu Val Phe Gln Thr Ser Val Gln Asp Leu Ile Leu Gly
           35                  40                  45
   Ala Thr Ile Tyr Leu Ile Gly Phe Asp Gly Thr Thr Val Thr Thr Arg
       50                  55                  60
   Ala Val Ala Ala Asn Asn Gly Leu Thr Ala Gly Thr Asp Asn Pro Ile
   65                  70                  75                  80
   Pro Phe Asn Leu Val Phe Pro Thr Asn Glu Ile
                   85                  90
<210> 7
   <211> 20
   <212> DNA
   <213> Artificial Sequence
<220>
   <223> Description of Artificial sequence: Primer
<400> 7
   ggtatgtgag gcttggtgac 20
<210> 8
   <211> 20
   <212> DNA
   <213> Artificial sequence
<220>
   <223> Description of Artificial sequence: Primer
<400> 8
   ttatctcgtt ggttggaatc 20
<210> 9
   <211> 20
   <212> DNA
   <213> Artificial sequence
<220>
   <223> Description of Artificial sequence: Primer
<400> 9
   tcttgggtat gtgaggcttg 20
<210> 10
   <211> 20
   <212> DNA
   <213> Artificial sequence
<220>
   <223> Description of Artificial sequence: Primer
<400> 10
   ggatgtgatt ggctgggtta 20
<210> 11
   <211> 271
   <212> DNA
   <213> Infectious bursal disease virus
<400> 11
   catcaggacc aaagagaatg tcagttccaa gcaggtggag tgacaatcac actgttctca 60
   gccaatatcg atgccatcac aagcctcagc atcgggggag aactcgtgtt tcaaacaagc 120
   gtccaaggcc ttatactggg cgctaccatc taccttatag gctttgatgg aactgcagta 180
   atcaccagag ctgtggccgc agataatggg ctgacggccg gcactgacaa ccttatgcca 240
   ttcaatattg taattccaac cagcgagata a 271
<210> 12
   <211> 90
   <212> PRT
   <213> Infectious bursal disease virus
<400> 12
   His Gln Asp Gln Arg Glu Cys Gln Phe Gln Ala Gly Gly Val Thr Ile
   1 5 10 15
   Thr Leu Phe Ser Ala Asn Ile Asp Ala Ile Thr Ser Leu Ser Ile Gly
   20 25 30
   Gly Glu Leu Val Phe Gln Thr Ser Val Gln Gly Leu Ile Leu Gly Ala
   35 40 45
   Thr Ile Tyr Leu Ile Gly Phe Asp Gly Thr Ala Val Ile Thr Arg Ala
   50 55 60
   Val Ala Ala Asp Asn Gly Leu Thr Ala Gly Thr Asp Asn Leu Met Pro
   65 70 75 80
   Phe Asn Ile Val Ile Pro Thr Ser Glu Ile
   85 90
<210> 13
   <211> 276
   <212> DNA
   <213> Infectious bursal disease virus
<400> 13
   cagccgacga ttaccaattc tcatcacagt accaagcagg tggggtaaca atcacactgt 60
   tctcagctaa tatcgatgcc atcacaagcc tcagcatcgg gggagaactc gtgtttcaaa 120
   caagcgtcca aggccttata ctgggtgcta ccatctacct tataggcttt gatgggactg 180
   cggtaatcac cagagctgtg gccgcagaca atgggctgac ggccggcact gacaacctta 240
   tgccattcaa tattgtgatt ccaaccagcg agataa                           276
<210> 14
   <211> 91
   <212> PRT
   <213> Infectious bursal disease virus
<400> 14
   Ala Asp Asp Tyr Gln Phe Ser Ser Gln Tyr Gln Ala Gly Gly Val Thr
   1               5                   10                  15
   Ile Thr Leu Phe Ser Ala Asn Ile Asp Ala Ile Thr Ser Leu Ser Ile
               20                  25                  30
   Gly Gly Glu Leu Val Phe Gln Thr Ser Val Gln Gly Leu Ile Leu Gly
           35                  40                  45
   Ala Thr Ile Tyr Leu Ile Gly Phe Asp Gly Thr Ala Val Ile Thr Arg
       50                  55                  60
   Ala Val Ala Ala Asp Asn Gly Leu Thr Ala Gly Thr Asp Asn Leu Met
   65                  70                  75                  80
   Pro Phe Asn Ile Val Ile Pro Thr Ser Glu Ile
                   85                  90
<210> 15
   <211> 276
   <212> DNA
   <213> Infectious bursal disease virus
<400> 15
   cagccgatga ttaccaattc tcatcacagt accaagcagg tggggtaaca atcacactgt 60
   tctcagctaa tatcgatgcc atcacaagcc tcagcatcgg gggagaactc gtgtttcaaa 120
   caagcgtcca aggccttata ctgggtgcta ccatctacct tataggcttt gatgggactg 180
   cggtaatcac cagagctgtg gccgcagaca atgggctaac ggccggcact gacaacctta 240
   tgccattcaa tattgtgatt ccaaccagcg agataa                           276
<210> 16
   <211> 91
   <212> PRT
   <213> Infectious bursal disease virus
<400> 16
   Ala Asp Asp Tyr Gln Phe Ser Ser Gln Tyr Gln Ala Gly Gly Val Thr
   1               5                   10                  15
   Ile Thr Leu Phe Ser Ala Asn Ile Asp Ala Ile Thr Ser Leu Ser Ile
               20                  25                  30
   Gly Gly Glu Leu Val Phe Gln Thr Ser Val Gln Gly Leu Ile Leu Gly
           35                  40                  45
   Ala Thr Ile Tyr Leu Ile Gly Phe Asp Gly Thr Ala Val Ile Thr Arg
       50                  55                  60
   Ala Val Ala Ala Asp Asn Gly Leu Thr Ala Gly Thr Asp Asn Leu Met
   65                  70                  75                  80
   Pro Phe Asn Ile Val Ile Pro Thr Ser Glu Ile
                   85                  90
<210> 17
   <211> 276
   <212> DNA
   <213> Infectious bursal disease virus
<400> 17
   cagccgatga ttaccaattc tcatcacagt accaaccagg tggggtaaca atcacactgt 60
   tctcagccaa cattgatgct atcacaagcc tcagcgttgg gggagagctc gtgtttcaaa 120
   caagcgtcca aggtcttgta ctgggcgcca ccatctactt tataggcttt gatgggacta 180
   cggtaatcac cagggctgtg gccgcagaca atgggctgac ggccggcacc gacaatctta 240
   tgccattcaa tcttgtgatt ccaaccaacg agataa                           276
<210> 18
   <211> 91
   <212> PRT
   <213> Infectious bursal disease virus
<400> 18
   Ala Asp Asp Tyr Gln Phe Ser Ser Gln Tyr Gln Pro Gly Gly Val Thr
   1               5                   10                  15
   Ile Thr Leu Phe Ser Ala Asn Ile Asp Ala Ile Thr Ser Leu Ser Val
               20                  25                  30
   Gly Gly Glu Leu Val Phe Gln Thr Ser Val Gln Gly Leu Val Leu Gly
           35                  40                  45
   Ala Thr Ile Tyr Phe Ile Gly Phe Asp Gly Thr Thr Val Ile Thr Arg
       50                  55                  60
   Ala Val Ala Ala Asp Asn Gly Leu Thr Ala Gly Thr Asp Asn Leu Met
   65                  70                  75                  80
   Pro Phe Asn Leu Val Ile Pro Thr Asn Glu Ile
                   85                  90
<210> 19
   <211> 276
   <212> DNA
   <213> Infectious bursal disease virus
<400> 19
   cagccgataa ttaccaattc tcatcacagt accaaacagg tggggtaaca atcacactgt 60
   tctcagccaa cattgatgcc atcacaagtc tcagcgttgg gggagagctc gtgttcaaaa 120
   caagcgtcca aagccttgta ctgggcgcca ccatctacct tataggcttt gatgggactg 180
   cggtaatcac cagagctgtg gccgcaaaca atgggctgac ggccggcatc gacaatctta 240
   tgccattcaa tcttgtgatt ccaaccaatg agataa                           276
<210> 20
   <211> 91
   <212> PRT
   <213> Infectious bursal disease virus
<400> 20
   Ala Asp Asn Tyr Gln Phe Ser Ser Gln Tyr Gln Thr Gly Gly Val Thr
   1               5                   10                  15
   Ile Thr Leu Phe Ser Ala Asn Ile Asp Ala Ile Thr Ser Leu Ser Val
               20                  25                  30
   Gly Gly Glu Leu Val Phe Lys Thr Ser Val Gln Ser Leu Val Leu Gly
           35                  40                  45
   Ala Thr Ile Tyr Leu Ile Gly Phe Asp Gly Thr Ala Val Ile Thr Arg
       50                  55                  60
   Ala Val Ala Ala Asn Asn Gly Leu Thr Ala Gly Ile Asp Asn Leu Met
   65                  70                  75                  80
   Pro Phe Asn Leu Val Ile Pro Thr Asn Glu Ile
                   85                  90
<210> 21
   <211> 276
   <212> DNA
   <213> Infectious bursal disease virus
<400> 21
   cagccgatga ttaccaattc tcatcacaat accaacaagg tggggtaacg atcacactgt 60
   tctcagccaa cattgatgcc atcacaagcc tcagcgttgg gggagagctt gtgtttaaaa 120
   caagcgtcca aagccttgta ctgggcgcca ccatctacct tataggcttt gatgggactg 180
   cggtaatcac tagagctgta gccgcaaaca atgggctgac ggccggcatc gacaatctta 240
   tgccattcaa tcttgtgatt ccaaccaacg agataa                           276
<210> 22
   <211> 91
   <212> PRT
   <213> Infectious bursal disease virus
<400> 22
   Ala Asp Asp Tyr Gln Phe Ser Ser Gln Tyr Gln Gln Gly Gly Val Thr
   1               5                   10                  15
   Ile Thr Leu Phe Ser Ala Asn Ile Asp Ala Ile Thr Ser Leu Ser Val
               20                  25                  30
   Gly Gly Glu Leu Val Phe Lys Thr Ser Val Gln Ser Leu Val Leu Gly
           35                  40                  45
   Ala Thr Ile Tyr Leu Ile Gly Phe Asp Gly Thr Ala Val Ile Thr Arg
       50                  55                  60
   Ala Val Ala Ala Asn Asn Gly Leu Thr Ala Gly Ile Asp Asn Leu Met
   65                  70                  75                  80
   Pro Phe Asn Leu Val Ile Pro Thr Asn Glu Ile
                   85                  90
<210> 23
   <211> 21
   <212> DNA
   <213> Artificial sequence
<220>
   <223> Description of Artificial Sequence: Primer
<400> 23
   aatggaacga tagcgtgtgg g 21
<210> 24
   <211> 19
   <212> DNA
   <213> Artificial sequence
<220>
   <223> Description of Artificial sequence: Primer
<400> 24
   tgaaactgga acaaaagac 19
<210> 25
   <211> 20
   <212> DNA
   <213> Artificial sequence
<220>
   <223> Description of Artificial sequence: Primer
<400> 25
   cataactaac ataagggcaa 20
<210> 26
   <211> 23
   <212> DNA
   <213> Artificial Sequence
<220>
   <223> Description of Artificial Sequence: Primer
<400> 26
   tacacctcat cccagacagg gtc 23
<210> 27
   <211> 23
   <212> DNA
   <213> Artificial sequence
<220>
   <223> Description of Artificial sequence: Primer
<400> 27
   aggcagggga agtgatttgt ggc 23
<210> 28
   <211> 20
   <212> DNA
   <213> Artificial sequence
<220>
   <223> Description of Artificial Sequence: Primer
<400> 28
   ctaagatctg caactgcgga 20
<210> 29
   <211> 20
   <212> DNA
   <213> Artificial Sequence
<220>
   <223> Description of Artificial Sequence: Primer
<400> 29
   ccttggaagc ggatagtcat 20
<210> 30
   <211> 19
   <212> DNA
   <213> Artificial Sequence
<220>
   <223> Description of Artificial Sequence: primer
<400> 30
   tggacatggg ggcgaccta 19
<210> 31
   <211> 18
   <212> DNA
   <213> Artificial Sequence
<220>
   <223> Description of Artificial Sequence: Primer
<400> 31
   agggattgac gttgtcca 18
<210> 32
   <211> 276
   <212> DNA
   <213> Infectious bursal disease virus
<400> 32
   cagccgatga ttaccaattc tcatcacagt accaaccagg tggggtaaca atcacactgt 60
   tctcagccaa cattgatgcc atcacaagcc tcagcgttgg gggagagctc gtgttccaaa 120
   caagcgtcca aggccttgta ctgggcgcca ccatctacct tataggcttt gatgggacta 180
   cggtaatcac cagagctgtg gccgcaaaca atgggctgac ggccggcacc gacaatctta 240
   tgccattcaa tcttgtgatt ccaaccaacg agataa                          276
<210> 33
   <211> 276
   <212> DNA
   <213> Infectious bursal disease virus
<400> 33
   cagccgatga ttaccaattc tcatcacaat accaacaagg tggggtaaca atcacactgt 60
   tctcagccaa cattgatgcc atcacaagcc tcagcgttgg gggagagctt gtgtttaaaa 120
   caagcgtcca aagccttgta ctgggcgcca ccatctacct tataggcttt gatgggactg 180
   cggtaatcac tagagctgta gccgcaaaca atgggctgac ggccggcatc gacaatctta 240
   tgccattcaa tcttgtgatt ccaaccaacg agataa                          276
<210> 34
   <211> 276
   <212> DNA
   <213> Infectious bursal disease virus
<400> 34
   cagccgatga ttaccaattc tcatcacagt accaaacagg tggggtaaca atcacactgt  60
   tctcagccaa cattgatgcc atcacaagtc tcagcgttgg gggagagctc gtgttcaaaa 120
   caagcgtcca aagccttgta ctgggcgcca ccatctacct tataggcttt gatgggactg 180
   cggtaatcac cagagctgtg gccgcaaaca atgggctgac ggccggcatc gacaatctta 240
   tgccattcaa tcttgtgatt ccaaccaatg agataa                          276
<210> 3S
   <211> 276
   <212> DNA
   <213> Infectious bursal disease virus
<400> 35
<210> 36
   <211> 276
   <212> DNA
   <213> Infectious bursal disease virus
<400> 36
   cagccgatga ttaccaattc tcatcacagt accaaccagg tggggtaaca atcacactgt 60
   tctcagccaa cattgatgct atcacaagcc tcagcgttgg gggagagctc gtgtttcaaa 120
   caagcgtcca aggtcttgta ctgggcgcca ccatctactt tataggcttt gatgggacta 180
   cggtaatcac cagggctgtg gccgcagaca atgggctgac ggccggcacc gacaatctta 240
   tgccattcaa tcttgtgatt ccaaccaacg agataa                           276
<210> 37
   <211> 276
   <212> DNA
   <213> Infectious bursal disease virus
<400> 37
   cagccgatga ttaccaattc tcatcacagt accaaccagg tggggtaaca atcacactgt 60
   tctcagccaa cattgatgcc atcacaagcc tcagcgttgg gggagagctc gtgtttcaaa 120
   caagcgtcca cggccttgta ctgggcgcca ccatctacct cataggcttt gatgggacaa 180
   cggtaatcac cagggctgtg gccgcaaaca atgggctgac aaccggcacc gacaacctta 240
   tgccattcaa tcttgtgatt ccaacaaacg agataa                           276
<210> 38
   <211> 276
   <212> DNA
   <213> Infectious bursal disease virus
<400> 38
   cagccgatga ttaccaattc tcatcacagt accaaccagg tggggtaaca atcacactgt 60
   tctcagccaa cattgatgcc atcacaagcc tcagcgttgg gggagagctc gtgtttcaaa 120
   caagcgtcca cggccttgta ctgggcgcca ccatctacct cataggcttt gatgggacaa 180
   cggtaatcac cagggctgtg gccgcaaaca atgggctgac gaccggcacc gacaacctta 240
   tgccattcaa tcttgtgatt ccaacaaacg agataa                           276
<210> 39
   <211> 276
   <212> DNA
   <213> Infectious bursal disease virus
<400> 39
   cagccgatga ttaccaattc tcatcacagt accaaccagg tggggtaaca atcacactgt 60
   tctcagccaa cattgatgcc atcacaagcc tcagcgttgg gggagagctc gtgtttcaaa 120
   caagcgtcca cggccttgta ctgggcgcca ccatctacct cataggcttt gatgggacaa 180
   cggtaatcac cagggctgtg gccgcaaaca atgggctgac gaccggcacc gacaatctta 240
   tgccattcaa tcttgtgatt ccaaccaatg agataa                           276
<210> 40
   <211> 276
   <212> DNA
   <213> Infectious bursal disease virus
<400> 40
   cagccgatga ttaccaattc tcatcacagt accaaccagg tggggtaaca atcacactgt 60
   tctcagccaa cattgatgct atcacaagcc tcagcgttgg gggagagctc gtgcttcaaa 120
   caagcgtcca aggccttgta ctgggcgcca ccatctactt tataggcttt gatgggacta 180
   cggtaatcac cagggctgtg gccgcagaca atgggctgac ggccggcacc gacaatctta 240
   tgccattcaa tcttgtgatt ccaaccaacg agataa 276
<210> 41
   <211> 276
   <212> DNA
   <213> Infectious bursal disease virus
<400> 41
   cagccgatga ttaccaattc ttatcacagt accaaccagg tggggtaaca atcacactgt 60
   tctcagccaa cattgatgct atcacaagcc tcagcgttgg gggagagctc gtgttctaaa 120
   caagcgttca aggccttgta ctgggcgcca ccatctacct tataggcttt gatgggacta 180
   cggtaatcac cagggctgtg gccgcagaca atgggctgac ggccggcacc gacaatctta 240
   tgccattcaa tattgtgatt ccaaccaacg agataa 276
<210> 42
   <211> 276
   <212> DNA
   <213> Infectious bursal disease virus
<400> 42
   cagccgatga ttaccaattc ttatcacagt accaaccagg tggggtaaca atcacactgt 60
   tctcagccaa cattgatgct atcacaagcc tcagcgttgg gggagagctc gtgtttcaaa 120
   caagcgttca aggccttgta ctgggcgcca ccatctacct tataggcttt gatgggacta 180
   cggtaatcac cagggctgtg gccgcagaca atgggctgac ggccggcacc gacaatctta 240
   tgccattcaa tattgtgatt ccaaccaacg agataa 276
<210> 43
   <211> 276
   <212> DNA
   <213> Infectious bursal disease virus
<400> 43
   cagccgataa ttaccagttc tcatcacagt accaaacagg tggggtaaca atcacactgt 60
   tctcagccaa cattgatgcc atcacaagtc tcagcgttgg gggagagctc gtgttcaaaa 120
   caagcatcca aaaccttgta ctgggcgcca ccatctacct tataggcttt gatgggactg 180
   cagtaatcac cagagctgtg gccgcaaaca atgggctgac ggccggcatc gacaatctta 240
   tgccattcaa ccttgtgatt ccaaccaatg agataa 276
<210> 44
   <211> 276
   <212> DNA
   <213> Infectious bursal disease virus
<400> 44
   cagccgatga ttaccaattc tcatcacagt accaaccagg tcgggtaaca atcacactgt 60
   tctcagccaa cattgatgct atcacaagcc tcagcgttgg gggagagctc gtgtttcaaa 120
   caagtgttca aggccttgta ctgggcgcca ccatctacct tataggcttt gatgggacta 180
   cggtaatcac cagggctgtg gccgctgaca atgggctgac ggccggcacc gacaatctta 240
   tgccattcaa cattgtgatt ccaaccaacg agataa 276
<210> 45
   <211> 276
   <212> DNA
   <213> Infectious bursal disease virus
<400> 45
   cagccgataa ttaccaattc tcatcacagt accaaacagg tggggtaaca atcacactgt 60
   tctcagccaa cattgatgcc atcacaagtc tcagcgttgg gggagagctc gtgttcaaaa 120
   caagcgtcca tagccttgta ctgggcgcca ccatctacct tataggcttt gatgggactg 180
   cggtaatcac cagagctgtg gccgcaaaca atgggctgac gaccggcatc gacaatctta 240
   tgccattcaa tcttgtgatt ccaaccaatg agataa                           276
<210> 46
   <211> 276
   <212> DNA
   <213> Infectious bursal disease virus
<400> 46
   cagccgataa ttaccaattc tcatcacagt accaaacagg tggggtaaca atcacactgt 60
   tctcggccaa cattgatgct atcacaagcc tcagcgttgg gggggagctt gtgttcaaaa 120
   caagcgtcca aaaccttgta ctgggcgcca ccatctacct tataggcttt gatgggactg 180
   cggtaatcac cagagccgtg gccgcaaaca atgggctgac ggccggcatc gacaatctta 240
   tgccattcaa tcttgtgatt ccaaccaacg agataa                           276
<210> 47
   <211> 276
   <212> DNA
   <213> Infectious bursal disease virus
<400> 47
   cagccgataa ttaccaattc tcatcacagt accaaacagg tggggtaaca atcacactgt 60
   tctcggccaa cattgatgcc atcacaagtc tcagcgttgg aggagagctc gtgttcaaaa 120
   caagcgtcca aaaccttgta ctgggcgcca ccatctacct tataggcttt gatgggactg 180
   cggtagtcac cagagctgta gccgcaaaca atgggctgac ggccggcatc gacaatctta 240
   tgccattcaa tcttgtgatt ccaaccaacg agataa                           276
<210> 48
   <211> 276
   <212> DNA
   <213> Infectious bursal disease virus
<400> 48
   cagctgacaa ttaccagttc tcatcacagt accaaacagg tggggttaca atcacactgt 60
   tctcagccaa cattgatgcc atcacgagtc tcagcgttgg gggagagctc gtgttcaaaa 120
   caagcgtcca aagccttgta ctgggcgcca ccatctacct tataggcttt gatgggactg 180
   cggtaatcac cagagctgtg gccgcaaaca atgggctgac ggccggcatc gacaatctta 240
   tgccattcaa ccttgtgatt ccaaccaacg agataa                           276
<210> 49
   <211> 276
   <212> DNA
   <213> Infectious bursal disease virus
<400> 49
   cagccgatga ttaccaattc ttatcacagt accaaccagg tggggtaaca atcacactgt 60
   tctcggccaa cattgatgct atcacaagcc tcagcgttgg gggagagctc gtgtttcaaa 120
   caagcgttca aggccttgta ctgggcgcca ccatctacct tataggcttt gatgggacta 180
   cggtaatcac cagggctgtg gccgcagaca atgggctgac ggccggcacc gacaatctta 240
   tgccattcaa tcttgtgatt ccaaccaatg agataa                           276
<210> 50
   <211> 276
   <212> DNA
   <213> Infectious bursal disease virus
<400> 50
   cagccgatga ttaccaattc tcatcacagt accaaccagg tggggtaaca atcacactgt 60
   tctcagccaa cattgatgcc atcacaagcc tcagcgttgg gggagagctc gcgtttcaaa 120
   caagcgtcca aggccttgta ctgggcgcca ccatctacct cataggcttt gatgggacaa 180
   cggtaatcac cagggctgtg gccgcaaaca atgggctgac gaccggcacc gacaacctta 240
   tgccattcaa tcttgtgatt ccaaccaacg agataa                           276
<210> 51
   <211> 276
   <212> DNA
   <213> Infectious bursal disease virus
<400> 51
   cagccgatga ttaccaattc tcatcacagt accaaccagg cggggtaaca atcacactgt 60
   tctcagccaa cattgatgct atcacaagcc tcagcgttgg gggagagctc gtgtttcaaa 120
   caagtgttca aggccttgta ctgggcgcca ccatctacct tataggcttt gatgggacta 180
   cggtaatcac cagggctgtg gccgctgaca atgggctgac ggccggcacc gacaatctta 240
   tgccattcaa cattgtgatt ccaaccaacg agataa                           276
<210> 52
   <211> 276
   <212> DNA
   <213> Infectious bursal disease virus
<400> 52
   cagccgacga ttaccaattc tcatcacagt accaagcagg tggggtaacg atcacactgt 60
   tctcagccaa catcgatgcc atcacaagcc tcagcatcgg gggagaactc gtgtttcaaa 120
   caagcgtcca aggccttata ctgggcgcta ccatctacct tataggcttt gatgggactg 180
   cggtaataac cagagctgtg gctgcagaca atgggctgac ggccggcact gacaacctta 240
   tgccattcaa tattgtgatt ccaaccaacg agataa                           276
<210> 53
   <211> 276
   <212> DNA
   <213> Infectious bursal disease virus
<220>
   <221> misc_feature
   <222> (41)
   <223> a, t, c or g
<400> 53
   cagccgataa ttaccaattc tcatcacagt accaaacagg nggggtaaca atcacactgt 60
   tctcagccaa cattgatgcc attacaagtc tcagcgttgg gggggagctc gtgttcaaaa 120
   caaacgtcca aaaccttgta ctgggcgcca ccatctacct tataggcttt gatgggactg 180
   cggtaatcac cagagctgtg gccgcaaaca atgggctgac ggccggcatc gacaatctta 240
   tgccattcaa tcttgtgatt ccaaccaacg agataa                            276
<210> 54
   <211> 276
   <212> DNA
   <213> Infectious bursal disease virus
<400> 54
   cagccgacga ttaccaattc tcatctcagt accaaacagg tggggtaaca atcgcactgt 60
   tctcagccaa cattgacgcc atcacaagtc tcagcgttgg gggagagctc gtgttcaaaa 120
   caagcgtcca aagccttgta ctgggcgcca ccatctacct tataggcttt gatgggactg 180
   cggtaatcac cagagctgtg gctgcaaaca atgggctgac ggccggcatc gacaatctta 240
   tgccattcaa tcttgtgatt ccaaccaacg agataa                            276
<210> 55
   <211> 276
   <212> DNA
   <213> Infectious bursal disease virus
<400> 55
   cagccgataa ttaccaattc tcatcacagt accaaacagg tggggtaaca atcacattgt 60
   tctcagctaa cattgatgtc atcacaagcc tcagcgttgg gggagagctc gtgttcaaga 120
   caagcgtcca aagccttgta ctgggcgcca ccatctacct tataggcttc gatgggactg 180
   cggtaatcac cagagctgtg gctgcaaaca atgggctgac ggccggtatc gacaatctta 240
   tgccattcaa tcttgtgatt ccaaccaacg agataa                            276
<210> 56
   <211> 276
   <212> DNA
   <213> Infectious bursal disease virus
<400> 56
   cagccgataa ttaccaattc tcatcacagt accaaacagg tggggtaacg atcagactgt 60
   tctcagccaa cattgatgcc atcacaagtc tcagtgttgg gggagagctc gtgttcaaaa 120
   caaacgtcca aggccttgta ctgggcgcca ccatctacct tataggcttt gatgggactg 180
   cggtgatcac cagagctgtg gccgcaaaca atgggctgac ggccggcatc gacaatctta 240
   tgccattcaa tcttgtgatt ccaaccaacg agataa                            276
<210> 57
   <211> 276
   <212> DNA
   <213> Infectious bursal disease virus
<400> 57
   cagccgatga ttaccaattc tcatcacagt accaaccagg tggggtaaca atcacactgt 60
   tctcagccaa cattgatgct atcacaagcc tcagcattgg gggagagctc gtgttccaaa 120
   caagcgtcca aggccttgta ctgggcgcta ccatctacct tataggcttt gatgggacta 180
   cagtaatcac cagagctgtg gcctcagaca atgggctgac tgccggcacc gacaatctta 240
   tgccattcaa tcttgtgatt ccaaccaacg agataa                            276
<210> 58
   <211> 276
   <212> DNA
   <213> Infectious bursal disease virus
<400> 58
   cagccgataa ttaccaattc tcatcacagt accaaccagg tggggtaaca atcacactgt 60
   tctcagccaa cattgatgct atcacaagcc tcagcattgg gggagagctc gtgttccaaa 120
   caagcgtcca aggccttgta ctgggcgcta ccatctacct tataggcttt gatgggacta 180
   cagtaatcac cagagctgtg gcctcaaaca atgggctgac tgccggcatc gacaatctta 240
   tgccattcaa tcttgtgatt ccaaccaacg agataa                          276
<210> 59
   <211> 276
   <212> DNA
   <213> Infectious bursal disease virus
<400> 59
   cagccgataa ttaccaattc tcatcacagt accaaccagg tggggtaaca atcacactgt 60
   tctcagccaa cattgatgct atcacaagcc tcagcattgg gggagagctc gtgttccaaa 120
   caagcgtcca aggccttgta ctgggcgcta ccatctacct tataggcttt gatgggacta 180
   cagtaatcac cagagctgtg gcctcagaca atgggctgac tgccggcatc gacaatctta 240
   tgccattcaa tcttgtgatt ccaaccaacg agataa                          276
<210> 60
   <211> 276
   <212> DNA
   <213> Infectious bursal disease virus
<400> 60
   cagccgataa ttaccaattc tcatcacagt accaaccagg tggggtaaca atcacactgt 60
   tctcagccaa cattgatgct atcacaagcc tcagcattgg gggagagctc gtgttccaaa 120
   caagcgtcca aggccttgta ctgggcgcta ccatctacct tataggcttt gatgggacta 180
   cagtaatcac cagagctgtg gcctcagaca atgggctgac tgccggcatc gacaatctta 240
   tgccattcaa tcttgtgatt ccaaccaacg agataa                          276
<210> 61
   <211> 24
   <212> DNA
   <213> Artificial Sequence
<220>
   <223> Description of Artificial sequence: Primer
<400> 61
   ggtgcgactg ctgtatttgg taac 24